# EUROPÄISCHE PATENTSCHRIFT

(11) **EP 0 835 248 B1**
(45) Veröffentlichungstag und Bekanntmachung des Hinweises auf die Patenterteilung: **21.02.2001**
(21) Anmeldenummer: 96922854.3
(22) Anmeldetag: 20.06.1996
(51) Int. Cl.: C07D 239/54, C07C 281/06, C07C 281/02, A01N 43/54

(54) **1-AMINO-3-BENZYLURACILE**
1-AMINO-3-BENZYLURACILS
1-AMINO-3-BENZYLURACILES

(30) Priorität: 29.06.1995 DE 19523372
(43) Veröffentlichungstag der Anmeldung: 15.04.1998
(73) Patentinhaber: BASF AKTIENGESELLSCHAFT, 67056 Ludwigshafen (DE)
(72) Erfinder: MENKE, Olaf, D-67317 Altleiningen (DE); KLINTZ, Ralf, D-67269 Grünstadt (DE); HAMPRECHT, Gerhard, D-69469 Weinheim (DE); HEISTRACHER, Elisabeth, D-67061 Ludwigshafen (DE); SCHÄFER, Peter, D-67308 Ottersheim (DE); ZAGAR, Cyrill, D-67061 Ludwigshafen (DE); GÖTZ, Norbert, D-67547 Worms (DE); WESTPHALEN, Karl-Otto, D-67346 Speyer (DE); MISSLITZ, Ulf, D-67433 Neustadt (DE); WALTER, Helmut, D-67283 Obrigheim (DE)
(86) Internationale Anmeldenummer: EP9602666
(87) Internationale Veröffentlichungsnummer: WO9701543

(56) Entgegenhaltungen:
- WO-A-95/04461

## Beschreibung

Die vorliegende Erfindung betrifft neue 1-Amino-3-benzyluracile der Formel I 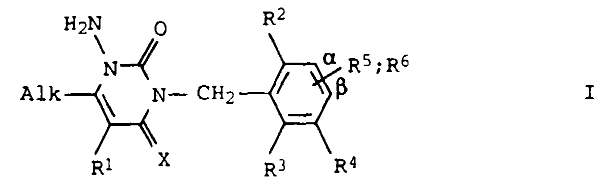 in der die Variablen folgende Bedeutungen haben:
- X: Sauerstoff oder Schwefel;
- Alk: C₁-C₄-Halogenalkyl;
- R¹: Wasserstoff oder Halogen;
- R², R³: unabhängig voneinander Wasserstoff, Cyano, Thiocyanato, Halogen, C₁-C₄-Halogenalkyl, C₁-C₄-Halogenalkoxy oder C₁-C₄-Halogenalkylthio;
- R⁴: Wasserstoff, Cyano, Thiocyanato, Halogen, C₁-C₄-Alkyl, C₁-C₄-Halogenalkyl, C₁-C₄-Alkoxy, C₁-C₄-Halogenalkoxy, C₁-C₄-Halogenalkylthio oder C₁-C₆-Alkylaminocarbonyl;
- R⁵: an Position a oder β
Wasserstoff, Cyano, Nitro, Hydroxy, Amino, Halogen, C₁-C₄-Alkylamino, das durch C₁-C₄-Alkyl, (C₁-C₄-Alkyl)carboxyl oder (C₁-C₄-Alkoxy)carbonyl substituiert sein kann,
C₁-C₆-Halogenalkoxy, C₁-C₆-Halogenalkylthio, C₁-C₆-Alkoxy, C₁-C₆-Alkylthio, C₃-C₆-Cycloalkoxy, C₃-C₆-Cycloalkylthio, C₃-C₆-Alkenyloxy, C₃-C₆-Alkenylthio, C₃-C₆-Alkinyloxy, C₃-C₆-Alkinylthio, (C₁-C₆-Alkyl)carbonyloxy, (C₁-C₆-Alkyl)carbonylthio, (C₃-C₆-Alkenyl)carbonyloxy, (C₃-C₆-Alkenyl)carbonylthio, (C₃-C₆-Alkinyl)carbonyloxy, (C₃-C₆-Alkinyl)carbonylthio, C₁-C₆-Alkylsulfonyloxy oder C₁-C₆-Alkylsulfonyl, wobei jeder der letztgenannten 16 Reste gewünschtenfalls ein bis drei Substituenten tragen kann, jeweils ausgewählt aus der Gruppe bestehend aus
- Halogen, Nitro, Cyano, Hydroxy, C₃-C₆-Cycloalkyl, C₁-C₆-Alkoxy, C₃-C₆-Cycloalkoxy, C₃-C₆-Alkenyloxy, C₃-C₆-Alkinyloxy, C₁-C₆-Alkoxy-C₁-C₆-alkoxy, C₁-C₆-Alkylthio, C₁-C₆-Alkylsulfinyl, C₁-C₆-Alkylsulfonyl, C₁-C₆-Alkylidenaminoxy,
- der Phenyl-, Phenoxy- oder Phenylsulfonylgruppe, die unsubstituiert sein oder ein bis drei Substituenten tragen kann, jeweils ausgewählt aus der Gruppe bestehend aus Halogen, Nitro, Cyano, C₁-C₆-Alkyl, C₁-C₆-Alkoxy und C₁-C₆-Halogenalkyl,
- einer 3- bis 7gliedrigen Heterocyclyl- oder Heterocyclyloxygruppe mit ein bis drei Heteroatomen, ausgewählt aus der Gruppe bestehend aus zwei Sauerstoffatomen, zwei Schwefelatomen und 3 Stickstoffatomen, wobei der Heterocyclus gesättigt, partiell oder vollständig ungesättigt oder aromatisch sein und gewünschtenfalls ein bis drei Substituenten tragen kann, jeweils ausgewählt aus der Gruppe bestehend aus Halogen, Nitro, Cyano, C₁-C₆-Alkyl, C₁-C₆-AlkoXy, C₁-C₆-Halogenalkyl und (C₁-C₆-Alkyl)carbonyl,
- einer Gruppe -CO-R⁷, -COOR⁷, -COSR⁷, -CON(R⁷)R⁸, -OCO-R⁷, -OCOOR⁷, -OCOSR⁷, -OCON(R⁷)R⁸ oder -N(R⁷)R⁸, wobei
- R⁷: für Wasserstoff, C₁-C₆-Alkyl, C₃-C₆-Cycloalkyl, C₃-C₆-Alkenyl, C₃-C₆-Alkinyl, C₁-C₆-Alkoxy-C₁-C₆-alkyl, (C₁-C₆-Alkoxy)carbonyl-C₁-C₆-alkyl, (C₃-C₆-Alkenyloxy)carbonyl-C₁-C₆-alkyl, Phenyl oder Phenyl-C₁-C₆-alkyl, wobei die Phenylgruppe und der Phenyl-Ring der Phenylalkylgruppe unsubstituiert sein oder einen bis drei Reste tragen können, jeweils ausgewählt aus der Gruppe bestehend aus Halogen, Nitro, Cyano, C₁-C₆-Alkyl, C₁-C₆-Halogenalkyl, C₁-C₆-Alkoxy und (C₁-C₆-Alkyl)carbonyl
und
- R⁸: für Wasserstoff, Hydroxy, C₁-C₆-Alkyl, C₃-C₆-Cycloalkyl, C₁-C₆-Alkoxy, (C₁-C₆-Alkoxy)carbonyl-C₁-C₆-alkoxy, C₃-C₆-Alkenyl oder C₃-C₆-Alkenyloxy,
stehen, oder R⁷ und R⁸ bilden zusammen mit dem gemeinsamen Stickstoff einen 3- bis 7gliedrigen Heterocyclus mit ein bis drei Heteroatomen, ausgewählt aus einer Gruppe bestehend aus ein oder zwei Sauerstoffatomen, ein oder zwei Schwefelatomen und ein bis drei Stickstoffatomen, wobei der Heterocyclus gesättigt, partiell oder vollständig ungesättigt oder aromatisch sein und gewünschtenfalls ein bis drei Substituenten tragen kann, jeweils ausgewählt aus der Gruppe bestehend aus Halogen, Nitro, Cyano, C₁-C₆-Alkyl, C₁-C₆-Halogenalkyl C₁-C₆-Alkoxy und (C₁-C₆-Alkyl)carbonyl;
- R⁶: an Position α, wobei R⁵ dann an Position β steht, oder an Position β, wobei R⁵ dann an Position α steht
1) Wasserstoff, Hydroxy, Mercapto, Halogen, C₁-C₆-Halogenalkoxy, C₁-C₆-Halogenalkylthio, C₁-C₆-Alkylthio(C₁-C₆-alkyl)carbonyl, (C₁-C₆-Alkyl)iminooxycarbonyl,
2) C₁-C₆-Alkoxy, C₁-C₆-Alkylthio, C₃-C₆-Cycloalkoxy, C₃-C₆-Cycloalkylthio, C₂-C₆-Alkenyloxy, C₂-C₆-Alkenylthio, C₂-C₆-Alkinyloxy, C₂-C₆-Alkinylthio, (C₁-C₆-Alkyl)carbonyloxy, (C₁-C₆-Alkyl)carbonylthio, (C₁-C₆-Alkoxy)carboxyloxy, (C₂-C₆-Alkenyl)carbonyloxy, (C₂-C₆-Alkenyl)carbonylthio, (C₂-C₆-Alkinyl)carbonyloxy, (C₂-C₆-Alkinyl)carbonylthio, C₁-C₆-Alkylsulfonyloxy oder C₁-C₆-Alkylsulfonyl, wobei jeder der letztgenannten 17 Reste gewünschtenfalls ein bis drei Substituenten tragen kann, jeweils ausgewählt aus der Gruppe bestehend aus
   - Halogen, Nitro, Cyano, Hydroxy, C₃-C₆-Cycloalkyl, C₁-C₆-Alkoxy, C₃-C₆-Cycloalkoxy, C₃-C₆-Alkenyloxy, C₃-C₆-Alkinyloxy, C₁-C₆-Alkoxy-C₁-C₆-alkoxy, C₁-C₆-Alkylthio, C₁-C₆-Alkylsulfinyl, C₁-C₆-Alkylsulfonyl, C₁-C₆-Alkylidenaminoxy,
   - der Phenyl-, Phenoxy- oder Phenylsulfonylgruppe, die unsubstituiert sein oder ein bis drei Substituenten tragen kann, jeweils ausgewählt aus der Gruppe bestehend aus Halogen, Nitro, Cyano, C₁-C₆-Alkyl, C₁-C₆-Halogenalkyl, C₁-C₆-Alkoxy und (C₁-C₆-Alkoxy)carbonyl,
   - einer 3- bis 7gliedrigen Heterocyclyl- oder Heterocyclyloxygruppe mit ein bis drei Heteroatomen, ausgewählt aus der Gruppe bestehend aus zwei Sauerstoffatomen, zwei Schwefelatomen und 3 Stickstoffatomen, wobei der Heterocyclus gesättigt, partiell oder vollständig ungesättigt oder aromatisch sein und gewünschtenfalls ein bis drei Substituenten tragen kann, jeweils ausgewählt aus der Gruppe bestehend aus Halogen, Nitro, Cyano, C₁-C₆-Alkyl, C₁-C₆-Alkoxy, C₁-C₆-Halogenalkyl und (C₁-C₆-Alkyl)carbonyl,
   - einer Gruppe -CO-R⁹, -COOR⁹, -COSR⁹, -CON(R⁹)R¹⁰, -OCO-R⁹, -OCOOR⁹, -OCOSR⁹, -OCON(R⁹)R¹⁰ oder -N(R⁹)R¹⁰, wobei R⁹ für eine der Bedeutungen von R⁷ und R¹⁰ für eine der Bedeutungen von R⁸ steht,
   - der Gruppe -C(R²¹)=N-OR²⁰;
3) -CY-R¹¹, -C(R¹¹) (Z¹R¹²) (Z²R¹³), wobei Z¹ und Z² jeweils für Sauerstoff oder Schwefel stehen,
   -C(R¹¹)=C(R¹⁴)-CN, -C(R¹¹)=C(R¹⁴)-CO-R¹⁵, -CH(R¹¹)-CH(R¹⁴)-CO-R¹⁵, -C(R¹¹)=C(R¹⁴)-CH₂-CO-R¹⁵, -C(R¹¹)=C(R¹⁴)-C(R¹⁶)=C(R¹⁷)-CO-R¹⁵, -C(R¹¹)=C(R¹⁴)-CH₂-CH(R¹⁸)-CO-R¹⁵, CO-OR¹⁹, -CO-SR¹⁹, -CO-N(R¹⁹)-OR²⁰, -C≡C-CO-NH-OR²⁰, -C≡C-CO-N(R¹⁹)-OR²⁰, -C≡C-CS-NH-OR²⁰, -C≡C-CS-N(R¹⁹)-OR²⁰, -C(R¹¹)=C(R¹⁴)-CO-NH-OR²⁰, -C(R¹¹)=C(R¹⁴)-CO-N(R¹⁹)-OR²⁰, -C(R¹¹)=C(R¹⁴)-CS-NH-OR²⁰, -C(R¹¹)=C(R¹⁴)-CS-N(R¹⁹)-OR²⁰, -C(R¹¹)=C(R¹⁴)-C(R²¹)=N-OR²⁰, -C(R²¹)=N-OR²⁰, -C≡C-C(R²¹)=N-OR²⁰, -C(Z¹R¹²) (Z²R¹³) -OR¹⁹, -C(Z¹R¹²)(Z²R¹³) -SR¹⁹, -C(Z¹R¹²)(Z²R¹³) -N(R²³)R²⁴, -N(R²³)R²⁴, -CON(R²³)R²⁴, 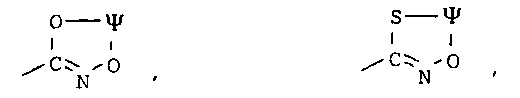 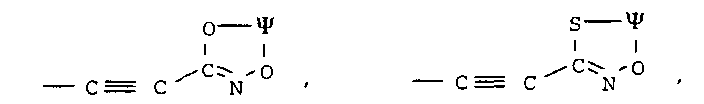 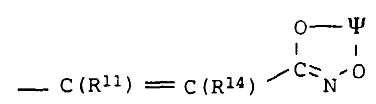 oder 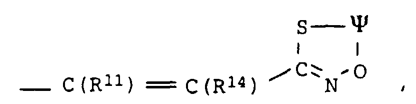 wobei Ψ für C₁-C₃-Alkylen steht, das einen C₁-C₆-Alkyl-substituenten tragen kann;
- R¹¹: Wasserstoff, Cyano, C₁-C₆-Alkyl, C₁-C₆-Halogenalkyl, C₂-C₆-Alkenyl, C₂-C₆-Alkinyl, C₃-C₆-Cycloalkyl, C₁-C₆-Alkoxy-C₁-C₆-alkyl oder (C₁-C₆-Alkoxy)carbonyl;
- R¹², R¹³: unabhängig voneinander C₁-C₆-Alkyl, C₁-C₆-Halogenalkyl, C₃-C₆-Alkenyl, C₃-C₆-Alkinyl, C₁-C₆-Alkoxy-C₁-C₆-alkyl oder
- R¹² und R¹³: zusammen eine gesättigte oder ungesättigte,
2- bis 4gliedrige Kohlenstoffkette, die einen Oxosubstituenten tragen kann, wobei ein Glied dieser Kette durch ein den variablen Z¹ und Z² nicht benachbartes Sauerstoff-, Schwefel- oder Stickstoffatom ersetzt sein kann,
und wobei die Kohlenstoffkette noch ein bis drei Reste tragen kann, jeweils ausgewählt aus der Gruppe bestehend aus Cyano, Nitro, Amino, Halogen, C₁-C₆-Alkyl, C₂-C₆-Alkenyl,
C₁-C₆-Alkoxy, C₂-C₆-Alkenyloxy, C₂-C₆-Alkinyloxy, C₁-C₆-Halogenalkyl, Cyano-C₁-C₆-alkyl, Hydroxy-C₁-C₆-alkyl, C₁-C₆-Alkoxy-C₁-C₆-alkyl, C₃-C₆-Alkenyloxy-C₁-C₆-alkyl, C₃-C₆-Alkinyloxy-C₁-C₆-alkyl, C₃-C₆-Cycloalkyl, C₃-C₆-Cycloalkoxy, Carboxy, (C₁-C₆-Alkoxy)carbonyl, (C₁-C₆-Alkyl)carbonyloxy-C₁-C₆-alkyl und Phenyl, das seinerseits unsubstituiert sein oder einen bis drei Substituenten tragen kann, jeweils ausgewählt aus der Gruppe bestehend aus Cyano, Nitro, Amino, Halogen, C₁-C₆-Alkyl, C₁-C₆-Halogenalkyl, C₁-C₆-Alkoxy und (C₁-C₆-Alkoxy)carbonyl,
und wobei die Kohlenstoffkette auch durch einen ankondensierten oder spiroverknüpften 3- bis 7gliedrigen Ring substituiert sein kann, der ein oder zwei Heteroatome als Ringglieder enthalten kann, ausgewählt aus Sauerstoff, Schwefel, Stickstoff und durch C₁-C₆-Alkyl substituiertem Stickstoff,
und der gewünschtenfalls seinerseits einen oder zwei der folgenden Substituenten tragen kann: Cyano, C₁-C₆-Alkyl, C₂-C₆-Alkenyl, C₁-C₆-Alkoxy, Cyano-C₁-C₆-alkyl, C₁-C₆-Halogenalkyl und (C₁-C₆-Alkoxy)carbonyl;
- R¹⁴: Wasserstoff, Cyano, Halogen, C₁-C₆-Alkyl, C₁-C₆-Halogenalkyl, C₁-C₆-Alkoxy, (C₁-C₆-Alkyl)carbonyl oder (C₁-C₆-Alkoxy)carbonyl;
- R¹⁵: Wasserstoff, O-R²², S-R²², C₁-C₆-Alkyl, das noch einen oder zwei C₁-C₆-Alkoxysubstituenten tragen kann, C₂-C₆-Alkenyl, C₂-C₆-Alkinyl, C₁-C₆-Halogenalkyl, C₃-C₆-Cycloalkyl, C₁-C₆-Alkylthio-C₁-C₆-alkyl, C₁-C₆-Alkyliminooxy, -N(R²³)R²⁴ oder Phenyl, das unsubstituiert sein oder ein bis drei Substituenten tragen kann, jeweils ausgewählt aus der Gruppe bestehend aus Cyano, Nitro, Halogen, C₁-C₆-Alkyl, C₂-C₆-Alkenyl, C₁-C₆-Halogenalkyl, C₁-C₆-Alkoxy und (C₁-C₆-Alkoxy)carbonyl,
wobei R²² für eine der Bedeutungen von R¹⁹ steht;
- R¹⁶: Wasserstoff, Cyano, Halogen, C₁-C₆-Alkyl, C₃-C₆-Alkenyl, C₃-C₆-Alkinyl, C₁-C₆-Alkoxy-C₁-C₆-alkyl, (C₁-C₆-Alkyl)carbonyl, (C₁-C₆-Alkoxy)carbonyl, -N(R²⁵)R²⁶, wobei R²⁵ und R²⁶ für eine der Bedeutungen von R²³ und R²⁴ stehen,
oder Phenyl, das seinerseits noch einen bis drei Substituenten tragen kann, jeweils ausgewählt aus der Gruppe bestehend aus Cyano, Nitro, Halogen, C₁-C₆-Alkyl, C₁-C₆-Halogenalkyl, C₃-C₆-Alkenyl, C₁-C₆-Alkoxy und (C₁-C₆-Alkoxy)carbonyl;
- R¹⁷: Wasserstoff, Cyano, Halogen, C₁-C₆-Alkyl, C₁-C₆-Alkoxy, C₁-C₆-Halogenalkyl, (C₁-C₆-Alkyl)carbonyl oder (C₁-C₆-Alkoxy) carbonyl;
- R¹⁸: Wasserstoff, Cyano, C₁-C₆-Alkyl oder (C₁-C₆-Alkoxy)carbonyl;
- R¹⁹: Wasserstoff, C₁-C₆-Alkyl, C₁-C₆-Halogenalkyl, C₂-C₆-Alkenyl oder C₂-C₆-Alkinyl, wobei die letztgenannten 4 Gruppen jeweils einen oder zwei der folgenden Reste tragen können: Cyano, Halogen, Hydroxy, Hydroxycarbonyl, C₁-C₆-Alkoxy, C₁-C₆-Alkylthio, (C₁-C₆-Alkyl)carbonyl, (C₁-C₆-Alkoxy)carbonyl, (C₁-C₆-Alkyl)carbonyloxy, (C₃-C₆-Alkenyloxy)carbonyl oder einen mit dem Stickstoffatom über eine Carbonylbrücke gebundenen 3- bis 7gliedrigen Azaheterocyclus, der neben Kohlenstoffringgliedern noch ein Sauerstoff- oder Schwefelatom als Ringglied enthalten kann;
oder (C₁-C₆-Alkyl)carbonyl, (C₁-C₆-Halogenalkyl)carbonyl, (C₁-C₆-Alkoxy)carbonyl, C₁-C₆-Alkylaminocarbonyl,
Di-(C₁-C₆-alkyl)aminocarbonyl, C₁-C₆-Alkyloximino-C₁-C₆-alkyl, C₃-C₆-Cycloalkyl,
Phenyl oder Phenyl-C₁-C₆-alkyl, wobei die Phenylringe unsubstituiert sein oder ihrerseits ein bis drei Substituenten tragen können, jeweils ausgewählt aus der Gruppe bestehend aus Cyano, Nitro, Halogen, C₁-C₆-Alkyl, C₁-C₆-Halogenalkyl, C₁-C₆-Alkoxy und (C₁-C₆-Alkoxy)carbonyl;
- R²⁰: Wasserstoff, C₁-C₆-Alkyl, C₁-C₆-Halogenalkyl, C₃-C₆-Cycloalkyl, C₃-C₆-Alkenyl, C₃-C₆-Alkinyl, Hydroxy-C₁-C₆-alkyl, C₁-C₆-Alkoxy-C₁-C₆-alkyl, C₁-C₆-Alkylthio-C₁-C₆-alkyl, Cyano-C₁-C₆-alkyl, (C₁-C₆-Alkyl)carbonyl-C₁-C₆-alkyl, (C₁-C₆-Alkoxy)carbonyl-C₁-C₆-alkyl, (C₁-C₆-Alkoxy)carbonyl-C₂-C₆-alkenyl, (C₁-C₆-Alkyl)carbonyloxy-C₁-C₆-alkyl oder Phenyl-C₁-C₆-alkyl, wobei der Phenylring gewünschtenfalls ein bis drei Substituenten tragen kann, jeweils ausgewählt aus der Gruppe bestehend aus Cyano, Nitro, Halogen, C₁-C₆-Alkyl, C₁-C₆-Halogenalkyl, C₁-C₆-Alkoxy und (C₁-C₆-Alkoxy)carbonyl;
- R²¹: - Wasserstoff, Halogen,
- C₁-C₆-Alkyl, C₁-C₆-Halogenalkyl, C₁-C₆-Alkoxy, C₁-C₆-Halogenalkoxy, C₃-C₆-Alkenyloxy, C₁-C₆-Alkylthio, C₁-C₆-Halogenalkylthio, (C₁-C₆-Alkyl)carbonyloxy, (C₁-C₆-Halogenalkyl)carbonyloxy, C₁-C₆-Alkylsulfonyloxy oder C₁-C₆-Halogenalkylsulfonyloxy,
   wobei die letztgenannten 11 Reste einen der folgenden Substituenten tragen können: Hydroxy, Cyano, Hydroxycarbonyl, C₁-C₆-Alkoxy, C₁-C₆-Alkylthio, (C₁-C₆-Alkyl)carbonyl, (C₁-C₆-Alkoxy)carbonyl, (C₁-C₆-Alkyl)aminocarbonyl, Di-(C₁-C₆-alkyl)aminocarbonyl, (C₁-C₆-Alkyl)carbonyloxy,
- einen mit dem Stickstoffatom über eine Carbonylbrücke gebundenen 3- bis 7gliedrigen Azaheterocyclus, der neben Kohlenstoffringgliedern noch ein Sauerstoff- oder Schwefelatom als Ringglied enthalten kann,
- (C₁-C₆-Alkyl)carbonyl, (C₁-C₆-Halogenalkyl)carbonyl, (C₁-C₆-Alkoxy)carbonyl, (C₁-C₆-Alkoxy)carbonyloxy, (C₁-C₆-Alkyl)carbonylthio, (C₁-C₆-Halogenalkyl)carbonylthio, (C₁-C₆-Alkoxy)carbonylthio, C₂-C₆-Alkenyl, C₂-C₆-Alkenylthio, C₃-C₆-Alkinyl, C₃-C₆-Alkinyloxy, C₃-C₆-Alkinylthio, (C₂-C₆-Alkinyl) carbonyloxy, C₃-C₆-Alkinylsulfonyloxy, C₃-C₆-Cycloalkyl, C₃-C₆-Cycloalkyloxy, C₃-C₆-Cycloalkylthio, (C₃-C₆-Cycloalkyl)carbonyloxy, C₃-C₆-Cycloalkylsulfonyloxy,
- Phenyl, Phenoxy, Phenylthio, Benzoyloxy, Phenylsulfonyloxy, Phenyl-C₁-C₆-alkyl, Phenyl-C₁-C₆-alkoxy, PhenylC₁-C₆-alkylthio, Phenyl-(C₁-C₆-alkyl)-carbonyloxy oder Phenyl-(C₁-C₆-alkyl)sulfonyloxy, wobei die Phenylringe der letztgenannten 10 Reste unsubstituiert sein oder ihrerseits ein bis drei Substituenten tragen können, jeweils ausgewählt aus der Gruppe bestehend aus Cyano, Nitro, Halogen, C₁-C₆-Alkyl, C₁-C₆-Halogenalkyl, C₁-C₆-Alkoxy und (C₁-C₆-Alkoxy)carbonyl;
- R²³, R²⁴: unabhängig voneinander
Wasserstoff, C₁-C₆-Alkyl, C₂-C₆-Alkenyl, C₂-C₆-Alkinyl, C₃-C₆-Cycloalkyl, C₁-C₆-Halogenalkyl, C₁-C₆-Alkoxy-C₁-C₆-alkyl, (C₁-C₆-Alkyl)carbonyl, (C₁-C₆-Alkoxy)carbonyl, (C₁-C₆-Alkoxy)carbonyl-C₁-C₆-alkyl, (C₁-C₆-Alkoxy)carbonyl-C₂-C₆-alkenyl, wobei die Alkenylkette zusätzlich ein bis drei Halogen- und/oder Cyano-Reste tragen kann, oder für C₁-C₆-Alkylsulfonyl, (C₁-C₆-Alkoxy)carbonyl-C₁-C₆-alkylsulfonyl, Phenyl oder Phenylsulfonyl, wobei die beiden Phenylringe unsubstituiert sein oder ihrerseits einen bis drei Substituenten tragen können, jeweils ausgewählt aus der Gruppe bestehend aus Cyano, Nitro, Halogen, C₁-C₆-Alkyl, C₁-C₆-Halogenalkyl, C₃-C₆-Alkenyl, C₁-C₆-Alkoxy und (C₁-C₆-Alkoxy)carbonyl,
oder R²³ und R²⁴ zusammen mit dem gemeinsamen Stickstoffatom einen gesättigten oder ungesättigten 4- bis 7gliedrigen Azaheterocyclus, der neben Kohlenstoffringgliedern gewünschtenfalls eines der folgenden Glieder enthalten kann: -O-, -S-, -N=, -NH- oder -N(C₁-C₆-Alkyl)-;
- Y: Sauerstoff, Schwefel oder -N(R²⁷)-;
- R²⁷: Wasserstoff, Hydroxy, C₁-C₆-Alkyl, C₃-C₆-Alkenyl,
C₃-C₆-Alkinyl, C₃-C₇-Cycloalkyl, C₁-C₆-Halogenalkyl, C₁-C₆-Alkoxy-C₁-C₆-alkyl, C₁-C₆-Alkoxy, C₃-C₆-Alkenyloxy, C₃-C₆-Alkinyloxy, C₃-C₆-Cycloalkoxy, C₅-C₇-Cycloalkenyloxy, C₁-C₆-Halogenalkoxy, C₃-C₆-Halogenalkenyloxy, Hydroxy-C₁-C₆-alkoxy, Cyano-C₁-C₆-alkoxy, C₃-C₆-Cycloalkyl-C₁-C₆-alkoxy, C₁-C₆-Alkoxy-C₁-C₆-alkoxy, C₁-C₆-Alkoxy-C₃-C₆-alkenyloxy, (C₁-C₆-Alkyl)carbonyloxy, (C₁-C₆-Halogenalkyl)carbonyloxy, (C₁-C₆-Alkyl)carbamoyloxy, (C₁-C₆-Halogenalkyl)carbamoyloxy, (C₁-C₆-Alkyl)carbonyl-C₁-C₆-alkyl, (C₁-C₆-Alkyl)carbonyl-C₁-C₆-alkoxy, (C₁-C₆-Alkoxy)carbonyl-C₁-C₆-alkyl, (C₁-C₆-Alkoxy)carbonyl-C₁-C₆-alkoxy, C₁-C₆-Alkylthio-C₁-C₆-alkoxy, Di-(C₁-C₆-alkyl)amino-C₁-C₆-alkoxy,
Phenyl, das seinerseits noch einen bis drei Substituenten tragen kann, jeweils ausgewählt aus der Gruppe bestehend aus Cyano, Nitro, Halogen, C₁-C₆-Alkyl, C₁-C₆-Halogenalkyl, C₂-C₆-Alkenyl, C₁-C₆-Alkoxy und (C₁-C₆-Alkoxy)carbonyl, Phenyl-C₁-C₆-alkoxy, Phenyl-C₃-C₆-alkenyloxy oder Phenyl-C₃-C₆-alkinyloxy, wobei jeweils eine oder zwei Methylengruppen der Kohlenstoffketten durch -O- , -S- oder -N(C₁-C₆-Alkyl)ersetzt sein können und wobei jeder Phenylring unsubstituiert oder seinerseits einen bis drei Substituenten tragen kann, jeweils ausgewählt aus der Gruppe bestehend aus Cyano, Nitro, Halogen, C₁-C₆-Alkyl, C₁-C₆-Halogenalkyl, C₂-C₆-Alkenyl, C₁-C₆-Alkoxy und (C₁-C₆-Alkoxy)carbonyl,
Heterocyclyl, Heterocyclyl-C₁-C₆-alkoxy, Heterocyclyl-C₃-C₆-alkenyloxy oder Heterocyclyl-C₃-C₆-alkinyloxy, wobei jeweils eine oder zwei Methylengruppen der Kohlenstoffketten durch -O- , -S- oder -N(C₁-C₆-Alkyl)- ersetzt sein können und wobei jeder Heterocyclus 3- bis 7gliedrig, gesättigt, ungesättigt oder aromatisch sein kann und ein bis vier Heteroatome enthält, ausgewählt aus der Gruppe bestehend aus zwei Sauerstoffatomen, zwei Schwefelatomen und 4 Stickstoffatomen, und entweder unsubstituiert ist oder seinerseits einen bis drei Substituenten trägt, jeweils ausgewählt aus der Gruppe bestehend aus Cyano, Nitro, Halogen, C₁-C₆-Alkyl, C₁-C₆-Halogenalkyl, C₂-C₆-Alkenyl, C₁-C₆-Alkoxy und (C₁-C₆-Alkoxy)carbonyl,
oder -N(R²⁸)R²⁹, wobei R²⁸ und R²⁹ jeweils für Wasserstoff, C₁-C₆-Alkyl, C₃-C₆-Alkenyl, C₃-C₆-Alkinyl, C₃-C₆-Cycloalkyl, C₁-C₆-Halogenalkyl, C₁-C₆-Alkoxy-C₁-C₆-alkyl, (C₁-C₆-Alkyl)carbonyl, (C₁-C₆-Alkoxy)carbonyl, (C₁-C₆-Alkoxy)carbonyl-C₁-C₆-alkyl, (C₁-C₆-Alkoxy)carbonyl-C₂-C₆-alkenyl, wobei die Alkenylkette zusätzlich ein bis drei Halogen- und/oder Cyano-Reste tragen kann,
oder für Phenyl, das unsubstituiert sein oder seinerseits noch einen bis drei Substituenten tragen kann, jeweils ausgewählt aus der Gruppe bestehend aus Cyano, Nitro, Halogen, C₁-C₆-Alkyl, C₁-C₆-Halogenalkyl, C₃-C₆-Alkenyl, C₁-C₆-Alkoxy und (C₁-C₆-Alkoxy)carbonyl,
stehen,
oder wobei R²⁸ und R²⁹ zusammen mit dem gemeinsamen Stickstoffatom einen gesättigten oder ungesättigten 4- bis 7gliedrigen Heterocyclus bilden, der neben Kohlenstoffringgliedern gewünschtenfalls noch eines der folgenden Glieder enthalten kann: -O-, -S-, -N=, -NH- oder -N(C₁-C₆-Alkyl)-;
sowie die landwirtschaftlich brauchbaren Salze der Verbindungen I.

Außerdem betrifft die Erfindung
- die Verwendung der Verbindungen I als Herbizide und/oder zur Desikkation und/oder Defoliation von Pflanzen,
- herbizide Mittel und Mittel zur Desikkation und/oder Defoliation von Pflanzen, welche die Verbindungen I als wirksame Substanzen enthalten,
- Verfahren zur Herstellung der Verbindungen I und von herbiziden Mitteln und Mitteln zur Desikkation und/oder Defoliation von Pflanzen unter Verwendung der Verbindungen I,
- Verfahren zur Bekämpfung von unerwünschtem Pflanzenwuchs und zur Desikkation und/oder Defoliation von Pflanzen mit den Verbindungen I, sowie
- neue Zwischenprodukte der Formeln III und IV.

In der WO 95/04461 werden bereits 3-Benzyl-1-methyl-6-trifluormethyluracile der Formel II 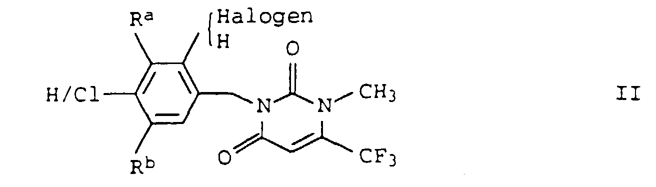 wobei
- R^{a} für: Wasserstoff, Cyano, Halogen, Niederalkoxy, Niederalkylaminocarbonyl oder Propargyloxy und
- R^{b} für: Wasserstoff, Cyano, Halogen, Niederalkylaminocarbonyl oder Carboxy stehen,
als Herbizide beschrieben.

Die herbizide Wirkung der bekannten Verbindungen bezüglich der Schadpflanzen ist jedoch nicht immer voll befriedigend. Aufgabe der vorliegenden Erfindung war es demnach, neue herbizid wirksame Verbindungen bereitzustellen, mit denen sich unerwünschte Pflanzen besser als bisher gezielt bekämpfen lassen. Die Aufgabe erstreckt sich auch auf die Bereitstellung neuer desikkant/defoliant wirksamer Verbindungen.

Demgemäß wurden die eingangs definierten 1-Amino-3-benzyluracile der Formel I gefunden. Ferner wurden herbizide Mittel gefunden, die die Verbindungen I enthalten und eine sehr gute herbizide Wirkung besitzen. Außerdem wurden Verfahren zur Herstellung dieser Mittel und Verfahren zur Bekämpfung von unerwünschtem Pflanzenwuchs mit den Verbindungen I gefunden.

Des weiteren wurde gefunden, daß die Verbindungen I auch zur Defoliation und Desikkation von Pflanzenteilen geeignet sind, wofür Kulturpflanzen wie Baumwolle, Kartoffel, Raps, Sonnenblume, Sojabohne oder Ackerbohnen, insbesondere Baumwolle, in Betracht kommen. Diesbezüglich wurden Mittel zur Desiccation und/oder Defoliation von Pflanzen, Verfahren zur Herstellung dieser Mittel und Verfahren zur Desikkation und/oder Defoliation von Pflanzen mit den Verbindungen I gefunden.

Die Verbindungen der Formel I können je nach Substitutionsmuster ein oder mehrere Chiralitätszentren enthalten und liegen dann als Enantiomeren- oder Diastereomerengemische vor. Gegenstand der Erfindung sind sowohl die reinen Enantiomeren oder Diastereomeren als auch deren Gemische.

Unter landwirtschaftlich brauchbaren Salzen kommen vor allem die Salze derjenigen Kationen oder die Säureadditionssalze derjenigen Säuren in Betracht, deren Kationen beziehungsweise Anionen die herbizide Wirkung der Verbindungen I nicht negativ beeinträchtigen. So kommen als Kationen insbesondere die Ionen der Alkalimetalle, vorzugsweise Natrium und Kalium, der Erdalkalimetalle, vorzugsweise Calcium, Magnesium und Barium, und der Übergangsmetalle, vorzugsweise Mangan, Kupfer, Zink und Eisen, sowie das Ammoniumion, das gewünschtenfalls ein bis vier C₁-C₄-Alkyl-substituenten und/oder einen Phenyl- oder Benzylsubstituenten tragen kann, vorzugsweise Diisopropylammonium, Tetramethylammonium, Tetrabutylammonium, Trimethylbenzylammonium, des weiteren Phosphoniumionen, Sulfoniumionen, vorzugsweise Tri(C₁-C₄-alkyl)sulfonium und Sulfoxoniumionen, vorzugsweise Tri(C₁-C₄-alkyl)sulfoxonium, in Betracht.

Anionen von brauchbaren Säureadditionssalzen sind in erster Linie Chlorid, Bromid, Fluorid, Hydrogensulfat, Sulfat, Dihydrogenphosphat, Hydrogenphosphat, Phosphat, Nitrat, Hydrogencarbonat, Carbonat, Hexafluorosilikat, Hexafluorophosphat, Benzoat, sowie die Anionen von C₁-C₄-Alkansäuren, vorzugsweise Formiat, Acetat, Propionat und Butyrat.

Die bei der Definition von Alk, R¹, R² bis R²⁹ und Ψ genannten organischen Molekülteile stellen Sammelbegriffe für individuelle Auf zählungen der einzelnen Bedeutungen dar. Sämtliche Kohlenstoffketten, also alle Alkyl-, Halogenalkyl-, Hydroxyalkyl-, Cyanoalkyl-, Phenylalkyl-, Alkylen-, Alkoxy-, Halogenalkoxy-, Hydroxyalkoxy-, Cyanoalkoxy-, Alkylthio-, Halogenalkylthio, Alkylsulfinyl-, Alkylsulfonyl-, Halogenalkylsulfonyl-, Alkylamino-, Dialkylamino-, Alkyloximino-, Alkyliminooxy-, Alkylidenaminoxy-, Alkenyl-, Alkenyloxy-, Halogenalkenyloxy-, Alkenylthio-, Alkinyl-, Alkinyloxy- und Alkinylthio-Teile können geradkettig oder verzweigt sein. Halogenierte Substituenten tragen vorzugsweise ein bis fünf gleiche oder verschiedene Halogenatome.

Die Bedeutung Halogen steht jeweils für Fluor, Brom, Chlor oder Iod, insbesondere für Fluor oder Chlor.

Ferner stehen beispielsweise:
- C₁-C₄-Alkyl für: Methyl, Ethyl, n-Propyl, 1-Methylethyl, n-Butyl, 1-Methylpropyl, 2-Methylpropyl oder 1,1-Dimethylethyl, insbesondere für Methyl oder Ethyl;
- -C₁-C₄-Halogenalkyl für: einen C₁-C₄-Alkylrest wie vorstehend genannt, der partiell oder vollständig durch Fluor, Chlor, Brom und/oder Iod substituiert ist, also z.B. Chlormethyl, Dichlormethyl, Trichlormethyl, Fluormethyl, Difluormethyl, Trifluormethyl, Chlorfluormethyl, Dichlorfluormethyl, Chlordifluormethyl, 2-Fluorethyl, 2-Chlorethyl, 2-Bromethyl, 2-Iodethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl, 2-Fluorpropyl, 3-Fluorpropyl, 2,2-Difluorpropyl, 2,3-Difluorpropyl, 2-Chlorpropyl, 3-Chlorpropyl, 2,3-Dichlorpropyl, 2-Brompropyl, 3-Brompropyl, 3,3,3-Trifluorpropyl, 3,3,3-Trichlorpropyl, 2,2,3,3,3-Pentafluorpropyl, Heptafluorpropyl, 1-(Fluormethyl)-2-fluorethyl, 1-(Chlormethyl)-2-chlorethyl, 1-(Brommethyl)-2-bromethyl, 4-Fluorbutyl, 4-Chlorbutyl, 4-Brombutyl oder Nonafluorbutyl, insbesondere für Chlormethyl, Fluormethyl, Difluormethyl, Trifluormethyl, 2-Fluorethyl, 2-Chlorethyl oder 2,2,2-Trifluorethyl;
- C₁-C₆-Alkyl für: C₁-C₄-Alkyl wie vorstehend genannt, sowie z.B. n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl oder 1-Ethyl-2-methylpropyl, insbesondere für Methyl, Ethyl, n-Propyl, 1-Methylethyl, n-Butyl, 1,1-Dimethylethyl, n-Pentyl oder n-Hexyl;
- C₁-C₆-Halogenalkyl für: C₁-C₆-Alkyl wie vorstehend genannt, das partiell oder vollständig durch Fluor, Chlor, Brom und/oder Iod substituiert ist, also z.B. einen der unter C₁-C₄-Halogenalkyl genannten Reste oder für 5-Fluor-1-pentyl, 5-Chlor-1-pentyl, 5-Brom-1-pentyl, 5-Iod-1-pentyl, 5,5,5-Trichlor-1-pentyl, Undecafluorpentyl, 6-Fluor-1-hexyl, 6-Chlor-1-hexyl, 6-Brom-1-hexyl, 6-Iod-1-hexyl, 6,6,6-Trichlor-1-hexyl oder Dodecafluorhexyl, insbesondere für Chlormethyl, Fluormethyl, Difluormethyl, Trifluormethyl, 2-Fluorethyl, 2-Chlorethyl oder 2,2,2-Trifluorethyl;
- Hydroxy-C₁-C₆-alkyl für: z.B. Hydroxymethyl, 2-Hydroxyeth-1-yl, 2-Hydroxy-prop-1-yl, 3-Hydroxy-prop-1-yl, 1-Hydroxy-prop-2-yl, 2-Hydroxy-but-1-yl, 3-Hydroxy-but-1-yl, 4-Hydroxy-but-1-yl, 1-Hydroxy-but-2-yl, 1-Hydroxy-but-3-yl, 2-Hydroxy-but-3-yl, 1-Hydroxy-2-methyl-prop-3-yl, 2-Hydroxy-2-methyl-prop-3-yl oder 2-Hydroxymethyl-prop-2-yl, insbesondere für 2-Hydroxyethyl;
- Cyano-C₁-C₆-alkyl für: z.B. Cyanomethyl, 1-Cyanoeth-1-yl, 2-Cyanoeth-1-yl, 1-Cyanoprop-1-yl, 2-Cyanoprop-1-yl, 3-Cyanoprop-1-yl, 1-Cyanoprop-2-yl, 2-Cyanoprop-2-yl, 1-Cyanobut-1-yl, 2-Cyanobut-1-yl, 3-Cyanobut-1-yl, 4-Cyanobut-1-yl, 1-Cyanobut-2-yl, 2-Cyanobut-2-yl, 1-Cyanobut-3-yl, 2-Cyano-but-3-yl, 1-Cyano-2-methyl-prop-3-yl, 2-Cyano-2-methyl-prop-3-yl, 3-Cyano-2-methyl-prop-3-yl oder 2-Cyanomethyl-prop-2-yl, insbesondere für Cyanomethyl oder 2-Cyanoethyl;
- Phenyl-C₁-C₆-alkyl für: z.B. Benzyl, 1-Phenylethyl, 2-Phenylethyl, 1-Phenylprop-1-yl, 2-Phenylprop-1-yl, 3-Phenylprop-1-yl, 1-Phenylbut-1-yl, 2-Phenylbut-1-yl, 3-Phenylbut-1-yl, 4-Phenylbut-1-yl, 1-Phenylbut-2-yl, 2-Phenylbut-2-yl, 3-Phenylbut-2-yl, 4-Phenylbut-2-yl, 1-(Phenylmethyl)-eth-1-yl, 1-(Phenylmethyl)-1-(methyl)-eth-1-yl oder 1-(Phenylmethyl)-prop-1-yl, insbesondere für Benzyl oder 2-Phenylethyl;
- Phenyl-(C₁-C₆-alkyl)carbonyloxy für: z.B. Benzylcarbonyloxy, 1-Phenylethylcarbonyloxy, 2-Phenylethylcarbonyloxy, 1-Phenyl-prop-1-ylcarbonyloxy, 2-Phenylprop-1-ylcarbonyloxy, 3-Phenyl-prop-1-ylcarbonyloxy, 1-Phenylbut-1-ylcarbonyloxy, 2-Phenyl-but-1-ylcarbonyloxy, 3-Phenylbut-1-ylcarbonyloxy, 4-Phenyl-but-1-ylcarbonyloxy, 1-Phenylbut-2-ylcarbonyloxy, 2-Phenyl-but-2-ylcarbonyloxy, 3-Phenylbut-2-ylcarbonyloxy, 4-Phenylbut-2-ylcarbonyloxy, 1-(Phenylmethyl)-eth-1-ylcarbonyloxy, 1-(Phenylmethyl)-1-(methyl)-eth-1-ylcarbonyloxy oder 1-(Phenylmethyl)-prop-1-ylcarbonyloxy, insbesondere für Benzylcarbonyloxy oder 2-Phenylethylcarbonyloxy;
- Phenyl-C₁-C₆-alkylsulfonyloxy für: z.B. Benzylsulfonyloxy, 1-Phenylethylsulfonyloxy, 2-Phenylethylsulfonyloxy, 1-Phenyl-prop-1-ylsulfonyloxy, 2-Phenylprop-1-ylsulfonyloxy, 3-Phenyl-prop-1-ylsulfonyloxy, 1-Phenylbut-1-ylsulfonyloxy, 2-Phenyl-but-1-ylsulfonyloxy, 3-Phenylbut-1-ylsulfonyloxy, 4-Phenyl-but-1-ylsulfonyloxy, 1-Phenylbut-2-ylsulfonyloxy, 2-Phenyl-but-2-ylsulfonyloxy, 3-Phenylbut-2-ylsulfonyloxy, 4-Phenyl-but-2-ylsulfonyloxy, 1-(Phenylmethyl)-eth-1-ylsulfonyloxy, 1-(Phenylmethyl)-1-(methyl)-eth-1-ylsulfonyloxy oder 1-(Phenylmethyl)-prop-1-ylsulfonyloxy, insbesondere für Benzylsulfonyloxy oder 2-Phenylethylsulfonyloxy;
- (C₁-C₆-Alkyl)carbonyl für: Methylcarbonyl, Ethylcarbonyl, n-Propylcarbonyl, 1-Methylethylcarbonyl, n-Butylcarbonyl, 1-Methylpropylcarbonyl, 2-Methylpropylcarbonyl, 1,1-Dimethylethylcarbonyl, n-Pentylcarbonyl, 1-Methylbutylcarbonyl, 2-Methylbutylcarbonyl, 3-Methylbutylcarbonyl, 1,1-Dimethylpropylcarbonyl, 1,2-Dimethylpropylcarbonyl, 2,2-Dimethylpropylcarbonyl, 1-Ethylpropylcarbonyl, n-Hexylcarbonyl, 1-Methylpentylcarbonyl, 2-Methylpentylcarbonyl, 3-Methylpentylcarbonyl, 4-Methylpentylcarbonyl, 1,1-Dimethylbutylcarbonyl, 1,2-Dimethylbutylcarbonyl, 1,3-Dimethylbutylcarbonyl, 2,2-Dimethylbutylcarbonyl, 2,3-Dimethylbutylcarbonyl, 3,3-Dimethylbutylcarbonyl, 1-Ethylbutylcarbonyl, 2-Ethylbutylcarbonyl, 1,1,2-Trimethylpropylcarbonyl, 1,2,2-Trimethylpropylcarbonyl, 1-Ethyl-1-methylpropylcarbonyl oder 1-Ethyl-2-methylpropylcarbonyl, insbesondere für Methylcarbonyl, Ethylcarbonyl oder 1-Methylethylcarbonyl;
- (C₁-C₆-Alkyl)carbonyl-C₁-C₆-alkyl für: durch (C₁-C₆-Alkyl)carbonyl wie vorstehend genannt substituiertes C₁-C₆-Alkyl, also z.B. für Methylcarbonylmethyl;
- (C₁-C₄-Alkyl)carboxyl für: Methylcarboxyl, Ethylcarboxyl, n-Propylcarboxyl, 1-Methylethylcarboxyl, n-Butylcarboxyl, 1-Methylpropylcarboxyl, 2-Methylpropylcarboxyl oder 1,1-Dimethylethylcarboxyl, insbesondere für Methylcarboxyl;
- (C₁-C₆-Halogenalkyl)carbonyl für: einen (C₁-C₆-Alkyl)carbonylrest wie vorstehend genannt, der partiell oder vollständig durch Fluor, Chlor, Brom und/oder Iod substituiert ist, also z.B. Chloracetyl, Dichloracetyl, Trichloracetyl, Fluoracetyl, Difluoracetyl, Trifluoracetyl, Chlorfluoracetyl, Dichlorfluoracetyl, Chlordifluoracetyl, 2-Fluorethylcarbonyl, 2-Chlorethylcarbonyl, 2-Bromethylcarbonyl, 2-Iodethylcarbonyl, 2,2-Difluorethylcarbonyl, 2,2,2-Trifluorethylcarbonyl, 2-Chlor-2-fluorethylcarbonyl, 2-Chlor-2,2-difluorethylcarbonyl, 2,2-Dichlor-2-fluorethylcarbonyl, 2,2,2-Trichlorethylcarbonyl, Pentafluorethylcarbonyl, 2-Fluorpropylcarbonyl, 3-Fluorpropylcarbonyl, 2,2-Difluorpropylcarbonyl, 2,3-Difluorpropylcarbonyl, 2-Chlorpropylcarbonyl, 3-Chlorpropylcarbonyl, 2,3-Dichlorpropylcarbonyl, 2-Brompropylcarbonyl, 3-Brompropylcarbonyl, 3,3,3-Trifluorpropylcarbonyl, 3,3,3-Trichlorpropylcarbonyl, 2,2,3,3,3-Pentafluorpropylcarbonyl, Heptafluorpropylcarbonyl, 1-(Fluormethyl)-2-fluorethylcarbonyl, 1-(Chlormethyl)-2-chlorethylcarbonyl, 1-(Brommethyl)-2-bromethylcarbonyl, 4-Fluorbutylcarbonyl, 4-Chlorbutylcarbonyl, 4-Brombutylcarbonyl, Nonafluorbutylcarbonyl, (5-Fluor-1-pentyl)carbonyl, (5-Chlor-1-pentyl)carbonyl, (5-Brom-1-pentyl)carbonyl, (5-Iod-1-pentyl)carbonyl, (5,5,5-Trichlor-1-pentyl)carbonyl, Undecafluorpentylcarbonyl, (6-Fluor-1-hexyl)carbonyl, (6-Chlor-1-hexyl)carbonyl, (6-Brom-1-hexyl)carbonyl, (6-Iod-1-hexyl)carbonyl, (6,6,6-Trichlor-1-hexyl)carbonyl oder Dodecafluorhexylcarbonyl, insbesondere für Trifluoracetyl;
- (C₁-C₆-Alkyl)carbonyloxy für: Acetyloxy, Ethylcarbonyloxy, n-Propylcarbonyloxy, 1-Methylethylcarbonyloxy, n-Butylcarbonyloxy, 1-Methylpropylcarbonyloxy, 2-Methylpropylcarbonyloxy, 1,1-Dimethylethylcarbonyloxy, n-Pentylcarbonyloxy, 1-Methylbutylcarbonyloxy, 2-Methylbutylcarbonyloxy, 3-Methylbutylcarbonyloxy, 1,1-Dimethylpropylcarbonyloxy, 1,2-Dimethylpropylcarbonyloxy, 2,2-Dimethylpropylcarbonyloxy, 1-Ethylpropylcarbonyloxy, n-Hexylcarbonyloxy, 1-Methylpentylcarbonyloxy, 2-Methylpentylcarbonyloxy, 3-Methylpentylcarbonyloxy, 4-Methylpentylcarbonyloxy, 1,1-Dimethylbutylcarbonyloxy, 1,2-Dimethylbutylcarbonyloxy, 1,3-Dimethylbutylcarbonyloxy, 2,2-Dimethylbutylcarbonyloxy, 2,3-Dimethylbutylcarbonyloxy, 3,3-Dimethylbutylcarbonyloxy, 1-Ethylbutylcarbonyloxy, 2-Ethylbutylcarbonyloxy, 1,1,2-Trimethylpropylcarbonyloxy, 1,2,2-Trimethylpropylcarbonyloxy, 1-Ethyl-1-methylpropylcarbonyloxy oder 1-Ethyl-2-methylpropylcarbonyloxy, insbesondere für Acetyloxy;
- (C₁-C₆-Halogenalkyl)carbonyloxy für: einen (C₁-C₆-Alkyl)carbonyloxy-Rest wie vorstehend genannt, der partiell oder vollständig durch Fluor, Chlor, Brom und/oder Iod substituiert ist, also z.B. Chloracetyloxy, Dichloracetyloxy, Trichloracetyloxy, Fluoracetyloxy, Difluoracetyloxy, Trifluoracetyloxy, Chlorfluoracetyloxy, Dichlorfluoracetyloxy, Chlordifluoracetyloxy, 2-Fluorethylcarbonyloxy, 2-Chlorethylcarbonyloxy, 2-Bromethylcarbonyloxy, 2-Iodethylcarbonyloxy, 2,2-Difluorethylcarbonyloxy, 2,2,2-Trifluorethylcarbonyloxy, 2-Chlor-2-fluorethylcarbonyloxy, 2-Chlor-2,2-difluorethylcarbonyloxy, 2,2-Dichlor-2-fluorethylcarbonyloxy, 2,2,2-Trichlorethylcarbonyloxy, Pentafluorethylcarbonyloxy, 2-Fluorpropylcarbonyloxy, 3-Fluorpropylcarbonyloxy, 2,2-Difluorpropylcarbonyloxy, 2,3-Difluorpropylcarbonyloxy, 2-Chlorpropylcarbonyloxy, 3-Chlorpropylcarbonyloxy, 2,3-Dichlorpropylcarbonyloxy, 2-Brompropylcarbonyloxy, 3-Brompropylcarbonyloxy, 3,3,3-Trifluorpropylcarbonyloxy, 3,3,3-Trichlorpropylcarbonyloxy, 2,2,3,3,3-Pentafluorpropylcarbonyloxy, Heptafluorpropylcarbonyloxy, 1-(Fluormethyl)-2-fluorethylcarbonyloxy, 1-(Chlormethyl)-2-chlorethylcarbonyloxy, 1-(Brommethyl)-2-bromethylcarbonyloxy, 4-Fluorbutylcarbonyloxy, 4-Chlorbutylcarbonyloxy, 4-Brombutylcarbonyloxy oder Nonafluorbutylcarbonyloxy, insbesondere für Trifluoracetyloxy;
- (C₁-C₆-Alkyl)carbonyloxy-C₁-C₆-alkyl für: durch (C₁-C₆-Alkyl)carbonyloxy wie vorstehend genannt substituiertes C₁-C₆-Alkyl, also z.B. für Methylcarbonyloxymethyl, Ethylcarbonyloxymethyl, 1-(Methylcarbonyloxy)ethyl, 2-(Methylcarbonyloxy)ethyl, 2-(Ethylcarbonyloxy)ethyl, 3-(Methylcarbonyloxy)propyl, 4-(Methoxycarbonyloxy)butyl, 5-(Methoxycarbonyloxy)pentyl oder 6-(Methoxycarbonyloxy)hexyl;
- C₁-C₆-Alkyl)carbonylthio für Acetylthio, Ethylcarbonylthio, n-Propylcarbonylthio, 1-Methylethylcarbonylthio, n-Butylcarbonylthio, 1-Methylpropylcarbonylthio, 2-Methylpropylcarbonylthio, 1,1-Dimethylethylcarbonylthio, n-Pentylcarbonylthio, 1-Methylbutylcarbonylthio, 2-Methylbutylcarbonylthio, 3-Methylbutylcarbonylthio, 1,1-Dimethylpropylcarbonylthio, 1,2-Dimethylpropylcarbonylthio, 2,2-Dimethylpropylcarbonylthio, 1-Ethylpropylcarbonylthio, n-Hexylcarbonylthio, 1-Methylpentylcarbonylthio, 2-Methylpentylcarbonylthio, 3-Methylpentylcarbonylthio, 4-Methylpentylcarbonylthio, 1,1-Dimethylbutylcarbonylthio, 1,2-Dimethylbutylcarbonylthio, 1,3-Dimethylbutylcarbonylthio, 2,2-Dimethylbutylcarbonylthio, 2,3-Dimethylbutylcarbonylthio, 3,3-Dimethylbutylcarbonylthio, 1-Ethylbutylcarbonylthio, 2-Ethylbutylcarbonylthio, 1,1,2-Trimethylpropylcarbonylthio, 1,2,2-Trimethylpropylcarbonylthio, 1-Ethyl-1-methylpropylcarbonylthio oder 1-Ethyl-2-methylpropylcarbonylthio, insbesondere für Acetylthio;
- (C₁-C₆-Halogenalkyl)carbonylthio für: einen (C₁-C₆-Alkyl)carbonylthio-Rest wie vorstehend genannt, der partiell oder vollständig durch Fluor, Chlor, Brom und/oder Iod substituiert ist, also z.B. Chloracetylthio, Dichloracetylthio, Trichloracetylthio, Fluoracetylthio, Difluoracetylthio, Trifluoracetylthio, Chlorfluoracetylthio, Dichlorfluoracetylthio, Chlordifluoracetylthio, 2-Fluorethylcarbonylthio, 2-Chlorethylcarbonylthio, 2-Bromethylcarbonylthio, 2-Iodethylcarbonylthio, 2,2-Difluorethylcarbonylthio, 2,2,2-Trifluorethylcarbonylthio, 2-Chlor-2-fluorethylcarbonylthio, 2-Chlor-2,2-difluorethylcarbonylthio, 2,2-Dichlor-2-fluorethylcarbonylthio, 2,2,2-Trichlorethylcarbonylthio, Pentafluorethylcarbonylthio, 2-Fluorpropylcarbonylthio, 3-Fluorpropylcarbonylthio, 2,2-Difluorpropylcarbonylthio, 2,3-Difluorpropylcarbonylthio, 2-Chlorpropylcarbonylthio, 3-Chlorpropylcarbonylthio, 2,3-Dichlorpropylcarbonylthio, 2-Brompropylcarbonylthio, 3-Brompropylcarbonylthio, 3,3,3-Trifluorpropylcarbonylthio, 3,3,3-Trichlorpropylcarbonylthio, 2,2,3,3,3-Pentafluorpropylcarbonylthio, Heptafluorpropylcarbonylthio, 1-(Fluormethyl)-2-fluorethylcarbonylthio, 1-(Chlormethyl)-2-chlorethylcarbonylthio, 1-(Brommethyl)-2-bromethylcarbonylthio, 4-Fluorbutylcarbonylthio, 4-Chlorbutylcarbonylthio, 4-Brombutylthio oder Nonafluorbutylthio, insbesondere für Trifluoracetylthio;
- (C₁-C₆-Alkyl)carbamoyloxy für: Methylcarbamoyloxy, Ethylcarbamoyloxy, n-Propylcarbamoyloxy, 1-Methylethylcarbamoyloxy, n-Butylcarbamoyloxy, 1-Methylpropylcarbamoyloxy, 2-Methylpropylcarbamoyloxy, 1,1-Dimethylethylcarbamoyloxy, n-Pentylcarbamoyloxy, 1-Methylbutylcarbamoyloxy, 2-Methylbutylcarbamoyloxy, 3-Methylbutylcarbamoyloxy, 1,1-Dimethylpropylcarbamoyloxy, 1,2-Dimethylpropylcarbamoyloxy, 2,2-Dimethylpropylcarbamoyloxy, 1-Ethylpropylcarbamoyloxy, n-Hexylcarbamoyloxy, 1-Methylpentylcarbamoyloxy, 2-Methylpentylcarbamoyloxy, 3-Methylpentylcarbamoyloxy, 4-Methylpentylcarbamoyloxy, 1,1-Dimethylbutylcarbamoyloxy, 1,2-Dimethylbutylcarbamoyloxy, 1,3-Dimethylbutylcarbamoyloxy, 2,2-Dimethylbutylcarbamoyloxy, 2,3-Dimethylbutylcarbamoyloxy, 3,3-Dimethylbutylcarbamoyloxy, 1-Ethylbutylcarbamoyloxy, 2-Ethylbutylcarbamoyloxy, 1,1,2-Trimethylpropylcarbamoyloxy, 1,2,2-Trimethylpropylcarbamoyloxy, 1-Ethyl-1-methylpropylcarbamoyloxy oder 1-Ethyl-2-methylpropylcarbamoyloxy, insbesondere für Methylcarbamoyloxy;
- (C₁-C₆-Halogenalkyl)carbamoyloxy für: einen (C₁-C₆-Alkyl)carbamoyloxy-Rest wie vorstehend genannt, der partiell oder vollständig durch Fluor, Chlor, Brom und/oder Iod substituiert ist, also z.B. Chlormethylcarbamoyloxy, Dichlormethylcarbamoyloxy, Trichlormethylcarbamoyloxy, Fluormethylcarbamoyloxy, Difluormethylcarbamoyloxy, Trifluormethylcarbamoyloxy, Chlorfluormethylcarbamoyloxy, Dichlorfluormethylcarbamoyloxy, Chlordifluormethylcarbamoyloxy, 2-Fluorethylcarbamoyloxy, 2-Chlorethylcarbamoyloxy, 2-Bromethylcarbamoyloxy, 2-Iodethylcarbamoyloxy, 2,2-Difluorethylcarbamoyloxy, 2,2,2-Trifluorethylcarbamoyloxy, 2-Chlor-2-fluorethylcarbamoyloxy, 2-Chlor-2,2-difluorethylcarbamoyloxy, 2,2-Dichlor-2-fluorethylcarbamoyloxy, 2,2,2-Trichlorethylcarbamoyloxy, Pentafluorethylcarbamoyloxy, 2-Fluorpropylcarbamoyloxy, 3-Fluorpropylcarbamoyloxy, 2,2-Difluorpropylcarbamoyloxy, 2,3-Difluorpropylcarbamoyloxy, 2-Chlorpropylcarbamoyloxy, 3-Chlorpropylcarbamoyloxy, 2,3-Dichlorpropylcarbamoyloxy, 2-Brompropylcarbamoyloxy, 3-Brompropylcarbamoyloxy, 3,3,3-Trifluorpropylcarbamoyloxy, 3,3,3-Trichlorpropylcarbamoyloxy, 2,2,3,3,3-Pentafluorpropylcarbamoyloxy, Heptafluorpropylcarbamoyloxy, 1-(Fluormethyl)-2-fluorethylcarbamoyloxy, 1-(Chlormethyl)-2-chlorethylcarbamoyloxy, 1-(Brommethyl) -2-bromethylcarbamoyloxy, 4-Fluorbutylcarbamoyloxy, 4-Chlorbutylcarbamoyloxy, 4-Brombutylcarbamoyloxy oder Nonafluorbutylcarbamoyloxy, insbesondere für Trifluormethylcarbamoyloxy;
- C₁-C₄-Alkoxy für: Methoxy, Ethoxy, n-Propoxy, 1-Methylethoxy, n-Butoxy, 1-Methylpropoxy, 2-Methylpropoxy oder 1,1-Dimethylethoxy, insbesondere für Methoxy oder Ethoxy;
- C₁-C₆-Alkoxy für: C₁-C₄-Alkoxy wie vorstehend genannt, sowie z.B. n-Pentoxy, 1-Methylbutoxy, 2-Methylbutoxy, 3-Methylbutoxy, 1,1-Dimethylpropoxy, 1,2-Dimethylpropoxy, 2,2-Dimethylpropoxy, 1-Ethylpropoxy, n-Hexoxy, 1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy und 1-Ethyl-2-methylpropoxy, insbesondere für Methoxy, Ethoxy oder 1-Methylethoxy;
- C₁-C₄-Halogenalkoxy für: einen C₁-C₄-Alkoxyrest wie vorstehend genannt, der partiell oder vollständig durch Fluor, Chlor, Brom und/oder Iod substituiert ist, also z.B. Chlormethoxy, Dichlormethoxy, Trichlormethoxy, Fluormethoxy, Difluormethoxy, Trifluormethoxy, Chlorfluormethoxy, Dichlorfluormethoxy, Chlordifluormethoxy, 2-Fluorethoxy, 2-Chlorethoxy, 2-Bromethoxy, 2-Iodethoxy, 2,2-Difluorethoxy, 2,2,2-Trifluorethoxy, 2-Chlor-2-fluorethoxy, 2-Chlor-2,2-difluorethoxy, 2,2-Dichlor-2-fluorethoxy, 2,2,2-Trichlorethoxy, Pentafluorethoxy, 2-Fluorpropoxy, 3-Fluorpropoxy, 2,2-Difluorpropoxy, 2,3-Difluorpropoxy, 2-Chlorpropoxy, 3-Chlorpropoxy, 2,3-Dichlorpropoxy, 2-Brompropoxy, 3-Brompropoxy, 3,3,3-Trifluorpropoxy, 3,3,3-Trichlorpropoxy, 2,2,3,3,3-Pentafluorpropoxy, Heptafluorpropoxy, 1-(Fluormethyl)-2-fluorethoxy, 1-(Chlormethyl)-2-chlorethoxy, 1-(Brommethyl)-2-bromethoxy, 4-Fluorbutoxy, 4-Chlorbutoxy, 4-Brombutoxy oder Nonafluorbutoxy, insbesondere für 2-Chlorethoxy oder 2,2,2-Trifluorethoxy;
- C₁-C₆-Halogenalkoxy für: einen C₁-C₆-Alkoxyrest wie vorstehend genannt, der partiell oder vollständig durch Fluor, Chlor, Brom und/oder Iod substituiert ist, also z.B. einen der unter C₁-C₄-Halogenalkoxy genannten Reste oder für 5-Fluor-1-pentoxy, 5-Chlor-1-pentoxy, 5-Brom-1-pentoxy, 5-Iod-1-pentoxy, 5,5,5-Trichlor-1-pentoxy, Undecafluorpentoxy, 6-Fluor-1-hexoxy, 6-Chlor-1-hexoxy, 6-Brom-1-hexoxy, 6-Iod-1-hexoxy, 6,6,6-Trichlor-1-hexoxy oder Dodecafluorhexoxy, insbesondere für Chlormethoxy, Fluormethoxy, Difluormethoxy, Trifluormethoxy, 2-Fluorethoxy, 2-Chlorethoxy oder 2,2,2-Trifluorethoxy;
- Phenyl-C₁-C₆-alkoxy für: z.B. Benzyloxy, 1-Phenylethoxy, 2-Phenylethoxy, 1-Phenylprop-1-yloxy, 2-Phenylprop-1-yloxy, 3-Phenylprop-1-yloxy, 1-Phenylbut-1-yloxy, 2-Phenylbut-1-yloxy, 3-Phenylbut-1-yloxy, 4-Phenylbut-1-yloxy, 1-Phenylbut-2-yloxy, 2-Phenylbut-2-yloxy, 3-Phenylbut-2-yloxy, 4-Phenylbut-2-yloxy, 1-(Phenylmethyl)-eth-1-yloxy, 1-(Phenylmethyl)-1-(methyl)-eth-1-yloxy oder 1-(Phenylmethyl)-prop-1-yloxy, insbesondere für Benzyloxy oder 2-Phenylethoxy;
- Heterocyclyl-C₁-C₆-alkoxy für: z.B. Heterocyclyl-methoxy, 1-(Heterocyclyl)ethoxy, 2-(Heterocyclyl)ethoxy, 1-(Heterocyclyl)prop-1-yloxy, 2-(Heterocyclyl)prop-1-yloxy, 3-(Heterocyclyl)prop-1-yloxy, 1-(Heterocyclyl)but-1-yloxy, 2-(Heterocyclyl)but-1-yloxy, 3-(Heterocyclyl)but-1-yloxy, 4-(Heterocyclyl)but-1-yloxy, 1-(Heterocyclyl)but-2-yloxy, 2-(Heterocyclyl)but-2-yloxy, 3-(Heterocyclyl)but-2-yloxy, 4-(Heterocyclyl)but-2-yloxy, 1-(Heterocyclylmethyl)-eth-1-yloxy, 1-(Heterocyclylmethyl)-1-(methyl)-eth-1-yloxy oder 1-(Heterocyclylmethyl)-prop-1-yloxy, insbesondere für Heterocyclylmethoxy oder 2-(Heterocyclyl)ethoxy;
- Phenyl-C₁-C₆-alkylthio für: z.B. Benzylthio, 1-Phenylethylthio, 2-Phenylethylthio, 1-Phenylprop-1-ylthio, 2-Phenylprop-1-ylthio, 3-Phenylprop-1-ylthio, 1-Phenylbut-1-ylthio, 2-Phenylbut-1-ylthio, 3-Phenylbut-1-ylthio, 4-Phenylbut-1-ylthio, 1-Phenylbut-2-ylthio, 2-Phenylbut-2-ylthio, 3-Phenylbut-2-ylthio, 3-Phenylbut-2-ylthio, 4-Phenylbut-2-ylthio, 1-(Phenylmethyl)-eth-1-ylthio, 1-(Phenylmethyl)-1-(methyl)-eth-1-ylthio oder 1-(Phenylmethyl)-prop-1-ylthio, insbesondere für Benzylthio oder 2-Phenylethylthio;
- (C₁-C₄-Alkoxy)carbonyl für: Methoxycarbonyl, Ethoxycarbonyl, n-Propoxycarbonyl, 1-Methylethoxycarbonyl, n-Butoxycarbonyl, 1-Methylpropoxycarbonyl, 2-Methylpropoxycarbonyl oder 1,1-Dimethylethoxycarbonyl, insbesondere für Methoxycarbonyl, Ethoxycarbonyl, Isopropoxycarbonyl oder Isobutoxycarbonyl;
- (C₁-C₆-Alkoxy)carbonyl für: (C₁-C₄-Alkoxy)carbonyl wie vorstehend genannt, sowie z.B. n-Pentoxycarbonyl, 1-Methylbutoxycarbonyl, 2-Methylbutoxycarbonyl, 3-Methylbutoxycarbonyl, 2,2-Dimethylpropoxycarbonyl, 1-Ethylpropoxycarbonyl, n-Hexoxycarbonyl, 1,1-Dimethylpropoxycarbonyl, 1,2-Dimethylpropoxycarbonyl, 1-Methylpentoxycarbonyl, 2-Methylpentoxycarbonyl, 3-Methylpentoxycarbonyl, 4-Methylpentoxycarbonyl, 1,1-Dimethylbutoxycarbonyl, 1,2-Dimethylbutoxycarbonyl, 1,3-Dimethylbutoxycarbonyl, 2,2-Dimethylbutoxycarbonyl, 2,3-Dimethylbutoxycarbonyl, 3,3-Dimethylbutoxycarbonyl, 1-Ethylbutoxycarbonyl, 2-Ethylbutoxycarbonyl, 1,1,2-Trimethylpropoxycarbonyl, 1,2,2-Trimethylpropoxycarbonyl, 1-Ethyl-1-methyl-propoxycarbonyl oder 1-Ethyl-2-methyl-propoxycarbonyl, insbesondere für Methoxycarbonyl, Ethoxycarbonyl oder 1-Methylethoxycarbonyl;
- (C₁-C₆-Alkoxy)carbonyloxy für: Methoxycarbonyloxy, Ethoxycarbonyloxy, n-Propoxycarbonyloxy, 1-Methylethoxycarbonyloxy, n-Butoxycarbonyloxy, 1-Methylpropoxycarbonyloxy, 2-Methylpropoxycarbonyloxy, 1,1-Dimethylethoxycarbonyloxy, n-Pentoxycarbonyloxy, 1-Methylbutoxycarbonyloxy, 2-Methylbutoxycarbonyloxy, 3-Methylbutoxycarbonyloxy, 2,2-Dimethylpropoxycarbonyloxy, 1-Ethylpropoxycarbonyloxy, n-Hexoxycarbonyloxy, 1,1-Dimethylpropoxycarbonyloxy, 1,2-Dimethylpropoxycarbonyloxy, 1-Methylpentoxycarbonyloxy, 2-Methylpentoxycarbonyloxy, 3-Methylpentoxycarbonyloxy, 4-Methylpentoxycarbonyloxy, 1,1-Dimethylbutoxycarbonyloxy, 1,2-Dimethylbutoxycarbonyloxy, 1,3-Dimethylbutoxycarbonyloxy, 2,2-Dimethylbutoxycarbonyloxy, 2,3-Dimethylbutoxycarbonyloxy, 3,3-Dimethylbutoxycarbonyloxy, 1-Ethylbutoxycarbonyloxy, 2-Ethylbutoxycarbonyloxy, 1,1,2-Trimethylpropoxycarbonyloxy, 1,2,2-Trimethylpropoxycarbonyloxy, 1-Ethyl-1-methyl-propoxycarbonyloxy oder l-Ethyl-2-methyl-propoxycarbonyloxy, insbesondere für Methoxycarbonyloxy, Ethoxycarbonyloxy oder 1-Methylethoxycarbonyloxy;
- (C₁-C₆-Alkoxy)carbonylthio für: Methoxycarbonylthio, Ethoxycarbonylthio, n-Propoxycarbonylthio, 1-Methylethoxycarbonylthio, n-Butoxycarbonylthio, 1-Methylpropoxycarbonylthio, 2-Methylpropoxycarbonylthio, 1,1-Dimethylethoxycarbonylthio, n-Pentoxycarbonylthio, 1-Methylbutoxycarbonylthio, 2-Methylbutoxycarbonylthio, 3-Methylbutoxycarbonylthio, 2,2-Dimethylpropoxycarbonylthio, 1-Ethylpropoxycarbonylthio, n-Hexoxycarbonylthio, 1,1-Dimethylpropoxycarbonylthio, 1,2-Dimethylpropoxycarbonylthio, 1-Methylpentoxycarbonylthio, 2-Methylpentoxycarbonylthio, 3-Methylpentoxycarbonylthio, 4-Methylpentoxycarbonylthio, 1,1-Dimethylbutoxycarbonylthio, 1,2-Dimethylbutoxycarbonylthio, 1,3-Dimethylbutoxycarbonylthio, 2,2-Dimethylbutoxycarbonylthio, 2,3-Dimethylbutoxycarbonylthio, 3,3-Dimethylbutoxycarbonylthio, 1-Ethylbutoxycarbonylthio, 2-Ethylbutoxycarbonylthio, 1,1,2-Trimethylpropoxycarbonylthio, 1,2,2-Trimethylpropoxycarbonylthio, 1-Ethyl-1-methyl-propoxycarbonylthio oder 1-Ethyl-2-methyl-propoxycarbonylthio, insbesondere für Methoxycarbonylthio, Ethoxycarbonylthio oder 1-Methylethoxycarbonylthio;
- C₁-C₆-Alkylthio für: Methylthio, Ethylthio, n-Propylthio, 1-Methylethylthio, n-Butylthio, 1-Methylpropylthio, 2-Methylpropylthio, 1,1-Dimethylethylthio, n-Pentylthio, 1-Methylbutylthio, 2-Methylbutylthio, 3-Methylbutylthio, 2,2-Dimethylpropylthio, 1-Ethylpropylthio, n-Hexylthio, 1,1-Dimethylpropylthio, 1,2-Dimethylpropylthio, 1-Methylpentylthio, 2-Methylpentylthio, 3-Methylpentylthio, 4-Methylpentylthio, 1,1-Dimethylbutylthio, 1,2-Dimethylbutylthio, 1,3-Dimethylbutylthio, 2,2-Dimethylbutylthio, 2,3-Dimethylbutylthio, 3,3-Dimethylbutylthio, 1-Ethylbutylthio, 2-Ethylbutylthio, 1,1,2-Trimethylpropylthio, 1,2,2-Trimethylpropylthio, 1-Ethyl-1-methylpropylthio und 1-Ethyl-2-methylpropylthio, insbesondere für Methylthio oder Ethylthio;
- C₁-C₄-Halogenalkylthio für: partiell oder vollständig durch Fluor, Chlor und/oder Brom substituiertes C₁-C₄-Alkylthio wie Methylthio, Ethylthio, n-Propylthio, 1-Methylethylthio, n-Butylthio, 1-Methylpropylthio, 2-Methylpropylthio und 1,1-Dimethylethylthio, vorzugsweise Methylthio oder Ethylthio,
   also z.B. Difluormethylthio, Trifluormethylthio, Chlordifluormethylthio, Bromdifluormethylthio, 2-Fluorethylthio, 2-Chlorethylthio, 2-Bromethylthio, 2-Iodethylthio, 2,2-Difluorethylthio, 2,2,2-Trifluorethylthio, 2,2,2-Trichlorethylthio, 2-Chlor-2-fluorethylthio, 2-chlor-2,2-difluorethylthio, 2,2-Dichlor-2-fluorethylthio, Pentafluorethylthio, 2-Fluorpropylthio, 3-Fluorpropylthio, 2-Chlorpropylthio, 3-Chlorpropylthio, 2-Brompropylthio, 3-Brompropylthio, 2,2-Difluorpropylthio, 2,3-Difluorpropylthio, 2,3-Dichlorpropylthio, 3,3,3-Trifluorpropylthio, 3,3,3-Trichlorpropylthio, 2,2,3,3,3-Pentafluorpropylthio, Heptafluorpropylthio, 1-(Fluormethyl)-2-fluorethylthio, 1-(Chlormethyl)-2-chlorethylthio, 1-(Brommethyl)-2-bromethylthio, 4-Fluorbutylthio, 4-Chlorbutylthio, 4-Brombutylthio oder Nonafluorbutylthio, insbesondere für Trifluormethylthio oder Difluormethylthio;
- C₁-C₆-Halogenalkylthio für: C₁-C₆-Alkylthio wie vorstehend genannt, das partiell oder vollständig durch Fluor, Chlor und/oder Brom substituiert ist, also z.B. einen der unter C₁-C₄-Halogenalkylthio genannten Reste oder für 5-Fluorpentylthio, 5-Chlorpentylthio, 5-Brompentylthio, 5-Iodpentylthio, Undecafluorpentylthio, 6-Fluorhexylthio oder 6-Chlorhexylthio, insbesondere für Chlormethylthio, Fluormethylthio, Difluormethylthio, Trifluormethylthio, 2-Fluorethylthio, 2-Chlorethylthio oder 2,2,2-Trifluorethylthio;
- C₁-C₆-Alkylsulfinyl für: Methylsulfinyl, Ethylsulfinyl, n-Propylsulfinyl, 1-Methylethylsulfinyl, n-Butylsulfinyl, 1-Methylpropylsulfinyl, 2-Methylpropylsulfinyl, 1,1-Dimethylethylsulfinyl, n-Pentylsulfinyl, 1-Methylbutylsulfinyl, 2-Methylbutylsulfinyl, 3-Methylbutylsulfinyl, 1,1-Dimethylpropylsulfinyl, 1,2-Dimethylpropylsulfinyl, 2,2-Dimethylpropylsulfinyl, 1-Ethylpropylsulfinyl, n-Hexylsulfinyl, 1-Methylpentylsulfinyl, 2-Methylpentylsulfinyl, 3-Methylpentylsulfinyl, 4-Methylpentylsulfinyl, 1,1-Dimethylbutylsulfinyl, 1,2-Dimethylbutylsulfinyl, 1,3-Dimethylbutylsulfinyl, 2,2-Dimethylbutylsulfinyl, 2,3-Dimethylbutylsulfinyl, 3,3-Dimethylbutylsulfinyl, 1-Ethylbutylsulfinyl, 2-Ethylbutylsulfinyl, 1,1,2-Trimethylpropylsulfinyl, 1,2,2-Trimethylpropylsulfinyl, 1-Ethyl-1-methylpropylsulfinyl oder 1-Ethyl-2-methylpropylsulfinyl, insbesondere für Methylsulfinyl;
- C₁-C₆-Alkylsulfonyl für: Methylsulfonyl, Ethylsulfonyl, n-Propylsulfonyl, 1-Methylethylsulfonyl, n-Butylsulfonyl, 1-Methylpropylsulfonyl, 2-Methylpropylsulfonyl, 1,1-Dimethylethylsulfonyl, n-Pentylsulfonyl, 1-Methylbutylsulfonyl, 2-Methylbutylsulfonyl, 3-Methylbutylsulfonyl, 1,1-Dimethylpropylsulfonyl, 1,2-Dimethylpropylsulfonyl, 2,2-Dimethylpropylsulfonyl, 1-Ethylpropylsulfonyl, n-Hexylsulfonyl, 1-Methylpentylsulfonyl, 2-Methylpentylsulfonyl, 3-Methylpentylsulfonyl, 4-Methylpentylsulfonyl, 1,1-Dimethylbutylsulfonyl, 1,2-Dimethylbutylsulfonyl, 1,3-Dimethylbutylsulfonyl, 2,2-Dimethylbutylsulfonyl, 2,3-Dimethylbutylsulfonyl, 3,3-Dimethylbutylsulfonyl, 1-Ethylbutylsulfonyl, 2-Ethylbutylsulfonyl, 1,1,2-Trimethylpropylsulfonyl, 1,2,2-Trimethylpropylsulfonyl, 1-Ethyl-1-methylpropylsulfonyl oder 1-Ethyl-2-methylpropylsulfonyl, insbesondere für Methylsulfonyl;
- C₁-C₆-Alkylsulfonyloxy für: Methylsulfonyloxy, Ethylsulfonyloxy, n-Propylsulfonyloxy, 1-Methylethylsulfonyloxy, n-Butylsulfonyloxy, 1-Methylpropylsulfonyloxy, 2-Methylpropylsulfonyloxy, 1,1-Dimethylethylsulfonyloxy, n-Pentylsulfonyloxy, 1-Methylbutylsulfonyloxy, 2-Methylbutylsulfonyloxy, 3-Methylbutylsulfonyloxy, 1,1-Dimethylpropylsulfonyloxy, 1,2-Dimethylpropylsulfonyloxy, 2,2-Dimethylpropylsulfonyloxy, 1-Ethylpropylsulfonyloxy, n-Hexylsulfonyloxy, 1-Methylpentylsulfonyloxy, 2-Methylpentylsulfonyloxy, 3-Methylpentylsulfonyloxy, 4-Methylpentylsulfonyloxy, 1,1-Dimethylbutylsulfonyloxy, 1,2-Dimethylbutylsulfonyloxy, 1,3-Dimethylbutylsulfonyloxy, 2,2-Dimethylbutylsulfonyloxy, 2,3-Dimethylbutylsulfonyloxy, 3,3-Dimethylbutylsulfonyloxy, 1-Ethylbutylsulfonyloxy, 2-Ethylbutylsulfonyloxy, 1,1,2-Trimethylpropylsulfonyloxy, 1,2,2-Trimethylpropylsulfonyloxy, 1-Ethyl-1-methylpropylsulfonyloxy oder 1-Ethyl-2-methylpropylsulfonyloxy, insbesondere für Methylsulfonyloxy;
- C₁-C₆-Halogenalkylsulfonyloxy für: C₁-C₆-Alkylsulfonyloxy wie vorstehend genannt, das partiell oder vollständig durch Fluor, Chlor, Brom und/oder Iod substituiert ist, also z.B. ClCH₂-SO₂-O-, CH(Cl)₂-SO₂-O-, C(Cl)₃-SO₂-O-, FCH₂-SO₂-O-, CHF₂-SO₂-O-, CF₃-SO₂-O-, Chlorfluormethyl-SO₂-O-, Dichlorfluormethyl-SO₂-O-, Chlordifluormethyl-SO₂-O-, 1-Fluorethyl-SO₂-O-, 2-Fluorethyl-SO₂-O-, 2-Chlorethyl-SO₂-O-, 2-Bromethyl-SO₂-O-, 2-Iodethyl-SO₂-O-, 2,2-Difluorethyl-SO₂-O-, 2,2,2-Trifluorethyl-SO₂-O-, 2-Chlor-2-fluorethyl-SO₂-O-, 2-Chlor-2,2-difluorethyl-SO₂-O-, 2,2-Dichlor-2-fluorethylSO₂-O-, 2,2,2-Trichlorethyl-SO₂-O-, C₂F₅-SO₂-O-, 2-Fluorpropyl-SO₂-O-, 3-Fluorpropyl-SO₂-O-, 2,2-Difluorpropyl-SO₂-O-, 2,3-Difluorpropyl-SO₂-O-, 2-Chlorpropyl-SO₂-O-, 3-Chlorpropyl-SO₂-O-, 2,3-Dichlorpropyl-SO₂-O-, 2-Brompropyl-SO₂-O-, 3-Brompropyl-SO₂-O-, 3,3,3-Trifluorpropyl-SO₂-O-, 3,3,3-Trichlorpropyl-SO₂-O-, 2,2,3,3,3-Pentafluorpropyl-SO₂-O-, C₂F₅-CF₂-SO₂-O-, 1-(Fluormethyl)-2-fluorethyl-SO₂-O-, 1-(Chlormethyl)-2-chlorethyl-SO₂-O-, 1-(Brommethyl)-2-bromethyl-SO₂-O-, 4-Fluorbutyl-SO₂-O-, 4-Chlorbutyl-SO₂-O-, 4-Brombutyl-SO₂-O-, C₂F₅-CF₂-CF₂-SO₂-O-, 5-Fluorpentyl-SO₂-O-, 5-Chlorpentyl-SO₂-O-, 5-Brompentyl-SO₂-O-, 5-Iodpentyl-SO₂-O-, 5,5,5-Trichlorpentyl-SO₂-O-, C₂F₅-CF₂-CF₂-CF₂-SO₂-O-, 6-Fluorhexyl-SO₂-O-, 6-Chlorhexyl-SO₂-O-, 6-Bromhexyl-SO₂-O-, 6-Iodhexyl-SO₂-O-, 6,6,6-Trichlorhexyl-SO₂-O- oder Dodecafluorhexyl-SO₂-O-, insbesondere für CF₃-SO₂-O-;
- C₁-C₄-Alkylamino für: Methylamino, Ethylamino, n-Propylamino, 1-Methylethylamino, n-Butylamino, 1-Methylpropylamino, 2-Methylpropylamino oder 1,1-Dimethylethylamino, insbesondere für Methylamino oder Ethylamino;
- (C₁-C₄-Alkylamino)carbonyl für: Methylaminocarbonyl, Ethylaminocarbonyl, n-Propylaminocarbonyl, 1-Methylethylaminocarbonyl, n-Butylaminocarbonyl, 1-Methylpropylaminocarbonyl, 2-Methylpropylaminocarbonyl oder 1,1-Dimethylethylaminocarbonyl, insbesondere für Methylaminocarbonyl oder Ethylaminocarbonyl;
- (C₁-C₆-Alkylamino)carbonyl für: (C₁-C₄-Alkylamino)carbonyl wie vorstehend genannt sowie z.B. n-Pentylaminocarbonyl, 1-Methylbutylaminocarbonyl, 2-Methylbutylaminocarbonyl, 3-Methylbutylaminocarbonyl, 2,2-Dimethylpropylaminocarbonyl, 1-Ethylpropylaminocarbonyl, n-Hexylaminocarbonyl, 1,1-Dimethylpropylaminocarbonyl, 1,2-Dimethylpropylaminocarbonyl, 1-Methylpentylaminocarbonyl, 2-Methylpentylaminocarbonyl, 3-Methylpentylaminocarbonyl, 4-Methylpentylaminocarbonyl, 1,1-Dimethylbutylaminocarbonyl, 1,2-Dimethylbutylaminocarbonyl, 1,3-Dimethylbutylaminocarbonyl, 2,2-Dimethylbutylaminocarbonyl, 2,3-Dimethylbutylaminocarbonyl, 3,3-Dimethylbutylaminocarbonyl, 1-Ethylbutylaminocarbonyl, 2-Ethylbutylaminocarbonyl, 1,1,2-Trimethylpropylaminocarbonyl, 1,2,2-Trimethylpropylaminocarbonyl, 1-Ethyl-1-methylpropylaminocarbonyl oder 1-Ethyl-2-methylpropylaminocarbonyl, insbesondere für Methylaminocarbonyl, Ethylaminocarbonyl oder Isopropylaminocarbonyl;
- Di-(C₁-C₆-alkyl)aminocarbonyl für: z.B. N,N-Dimethylaminocarbonyl, N,N-Diethylaminocarbonyl, N,N-Dipropylaminocarbonyl, N,N-Di-(1-methylethyl) aminocarbonyl, N,N-Dibutylaminocarbonyl, N,N-Di-(1-methylpropyl)aminocarbonyl, N,N-Di-(2-methylpropyl)aminocarbonyl, N,N-Di-(1,1-dimethylethyl)-aminocarbonyl, N-Ethyl-N-methylaminocarbonyl, N-Methyl-N-propylaminocarbonyl, N-Methyl-N-(1-methylethyl)aminocarbonyl, N-Butyl-N-methylaminocarbonyl, N-Methyl-N-(1-methylpropyl)aminocarbonyl, N-Methyl-N-(2-methylpropyl)aminocarbonyl, N-(1,1-Dimethylethyl)-N-methylaminocarbonyl, N-Ethyl-N-propylaminocarbonyl, N-Ethyl-N-(1-methylethyl)aminocarbonyl, N-Butyl-N-ethylaminocarbonyl, N-Ethyl-N-(1-methylpropyl)aminocarbonyl, N-Ethyl-N-(2-methylpropyl)aminocarbonyl, N-Ethyl-N-(1,1-dimethylethyl)aminocarbonyl, N-(1-Methylethyl)-N-propylaminocarbonyl, N-Butyl-N-propylaminocarbonyl, N-(1-Methylpropyl)-N-propylaminocarbonyl, N-(2-Methylpropyl)-N-propylaminocarbonyl, N-(1,1-Dimethylethyl)-N-propylaminocarbonyl, N-Butyl-N-(1-methylethyl)aminocarbonyl, N-(1-Methylethyl)-N-(1-methylpropyl)aminocarbonyl, N-(1-Methylethyl)-N-(2-methylpropyl)aminocarbonyl, N-(1,1-Dimethylethyl)-N-(1-methylethyl)aminocarbonyl, N-Butyl-N-(1-methylpropyl)aminocarbonyl, N-Butyl-N-(2-methylpropyl)aminocarbonyl, N-Butyl-N-(1,1-dimethylethyl)aminocarbonyl, N-(1-Methylpropyl)-N-(2-methylpropyl)aminocarbonyl, N-(1,1-Dimethylethyl)-N-(1-methylpropyl)aminocarbonyl oder N-(1,1-Dimethylethyl)-N-(2-methylpropyl)aminocarbonyl, insbesondere für N,N-Dimethylaminocarbonyl oder N,N-Diethylaminocarbonyl;
- (C₁-C₆-Alkyl)aminooxycarbonyl für: Methyliminooxycarbonyl, Ethyliminooxycarbonyl, n-Propyliminooxycarbonyl, 1-Methylethyliminooxycarbonyl, n-Butyliminooxycarbonyl, 1-Methylpropyliminooxycarbonyl, 2-Methylpropyliminooxycarbonyl, 1,1-Dimethylethyliminooxycarbonyl, n-Pentyliminooxycarbonyl, 1-Methylbutyliminooxycarbonyl, 2-Methylbutyliminooxycarbonyl, 3-Methylbutyliminooxycarbonyl, 1,1-Dimethylpropyliminooxycarbonyl, 1,2-Dimethylpropyliminooxycarbonyl, 2,2-Dimethylpropyliminooxycarbonyl, 1-Ethylpropyliminooxycarbonyl, n-Hexyliminooxycarbonyl, 1-Methylpentyliminooxycarbonyl, 2-Methylpentyliminooxycarbonyl, 3-Methylpentyliminooxycarbonyl, 4-Methylpentyliminooxycarbonyl, 1,1-Dimethylbutyliminooxycarbonyl, 1,2-Dimethylbutyliminooxycarbonyl, 1,3-Dimethylbutyliminooxycarbonyl, 2,2-Dimethylbutyliminooxycarbonyl, 2,3-Dimethylbutyliminooxycarbonyl, 3,3-Dimethylbutyliminooxycarbonyl, 1-Ethylbutyliminooxycarbonyl, 2-Ethylbutyliminooxycarbonyl, 1,1,2-Trimethylpropyliminooxycarbonyl, 1,2,2-Trimethylpropyliminooxycarbonyl, 1-Ethyl-1-methylpropyliminooxycarbonyl oder 1-Ethyl-2-methylpropyliminooxycarbonyl, insbesondere für Methyliminooxycarbonyl, Ethyliminooxycarbonyl oder 1-Methylethyliminooxycarbonyl;
- C₁-C₆-Alkylidenaminoxy für: Methylidenaminoxy, Ethylidenaminoxy, 1-Propylidenaminoxy, 2-Propylidenaminoxy, 1-Butylidenaminoxy, 2-Butylidenaminoxy oder 2-Hexylidenaminoxy, insbesondere für Methylidenaminoxy oder 2-Propylidenaminoxy;
- C₁-C₆-Alkyliminooxy für: Methyliminooxy, Ethyliminooxy, n-Propyliminooxy, 1-Methylethyliminooxy, n-Butyliminooxy, 1-Methylpropyliminooxy, 2-Methylpropyliminooxy, n-Pentyliminooxy, n-Hexyliminooxy, 1-Methylpentyliminooxy, 2-Methylpentyliminooxy, 3-Methylpentyliminooxy oder 4-Methylpentyliminooxy, insbesondere für Methyliminooxy, Ethyliminooxy oder Isopropyliminooxy;
- C₁-C₆-Alkyloximino-C₁-C₆-alkyl für: durch C₁-C₆-Alkyloximino wie Methoxyimino, Ethoxyimino, 1-Propoxyimino, 2-Propoxyimino, 1-Methylethoxyimino, n-Butoxyimino, sec.-Butoxyimino, tert.-Butoxyimino, 1-Methyl-1-propoxyimino, 2-Methyl-1-propoxyimino, 1-Methyl-2-propoxyimino, 2-Methyl-2-propoxyimino, n-Pentoxyimino, 2-Pentoxyimino, 3-Pentoxyimino, 4-Pentoxyimino, 1-Methyl-1-butoxyimino, 2-Methyl-1-butoxyimino, 3-Methyl-1-butoxyimino, 1-Methyl-2-butoxyimino, 2-Methyl-2-butoxyimino, 3-Methyl-2-butoxyimino, 1-Methyl-3-butoxyimino, 2-Methyl-3-butoxyimino, 3-Methyl-3-butoxyimino, 1,1-Dimethyl-2-propoxyimino, 1,2-Dimethyl-1-propoxyimino, 1,2-Dimethyl-2-propoxyimino, 1-Ethyl-1-propoxyimino, 1-Ethyl-2-propoxyimino, n-Hexoxyimino, 2-Hexoxyimino, 3-Hexoxyimino, 4-Hexoxyimino, 5-Hexoxyimino, 1-Methyl-1-pentoxyimino, 2-Methyl-1-pentoxyimino, 3-Methyl-1-pentoxyimino, 4-Methyl-1-pentoxyimino, 1-Methyl-2-pentoxyimino, 2-Methyl-2-pentoxyimino, 3-Methyl-2-pentoxyimino, 4-Methyl-2-pentoxyimino, 1-Methyl-3-pentoxyimino, 2-Methyl-3-pentoxyimino, 3-Methyl-3-pentoxyimino, 4-Methyl-3-pentoxyimino, 1-Methyl-4-pentoxyimino, 2-Methyl-4-pentoxyimino, 3-Methyl-4-pentoxyimino, 4-Methyl-4-pentoxyimino, 1,1-Dimethyl-2-butoxyimino, 1,1-Dimethyl-3-butoxyimino, 1,2-Dimethyl-1-butoxyimino, 1,2-Dimethyl-2-butoxyimino, 1,2-Dimethyl-3-butoxyimino, 1,3-Dimethyl-1-butoxyimino, 1,3-Dimethyl-2-butoxyimino, 1,3-Dimethyl-3-butoxyimino, 2,2-Dimethyl-3-butoxyimino, 2,3-Dimethyl-1-butoxyimino, 2,3-Dimethyl-2-butoxyimino, 2,3-Dimethyl-3-butoxyimino, 3,3-Dimethyl-1-butoxyimino, 3,3-Dimethyl-2-butoxyimino, 1-Ethyl-1-butoxyimino, 1-Ethyl-2-butoxyimino, 1-Ethyl-3-butoxyimino, 2-Ethyl-1-butoxyimino, 2-Ethyl-2-butoxyimino, 2-Ethyl-3-butoxyimino, 1,1,2-Trimethyl-2-propoxyimino, 1-Ethyl-1-methyl-2-propoxyimino, 1-Ethyl-2-methyl-1-propoxyimino und 1-Ethyl-2-methyl-2-propoxyimino, substituiertes C₁-C₆-Alkyl, also z.B. für Methoxyiminomethyl;
- C₁-C₆-Alkoxy-C₁-C₆-alkyl für: durch C₁-C₆-Alkoxy wie vorstehend genannt substituiertes C₁-C₆-Alkyl, also z.B. für Methoxymethyl, Ethoxymethyl, n-Propoxymethyl, (1-Methylethoxy)methyl, n-Butoxymethyl, (1-Methylpropoxy)methyl, (2-Methylpropoxy)methyl, (1,1-Dimethylethoxy)methyl, 2-(Methoxy)ethyl, 2-(Ethoxy)ethyl, 2-(n-Propoxy)ethyl, 2-(1-Methylethoxy)ethyl, 2-(n-Butoxy)ethyl, 2-(1-Methylpropoxy)ethyl, 2-(2-Methylpropoxy)ethyl, 2-(1,1-Dimethylethoxy)ethyl, 2-(Methoxy)propyl, 2-(Ethoxy)propyl, 2-(n-Propoxy)propyl, 2-(1-Methylethoxy)propyl, 2-(n-Butoxy)propyl, 2-(1-Methylpropoxy)propyl, 2-(2-Methylpropoxy)propyl, 2-(1,1-Dimethylethoxy)propyl, 3-(Methoxy)propyl, 3-(Ethoxy)propyl, 3-(n-Propoxy)propyl, 3-(1-Methylethoxy)propyl, 3-(n-Butoxy)propyl, 3-(1-Methylpropoxy)propyl, 3-(2-Methylpropoxy)propyl, 3-(1,1-Dimethylethoxy)propyl, 2-(Methoxy)butyl, 2-(Ethoxy)butyl, 2-(n-Propoxy)butyl, 2-(1-Methylethoxy)butyl, 2-(n-Butoxy)butyl, 2-(1-Methylpropoxy)butyl, 2-(2-Methylpropoxy)butyl, 2-(1,1-Dimethylethoxy)butyl, 3-(Methoxy)butyl, 3-(Ethoxy)butyl, 3-(n-Propoxy)butyl, 3-(1-Methylethoxy)butyl, 3-(n-Butoxy)butyl, 3-(1-Methylpropoxy)butyl, 3-(2-Methylpropoxy)butyl, 3-(1,1-Dimethylethoxy)butyl, 4-(Methoxy)butyl, 4-(Ethoxy)butyl, 4-(n-Propoxy)butyl, 4-(1-Methylethoxy)butyl, 4-(n-Butoxy)butyl, 4-(1-Methylpropoxy)butyl, 4-(2-Methylpropoxy)butyl oder 4-(1,1-Dimethylethoxy)butyl, insbesondere für Methoxymethyl oder 2-Methoxyethyl;
- C₁-C₆-Alkoxy-C₁-C₆-alkoxy für: durch C₁-C₆-Alkoxy wie vorstehend genannt substituiertes C₁-C₆-Alkoxy, also z.B. für Methoxymethoxy, Ethoxymethoxy, n-Propoxymethoxy, (1-Methylethoxy)methoxy, n-Butoxymethoxy, (1-Methylpropoxy)methoxy, (2-Methylpropoxy)methoxy, (1,1-Dimethylethoxy)methoxy, 2-(Methoxy)ethoxy, 2-(Ethoxy)ethoxy, 2-(n-Propoxy)ethoxy, 2-(1-Methylethoxy)ethoxy, 2-(n-Butoxy)ethoxy, 2-(1-Methylpropoxy)ethoxy, 2-(2-Methylpropoxy)ethoxy, 2-(1,1-Dimethylethoxy)ethoxy, 2-(Methoxy)propoxy, 2-(Ethoxy)propoxy, 2-(n-Propoxy)propoxy, 2-(1-Methylethoxy)propoxy, 2-(n-Butoxy)propoxy, 2-(1-Methylpropoxy)propoxy, 2-(2-Methylpropoxy)propoxy, 2-(1,1-Dimethylethoxy)propoxy, 3-(Methoxy)propoxy, 3-(Ethoxy)propoxy, 3-(n-Propoxy)propoxy, 3-(1-Methylethoxy)propoxy, 3-(n-Butoxy)propoxy, 3-(1-Methylpropoxy)propoxy, 3-(2-Methylpropoxy)propoxy, 3-(1,1-Dimethylethoxy)propoxy, 2-(Methoxy)butoxy, 2-(Ethoxy)butoxy, 2-(n-Propoxy)butoxy, 2-(1-Methylethoxy)butoxy, 2-(n-Butoxy)butoxy, 2-(1-Methylpropoxy)butoxy, 2-(2-Methylpropoxy)butoxy, 2-(1,1-Dimethylethoxy)butoxy, 3-(Methoxy)butoxy, 3-(Ethoxy)butoxy, 3-(n-Propoxy)butoxy, 3-(1-Methylethoxy)butoxy, 3-(n-Butoxy)butoxy, 3-(1-Methylpropoxy)butoxy, 3-(2-Methylpropoxy)butoxy, 3-(1,1-Dimethylethoxy)butoxy, 4-(Methoxy)butoxy, 4-(Ethoxy)butoxy, 4-(n-Propoxy)butoxy, 4-(1-Methylethoxy)butoxy, 4-(n-Butoxy)butoxy, 4-(1-Methylpropoxy)butoxy, 4-(2-Methylpropoxy)butoxy, 4-(1,1-Dimethylethoxy)butoxy, 5-(Methoxy)pentoxy, 5-(Ethoxy)pentoxy, 5-(n-Propoxy)pentoxy, 5-(1-Methylethoxy)pentoxy, 5-(n-Butoxy)pentoxy, 5-(1-Methylpropoxy)pentoxy, 5-(2-Methylpropoxy)pentoxy, 5-(1,1-Dimethylethoxy)pentoxy, 6-(Methoxy)hexoxy, 6-(Ethoxy)hexoxy, 6-(n-Propoxy)hexoxy, 6-(1-Methylethoxy)hexoxy, 6-(n-Butoxy)hexoxy, 6-(1-Methylpropoxy)hexoxy, 6-(2-Methylpropoxy)hexoxy oder 6-(1,1-Dimethylethoxy)hexoxy, insbesondere für Methoxymethoxy oder Ethoxymethoxy;
- C₁-C₆-Alkylthio-C₁-C₆-alkoxy für: durch C₁-C₆-Alkylthio wie vorstehend genannt substituiertes C₁-C₆-Alkoxy, also z.B. für Methylthiomethoxy, Ethylthiomethoxy, n-Propylthiomethoxy, (1-Methylethylthio)methoxy, n-Butylthiomethoxy, (1-Methylpropylthio)methoxy, (2-Methylpropylthio)methoxy, (1,1-Dimethylethylthio)methoxy, 2-(Methylthio)ethoxy, 2-(Ethylthio)ethoxy, 2-(n-Propylthio)ethoxy, 2-(1-Methylethylthio)ethoxy, 2-(n-Butylthio)ethoxy, 2-(1-Methylpropylthio)ethoxy, 2-(2-Methylpropylthio)ethoxy, 2-(1,1-Dimethylethylthio)ethoxy, 2-(Methylthio)propoxy, 2-(Ethylthio)propoxy, 2-(n-Propylthio)propoxy, 2-(1-Methylethylthio)propoxy, 2-(n-Butylthio)propoxy, 2-(1-Methylpropylthio)propoxy, 2-(2-Methylpropylthio)propoxy, 2-(1,1-Dimethylethylthio)propoxy, 3-(Methylthio)propoxy, 3-(Ethylthio)propoxy, 3-(n-Propylthio)propoxy, 3-(1-Methylethylthio)propoxy, 3-(n-Butylthio)propoxy, 3-(1-Methylpropylthio)propoxy, 3-(2-Methylpropylthio)propoxy, 3-(1,1-Dimethylethylthio)propoxy, 2-(Methylthio)butoxy, 2-(Ethylthio)butoxy, 2-(n-Propylthio)butoxy, 2-(1-Methylethylthio)butoxy, 2-(n-Butylthio)butoxy, 2-(1-Methylpropylthio)butoxy, 2-(2-Methylpropylthio)butoxy, 2-(1,1-Dimethylethylthio)butoxy, 3-(Methylthio)butoxy, 3-(Ethylthio)butoxy, 3-(n-Propylthio)butoxy, 3-(1-Methylethylthio)butoxy, 3-(n-Butylthio)butoxy, 3-(1-Methylpropylthio)butoxy, 3-(2-Methylpropylthio)butoxy, 3-(1,1-Dimethylethylthio)butoxy, 4-(Methylthio)butoxy, 4-(Ethylthio)butoxy, 4-(n-Propylthio)butoxy, 4-(1-Methylethylthio)butoxy, 4-(n-Butylthio)butoxy, 4-(1-Methylpropylthio)butoxy, 4-(2-Methylpropylthio)butoxy, 4-(1,1-Dimethylethylthio)butoxy, 5-(Methylthio)pentoxy, 5-(Ethylthio)pentoxy, 5-(n-Propylthio)pentoxy, 5-(1-Methylethylthio)pentoxy, 5-(n-Butylthio)pentoxy, 5-(1-Methylpropylthio)pentoxy, 5-(2-Methylpropylthio)pentoxy, 5-(1,1-Dimethylethylthio)pentoxy, 6-(Methylthio)hexoxy, 6-(Ethylthio)hexoxy, 6-(n-Propylthio)hexoxy, 6-(1-Methylethylthio)hexoxy, 6-(n-Butylthio)hexoxy, 6-(1-Methylpropylthio)hexoxy, 6-(2-Methylpropylthio)hexoxy oder 6-(1,1-Dimethylethylthio)hexoxy, insbesondere für Methylthiomethoxy oder Ethylthioethoxy;
- (C₁-C₆-Alkyl)carbonyl-C₁-C₆-alkoxy für: durch C₁-C₆-Alkyl wie vorstehend genannt substituiertes C₁-C₆-Alkoxy, also z.B. für Methylcarbonyl-methoxy, Ethylcarbonyl-methoxy, n-Propylcarbonyl-methoxy, n-Butylcarbonyl-methoxy, 1-(Methylcarbonyl)ethoxy, 2-(Methylcarbonyl)ethoxy, 2-(Ethylcarbonyl)ethoxy, 2-(n-Propylcarbonyl)ethoxy, 2-(n-Butylcarbonyl)ethoxy, 3-(Methylcarbonyl)propoxy, 3-(Ethylcarbonyl)propoxy, 3-(n-Propylcarbonyl)propoxy, 3-(n-Butylcarbonyl)propoxy, 4-(Methylcarbonyl)butoxy, 4-(Ethylcarbonyl)butoxy, 4-(n-Propylcarbonyl)butoxy, 4-(n-Butylcarbonyl)butoxy, 5-(Methylcarbonyl)pentoxy, 5-(Ethylcarbonyl)pentoxy, 5-(n-Propylcarbonyl)pentoxy, 5-(n-Butylcarbonyl)butoxy, 6-(Methylcarbonyl)hexoxy, 6-(Ethylcarbonyl)hexoxy, 6-(n-Propylcarbonyl)hexoxy oder 6-(n-Butylcarbonyl)hexoxy, insbesondere für Methylcarbonylmethoxy oder 1-(Methylcarbonyl)ethoxy;
- (C₁-C₆-Alkoxy)carbonyl-C₁-C₆-alkoxy für: durch (C₁-C₆-Alkoxy)carbonyl wie vorstehend genannt substituiertes C₁-C₆-Alkoxy, also z.B. für Methoxycarbonyl-methoxy, Ethoxycarbonylmethoxy, n-Propoxycarbonyl-methoxy, n-Butoxycarbonyl-methoxy, 1-(Methoxycarbonyl)ethoxy, 2-(Methoxycarbonyl)ethoxy, 2-(Ethoxycarbonyl)ethoxy, 2-(n-Propoxycarbonyl)ethoxy, 2-(n-Butoxycarbonyl)ethoxy, 3-(Methoxycarbonyl)propoxy, 3-(Ethoxycarbonyl)propoxy, 3-(n-Propoxycarbonyl)propoxy, 3-(n-Butoxycarbonyl)propoxy, 4-(Methoxycarbonyl)butoxy, 4-(Ethoxycarbonyl)butoxy, 4-(n-Propoxycarbonyl)butoxy, 4-(n-Butoxycarbonyl)butoxy, 5-(Methoxycarbonyl)pentoxy, 5-(Ethoxycarbonyl)pentoxy, 5-(n-Propoxycarbonyl)pentoxy, 5-(n-Butoxycarbonyl)butoxy, 6-(Methoxycarbonyl)hexoxy, 6-(Ethoxycarbonyl)hexoxy, 6-(n-Propoxycarbonyl)hexoxy oder 6-(n-Butoxycarbonyl)hexoxy, insbesondere für Methoxycarbonylmethoxy oder 1-(Methoxycarbonyl)ethoxy;
- (C₁-C₆-Alkoxy)carbonyl-C₁-C₆-alkyl für: durch (C₁-C₆-Alkoxy)carbonyl wie vorstehend genannt substituiertes C₁-C₆-Alkyl, also z.B. für Methoxycarbonylmethyl, Ethoxycarbonylmethyl, 1-(Methoxycarbonyl)ethyl, 2-(Methoxycarbonyl)ethyl, 2-(Ethoxycarbonyl)ethyl, 3-(Methoxycarbonyl)propyl, 4-(Methoxycarbonyl)butyl, 5-(Methoxycarbonyl)pentyl oder 6-(Methoxycarbonyl)hexyl;
- (C₁-C₆-Alkoxy)carbonyl-C₁-C₆-alkylsulfonyl für: durch (C₁-C₆-Alkoxy)carbonyl wie vorstehend genannt substituiertes C₁-C₆-Alkyl, also z.B. für Methoxycarbonylmethylsulfonyl, Ethoxycarbonylmethylsulfonyl, 1-(Methoxycarbonyl)ethylsulfonyl, 2-(Methoxycarbonyl)ethylsulfonyl, 2-(Ethoxycarbonyl)ethylsulfonyl, 3-(Methoxycarbonyl)propylsulfonyl, 4-(Methoxycarbonyl)butylsulfonyl, 5-(Methoxycarbonyl)pentylsulfonyl oder 6-(Methoxycarbonyl)hexylsulfonyl;
- C₁-C₆-Alkylthio-C₁-C₆-alkyl für: durch C₁-C₆-Alkylthio wie vorstehend genannt substituiertes C₁-C₆-Alkyl, also z.B. für Methylthiomethyl, Ethylthiomethyl, n-Propylthiomethyl, (1-Methylethylthio)methyl, n-Butylthiomethyl, (1-Methylpropylthio)methyl, (2-Methylpropylthio)methyl, (1,1-Dimethylethylthio)methyl, 2-Methylthioethyl, 2-Ethylthioethyl, 2-(n-Propylthio)ethyl, 2-(1-Methylethylthio)ethyl, 2-(n-Butylthio)ethyl, 2-(1-Methylpropylthio)ethyl, 2-(2-Methylpropylthio)ethyl, 2-(1,1-Dimethylethylthio)ethyl, 2-(Methylthio)propyl, 3-(Methylthio)propyl, 2-(Ethylthio)propyl, 3-(Ethylthio)propyl, 3-(Propylthio)propyl, 3-(Butylthio)propyl, 4-(Methylthio)butyl, 4-(Ethylthio)butyl, 4-(n-Propylthio)butyl oder 4-(n-Butylthio)butyl, insbesondere für 2-(Methylthio)ethyl;
- C₁-C₆-Alkylthio-(C₁-C₆-alkyl)carbonyl für: durch C₁-C₆-Alkylthio wie vorstehend genannt, vorzugsweise Methylthio oder Ethylthio, substituiertes (C₁-C₆-Alkyl)carbonyl, also z.B. für Methylthiomethylcarbonyl, Ethylthiomethylcarbonyl, 1-(Methylthio)ethylcarbonyl, 2-(Methylthio)ethylcarbonyl, 3-(Methylthio)propylcarbonyl, 4-(Methylthio)butylcarbonyl, 5-(Methylthio)pentylcarbonyl oder 6-(Methylthio)hexylcarbonyl, insbesondere für (Methylthio)methylcarbonyl oder 1-(Methylthio)ethylcarbonyl;
- Di-(C₁-C₆-alkyl)amino-C₁-C₆-alkoxy: durch Di-(C₁-C₆-alkyl)amino wie N,N-Dimethylamino, N,N-Diethylamino, N,N-Dipropylamino, N,N-Di-(1-methylethyl)amino, N,N-Dibutylamino, N,N-Di-(1-methylpropyl)amino, N,N-Di-(2-methylpropyl)amino, N,N-Di-(1,1-dimethylethyl)amino, N-Ethyl-N-methylamino, N-Methyl-N-propylamino, N-Methyl-N-(1-methylethyl)amino, N-Butyl-N-methylamino, N-Methyl-N-(1-methylpropyl)amino, N-Methyl-N-(2-methylpropyl)amino, N-(1,1-Dimethylethyl)-N-methylamino, N-Ethyl-N-propylamino, N-Ethyl-N-(1-methylethyl)amino, N-Butyl-N-ethylamino, N-Ethyl-N-(1-methylpropyl)amino, N-Ethyl-N-(2-methylpropyl)amino, N-Ethyl-N-(1,1-dimethylethyl)amino, N-(1-Methylethyl)-N-propylamino, N-Butyl-N-propylamino, N-(1-Methylpropyl)-N-propylamino, N-(2-Methylpropyl)-N-propylamino, N-(1,1-Dimethylethyl)-N-propylamino, N-Butyl-N-(1-methylethyl)amino, N-(1-Methylethyl)-N-(1-methylpropyl)amino, N-(1-Methylethyl)-N-(2-methylpropyl)amino, N-(1,1-Dimethylethyl)-N-(1-methylethyl)amino, N-Butyl-N-(1-methylpropyl)amino, N-Butyl-N-(2-methylpropyl)amino, N-Butyl-N-(1,1-dimethylethyl)amino, N-(1-Methylpropyl)-N-(2-methylpropyl)amino, N-(1,1-Dimethylethyl)-N-(1-methylpropyl)amino oder N-(1,1-Dimethylethyl)-N-(2-methylpropyl)amino, vorzugsweise N,N-Dimethylamino oder N,N-Diethylamino, substituiertes C₁-C₆-Alkoxy, also z.B. für Dimethylaminomethoxy, Diethylaminomethoxy, 1-(Dimethylamino)ethoxy, 2-(Dimethylamino)ethoxy, 3-(Dimethylamino)propoxy, 4-(Dimethylamino)butoxy, 5-(Dimethylamino)pentoxy oder 6-(Dimethylamino)hexoxy, insbesondere für Dimethylaminomethoxy oder 1-(Dimethylamino)ethoxy;
- C₃-C₆-Alkenyl für: z.B. Prop-2-en-1-yl, n-Buten-4-yl, 1-Methyl-prop-2-en-1-yl, 2-Methyl-prop-2-en-1-yl, 2-Buten-1-yl, n-Penten-3-yl, n-Penten-4-yl, 1-Methyl-but-2-en-1-yl, 2-Methyl-but-2-en-1-yl, 3-Methyl-but-2-en-1-yl, 1-Methyl-but-3-en-1-yl, 2-Methyl-but-3-en-1-yl, 3-Methyl-but-3-en-1-yl, 1,1-Dimethyl-prop-2-en-1-yl, 1,2-Dimethyl-prop-2-en1-yl, 1-Ethyl-prop-2-en-1-yl, n-Hex-3-en-1-yl, n-Hex-4-en-1-yl, n-Hex-5-en-1-yl, 1-Methyl-pent-3-en-1-yl, 2-Methyl-pent-3-en-1-yl, 3-Methyl-pent-3-en-1-yl, 4-Methyl-pent-3-en-1-yl, 1-Methyl-pent-4-en-1-yl, 2-Methyl-pent-4-en-1-yl, 3-Methyl-pent-4-en-1-yl, 4-Methyl-pent-4-en-1-yl, 1,1-Dimethyl-but-2-en-1-yl, 1,1-Dimethyl-but-3-en-1-yl, 1,2-Dimethyl-but-2-en-1-yl, 1,2-Dimethyl-but-3-en-1-yl, 1,3-Dimethyl-but-2-en-1-yl, 1,3-Dimethyl-but-3-en-1-yl, 2,2-Dimethyl-but-3-en-1-yl, 2,3-Dimethyl-but-2-en-1-yl, 2,3-Dimethyl-but-3-en-1-yl, 3,3-Dimethyl-but-2-en-1-yl, 1-Ethyl-but-2-en-1-yl, 1-Ethyl-but-3-en-1-yl, 2-Ethyl-but-2-en-1-yl, 2-Ethyl-but-3-en-1-yl, 1,1,2-Trimethyl-prop-2-en-1-yl, 1-Ethyl-1-methyl-prop-2-en-1-yl oder 1-Ethyl-2-methyl-prop-2-en-1-yl, insbesondere für Prop-2-en-1-yl oder n-Buten-4-yl;
- C₂-C₆-Alkenyl für: Ethenyl oder einen der unter C₃-C₆-Alkenyl genannten Reste, insbesondere für Ethenyl oder Prop-2-en-1-yl;
- C₃-C₆-Halogenalkenyl für: C₃-C₆-Alkenyl wie vorstehend genannt, das partiell oder vollständig durch Fluor, Chlor und/oder Brom substituiert ist, also z.B. 2-Chlorallyl, 3-Chlorallyl, 2,3-Dichlorallyl, 3,3-Dichlorallyl, 2,3,3-Trichlorallyl, 2,3-Dichlorbut-2-enyl, 2-Bromallyl, 3-Bromallyl, 2,3-Dibromallyl, 3,3-Dibromallyl, 2,3,3-Tribromallyl oder 2,3-Dibrombut-2-enyl, insbesondere für 2-Chlorallyl oder 3,3-Dichlorallyl;
- (C₁-C₆-Alkoxy)carbonyl-C₂-C₆-alkenyl für: durch (C₁-C₆-Alkoxy)carbonyl wie vorstehend genannt substituiertes C₂-C₆-Alkenyl, also z.B. für 3-(Methoxycarbonyl)allyl, 3-(Ethoxycarbonyl)allyl, 3-(n-Propoxycarbonyl)allyl, 3-(n-Butoxycarbonyl)allyl, 3-(n-Pentoxycarbonyl)allyl, 3-(n-Hexoxycarbonyl)allyl, 3-(Methoxycarbonyl)-2-methylallyl, 3-(Methoxycarbonyl)-3-methylallyl, 4-(Methoxycarbonyl)but-2-enyl, 4-(Ethoxycarbonyl)but-2-enyl, 4-(n-Propoxycarbonyl)but-2-enyl, 4-(n-Butoxycarbonyl)but-2-enyl, 4-(n-Pentoxycarbonyl)but-2-enyl, 4-(n-Hexoxycarbonyl)but-2-enyl, 4-(Methoxycarbonyl)but-3-enyl, 4-(Ethoxycarbonyl)but-3-enyl, 4-(n-Propoxycarbonyl)but-3-enyl), 4-(n-Butoxycarbonyl)but-3-enyl, 4-(n-Pentoxycarbonyl)but-3-enyl, 4-(n-Hexoxycarbonyl)but-3-enyl, 4-(Methoxycarbonyl)but-3-en-2-yl, 5-(Methoxycarbonyl)pent-4-enyl, 5-(Ethoxycarbonyl)pent-4-enyl, 5-(n-Propoxycarbonyl)pent-4-enyl, 5-(n-Butoxycarbonyl)pent-4-enyl, 5-(n-Pentoxycarbonyl)pent-4-enyl, 5-(n-Hexoxycarbonyl)pent-4-enyl, insbesondere für 3-(Methoxycarbonyl)allyl, 3-(Methoxycarbonyl)-2-methylallyl, 3-(Methoxycarbonyl)-3-methylallyl oder 4-(Methoxycarbonyl)but-3-en-2-yl;
- Phenyl-C₃-C₆-alkenyloxy für: z.B. 3-Phenyl-allyloxy, 4-Phenyl-but-2-enyloxy, 4-Phenyl-but-3-enyloxy oder 5-Phenyl-pent-4-enyloxy, vorzugsweise 3-Phenylallyloxy oder 4-Phenyl-but-2-enyloxy, insbesondere für 3-Phenylallyloxy;
- Heterocyclyl-C₃-C₆-alkenyloxy für: z.B. 3-Heterocyclyl-allyloxy, 4-Heterocyclyl-but-2-enyloxy, 4-Heterocyclyl-but-3-enyloxy oder 5-Heterocyclyl-pent-4-enyloxy, vorzugsweise 3-Heterocyclyl-allyloxy oder 4-Heterocyclyl-but-2-enyloxy, insbesondere für 3-Heterocyclyl-allyloxy;
- C₃-C₆-Alkenyloxy für: Prop-1-en-1-yloxy, Prop-2-en-1-yloxy, 1-Methylethenyloxy, n-Buten-1-yloxy, n-Buten-2-yloxy, n-Buten-3-yloxy, 1-Methyl-prop-1-en-1-yloxy, 2-Methyl-prop-1-en-1-yloxy, 1-Methyl-prop-2-en-1-yloxy, 2-Methyl-prop-2-en-1-yloxy, n-Penten-1-yloxy, n-Penten-2-yloxy, n-Penten-3-yloxy, n-Penten-4-yloxy, 1-Methyl-but-1-en-1-yl-oxy, 2-Methyl-but-1-en-1-yloxy, 3-Methyl-but-1-en-1-yloxy, 1-Methyl-but-2-en-1-yloxy, 2-Methyl-but-2-en-1-yloxy, 3-Methyl-but-2-en-1-yloxy, 1-Methyl-but-3-en-1-yloxy, 2-Methyl-but-3-en-1-yloxy, 3-Methyl-but-3-en-1-yloxy, 1,1-Dimethyl-prop-2-en-1-yloxy, 1,2-Dimethyl-prop-1-en-1-yl-oxy, 1,2-Dimethyl-prop-2-en-1-yloxy, 1-Ethyl-prop-1-en-2-yl-oxy, 1-Ethyl-prop-2-en-1-yloxy, n-Hex-1-en-1-yloxy, n-Hex-2-en-1-yloxy, n-Hex-3-en-1-yloxy, n-Hex-4-en-1-yloxy, n-Hex-5-en-1-yloxy, 1-Methyl-pent-1-en-1-yloxy, 2-Methyl-pent-1-en-1-yloxy, 3-Methyl-pent-1-en-1-yloxy, 4-Methyl-pent-1-en-1-yl-oxy, 1-Methyl-pent-2-en-1-yloxy, 2-Methyl-pent-2-en-1-yloxy, 3-Methyl-pent-2-en-1-yloxy, 4-Methyl-pent-2-en-1-yloxy, 1-Methyl-pent-3-en-1-yloxy, 2-Methyl-pent-3-en-1-yloxy, 3-Methyl-pent-3-en-1-yloxy, 4-Methyl-pent-3-en-1-yloxy, 1-Methyl-pent-4-en-1-yloxy, 2-Methyl-pent-4-en-1-yloxy, 3-Methyl-pent-4-en-1-yloxy, 4-Methyl-pent-4-en-1-yloxy, 1,1-Dimethyl-but-2-en-1-yloxy, 1,1-Dimethyl-but-3-en-1-yloxy, 1,2-Dimethyl-but-1-en-1-yloxy, 1,2-Dimethyl-but-2-en-1-yloxy, 1,2-Dimethyl-but-3-en-1-yloxy, 1,3-Dimethyl-but-1-en-1-yloxy, 1,3-Dimethyl-but-2-en-1-yloxy, 1,3-Dimethyl-but-3-en-1-yloxy, 2,2-Dimethyl-but-3-en-1-yloxy, 2,3-Dimethyl-but-1-en-1-yloxy, 2,3-Dimethyl-but-2-en-1-yloxy, 2,3-Dimethyl-but-3-en-1-yloxy, 3,3-Dimethyl-but-1-en-1-yloxy, 3,3-Dimethyl-but-2-en-1-yloxy, 1-Ethyl-but-1-en-1-yloxy, 1-Ethyl-but-2-en-1-yloxy, 1-Ethyl-but-3-en-1-yloxy, 2-Ethyl-but-1-en-1-yloxy, 2-Ethyl-but-2-en-1-yloxy, 2-Ethyl-but-3-en-1-yloxy, 1,1,2-Trimethyl-prop-2-en-1-yloxy, 1-Ethyl-1-methyl-prop-2-en-1-yloxy, 1-Ethyl-2-methyl-prop-1-en-1-yloxy oder 1-Ethyl-2-methyl-prop-2-en-1-yloxy, insbesondere für Prop-2-en-1-yloxy;
- C₂-C₆-Alkenyloxy für: Ethenyloxy oder einen der unter C₃-C₆-Alkenyloxy genannten Reste, insbesondere für Ethenyloxy oder Prop-2-en-1-yloxy;
- C₃-C₆-Halogenalkenyloxy für: C₃-C₆-Alkenyloxy wie vorstehend genannt, das partiell oder vollständig durch Fluor, Chlor und/oder Brom substituiert ist, also z.B. 2-Chlorallyloxy, 3-Chlorallyloxy, 2,3-Dichlorallyloxy, 3,3-Dichlorallyloxy, 2,3,3-Trichlorallyloxy, 2,3-Dichlorbut-2-enyloxy, 2-Bromallyloxy, 3-Bromallyloxy, 2,3-Dibromallyloxy, 3,3-Dibromallyloxy, 2,3,3-Tribromallyloxy oder 2,3-Dibrombut-2-enyloxy, insbesondere für 2-Chlorallyloxy oder 3,3-Dichlorallyloxy;
- C₁-C₆-Alkoxy-C₃-C₆-alkenyloxy für: durch C₁-C₆-Alkoxy wie vorstehend genannt substituiertes C₃-C₆-Alkenyloxy, also z.B. für 3-(Methoxy)allyloxy, 3-(Ethoxy)allyloxy, 3-(n-Propoxy)allyloxy, 3-(n-Butoxy)allyloxy, 3-(n-Pentoxy)allyloxy, 3-(n-Hexoxy)allyloxy, 3-(Methoxy)-1-methylallyloxy, 3-(Methoxy)-2-methylallyloxy, 3-(Methoxy)-3-methylallyloxy, 4-(Methoxy)but-2-enyloxy, 4-(Ethoxy)but-2-enyloxy, 4-(n-Propoxy)but-2-enyloxy, 4-(n-Butoxy)but-2-enyloxy, 4-(n-Pentoxy)but-2-enyloxy, 4-(n-Hexoxy)but-2-enyloxy, 4-(Methoxy)but-3-enyloxy, 4-(Ethoxy)but-3-enyloxy, 4-(n-Propoxy)but-3-enyloxy, 4-(n-Butoxy)but-3-enyloxy, 4-(n-Pentoxy)but-3-enyloxy, 4-(n-Hexoxy)but-3-enyloxy, 5-(Methoxy)pent-4-enyloxy, 5-(Ethoxy)pent-4-enyloxy, 5-(n-Propoxy)pent-4-enyloxy, 5-(n-Butoxy)pent-4-enyloxy, 5-(n-Pentoxy)pent-4-enyloxy, 5-(n-Hexoxy)pent-4-enyloxy, insbesondere für 3-(Methoxy)allyloxy, 3-(Methoxy)-1-methylallyloxy, 3-(Methoxy)-2-methylallyloxy oder 3-(Methoxy)-3-methylallyloxy;
- C₃-C₆-Alkenylthio für: Prop-1-en-1-ylthio, Prop-2-en-1-ylthio, 1-Methylethenylthio, n-Buten-1-ylthio, n-Buten-2-ylthio, n-Buten-3-ylthio, 1-Methyl-prop-1-en-1-ylthio, 2-Methyl-prop-1-en-1-ylthio, 1-Methyl-prop-2-en-1-ylthio, 2-Methyl-prop-2-en-1-ylthio, n-Penten-1-ylthio, n-Penten-2-ylthio, n-Penten-3-ylthio, n-Penten-4-ylthio, 1-Methyl-but-1-en-1-ylthio, 2-Methyl-but-1-en-1-ylthio, 3-Methyl-but-1-en-1-ylthio, 1-Methyl-but-2-en-1-ylthio, 2-Methyl-but-2-en-1-ylthio, 3-Methyl-but-2-en-1-ylthio, 1-Methyl-but-3-en-1-ylthio, 2-Methyl-but-3-en-1-ylthio, 3-Methyl-but-3-en-1-ylthio, 1,1-Dimethyl-prop-2-en-1-ylthio, 1,2-Dimethyl-prop-1-en-1-ylthio, 1,2-Dimethyl-prop-2-en-1-ylthio, 1-Ethyl-prop-1-en-2-ylthio, 1-Ethyl-prop-2-en-1-ylthio, n-Hex-1-en-1-ylthio, n-Hex-2-en-1-ylthio, n-Hex-3-en-1-ylthio, n-Hex-4-en-1-ylthio, n-Hex-5-en-1-ylthio, 1-Methyl-pent-1-en-1-ylthio, 2-Methyl-pent-1-en-1-ylthio, 3-Methyl-pent-1-en-1-ylthio, 4-Methyl-pent-1-en-1-ylthio, 1-Methyl-pent-2-en-1-ylthio, 2-Methyl-pent-2-en-1-ylthio, 3-Methyl-pent-2-en-1-ylthio, 4-Methyl-pent-2-en-1-ylthio, 1-Methyl-pent-3-en-1-ylthio, 2-Methyl-pent-3-en-1-ylthio, 3-Methyl-pent-3-en-1-ylthio, 4-Methyl-pent-3-en-1-ylthio, 1-Methyl-pent-4-en-1-ylthio, 2-Methyl-pent-4-en-1-ylthio, 3-Methyl-pent-4-en-1-ylthio, 4-Methyl-pent-4-en-1-ylthio, 1,1-Dimethyl-but-2-en-1-ylthio, 1,1-Dimethyl-but-3-en-1-ylthio, 1,2-Dimethyl-but-1-en-1-ylthio, 1,2-Dimethyl-but-2-en-1-ylthio, 1,2-Dimethyl-but-3-en-1-ylthio, 1,3-Dimethyl-but-1-en-1-ylthio, 1,3-Dimethyl-but-2-en-1-ylthio, 1,3-Dimethyl-but-3-en-1-ylthio, 2,2-Dimethyl-but-3-en-1-ylthio, 2,3-Dimethyl-but-1-en-1-ylthio, 2,3-Dimethyl-but-2-en-1-ylthio, 2,3-Dimethyl-but-3-en-1-ylthio, 3,3-Dimethyl-but-1-en-1-ylthio, 3,3-Dimethyl-but-2-en-1-ylthio, 1-Ethyl-but-1-en-1-ylthio, 1-Ethyl-but-2-en-1-ylthio, 1-Ethyl-but-3-en-1-ylthio, 2-Ethyl-but-1-en-1-ylthio, 2-Ethyl-but-2-en-1-ylthio, 2-Ethyl-but-3-en-1-ylthio, 1,1,2-Trimethyl-prop-2-en-1-ylthio, 1-Ethyl-1-methyl-prop-2-en-1-ylthio, 1-Ethyl-2-methyl-prop-1-en-1-ylthio oder 1-Ethyl-2-methyl-prop-2-en-1-ylthio, insbesondere für Prop-2-en-1-ylthio;
- C₂-C₆-Alkenylthio für: Ethenylthio oder einen der unter C₃-C₆-Alkenylthio genannten Reste, insbesondere für Ethenylthio oder Prop-2-en-1-ylthio;
- C₃-C₆-Alkinyl für: Prop-1-in-1-yl, Prop-2-in-1-yl, n-But-1-in-1-yl, n-But-1-in-3-yl, n-But-1-in-4-yl, n-But-2-in-1-yl, n-Pent-1-in-1-yl, n-Pent-1-in-3-yl, n-Pent-1-in-4-yl, n-Pent-1-in-5-yl, n-Pent-2-in-1-yl, n-Pent-2-in-4-yl, n-Pent-2-in-5-yl, 3-Methyl-but-1-in-3-yl, 3-Methyl-but-1-in-4-yl, n-Hex-1-in-1-yl, n-Hex-1-in-3-yl, n-Hex-1-in-4-yl, n-Hex-1-in-5-yl, n-Hex-1-in-6-yl, n-Hex-2-in-1-yl, n-Hex-2-in-4-yl, n-Hex-2-in-5-yl, n-Hex-2-in-6-yl, n-Hex-3-in-1-yl, n-Hex-3-in-2-yl, 3-Methyl-pent-1-in-1-yl, 3-Methyl-pent-1-in-3-yl, 3-Methyl-pent-1-in-4-yl, 3-Methyl-pent-1-in-5-yl, 4-Methyl-pent-1-in-1-yl, 4-Methyl-pent-2-in-4-yl oder 4-Methyl-pent-2-in-5-yl, insbesondere für Prop-2-in-1-yl;
- C₂-C₆-Alkinyl für: Ethinyl oder einen der unter C₃-C₆-Alkinyl genannten Reste, insbesondere für Ethinyl oder Prop-2-in-1-yl;
- C₃-C₆-Alkinyloxy für: Prop-1-in-1-yloxy, Prop-2-in-1-yloxy, n-But-1-in-1-yloxy, n-But-1-in-3-yloxy, n-But-1-in-4-yloxy, n-But-2-in-1-yloxy, n-Pent-1-in-1-yloxy, n-Pent-1-in-3-yloxy, n-Pent-1-in-4-yloxy, n-Pent-1-in-5-yloxy, n-Pent-2-in-1-yl-oxy, n-Pent-2-in-4-yloxy, n-Pent-2-in-5-yloxy, 3-Methyl-but-1-in-3-yloxy, 3-Methyl-but-1-in-4-yloxy, n-Hex-1-in-1-yloxy, n-Hex-1-in-3-yloxy, n-Hex-1-in-4-yloxy, n-Hex-1-in-5-yloxy, n-Hex-1-in-6-yloxy, n-Hex-2-in-1-yloxy, n-Hex-2-in-4-yloxy, n-Hex-2-in-5-yloxy, n-Hex-2-in-6-yloxy, n-Hex-3-in-1-yloxy, n-Hex-3-in-2-yloxy, 3-Methylpent-1-in-1-yloxy, 3-Methyl-pent-1-in-3-yloxy, 3-Methyl-pent-1-in-4-yloxy, 3-Methyl-pent-1-in-5-yloxy, 4-Methyl-pent-1-in-1-yloxy, 4-Methyl-pent-2-in-4-yloxy oder 4-Methylpent-2-in-5-yloxy, insbesondere für Prop-2-in-1-yloxy;
- C₂-C₆-Alkinyloxy für: Ethinyloxy oder einen der unter C₃-C₆-Alkinyloxy genannten Reste, insbesondere für Ethinyloxy oder Prop-2-in-1-yloxy;
- Phenyl-C₃-C₆-alkinyloxy für: z.B. 3-Phenylprop-2-in-1-yloxy, 4-Phenylbut-2-in-1-yloxy, 3-Phenylbut-3-in-2-yloxy, 5-Phenyl-pent-3-in-1-yloxy oder 6-Phenylhex-4-in-1-yloxy, insbesondere für 3-Phenylprop-2-in-1-yloxy oder 3-Phenylbut-3-in-2-yloxy;
- Heterocyclyl-C₃-C₆-alkinyloxy für: z.B. 3-(Heterocyclyl)prop-2-in-1-yloxy, 4-(Heterocyclyl)but-2-in-1-yloxy, 3-(Heterocyclyl)but-3-in-2-yloxy, 5-(Heterocyclyl)pent-3-in-1-yloxy oder 6-(Heterocyclyl)hex-4-in-1-yloxy, insbesondere für 3-(Heterocyclyl)prop-2-in-1-yloxy oder 3-(Heterocyclyl)but-3-in-2-yloxy;
- C₃-C₆-Alkinylthio für: Prop-1-in-1-ylthio, Prop-2-in-1-ylthio, n-But-1-in-1-ylthio, n-But-1-in-3-ylthio, n-But-1-in-4-ylthio, n-But-2-in-1-ylthio, n-Pent-1-in-1-ylthio, n-Pent-1-in-3-ylthio, n-Pent-1-in-4-ylthio, n-Pent-1-in-5-ylthio, n-Pent-2-in-1-ylthio, n-Pent-2-in-4-ylthio, n-Pent-2-in-5-ylthio, 3-Methyl-but-1-in-3-ylthio, 3-Methylbut-1-in-4-ylthio, n-Hex-1-in-1-ylthio, n-Hex-1-in-3-ylthio, n-Hex-1-in-4-ylthio, n-Hex-1-in-5-ylthio, n-Hex-1-in-6-ylthio, n-Hex-2-in-1-ylthio, n-Hex-2-in-4-ylthio, n-Hex-2-in-5-ylthio, n-Hex-2-in-6-ylthio, n-Hex-3-in-1-ylthio, n-Hex-3-in-2-ylthio, 3-Methylpent-1-in-1-ylthio, 3-Methyl-pent-1-in-3-ylthio, 3-Methyl-pent-1-in-4-ylthio, 3-Methylpent-1-in-5-ylthio, 4-Methyl-pent-1-in-1-ylthio, 4-Methyl-pent-2-in-4-ylthio oder 4-Methyl-pent-2-in-5-ylthio, insbesondere für Prop-2-in-1-ylthio;
- C₂-C₆-Alkinylthio für: Ethinylthio oder einen der unter C₃-C₆-Alkinylthio genannten Reste, insbesondere für Ethinylthio oder Prop-2-in-1-ylthio;
- (C₃-C₆-Alkenyl)oxycarbonyl für: Prop-1-en-1-yloxycarbonyl, Prop-2-en-1-yloxycarbonyl, 1-Methylethenyloxycarbonyl, n-Buten-1-yloxycarbonyl, n-Buten-2-yloxycarbonyl, n-Buten-3-yloxycarbonyl, 1-Methyl-prop-1-en-1-yloxycarbonyl, 2-Methyl-prop-1-en-1-yloxycarbonyl, 1-Methyl-prop-2-en-1-yloxycarbonyl, 2-Methyl-prop-2-en-1-yloxycarbonyl, n-Penten-1-yloxycarbonyl, n-Penten-2-yloxycarbonyl, n-Penten-3-yloxycarbonyl, n-Penten-4-yloxycarbonyl, 1-Methyl-but-1-en-1-yloxycarbonyl, 2-Methyl-but-1-en-1-yloxycarbonyl, 3-Methyl-but-1-en-1-yloxycarbonyl, 1-Methyl-but-2-en-1-yloxycarbonyl, 2-Methyl-but-2-en-1-yloxycarbonyl, 3-Methyl-but-2-en-1-yloxycarbonyl, 1-Methyl-but-3-en-1-yloxycarbonyl, 2-Methyl-but-3-en-1-yloxycarbonyl, 3-Methyl-but-3-en-1-yloxycarbonyl, 1,1-Dimethyl-prop-2-en-1-yloxycarbonyl, 1,2-Dimethyl-prop-1-en-1-yloxycarbonyl, 1,2-Dimethyl-prop-2-en-1-yloxycarbonyl, 1-Ethyl-prop-1-en-2-yloxycarbonyl, 1-Ethyl-prop-2-en-1-yloxycarbonyl, n-Hex-1-en-1-yloxycarbonyl, n-Hex-2-en-1-yloxycarbonyl, n-Hex-3-en-1-yloxycarbonyl, n-Hex-4-en-1-yloxycarbonyl, n-Hex-5-en-1-yloxycarbonyl, 1-Methyl-pent-1-en-1-yloxycarbonyl, 2-Methyl-pent-1-en-1-yloxycarbonyl, 3-Methyl-pent-1-en-1-yloxycarbonyl, 4-Methyl-pent-1-en-1-yloxycarbonyl, 1-Methyl-pent-2-en-1-yloxycarbonyl, 2-Methyl-pent-2-en-1-yloxycarbonyl, 3-Methyl-pent-2-en-1-yloxycarbonyl, 4-Methyl-pent-2-en-1-yloxycarbonyl, 1-Methyl-pent-3-en-1-yloxycarbonyl, 2-Methyl-pent-3-en-1-yloxycarbonyl, 3-Methyl-pent-3-en-1-yloxycarbonyl, 4-Methyl-pent-3-en-1-yloxycarbonyl, 1-Methyl-pent-4-en-1-yloxycarbonyl, 2-Methyl-pent-4-en-1-yloxycarbonyl, 3-Methyl-pent-4-en-1-yloxycarbonyl, 4-Methyl-pent-4-en-1-yloxycarbonyl, 1,1-Dimethyl-but-2-en-1-yloxycarbonyl, 1,1-Dimethyl-but-3-en-1-yloxycarbonyl, 1,2-Dimethyl-but-1-en-1-yloxycarbonyl, 1,2-Dimethyl-but-2-en-1-yloxycarbonyl, 1,2-Dimethyl-but-3-en-1-yloxycarbonyl, 1,3-Dimethyl-but-1-en-1-yloxycarbonyl, 1,3-Dimethyl-but-2-en-1-yloxycarbonyl, 1,3-Dimethyl-but-3-en-1-yloxycarbonyl, 2,2-Dimethyl-but-3-en-1-yloxycarbonyl, 2,3-Dimethyl-but-1-en-1-yloxycarbonyl, 2,3-Dimethyl-but-2-en-1-yloxycarbonyl, 2,3-Dimethyl-but-3-en-1-yloxycarbonyl, 3,3-Dimethyl-but-1-en-1-yloxycarbonyl, 3,3-Dimethyl-but-2-en-1-yloxycarbonyl, 1-Ethyl-but-1-en-1-yloxycarbonyl, 1-Ethyl-but-2-en-1-yloxycarbonyl, 1-Ethyl-but-3-en-1-yloxycarbonyl, 2-Ethyl-but-1-en-1-yloxycarbonyl, 2-Ethyl-but-2-en-1-yloxycarbonyl, 2-Ethyl-but-3-en-1-yloxycarbonyl, 1,1,2-Trimethyl-prop-2-en-1-yloxycarbonyl, 1-Ethyl-1-methyl-prop-2-en-1-yloxycarbonyl, 1-Ethyl-2-methyl-prop-1-en-1-yloxycarbonyl oder 1-Ethyl-2-methyl-prop-2-en-1-yloxycarbonyl, insbesondere für Prop-2-en-1-yloxycarbonyl;
- (C₃-C₆-Alkenyloxy)carbonyl-C₁-C₆-alkyl für: durch (C₃-C₆-Alkenyloxy)carbonyl wie vorstehend genannt, vorzugsweise Prop-2-en-1-yl-oxycarbonyl, substituiertes C₁-C₆-Alkyl, also beispielsweise Prop-2-en-1-yl-oxycarbonyl-methyl;
- (C₃-C₆-Alkenyl)carbonyloxy für: Prop-1-en-1-ylcarbonyloxy, Prop-2-en-1-ylcarbonyloxy, 1-Methylethenylcarbonyloxy, n-Buten-1-ylcarbonyloxy, n-Buten-2-ylcarbonyloxy, n-Buten-3-ylcarbonyloxy, 1-Methyl-prop-1-en-1-ylcarbonyloxy, 2-Methyl-prop-1-en-1-ylcarbonyloxy, 1-Methyl-prop-2-en-1-yl-carbonyloxy, 2-Methyl-prop-2-en-1-ylcarbonyloxy, n-Penten-1-ylcarbonyloxy, n-Penten-2-ylcarbonyloxy, n-Penten-3-ylcarbonyloxy, n-Penten-4-ylcarbonyloxy, 1-Methyl-but-1-en-1-ylcarbonyloxy, 2-Methyl-but-1-en-1-ylcarbonyloxy, 3-Methyl-but-1-en-1-ylcarbonyloxy, 1-Methyl-but-2-en-1-ylcarbonyloxy, 2-Methyl-but-2-en-1-ylcarbonyloxy, -3-Methyl-but-2-en-1-ylcarbonyloxy, 1-Methyl-but-3-en-1-ylcarbonyloxy, 2-Methyl-but-3-en-1-ylcarbonyloxy, 3-Methyl-but-3-en-1-ylcarbonyloxy, 1,1-Dimethyl-prop-2-en-1-ylcarbonyloxy, 1,2-Dimethyl-prop-1-en-1-ylcarbonyloxy, 1,2-Dimethyl-prop-2-en-1-ylcarbonyloxy, 1-Ethyl-prop-1-en-2-ylcarbonyloxy, 1-Ethyl-prop-2-en-1-ylcarbonyloxy, n-Hex-1-en-1-ylcarbonyloxy, n-Hex-2-en-1-ylcarbonyloxy, n-Hex-3-en-1-ylcarbonyloxy, n-Hex-4-en-1-ylcarbonyloxy, n-Hex-5-en-1-ylcarbonyloxy, 1-Methyl-pent-1-en-1-ylcarbonyloxy, 2-Methyl-pent-1-en-1-ylcarbonyloxy, 3-Methyl-pent-1-en-1-ylcarbonyloxy, 4-Methyl-pent-1-en-1-ylcarbonyloxy, 1-Methyl-pent-2-en-1-ylcarbonyloxy, 2-Methyl-pent-2-en-1-ylcarbonyloxy, 3-Methyl-pent-2-en-1-ylcarbonyloxy, 4-Methyl-pent-2-en-1-ylcarbonyloxy, 1-Methyl-pent-3-en-1-ylcarbonyloxy, 2-Methyl-pent-3-en-1-ylcarbonyloxy, 3-Methyl-pent-3-en-1-ylcarbonyloxy, 4-Methyl-pent-3-en-1-ylcarbonyloxy, 1-Methyl-pent-4-en-1-ylcarbonyloxy, 2-Methylpent-4-en-1-ylcarbonyloxy, 3-Methyl-pent-4-en-1-ylcarbonyloxy, 4-Methyl-pent-4-en-1-ylcarbonyloxy, 1,1-Dimethyl-but-2-en-1-ylcarbonyloxy, 1,1-Dimethyl-but-3-en-1-ylcarbonyloxy, 1,2-Dimethyl-but-1-en-1-ylcarbonyloxy, 1,2-Dimethyl-but-2-en-1-ylcarbonyloxy, 1,2-Dimethyl-but-3-en-1-ylcarbonyloxy, 1,3-Dimethyl-but-1-en-1-ylcarbonyloxy, 1,3-Dimethyl-but-2-en-1-ylcarbonyloxy, 1,3-Dimethyl-but-3-en-1-ylcarbonyloxy, 2,2-Dimethyl-but-3-en-1-ylcarbonyloxy, 2,3-Dimethyl-but-1-en-1-ylcarbonyloxy, 2,3-Dimethyl-but-2-en-1-ylcarbonyloxy, 2,3-Dimethyl-but-3-en-1-ylcarbonyloxy, 3,3-Dimethyl-but-1-en-1-ylcarbonyloxy, 3,3-Dimethyl-but-2-en-1-ylcarbonyloxy, 1-Ethyl-but-1-en-1-ylcarbonyloxy, 1-Ethyl-but-2-en-1-ylcarbonyloxy, 1-Ethyl-but-3-en-1-ylcarbonyloxy, 2-Ethyl-but-1-en-1-ylcarbonyloxy, 2-Ethyl-but-2-en-1-ylcarbonyloxy, 2-Ethyl-but-3-en-1-ylcarbonyloxy, 1,1,2-Trimethyl-prop-2-en-1-ylcarbonyloxy, 1-Ethyl-1-methyl-prop-2-en-1-ylcarbonyloxy, 1-Ethyl-2-methyl-prop-1-en-1-ylcarbonyloxy oder 1-Ethyl-2-methyl-prop-2-en-1-ylcarbonyloxy, insbesondere für Prop-2-en-1-ylcarbonyloxy;
- (C₂-C₆-Alkenyl)carbonyloxy für: Ethenylcarbonyloxy oder einen der unter (C₃-C₆-Alkenyl)carbonyloxy genannten Reste, insbesondere für Ethenylcarbonyloxy oder Prop-2-en-1-ylcarbonyloxy;
- (C₃-C₆-Alkenyl)carbonylthio für: Prop-1-en-1-ylcarbonylthio, Prop-2-en-1-ylcarbonylthio, 1-Methylethenylcarbonylthio, n-Buten-1-ylcarbonylthio, n-Buten-2-ylcarbonylthio, n-Buten-3-ylcarbonylthio, 1-Methyl-prop-1-en-1-ylcarbonylthio, 2-Methyl-prop-1-en-1-ylcarbonylthio, 1-Methyl-prop-2-en-1-ylcarbonylthio, 2-Methyl-prop-2-en-1-ylcarbonylthio, n-Penten-1-ylcarbonylthio, n-Penten-2-ylcarbonylthio, n-Penten-3-ylcarbonylthio, n-Penten-4-ylcarbonylthio, 1-Methyl-but-1-en-1-ylcarbonylthio, 2-Methyl-but-1-en-1-ylcarbonylthio, 3-Methyl-but-1-en-1-ylcarbonylthio, 1-Methyl-but-2-en-1-ylcarbonylthio, 2-Methyl-but-2-en-1-ylcarbonylthio, 3-Methyl-but-2-en-1-ylcarbonylthio, 1-Methyl-but-3-en-1-ylcarbonylthio, 2-Methyl-but-3-en-1-ylcarbonylthio, 3-Methyl-but-3-en-1-ylcarbonylthio, 1,1-Dimethyl-prop-2-en-1-ylcarbonylthio, 1,2-Dimethyl-prop-1-en-1-ylcarbonylthio, 1,2-Dimethyl-prop-2-en-1-ylcarbonylthio, 1-Ethyl-prop-1-en-2-ylcarbonylthio, 1-Ethyl-prop-2-en-1-ylcarbonylthio, n-Hex-1-en-1-ylcarbonylthio, n-Hex-2-en-1-ylcarbonylthio, n-Hex-3-en-1-ylcarbonylthio, n-Hex-4-en-1-ylcarbonylthio, n-Hex-5-en-1-ylcarbonylthio, 1-Methyl-pent-1-en-1-ylcarbonylthio, 2-Methyl-pent-1-en-1-ylcarbonylthio, 3-Methyl-pent-1-en-1-ylcarbonylthio, 4-Methyl-pent-1-en-1-ylcarbonylthio, 1-Methyl-pent-2-en-1-ylcarbonylthio, 2-Methyl-pent-2-en-1-ylcarbonylthio, 3-Methyl-pent-2-en-1-ylcarbonylthio, 4-Methyl-pent-2-en-1-ylcarbonylthio, 1-Methyl-pent-3-en-1-ylcarbonylthio, 2-Methyl-pent-3-en-1-ylcarbonylthio, 3-Methyl-pent-3-en-1-ylcarbonylthio, 4-Methyl-pent-3-en-1-ylcarbonylthio, 1-Methyl-pent-4-en-1-ylcarbonylthio, 2-Methyl-pent-4-en-1-ylcarbonylthio, 3-Methyl-pent-4-en-1-ylcarbonylthio, 4-Methyl-pent-4-en-1-ylcarbonylthio, 1,1-Dimethyl-but-2-en-1-ylcarbonylthio, 1,1-Dimethyl-but-3-en-1-ylcarbonylthio, 1,2-Dimethyl-but-1-en-1-ylcarbonylthio, 1,2-Dimethyl-but-2-en-1-ylcarbonylthio, 1,2-Dimethyl-but-3-en-1-ylcarbonylthio, 1,3-Dimethyl-but-1-en-1-ylcarbonylthio, 1,3-Dimethyl-but-2-en-1-ylcarbonylthio, 1,3-Dimethyl-but-3-en-1-ylcarbonylthio, 2,2-Dimethyl-but-3-en-1-ylcarbonylthio, 2,3-Dimethyl-but-1-en-1-ylcarbonylthio, 2,3-Dimethyl-but-2-en-1-ylcarbonylthio, 2,3-Dimethyl-but-3-en-1-ylcarbonylthio, 3,3-Dimethyl-but-1-en-1-ylcarbonylthio, 3,3-Dimethyl-but-2-en-1-ylcarbonylthio, 1-Ethyl-but-1-en-1-ylcarbonylthio, 1-Ethyl-but-2-en-1-ylcarbonylthio, 1-Ethyl-but-3-en-1-ylcarbonylthio, 2-Ethyl-but-1-en-1-ylcarbonylthio, 2-Ethyl-but-2-en-1-ylcarbonylthio, 2-Ethyl-but-3-en-1-ylcarbonylthio, 1,1,2-Trimethyl-prop-2-en-1-ylcarbonylthio, 1-Ethyl-1-methyl-prop-2-en-1-ylcarbonylthio, 1-Ethyl-2-methyl-prop-1-en-1-ylcarbonylthio oder 1-Ethyl-2-methyl-prop-2-en-1-ylcarbonylthio, insbesondere für Prop-2-en-1-ylcarbonylthio;
- (C₂-C₆-Alkenyl)carbonylthio für: Ethenylcarbonylthio oder einen der unter (C₃-C₆-Alkenyl)carbonylthio genannten Reste, insbesondere für Prop-2-en-1-yl-carbonylthio;
- (C₃-C₆-Alkinyl)carbonyloxy für: Prop-1-in-1-ylcarbonyloxy, Prop-2-in-1-ylcarbonyloxy, n-But-1-in-1-ylcarbonyloxy, n-But-1-in-3-ylcarbonyloxy, n-But-1-in-4-ylcarbonyloxy, n-But-2-in-1-ylcarbonyloxy, n-Pent-1-in-1-ylcarbonyloxy, n-Pent-1-in-3-ylcarbonyloxy, n-Pent-1-in-4-ylcarbonyloxy, n-Pent-1-in-5-ylcarbonyloxy, n-Pent-2-in-1-ylcarbonyloxy, n-Pent-2-in-4-ylcarbonyloxy, n-Pent-2-in-5-ylcarbonyloxy, 3-Methyl-but-1-in-3-ylcarbonyloxy, 3-Methyl-but-1-in-4-ylcarbonyloxy, n-Hex-1-in-1-ylcarbonyloxy, n-Hex-1-in-3-ylcarbonyloxy, n-Hex-1-in-4-ylcarbonyloxy, n-Hex-1-in-5-ylcarbonyloxy, n-Hex-1-in-6-ylcarbonyloxy, n-Hex-2-in-1-ylcarbonyloxy, n-Hex-2-in-4-ylcarbonyloxy, n-Hex-2-in-5-ylcarbonyloxy, n-Hex-2-in-6-ylcarbonyloxy, n-Hex-3-in-1-ylcarbonyloxy, n-Hex-3-in-2-ylcarbonyloxy, 3-Methylpent-1-in-1-ylcarbonyloxy, 3-Methyl-pent-1-in-3-ylcarbonyloxy, 3-Methyl-pent-1-in-4-ylcarbonyloxy, 3-Methyl-pent-1-in-5-ylcarbonyloxy, 4-Methyl-pent-1-in-1-ylcarbonyloxy, 4-Methyl-pent-2-in-4-ylcarbonyloxy oder 4-Methylpent-2-in-5-ylcarbonyloxy, insbesonere für Prop-2-in-1-ylcarbonyloxy;
- (C₂-C₆-Alkinyl)carbonyloxy für: Ethinylcarbonyloxy oder einen der unter (C₃-C₆-Alkinyl)carbonyloxy genannten Reste, insbesondere für Ethinylcarbonyloxy oder Prop-2-in-1-ylcarbonyloxy;
- C₃-C₆-Alkinylsulfonyloxy für: Prop-1-in-1-ylsulfonyloxy, Prop-2-in-1-ylsulfonyloxy, n-But-1-in-1-ylsulfonyloxy, n-But-1-in-3-ylsulfonyloxy, n-But-1-in-4-ylsulfonyloxy, n-But-2-in-1-ylsulfonyloxy, n-Pent-1-in-1-ylsulfonyloxy, n-Pent-1-in-3-ylsulfonyloxy, n-Pent-1-in-4-ylsulfonyloxy, n-Pent-1-in-5-ylsulfonyloxy, n-Pent-2-in-1-ylsulfonyloxy, n-Pent-2-in-4-ylsulfonyloxy, n-Pent-2-in-5-ylsulfonyloxy, 3-Methyl-but-1-in-3-ylsulfonyloxy, 3-Methyl-but-1-in-4-ylsulfonyloxy, n-Hex-1-in-1-ylsulfonyloxy, n-Hex-1-in-3-ylsulfonyloxy, n-Hex-1-in-4-ylsulfonyloxy, n-Hex-1-in-5-ylsulfonyloxy, n-Hex-1-in-6-ylsulfonyloxy, n-Hex-2-in-1-ylsulfonyloxy, n-Hex-2-in-4-ylsulfonyloxy, n-Hex-2-in-5-ylsulfonyloxy, n-Hex-2-in-6-ylsulfonyloxy, n-Hex-3-in-1-ylsulfonyloxy, n-Hex-3-in-2-ylsulfonyloxy, 3-Methylpent-1-in-1-ylsulfonyloxy, 3-Methyl-pent-1-in-3-ylsulfonyloxy, 3-Methyl-pent-1-in-4-ylsulfonyloxy, 3-Methyl-pent-1-in-5-ylsulfonyloxy, 4-Methyl-pent-1-in-1-ylsulfonyloxy, 4-Methyl-pent-2-in-4-ylsulfonyloxy oder 4-Methylpent-2-in-5-ylsulfonyloxy, insbesonere für Prop-2-in-1-ylsulfonyloxy;
- (C₃-C₆-Alkinyl)carbonylthio für: Prop-1-in-1-ylcarbonylthio, Prop-2-in-1-ylcarbonylthio, n-But-1-in-1-ylcarbonylthio, n-But-1-in-3-ylcarbonylthio, n-But-1-in-4-ylcarbonylthio, n-But-2-in-1-ylcarbonylthio, n-Pent-1-in-1-ylcarbonylthio, n-Pent-1-in-3-ylcarbonylthio, n-Pent-1-in-4-ylcarbonylthio, n-Pent-1-in-5-ylcarbonylthio, n-Pent-2-in-1-ylcarbonylthio, n-Pent-2-in-4-ylcarbonylthio, n-Pent-2-in-5-ylcarbonylthio, 3-Methyl-but-1-in-3-ylcarbonylthio, 3-Methylbut-1-in-4-ylcarbonylthio, n-Hex-1-in-1-ylcarbonylthio, n-Hex-1-in-3-ylcarbonylthio, n-Hex-1-in-4-ylcarbonylthio, n-Hex-1-in-5-ylcarbonylthio, n-Hex-1-in-6-ylcarbonylthio, n-Hex-2-in-1-ylcarbonylthio, n-Hex-2-in-4-ylcarbonylthio, n-Hex-2-in-5-ylcarbonylthio, n-Hex-2-in-6-ylcarbonylthio, n-Hex-3-in-1-ylcarbonylthio, n-Hex-3-in-2-ylcarbonylthio, 3-Methylpent-1-in-1-ylcarbonylthio, 3-Methyl-pent-1-in-3-ylcarbonylthio, 3-Methyl-pent-1-in-4-ylcarbonylthio, 3-Methylpent-1-in-5-ylcarbonylthio, 4-Methyl-pent-1-in-1-ylcarbonylthio, 4-Methyl-pent-2-in-4-ylcarbonylthio oder 4-Methyl-pent-2-in-5-ylcarbonylthio, insbesondere für Prop-2-in-1-ylcarbonylthio; - (C₂-C₆-Alkinyl)carbonylthio für: Ethinylcarbonylthio oder einen der unter (C₃-C₃-Alkinyl)carbonylthio genannten Reste, insbesondere für Ethinylcarbonylthio oder Prop-2-in-1-ylcarbonylthio;
- (C₁-C₆-Alkoxy)carbonyl-C₂-C₆-alkenyl für: durch (C₁-C₆-Alkoxy)carbonyl wie vorsthend genannt substituiertes C₂-C₆-Alkenyl, als beispielsweise für Methoxycarbonylprop-2-en-1-yl;
- C₃-C₆-Alkenyloxy-C₁-C₆-alkyl für: durch C₃-C₆-Alkenyloxy wie vorstehend genannt, vorzugsweise Allyloxy, 2-Methyl-prop-2-en-1-yloxy, But-1-en-3-yloxy, But-1-en-4-yloxy oder But-2-en-1-yloxy substituiertes C₁-C₆-Alkyl, also beispielsweise für Allyloxymethyl, 2-Allyloxyethyl oder But-1-en-4-yloxymethyl;
- C₃-C₆-Alkinyloxy-C₁-C₆-alkyl für: durch C₃-C₆-Alkinyloxy wie vorstehend genannt, vorzugsweise Propargyloxy, But-1-in-3-yloxy, But-1-in-4-yloxy oder But-2-in-1-yloxy, substituiertes C₁-C₆-Alkyl, also beispielsweise für Propargyloxymethyl oder 2-Propargyloxyethyl;
- C₃-C₇-Cycloalkyl für: Cyclopropyl, Cyclobutyl, Cyclopentyl, Cyclohexyl oder Cycloheptyl, insbesondere für Cyclopentyl oder Cyclohexyl;
- C₃-C₆-Cycloalkyl für: Cyclopropyl, Cyclobutyl, Cyclopentyl oder Cyclohexyl;
- C₃-C₈-Cycloalkyl für: Cyclopropyl, Cyclobutyl, Cyclopentyl, Cyclohexyl, Cycloheptyl oder Cyclooctyl, insbesondere für Cyclopentyl oder Cyclohexyl;
- C₃-C₈-Cycloalkyl-C₁-C₄-alkyl für: Cyclopropylmethyl, Cyclobutylmethyl, Cyclopentylmethyl, Cyclohexylmethyl, Cycloheptylmethyl, Cyclooctylmethyl, 1-(Cyclopropyl)ethyl, 1-(cyclobutyl)ethyl, 1-(Cyclopentyl)ethyl, 1-(Cyclohexyl)ethyl, 1-(Cycloheptyl)ethyl, 1-(cyclooctyl)ethyl, 2-(Cyclopropyl)ethyl, 2-(Cyclobutyl)ethyl, 2-(Cyclopentyl)ethyl, 2-(Cyclohexyl)ethyl, 2-(Cycloheptyl)ethyl, 2-(Cyclooctyl)ethyl, 3-(Cyclopropyl)propyl, 3-(Cyclobutyl)propyl, 3-(Cyclopentyl)propyl, 3-(Cyclohexyl)propyl, 3-(Cycloheptyl)propyl, 3-(Cyclooctyl)propyl, 4-(Cyclopropyl)butyl, 4-(Cyclobutyl)butyl, 4-(Cyclopentyl)butyl, 4-(Cyclohexyl)butyl, 4-(Cycloheptyl)butyl oder 4-(Cyclooctyl)butyl, insbesondere für Cyclopentylmethyl oder Cyclohexylmethyl;
- C₃-C₆-Cycloalkoxy für: Cyclopropyloxy, Cyclobutyloxy, Cyclopentyloxy oder Cyclohexyloxy;
- C₃-C₆-Cycloalkylthio für: Cyclopropylthio, Cyclobutylthio, Cyclopentylthio oder Cyclohexylthio;
- C₃-C₆-Cycloalkylcarbonyloxy für: Cyclopropylcarbonyloxy, Cyclobutylcarbonyloxy, Cyclopentylcarbonyloxy oder Cyclohexylcarbonyloxy;
- C₃-C₆-Cycloalkylsulfonyloxy für: Cyclopropylsulfonyloxy, Cyclobutylsulfonyloxy, Cyclopentylsulfonyloxy oder Cyclohexylsulfonyloxy;
- C₃-C₆-Cycloalkoxy-C₁-C₄-alkyl für: Cyclopropyloxymethyl, Cyclobutyloxymethyl, Cyclopentyloxymethyl, Cyclohexyloxymethyl, 1-(Cyclopropyloxy)ethyl, 1-(Cyclobutyloxy)ethyl, 1-(Cyclopentyloxy)ethyl, 1-(Cyclohexyloxy)ethyl, 2-(Cyclopropyloxy)ethyl, 2-(Cyclobutyloxy)ethyl, 2-(Cyclopentyloxy)ethyl, 2-(Cyclohexyloxy)ethyl, 3-(Cyclopropyloxy)propyl, 3-(Cyclobutyloxy)propyl, 3-(Cyclopentyloxy)propyl, 3-(Cyclohexyloxy)propyl, 4-(Cyclopropyloxy)butyl, 4-(Cyclobutyloxy)butyl, 4-(Cyclopentyloxy)butyl oder 4-(Cyclohexyloxy)butyl, insbesondere für Cyclopentyloxymethyl, Cyclohexyloxymethyl oder 2-(Cyclopentyloxy)ethyl;
- C₅-C₇-Cycloalkenyloxy für: Cyclopent-1-enyloxy, Cyclopent-2-enyloxy, Cylopent-3-enyloxy, Cyclohex-1-enyloxy, Cyclohex-2-enyloxy, Cyclohex-3-enyloxy, Cyclohept-1-enyloxy, Cyclohept-2-enyloxy, Cyclohept-3-enyloxy oder Cyclohept-4-enyloxy;
- C₁-C₃-Alkylen für: Methylen, 1,2-Ethylen oder 1,3-Propylen.

Unter 3- bis 7-gliedrigem Heterocyclyl sind sowohl gesättigte, partiell oder vollständig ungesättigte als auch aromatische Heterocyclen mit ein bis drei Heteroatomen, ausgewählt aus einer Gruppe bestehend aus
- ein bis drei Stickstoffatomen,
- einem oder zwei Sauerstoff- und
- einem oder zwei Schwefelatomen,
zu verstehen.

Beispiele für 3- bis 7-gliedrige Heterocyclen sind Oxiranyl, Aziridiny, Oxetanyl, Tetrahydrofuranyl, Tetrahydrothienyl, Pyrrolidinyl, Isoxazolidinyl, Isothiazolidinyl, Pyrazolidinyl, Oxazolidinyl, Thiazolidinyl, Imidazolidinyl, Dioxolanyl wie 1,3-Dioxolan-2-yl und 1,3-Dioxolan-4-yl, Dioxanyl wie 1,3-Dioxan-2-yl und 1,3-Dioxan-4-yl, Dithianyl wie 1,3-Dithian-2-yl, des weiteren 1,2,4-Oxadiazolidinyl, 1,3,4-Oxadiazolidinyl, 1,2,4-Thiadiazolidinyl, 1,3,4-Thiadiazolidinyl, 1,2,4-Triazolidinyl, 1,3,4-Triazolidinyl, 2,3-Dihydrofuryl, 2,5-Dihydrofuryl, 2,3-Dihydrothienyl, 2,5-Dihydrothienyl, 2,3-Pyrrolinyl, 2,5-Pyrrolinyl, 2,3-Isoxazolinyl, 3,4-Isoxazolinyl, 4,5-Isoxazolinyl, 2,3-Isothiazolinyl, 3,4-Isothiazolinyl, 4,5-Isothiazolinyl, 2,3-Dihydropyrazolyl, 3,4-Dihydropyrazolyl, 4,5-Dihydropyrazolyl, 2,3-Dihydrooxazolyl, 3,4-Dihydrooxazolyl, Piperidinyl, Tetrahydropyridazinyl, Tetrahydropyrimidinyl, Tetrahydropyrazinyl, 1,3,5-Tetrahydrotriazinyl und 1,2,4-Tetrahydrotriazinyl,
sowie die folgenden Heteroaromaten:
Furyl wie 2-Furyl und 3-Furyl, Thienyl wie 2-Thienyl und 3-Thienyl, Pyrrolyl wie 2-Pyrrolyl und 3-Pyrrolyl, Isoxazolyl wie 3-Isoxazolyl, 4-Isoxazolyl und 5-Isoxazolyl, Isothiazolyl wie 3-Isothiazolyl, 4-Isothiazolyl und 5-Isothiazolyl, Pyrazolyl wie 3-Pyrazolyl, 4-Pyrazolyl und 5-Pyrazolyl, Oxazolyl wie 2-Oxazolyl, 4-Oxazolyl und 5-Oxazolyl, Thiazolyl wie 2-Thiazolyl, 4-Thiazolyl und 5-Thiazolyl, Imidazolyl wie 2-Imidazolyl und 4-Imidazolyl, Oxadiazolyl wie 1,2,4-Oxadiazol-3-yl,
1,2,4-Oxadiazol-5-yl und 1,3,4-Oxadiazol-2-yl, Thiadiazolyl wie 1,2,4-Thiadiazol-3-yl, 1,2,4-Thiadiazol-5-yl und l,3,4-Thiadiazol-2-yl, Triazolyl wie 1,2,4-Triazol-1-yl, 1,2,4-Triazol-3-yl und 1,2,4-Triazol-4-yl, Pyridinyl wie 2-Pyridinyl, 3-Pyridinyl und 4-Pyridinyl, Pyridazinyl wie 3-Pyridazinyl und 4-Pyridazinyl, Pyrimidinyl wie 2-Pyrimidinyl, 4-Pyrimidinyl und 5-Pyrimidinyl, des weiteren 2-Pyrazinyl, 1,3,5-Triazin-2-yl und 1,2,4-Triazin-3-yl, insbesondere Pyridyl, Pyrimidyl, Furanyl und Thienyl.

Im Hinblick auf die Verwendung der erfindungsgemäßen Verbindungen der Formel I als Herbizide und/oder als defoliant/desikkant wirksame Verbindungen haben die Variablen vorzugsweise folgende Bedeutungen, und zwar jeweils für sich allein oder in Kombination:
- X: Sauerstoff;
- Alk: Trifluormethyl;
- R¹: Wasserstoff;
- R²: Wasserstoff;
- R³: Halogen, insbesondere Chlor;
- R⁴: Cyano, Halogen oder C₁-C₄-Alkoxy, insbesondere Chlor oder C₁-C₃-Alkoxy; besonders bevorzugt ist Chlor;
- R⁵: Wasserstoff, Halogen oder C₁-C₄-Alkoxy, insbesondere Wasserstoff, Chlor oder Methoxy;
besonders bevorzugt ist Wasserstoff;
- R⁶: C₁-C₆-Alkoxy, C₃-C₆-Alkenyloxy oder C₃-C₆-Alkinyloxy, wobei jeder dieser Reste einen der folgenden Substituenten tragen kann:
- die Phenylgruppe, die unsubstituiert sein oder ein bis drei Substituenten tragen kann, jeweils ausgewählt aus der Gruppe bestehend aus Halogen, Nitro, Cyano, C₁-C₆-Alkyl, C₁-C₆-Halogenalkyl, C₁-C₆-Alkoxy und (C₁-C₆-Alkoxy)carbonyl,
- einer Gruppe -CO-R⁹, -CO-OR⁹, -CO-SR⁹ oder -CO-N(R⁹)R¹⁰,
- der Gruppe -C(R²¹)=N-OR²⁰;
   -CY-R¹¹, -C(R¹¹)=C(R¹⁴)-CO-R¹⁵, -CO-OR¹⁹, -CO-N(R¹⁹)-OR²⁰, -C(R¹¹)=C(R¹⁴)-CO-N(R¹⁹)-OR²⁰, -C(R¹¹)=C(R¹⁴)-C(R²¹)=N-OR²⁰, -C(R²¹)=N-OR²⁰, -N(R²³)R²⁴ oder -CON(R²³)R²⁴;
insbesondere
- C₁-C₆-Alkoxy, das einen der folgenden Substituenten trägt:
   - Phenyl, substituiert durch Halogen oder C₁-C₃-Alkoxy, oder
   - eine Gruppe -CO-OR⁹, -CO-N(R⁹)R¹⁰ oder -C(R²¹)=N-OR²⁰;
- oder C₃-C₆-Alkenyloxy, C₃-C₆-Alkinyloxy, durch Halogen oder -CO-OR⁹ substituiertes C₃-C₆-Alkenyloxy, durch Halogen oder -CO-OR⁹ substituiertes C₃-C₆-Alkinyloxy, -CYR¹¹, -C(R¹¹)=C(R¹⁴)-CO-R¹⁵, -C(R¹¹)=C(R¹⁴) -CO-N(R¹⁹)-OR²⁰, -C(R¹¹)=C(R¹⁴)-C(R²¹)=N-OR²⁰ oder -N(R²³)R²⁴;
- R⁷: für Wasserstoff, C₁-C₆-Alkyl, C₃-C₆-Alkenyl, (C₁-C₆-Alkoxy)carbonyl-C₁-C₆-alkyl oder Phenyl, das unsubstituiert sein oder einen bis drei Reste tragen kann, jeweils ausgewählt aus der Gruppe bestehend aus Halogen, Nitro, C₁-C₆-Alkyl, C₁-C₆-Alkoxy und (C₁-C₆-Alkyl)carbonyl,
insbesondere C₁-C₃-Alkyl, C₃-C₄-Alkenyl oder (C₁-C₃-Alkoxy)carbonyl-C₁-C₃-alkyl;
- R⁸: für Wasserstoff, C₁-C₃-Alkyl, C₁-C₃-Alkoxy, (C₁-C₃-Alkoxy)carbonyl-C₁-C₃-alkoxy oder C₃-C₄-Alkenyloxy,
insbesondere Wasserstoff, C₁-C₃-Alkoxy, (C₁-C₃-Alkoxy)carbonyl-C₁-C₃-alkoxy oder C₃-C₄-Alkenyloxy;
- R⁹: für Wasserstoff, C₁-C₆-Alkyl, C₃-C₆-Alkenyl, (C₁-C₆-Alkoxy)carbonyl-C₁-C₆-alkyl oder Phenyl, das unsubstituiert sein oder einen bis drei Reste tragen kann, jeweils ausgewählt aus der Gruppe bestehend aus Halogen, Nitro, C₁-C₆-Alkyl, C₁-C₆-Alkoxy und (C₁-C₆-Alkyl)carbonyl, insbesondere C₁-C₃-Alkyl, C₃-C₄-Alkenyl oder (C₁-C₃-Alkoxy)carbonyl-C₁-C₃-alkyl;
- R¹⁰: für Wasserstoff, C₁-C₃-Alkyl, C₁-C₃-Alkoxy, (C₁-C₃-Alkoxy)carbonyl-C₁-C₃-alkoxy oder C₃-C₄-Alkenyloxy,
insbesondere C₁-C₃-Alkyl, C₁-C₃-Alkoxy, (C₁-C₃-Alkoxy)carbonyl-C₁-C₃-alkoxy oder C₃-C₄-Alkenyloxy;
- R¹¹: Wasserstoff oder Methyl, insbesondere Wasserstoff;
- R¹², R¹³: unabhängig voneinander C₁-C₆-Alkyl, insbesondere C₁-C₃-Alkyl oder
- R¹² und R¹³: zusammen eine gesättigte oder ungesättigte,
zwei- bis viergliedrige Kohlenstoffkette, die einen Oxo-substituenten tragen kann, wobei die Kohlenstoffkette noch ein oder zwei Reste tragen kann, jeweils ausgewählt aus der Gruppe bestehend aus C₁-C₆-Alkyl, C₂-C₆-Alkenyl, C₂-C₆-Alkenyloxy und (C₁-C₆-Alkoxy)carbonyl,
insbesondere eine gesättigte zwei- oder dreigliedrige Kohlenstoffkette, die unsubstituiert sein oder ein oder zwei Reste tragen kann, jeweils ausgewählt aus der Gruppe, bestehend aus C₁-C₃-Alkyl, C₂-C₄-Alkenyl und (C₁-C₃-Alkoxy)carbonyl;
- R¹⁴: Wasserstoff, Halogen oder C₁-C₆-Alkyl,
insbesondere Wasserstoff, Chlor oder Methyl;
besonders bevorzugt ist Chlor;
- R¹⁵: O-R²², C₁-C₄-Alkyl, C₃-C₄-Alkenyl, C₃-C₄-Alkinyl, C₁-C₆-Alkyliminooxy oder -N(R²³)R²⁴,
insbesondere O-R²² oder -N(R²³)R²⁴;
- R¹⁶: Wasserstoff, Halogen oder C₁-C₆-Alkyl,
insbesondere Wasserstoff, Chlor oder Methyl;
- R¹⁷: Wasserstoff, Halogen oder C₁-C₆-Alkyl,
insbesondere Wasserstoff, Chlor oder Methyl;
- R¹⁸: Wasserstoff, C₁-C₄-Alkyl oder (C₁-C₄-Alkoxy)carbonyl, insbesondere Wasserstoff, Methyl oder (C₁-C₃-Alkoxy)carbonyl;
- R¹⁹: Wasserstoff, C₁-C₃-Alkyl oder C₃-C₄-Alkenyl, wobei die beiden letzten Gruppen jeweils einen der folgenden Reste tragen können: Chlor, (C₁-C₆-Alkyl)carbonyl, (C₁-C₆-Alkoxy)carbonyl oder (C₃-C₆-Alkenyloxy)carbonyl;
oder (C₁-C₆-Alkylamino)carbonyl oder Di-(C₁-C₆-alkyl)aminocarbonyl;
insbesondere Wasserstoff, C₁-C₃-Alkyl oder C₃-C₄-Alkenyl, wobei die beiden letzten Gruppen jeweils einen der folgenden Reste tragen können: Chlor, (C₁-C₃-Alkoxy)carbonyl oder (C₃-C₄-Alkenyloxy)carbonyl;
oder (C₁-C₂-Alkyl)aminocarbonyl oder Dimethylaminocarbonyl;
- R²⁰: C₁-C₆-Alkyl, C₃-C₆-Alkenyl, C₃-C₆-Alkinyl, (C₁-C₆-Alkyl)carbonyl-C₁-C₆-alkyl, (C₁-C₆-Alkoxy)carbonyl-C₁-C₆-alkyl oder (C₁-C₆-Alkoxy)carbonyl-C₂-C₆-alkenyl,
insbesondere C₁-C₄-Alkyl, C₃-C₄-Alkenyl oder (C₁-C₃-Alkoxy)carbonyl-C₁-C₃-alkyl;
- R²¹: Wasserstoff, Methyl, C₁-C₆-Alkoxy, C₃-C₆-Alkenyloxy oder (C₁-C₆-Alkyl)carbonyloxy, wobei die letztgenannten 4 Reste einen der folgenden Substituenten tragen können:
(C₁-C₆-Alkyl)carbonyl, (C₁-C₆-Alkoxy)carbonyl, (C₁-C₆-Alkylamino)carbonyl oder Di-(C₁-C₆-alkyl)aminocarbonyl; oder C₃-C₆-Alkinyloxy, (C₂-C₆-Alkinyl)carbonyloxy, Phenoxy oder Benzoyloxy, wobei die Phenylringe der beiden letztgenannten Reste unsubstituiert sein oder ihrerseits ein bis drei Substituenten tragen können, jeweils ausgewählt aus der Gruppe bestehend aus Halogen, C₁-C₆-Alkyl, C₁-C₆-Halogenalkyl und C₁-C₆-Alkoxy;
insbesondere C₃-C₄-Alkinyloxy, C₁-C₃-Alkoxy oder C₃-C₆-Alkenyloxy, wobei die beiden letzten Reste jeweils einen der folgenden Substituenten tragen können: (C₁-C₃-Alkoxy)carbonyl, (C₁-C₃-Alkyl)aminocarbonyl oder Dimethylaminocarbonyl;
- R²²: eine der bevorzugten Bedeutungen von R¹⁹,
insbesondere Wasserstoff, C₁-C₃-Alkyl oder C₃-C₄-Alkenyl, wobei die beiden letzten Gruppen jeweils einen der folgenden Reste tragen können: Chlor, (C₁-C₃-Alkoxy)carbonyl oder (C₃-C₄-Alkenyloxy)carbonyl;
oder (C₁-C₂-Alkyl)aminocarbonyl oder Dimethylaminocarbonyl;
- R²³, R²⁴: unabhängig voneinander für
Wasserstoff, C₁-C₄-Alkyl, C₃-C₄-Alkenyl, C₃-C₆-Cycloalkyl, (C₁-C₆-Alkyl)carbonyl, (C₁-C₆-Alkoxy)carbonyl, (C₁-C₆-Alkoxy)carbonyl-C₁-C₆-alkyl, C₁-C₆-Alkylsulfonyl, (C₁-C₆-Alkoxy)carbonyl-C₁-C₆-alkylsulfonyl oder für Phenylsulfonyl, das unsubstituiert sein oder seinerseits noch einen bis drei Substituenten tragen kann, jeweils ausgewählt aus der Gruppe bestehend aus Nitro, Halogen, C₁-C₆-Alkyl, C₃-C₆-Alkenyl, C₁-C₆-Alkoxy und (C₁-C₆-Alkoxy)carbonyl,
insbesondere Wasserstoff, C₁-C₃-Alkyl, (C₁-C₃-Alkyl)carbonyl, C₁-C₃-Alkylsulfonyl oder Phenylsulfonyl, dessen Phenylring einen Halogen-, C₁-C₃-Alkyl- oder C₁-C₃-Alkoxy-Substituenten trägt;
besonders bevorzugt sind Wasserstoff, Methyl, Ethyl, Methylsulfonyl und Ethylsulfonyl;
- Y: -N(R²⁷)-, wobei
- R²⁷: für C₁-C₆-Alkyl, C₃-C₆-Alkenyl, C₃-C₆-Alkinyl, C₁-C₆-Alkoxy, C₃-C₆-Alkenyloxy, C₃-C₆-Alkinyloxy, (C₁-C₆-Alkyl)carbonyloxy, (C₁-C₆-Alkyl)carbamoyloxy, (C₁-C₆-Alkyl) carbonyl-C₁-C₆-alkoxy, (C₁-C₆-Alkoxy)carbonyl-C₁-C₆-alkyl, (C₁-C₆-Alkoxy) carbonyl-C₁-C₆-alkoxy oder Phenyl, das seinerseits noch einen bis drei Substituenten tragen kann, jeweils ausgewählt aus der Gruppe bestehend aus Nitro, Halogen, C₁-C₆-Alkyl, C₁-C₆-Alkoxy und (C₁-C₆-Alkoxy)carbonyl,
insbesondere C₁-C₆-Alkoxy, C₃-C₆-Alkenyloxy oder (C₁-C₆-Alkoxy)carbonyl-C₁-C₆-alkoxy, steht;
besonders bevorzugt sind C₁-C₃-Alkoxy, C₃-C₄-Alkenyloxy und (C₁-C₃-Alkoxy)carbonyl-C₁-C₃-alkoxy.

Bevorzugt sind ferner
- diejenigen Verbindungen I, bei denen R⁶ C₁-C₄-Alkoxy, das eine Gruppe -CO-OR⁹, -CO-N(R⁹)R¹⁰ oder -C(R²¹)=N-OR²⁰ trägt, bedeutet, und
- diejenigen Verbindungen I, bei denen R⁶ -CY-R¹¹ bedeutet, und
- diejenigen Verbindungen I, bei denen R⁶ -C (R¹¹)=C(R¹⁴)-CO-R¹⁵ bedeutet, und
- diejenigen Verbindungen I, bei denen R⁶ -C(R¹¹)=C(R¹⁴) -CO-N(R¹⁹)-OR²⁰ bedeutet, und
- diejenigen Verbindungen I, bei denen R⁶ -C(R¹¹)=C(R¹⁴)-C(R²¹)=N-OR²⁰ bedeutet.

Außerdem sind ganz allgemein diejenigen Verbindungen I bevorzugt, bei denen R⁶ in Position β steht.

Ganz besonders bevorzugt sind die in den folgenden Tabellen 1 und 2 aufgeführten Verbindungen Ia (≙ I mit X = Sauerstoff, Alk = Trifluormethyl, R¹, R² = Wasserstoff, R³, R⁴ = Chlor):

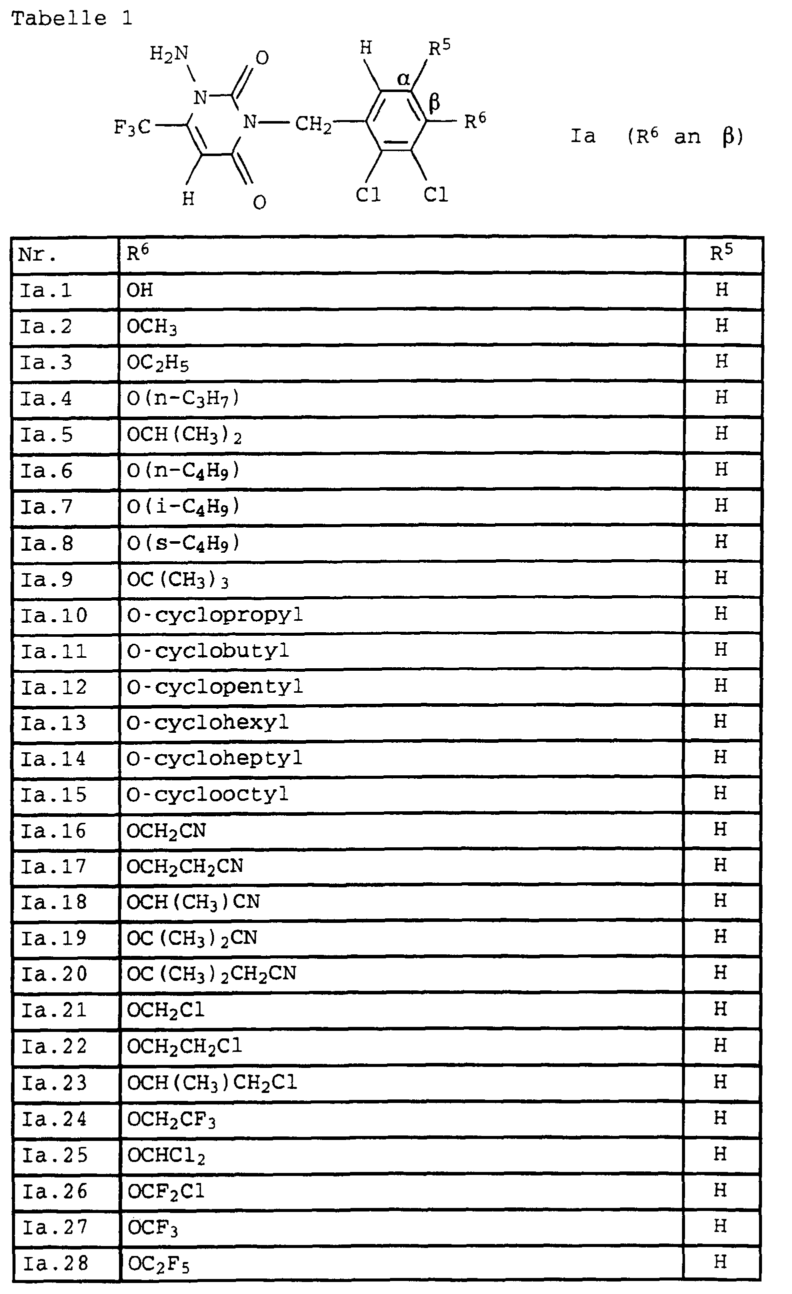

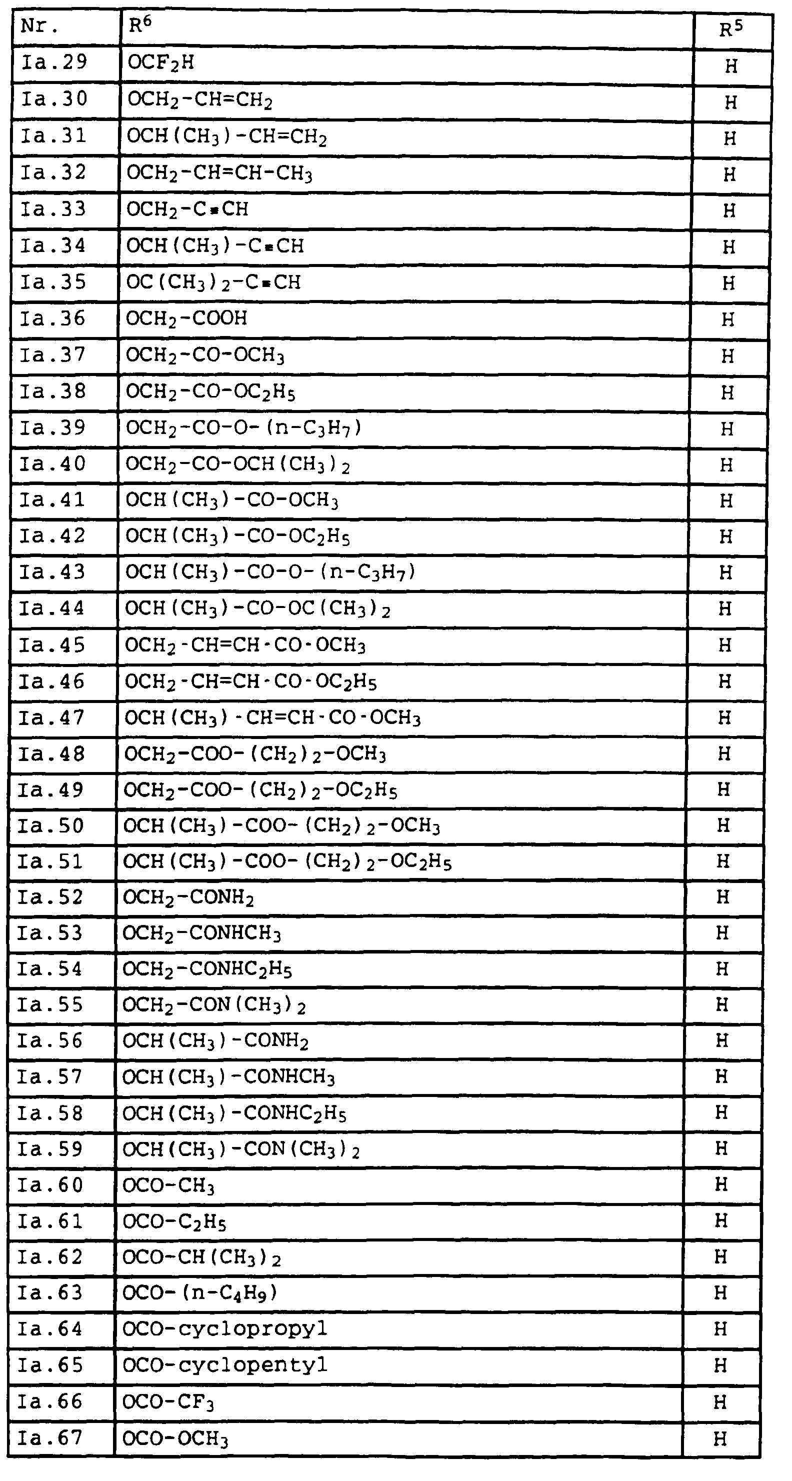

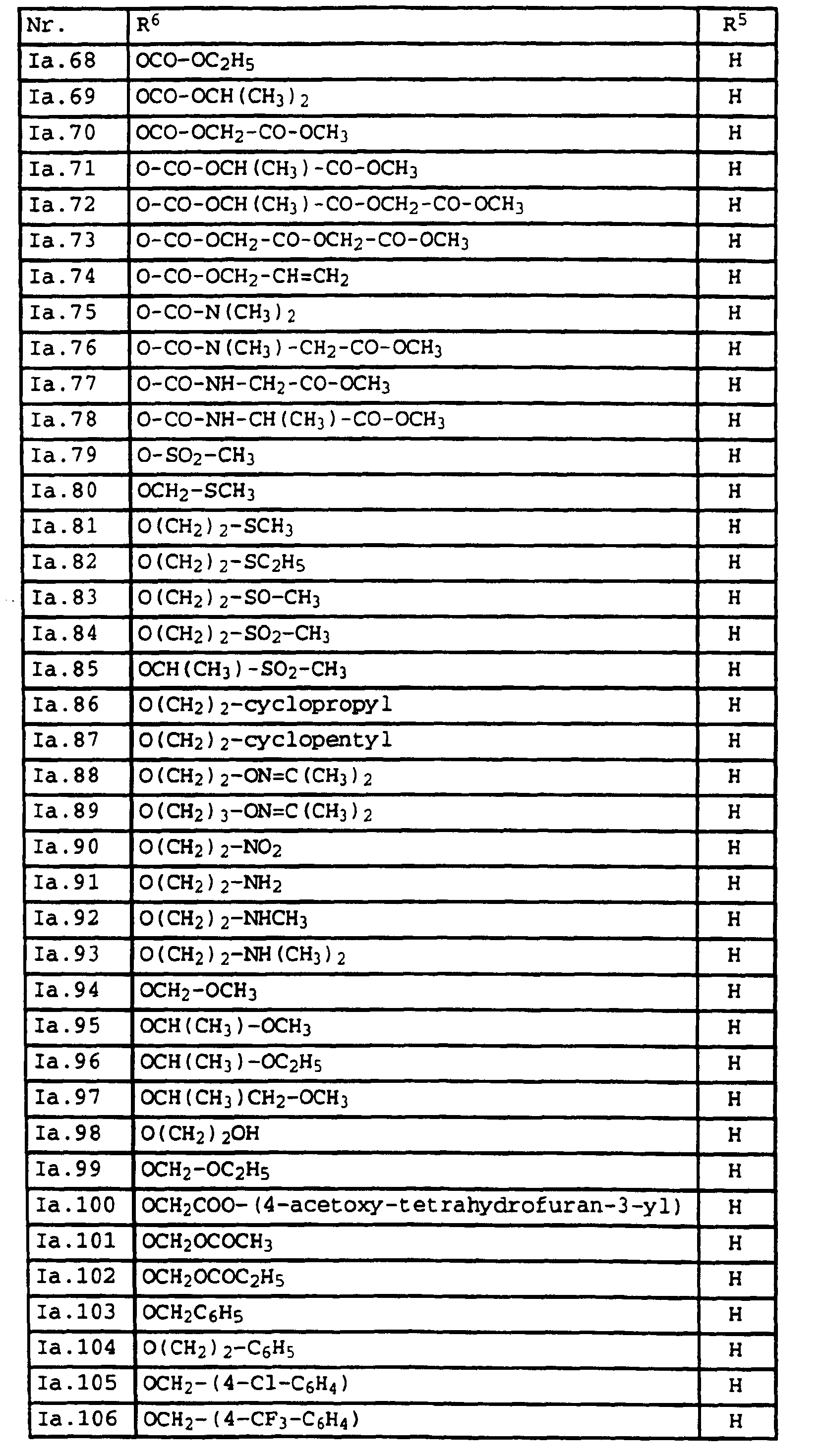

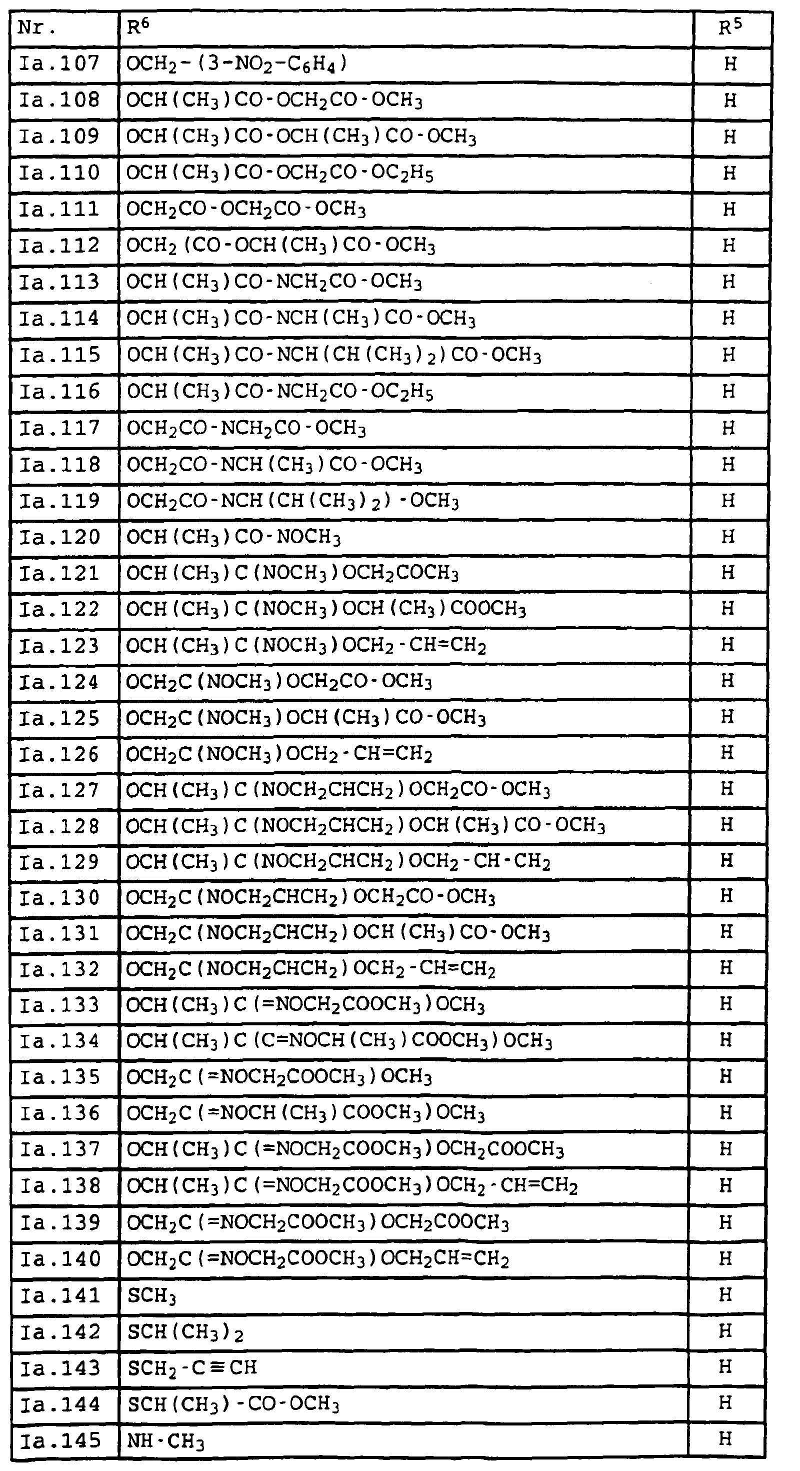

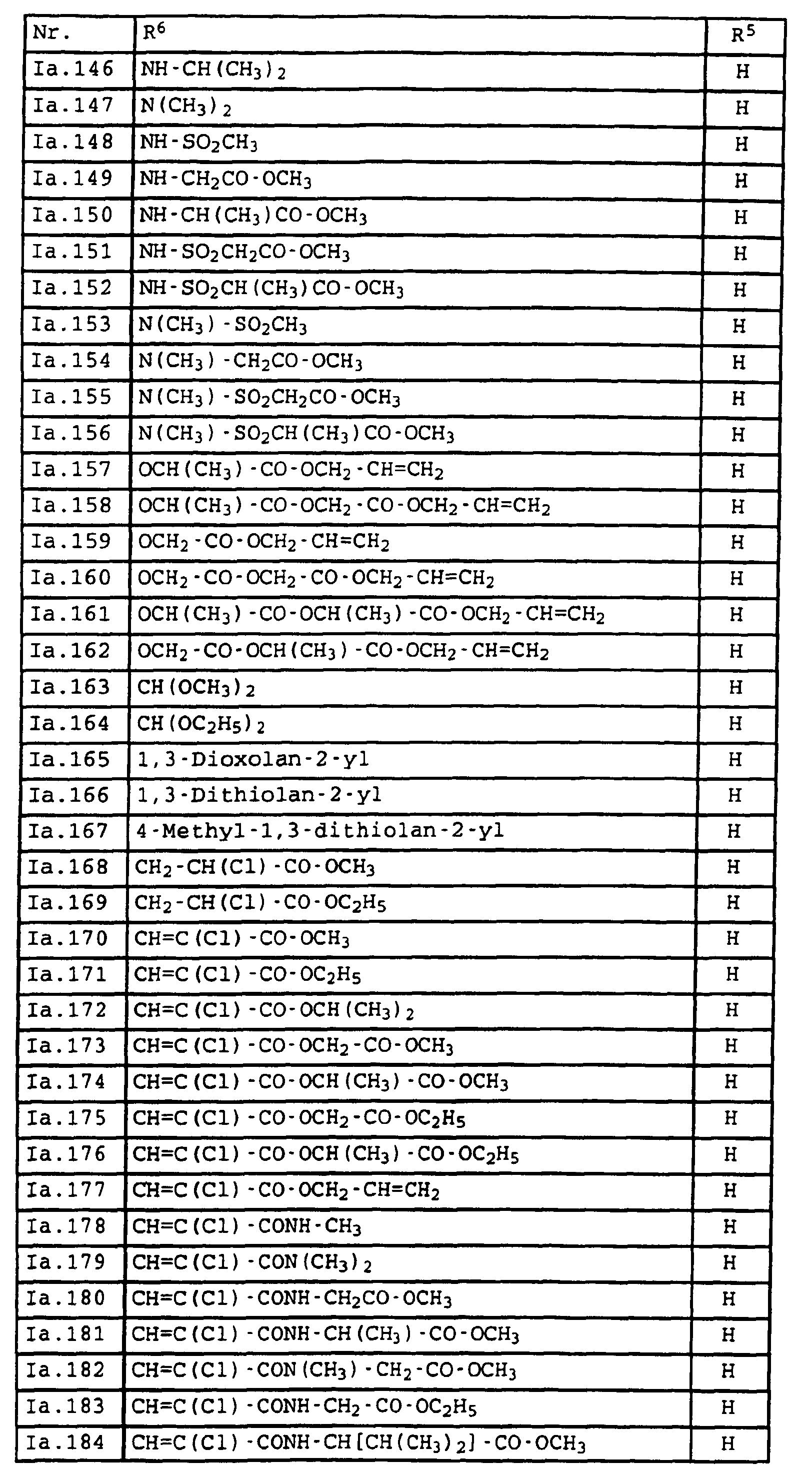

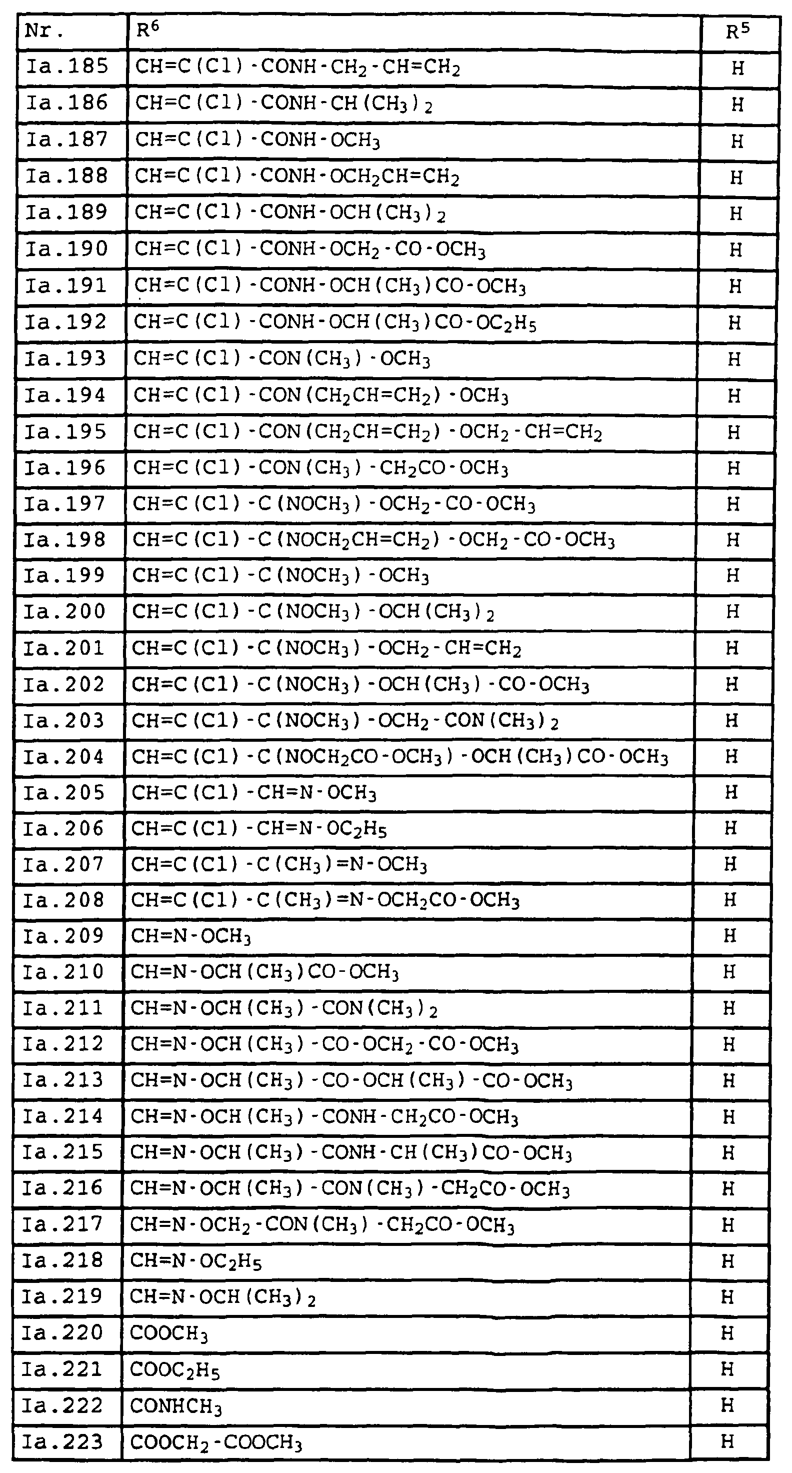

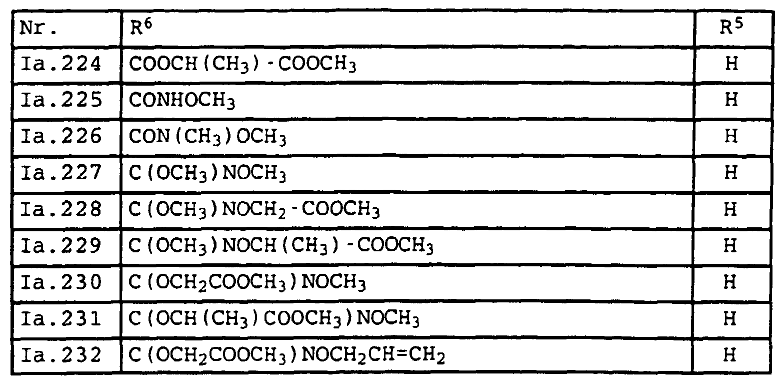

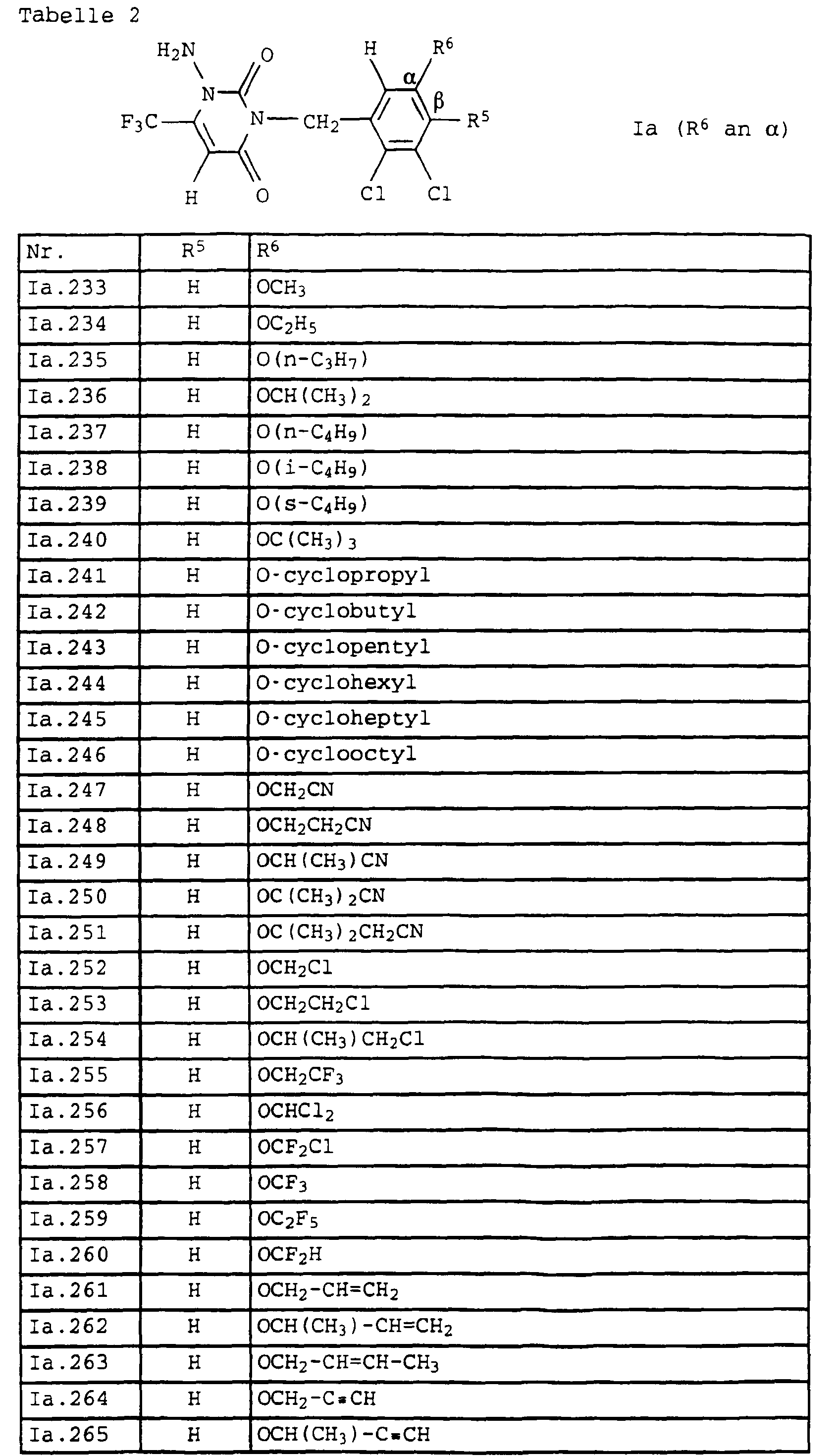

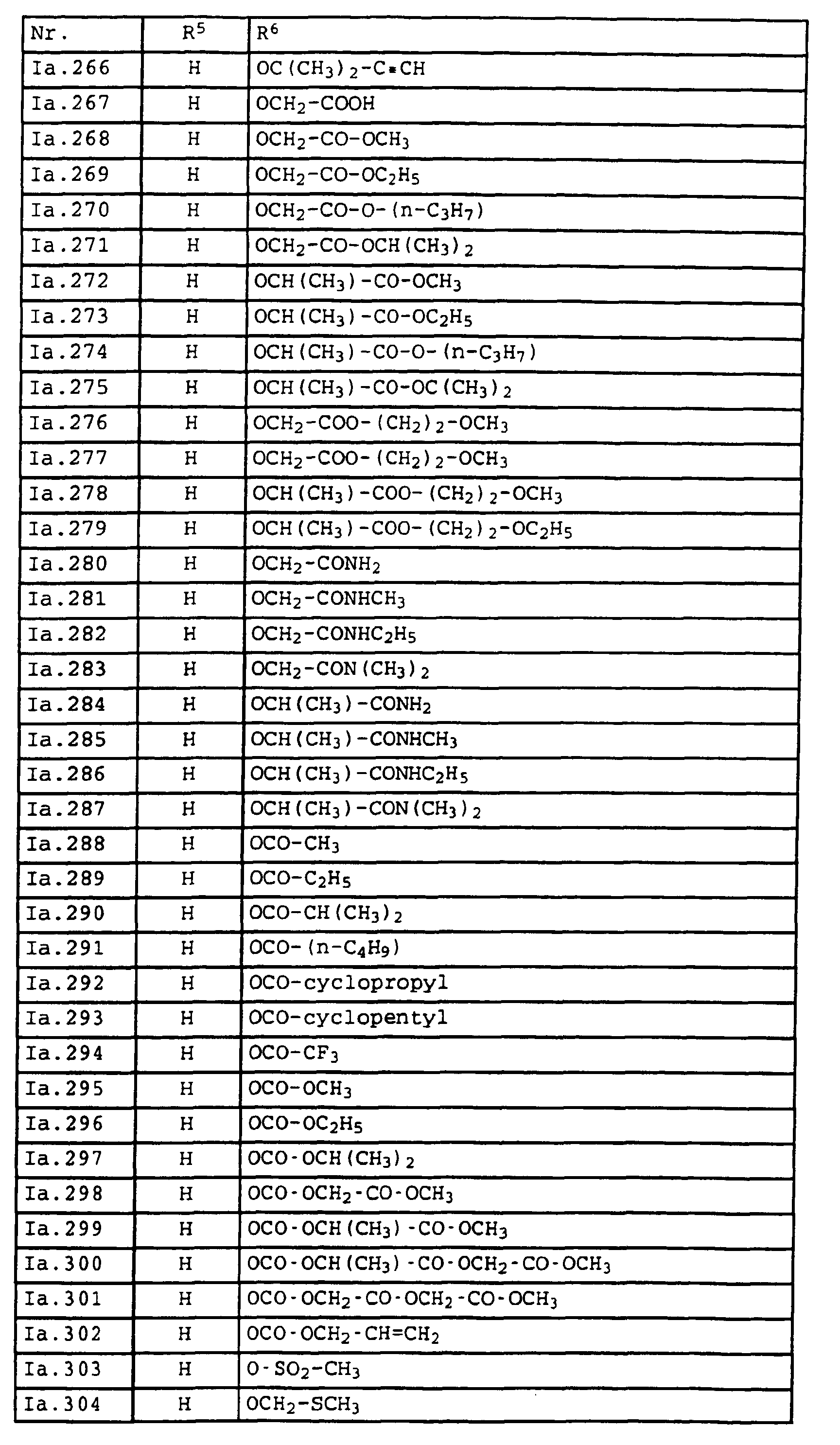

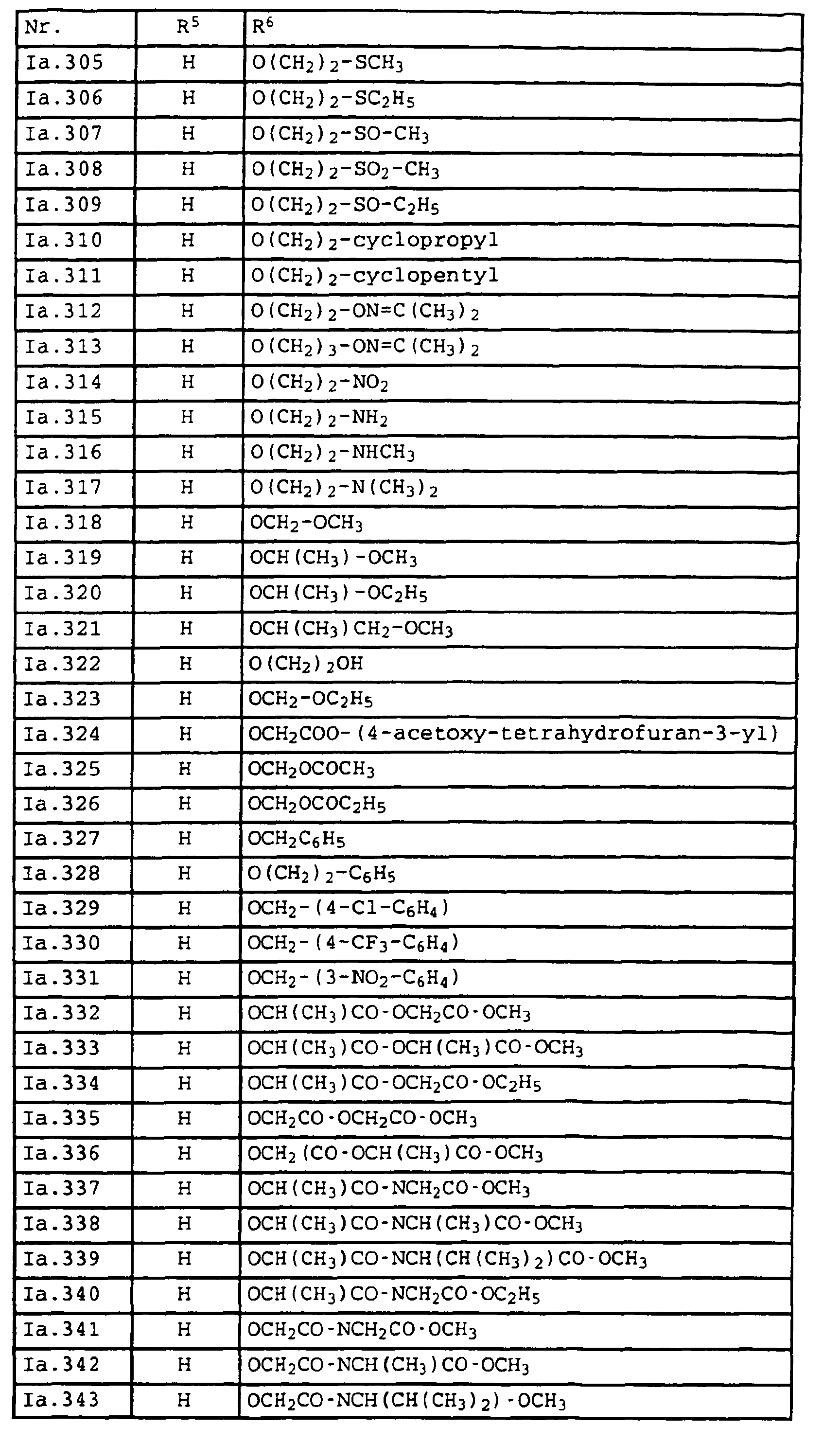

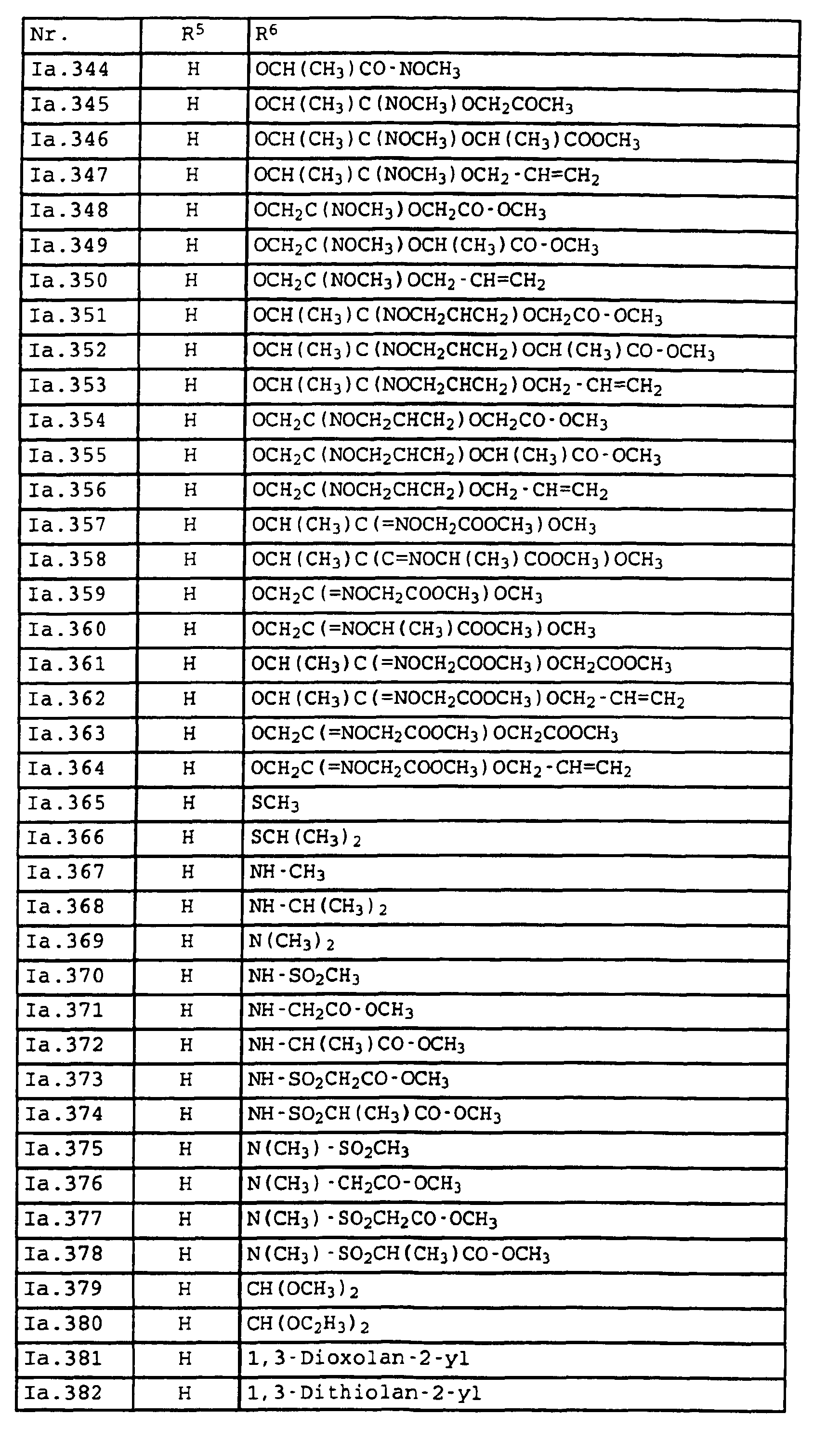

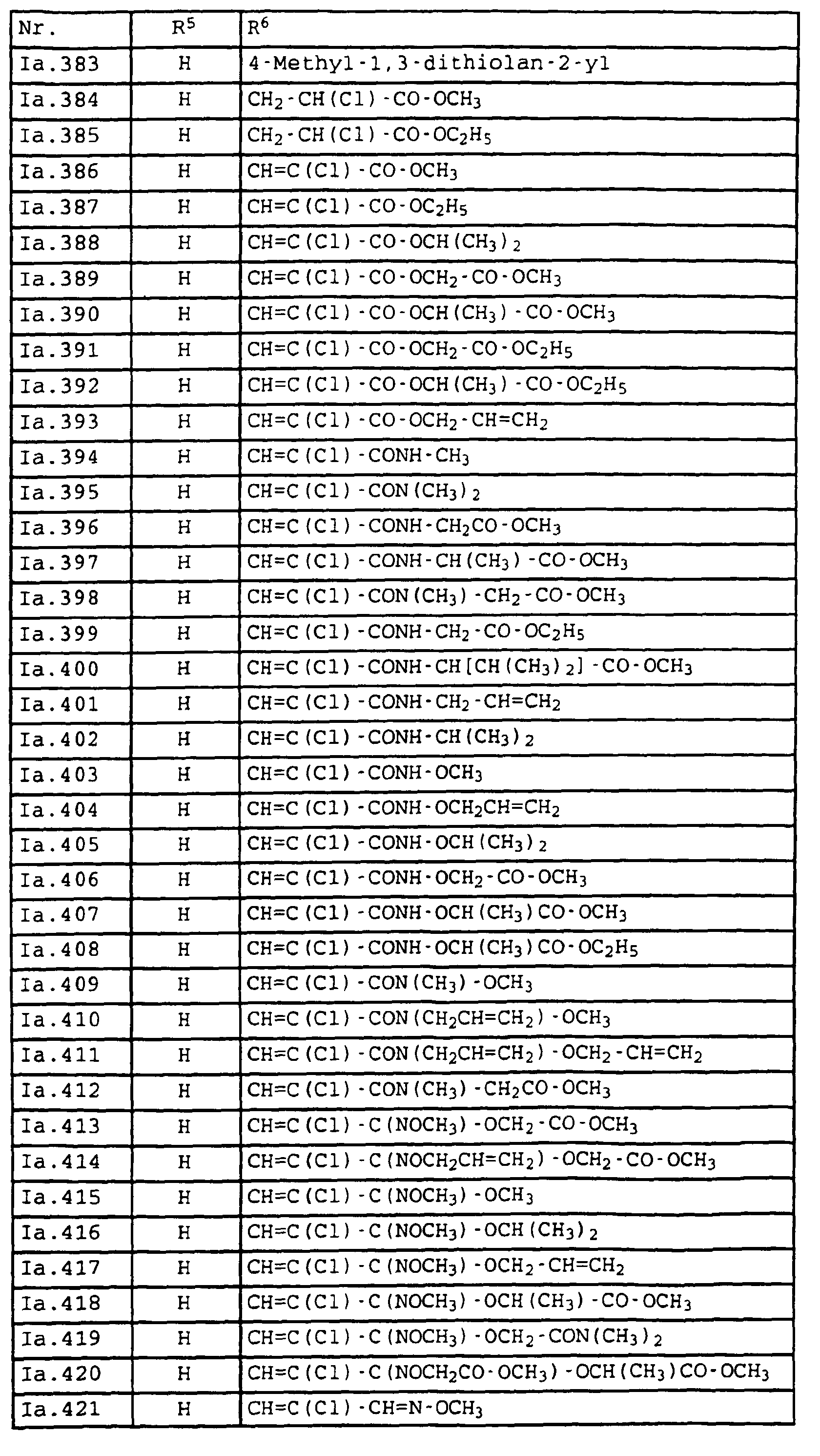

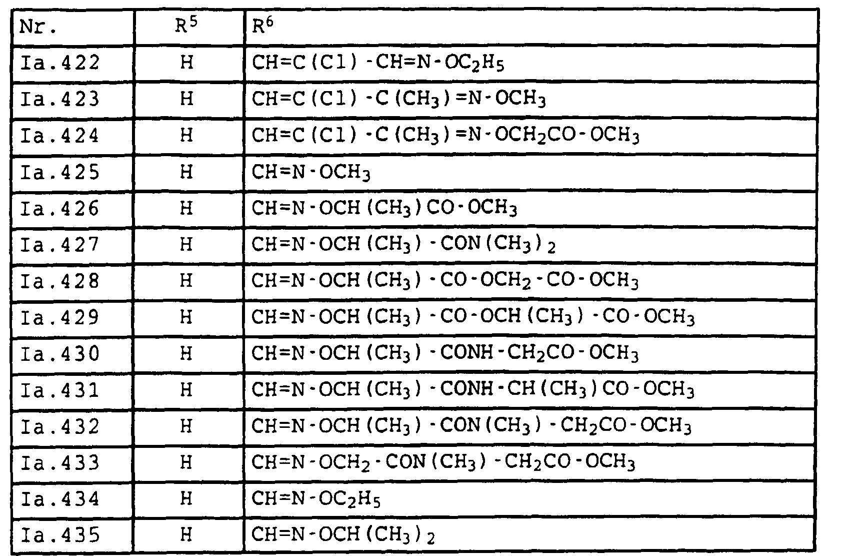

Des weiteren sind die folgenden 1-Amino-3-benzyluracile der Formeln Ib bis Ig besonders bevorzugt, insbesondere
- die Verbindungen Ib.1 - Ib.232 und Ib.233 - Ib.435, die sich von den entsprechenden Verbindungen Ia.1 - Ia.232 und Ia.233 - Ia.435 lediglich dadurch unterscheiden, daß R⁴ Methoxy bedeutet: 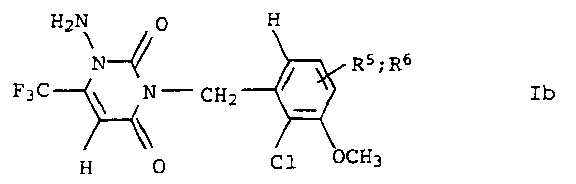
- die Verbindungen Ic.1 - Ic.232 und Ic.233 - Ic.435, die sich von den entsprechenden Verbindungen Ia.1 - Ia.232 und Ia.233 - Ia.435 lediglich dadurch unterscheiden, daß R⁴ Methoxy und R⁶ Chlor bedeuten: 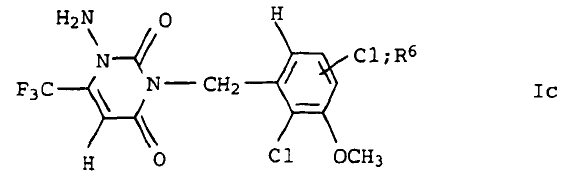
- die Verbindungen Id.1 - Id.232 und Id.233 - Id.435, die sich von den entsprechenden Verbindungen Ia.1 - Ia.232 und Ia.233 - Ia.435 lediglich dadurch unterscheiden, daß R⁶ Methoxy bedeutet: 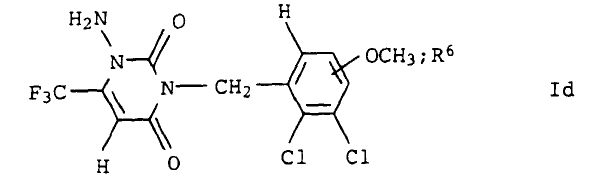
- die Verbindungen Ie.1 - Ie. 232 und Ie.233 - Ie.435, die sich von den entsprechenden Verbindungen Ia.1 - Ia.232 und Ia.233 - Ia.435 lediglich dadurch unterscheiden, daß R⁴ und R⁶ Methoxy bedeuten: 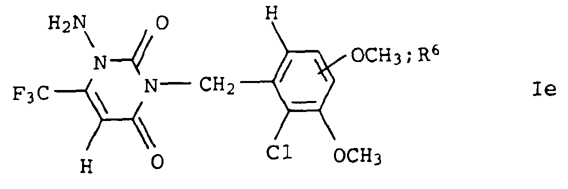
- die Verbindungen If.1 - If.232 und If.233 - If.435, die sich von den entsprechenden Verbindungen Ia.1 - Ia.232 und Ia.233 - Ia.435 lediglich dadurch unterscheiden, daß X Schwefel bedeutet: 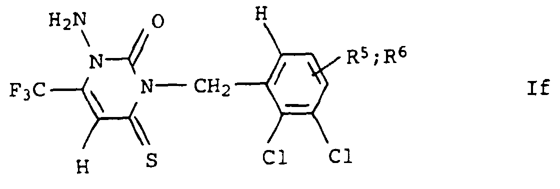
- die Verbindungen Ig.1 - Ig.232 und Ig.233 - Ig.435, die sich von den entsprechenden Verbindungen Ia.1 - Ia.232 und Ia.233 - Ia.435 lediglich dadurch unterscheiden, daß R⁵ Chlor bedeutet:

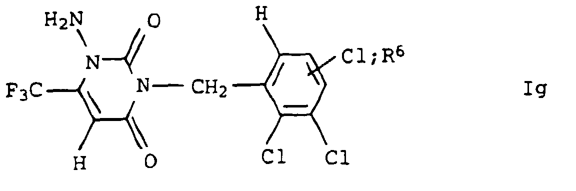

Die 1-Amino-3-benzyluracile der Formel I sind auf verschiedene Weise erhältlich, insbesondere nach einem der folgenden Verfahren:
Verfahren A₎
   Cyclisierung eines Enaminesters der Formel III oder eines Enamincarboxylates der Formel IV in Gegenwart einer Base: 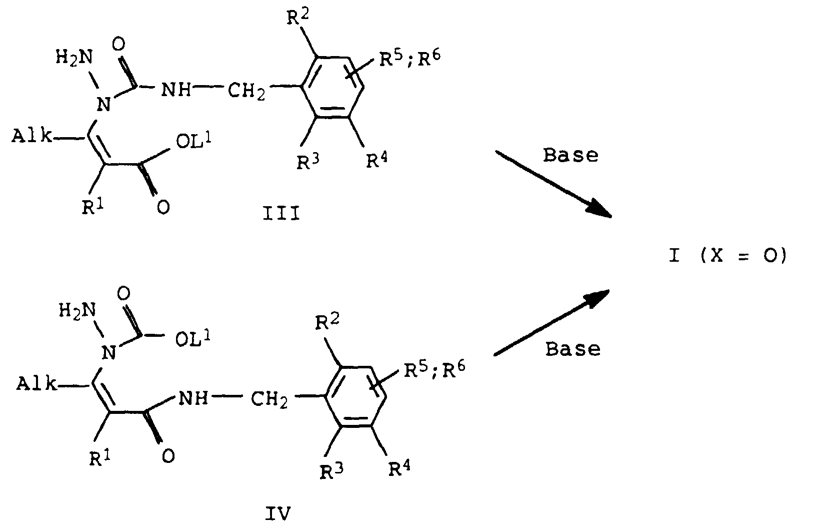
   L¹ bedeutet niedermolekulares Alkyl, vorzugsweise C₁-C₆-Alkyl, oder Phenyl.
   In der Regel cyclisiert man in einem inerten organischen Lösungs- oder Verdünnungsmittel, das aprotisch ist, beispielsweise in einem aliphatischen oder cyclischen Ether wie 1,2-Dimethoxyethan, Tetrahydrofuran und Dioxan, in einem Aromaten wie Benzol und Toluol oder in einem polaren Solvens wie Dimethylformamid und Dimethylsulfoxid. Auch Mischungen aus polarem Solvens und einem Kohlenwasserstoff wie n-Hexan sind geeignet. Je nach Ausgangsverbindung kann sich auch Wasser als Verdünnungsmittel eignen.
   Als Basen kommen vorzugsweise Alkalimetallalkoholate, insbesondere die Natriumalkoholate, Alkalimetallhydroxide, insbesondere Natrium- und Kaliumhydroxid, Alkalimetallcarbonate, insbesondere Natrium- und Kaliumcarbonat, und Metallhydride, insbesondere Natriumhydrid, in Betracht. Bei der Verwendung von Natriumhydrid als Base hat es sich als vorteilhaft erwiesen, in einem aliphatischen oder cyclischen Äther, in Dimethylformamid oder in Dimethylsulfoxid zu arbeiten.
   Im allgemeinen ist die 0,5- bis zweifache molare Menge an Base, bezogen auf die Menge an III oder IV, für das Gelingen der Reaktion ausreichend.
   In der Regel liegt die Reaktionstemperatur zwischen (-78)°C und der Siedetemperatur des jeweiligen Reaktionsgemisches, insbesondere bei (-60) bis 60°C.
   Üblicherweise wird das Verfahrensprodukt als Metallsalz erhalten, wobei das Metall dem Kation der verwendeten Base entspricht. Das Salz kann auf an sich bekannte Weise isoliert und gereinigt oder gewünschtenfalls mittels Säure in die freie Verbindung III übergeführt werden.
Verfahren B₎
   Schwefelung eines 1-Amino-3-benzyluracils der Formel I mit X = Sauerstoff: 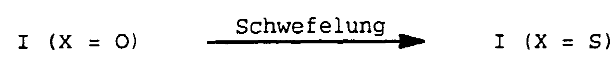
   Die Schwefelung erfolgt in der Regel in einem inerten Lösungs- oder Verdünnungsmittel, beispielsweise in einem aromatischen Kohlenwasserstoff wie Toluol und den Xylolen, in einem Ether wie Diethylether, 1,2-Dimethoxyethan und Tetrahydrofuran, oder in einem organischen Amin wie Pyridin.
   Als Schwefelungsreagenz eignen sich besonders gut Phosphor(V)-sulfid und 2,4-Bis(4-methoxyphenyl)-1,3,2,4-dithiadiphosphetan-2,4-dithion ("Lawesson-Reagenz").
   Üblicherweise ist die 1- bis 5-fache molare Menge, bezogen auf die zu schwefelnde Ausgangsverbindung, für eine weitgehend vollständige Umsetzung ausreichend.
   Die Reaktionstemperatur liegt normalerweise bei 20 bis 200°C, vorzugsweise bei 40°C bis zur Siedetemperatur des Reaktionsgemisches.
Verfahren C₎
   Umsetzung eines 1-H-3-Phenyluracils der Formel V in Gegenwart einer Base mit einem elektrophilen Aminierungsreagenz: 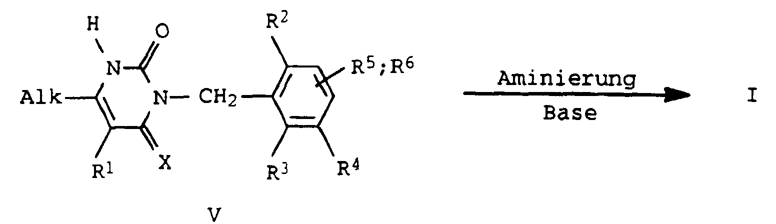
   Als Aminierungsreagenz hat sich bisher 2,4-Dinitrophenoxyamin besonders bewährt, jedoch kann z.B. auch Hydroxylamin-O-sulfonsäure (HOSA) verwendet werden, die aus der Literatur bereits als Aminierungsreagenz bekannt ist (vgl. z.B. E. Hofer et al., Synthesis 1983, 466; W. Friedrichsen et al., Heterocycles 20 (1983) 1271; H. Hart et al., Tetrahedron Lett. 25 (1984) 2073; B. Vercek et al., Monatsh. Chem. 114 (1983) 789; G. Sosnousky et al., Z. Naturforsch. 38 (1983) 884; R.S. Atkinson et al., J. Chem. Soc. Perkin Trans. 1987, 2787).
   Die Aminierung kann auf an sich bekannte Weise durchgeführt werden (siehe z.B. T. Sheradsky, Tetrahedron Lett. 1968, 1909, M.P. Wentland et al., J. Med. Chem. 27 (1984) 1103 und insbesondere EP-A 240 194, EP-A 476 697 und EP-A 517 181, wo die Aminierung von Uracilen gelehrt wird).
   Als Basen eignen sich beispielsweise Alkalimetallalkoholate wie Natriummethylat und Kalium-tert.-butanolat, Alkalimetallcarbonate wie Natrium- und Kaliumcarbonat, oder Alkalimetallhydride wie Natriumhydrid und Kaliumhydrid.
   Normalerweise führt man die Umsetzung in einem polaren Lösungsmittel durch, z.B. in Dimethylformamid, N-Methylpyrrolidon, in einem Sulfoxid wie Dimethylsulfoxid oder in einem Carbonsäureester wie Ethylacetat, das sich bisher als besonders geeignet erwiesen hat.
   Die Menge an Base und Aminierungsmittel liegt vorzugsweise jeweils bei der 0,5- bis zweifachen molaren Menge, bezogen auf die Menge an III.
   Im allgemeinen arbeitet man bei (-10)°C bis zur Siedetemperatur des Reaktionsgemisches, insbesondere bei 10 bis 70°C.
   Die 1-H-3-Phenyluracile V sind ihrerseits z.B. durch Cyclisierung von Enaminestern VI oder Enamincarboxylaten VII erhältlich: 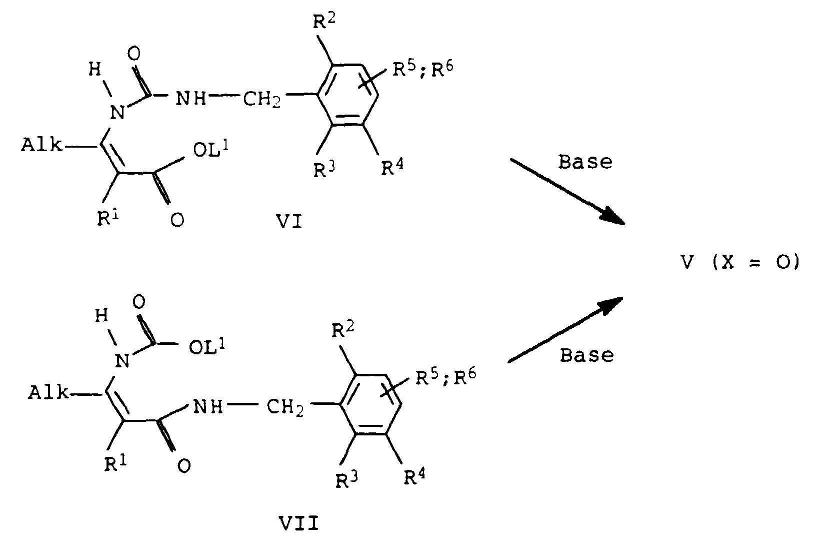
   Bezüglich der Lösungs-/Verdünnungsmittel, Basen, Mengenverhältnisse und der Reaktionstemperatur gelten die unter A₎ gemachten Angaben.
   Gewünschtenfalls kann das Verfahrensprodukt V mit X = Sauerstoff gemäß Verfahren B₎ in V mit X = Schwefel übergeführt werden.
Verfahren D₎
   Umsetzung eines 1-Amino-6-halogenalkyluracils VIII in Gegenwart einer Base mit einem Benzylhalogenid IX: 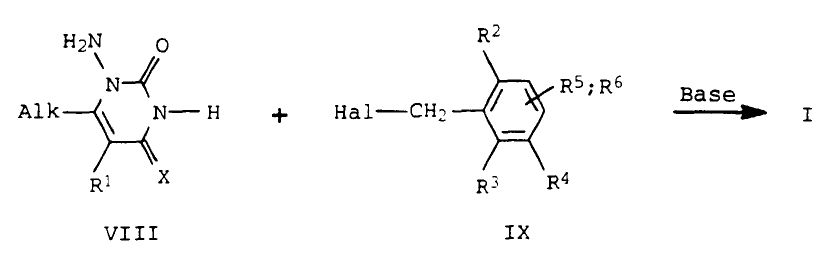
   Hal steht für Halogen, besonders bevorzugt Brom.
   Geeignete Basen sind beispielsweise Alkalimetallalkoholate wie Natriummethylat, Alkalimetallcarbonate wie Natrium- und Kaliumcarbonat oder Alkalimetallhydride wie Natrium- und Kaliumhydrid.
   Es besteht auch die Möglichkeit, VIII mittels Base zunächst in das Alkalimetallsalz zu überführen und dieses dann anschließend mit IX umzusetzen.
   Üblicherweise arbeitet man in einem inerten polaren Lösungs- oder Verdünnungsmittel, z.B. in Dimethylformamid, N-Methylpyrrolidon, in einem Sulfoxid wie Dimethylsulfoxid, in einem Carbonsäureester wie Ethylacetat oder in einem Keton wie Aceton.
   In allgemeinen liegt die Reaktionstemperatur bei 0°C bis zur Siedetemperatur des Reaktionsgemisches.
Verfahren E₎
   Etherspaltung eines 1-Methyl-3-benzyluracils der Formel I, bei dem R⁵ und/oder R⁶ unsubstituiertes oder substituiertes Alkoxy, Cycloalkoxy, Alkenyloxy, Alkinyloxy oder Benzyloxy bedeuten: 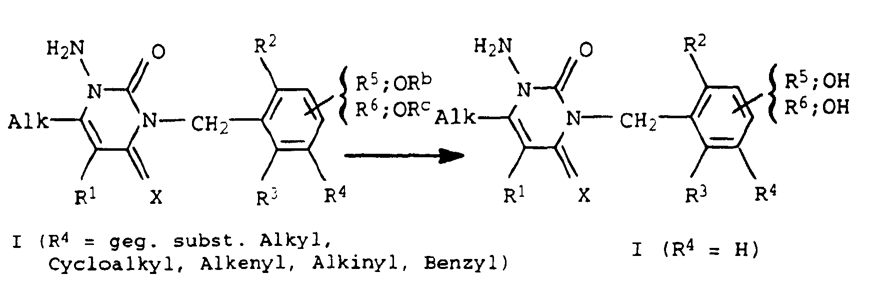
   OR^{b} und OR^{c} stehen für die unter R⁵ bzw. R⁶ definierten Alkoholreste.
   Die Etherspaltung erfolgt üblicherweise mittels Säure, z.B. mittels Bromwasserstoff, Iodwasserstoff oder Pyridiniumhydrochlorid, mittels einer Lewis-Säure wie Aluminiumtrichlorid, -tribromid, -triiodid, Bortrichlorid, -tribromid, -trifluorid und Eisentrichlorid, oder mittels Trimethylsilyliodid. Daneben sind aber auch Lithiumsalze wie Lithiumchlorid oder Mischungen aus einem anorganischen Iodid und Trimethylsilylchlorid brauchbar, um die Etherbindung zu spalten. In Einzelfällen, z.B. wenn R⁶ Benzyloxy bedeutet, kann die Bindung auch unter Hydrierbedingungen mittels Wasserstoff in Gegenwart eines Hydrierkatalysators wie Platin und Palladium auf Aktivkohle gespalten werden.
   Allylether (R⁵ oder R⁶ = Allyloxy) können ferner auf hierfür an sich bekannte Weise in die entsprechenden Phenole übergeführt werden, z.B. durch Isomerisierung in Gegenwart eines Übergangsmetallkatalysators zum Enolether und Spaltung von Letzterem, vorzugsweise unter leicht sauren Bedingungen (vgl. z.B. T. Greene u. P.G.M. Wutz in "Protective Groups in Organic Synthesis", John Wiley & Sons, 2. Auflage New York 1991, S. 42ff.).
   Üblicherweise arbeitet man in einem inerten Lösungs- oder Verdünnungsmittel, z. B. in einem aliphatischen, cyclischen oder aromatischen Kohlenwasserstoff wie n-Pentan, Petrolether, Cyclohexan, Benzol, Toluol oder Xylol, einem aliphatischen oder cyclischen Ether wie Diethylether, tert.-Butylmethylether, Dimethoxyethan und Tetrahydrofuran, einem aliphatischen oder aromatischen Halogenkohlenwasserstoff wie Dichlormethan, Chloroform, Chlorbenzol, 1,2-Dichlorethan und den Dichlorbenzolen, einem Alkohol wie Methanol, Ethanol und tert.-Butanol, einem Amid wie Dimethylformamid und N-Methylpyrrolidon, einem Amin wie Ammoniak, oder in einem Gemisch derartiger Solventien.
   Eine Reaktionsführung ohne Lösungsmittel kann auch vorteilhaft sein.
   Bezüglich besonders bevorzugter Ausführungsformen sei auf die Ausführungen in Houben-Weyl, "Methoden der Organischen Chemie', Georg Thieme Verlag, 4. Auflage, Stuttgart 1979, Bd. 6/1a/1, S. 309ff und in R. C. Larock, "Comprehensive Organic Transformations", VCH-Publishers, Weinheim 1989, S. 501ff sowie auf die dort zitierte Literatur verwiesen.
Verfahren F₎
   Alkylierung eines 1-Methyl-3-benzyluracils der Formel I, bei dem R⁵ und/oder R⁶ Hydroxy bedeutet, in Gegenwart einer Base: 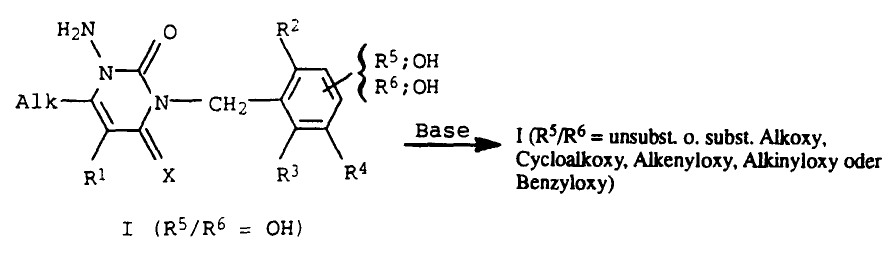
   Die Alkylierung kann beispielsweise mit dem Halogenid, vorzugsweise dem Chlorid oder Bromid, dem Sulfat, Sulfonat, vorzugsweise dem Methansulfonat (Mesylat), Benzolsulfonat, p-Toluolsulfonat (Tosylat), p-Brombenzolsulfonat (Brosylat), dem Trifluormethansulfonat (Triflat) oder der Diazoverbindung eines unsubstituierten oder substituierten Alkans, Cycloalkans, Halogenalkans, Alkens oder Alkins vorgenommen werden.
   Normalerweise arbeitet man in einem inerten organischen Lösungsmittel, wobei besonders aprotische Lösungsmittel, z.B. aliphatische und cyclische Ether wie 1,2-Dimethoxyethan, Tetrahydrofuran und Dioxan, aliphatische Ketone wie Aceton, Amide wie Dimethylformamid, Sulfoxide wie Dimethylsulfoxid, Harnstoffen wie Tetramethylharnstoff und 1,3-Dimethyltetrahydro-2(1H)-pyrimidinon, Carbonsäureester wie Essigsäureethylester, oder halogenierte aliphatische oder aromatische Kohlenwasserstoffe wie Dichlormethan und Chlorbenzol, in Betracht kommen.
   Als Base eignen sich sowohl anorganische Basen, z.B. Alkalimetallcarbonate wie Natriumcarbonat und Kaliumcarbonat, Alkalimetallhydrogencarbonate wie Natrium- und Kaliumhydrogencarbonat, oder Alkalimetallhydride wie Natriumhydrid und Kaliumhydrid, als auch organische Basen, z.B. Amine wie Triethylamin, Pyridin und N,N-Diethylanilin, oder Alkalimetallalkoholate wie Natriummethanolat, -ethanolat und Kalium-tert.-butanolat.
   Die Menge an Base und Alkylierungsmittel liegt vorzugsweise bei der 0,5- bis 2-fachen molaren Menge, bezogen auf die Menge an I mit R⁵/R⁶ = Hydroxy.
   Im allgemeinen empfiehlt sich eine Reaktionstemperatur von 0°C bis zur Siedetemperatur des Reaktionsgemisches, insbesondere von 0 bis 60°C.
   Mit dieser Methode lassen sich auch Verbindungen I mit R⁴ = C₁-C₄-Alkoxy aus den entsprechenden Hydroxy-Vorprodukten herstellen
Verfahren G₎
   Acylierung eines 1-Amino-3-benzyluracils der Formel I, bei dem R⁵ und/oder R⁶ Hydroxy bedeutet, mit einem geeigneten Acylierungsmittel: 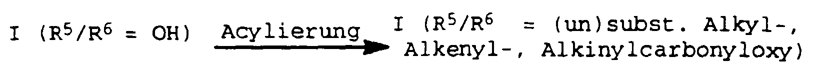
   Geeignete Acylierungsmittel sind z.B. die Säurehalogenide, insbesondere die Säurechloride, die Anhydride oder Isocyanate von Alkan-, Cycloalkan-, Alken-, Alkin-, Phenyl- oder Phenylalkancarbonsäuren. Es kommen aber auch die freien Säuren oder deren Anhydride in Betracht, sofern dann in Gegenwart eines Kondensationsmittel wie Carbonyldiimidazol und Dicyclohexylcarbondiimid gearbeitet wird.
   In der Regel arbeitet man in einem inerten organischen Lösungs- oder Verdünnungsmittel, das vorzugsweise aprotisch ist, z.B. in einem aliphatischen oder cyclischen Ether wie 1,2-Dimethoxyethan, Tetrahydrofuran und Dioxan, einem aliphatischen Keton wie Aceton, einem Amid wie Dimethylformamid, einem Harnstoff wie Tetramethylharnstoff und 1,3-Dimethyltetrahydro2(1H)-pyrimidinon, einem Carbonsäureester wie Essigsäureethylester, oder einem aliphatischen oder aromatischen Halogenkohlenwasserstoff wie Dichlormethan und Chlorbenzol.
   Bezüglich geeigneter Basen, der Mengenverhältnisse und der Reaktkionstemperatur sei auf die Ausführungen unter Verfahren F₎ verwiesen.
Verfahren H₎
   Substitution von Halogenid durch Cyanid: 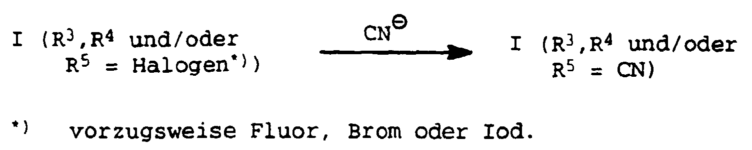
   Geeignete Cyanide sind insbesondere Metallcyanide, z.B. die Alkalimetallcyanide wie Lithium-, Natrium- und Kaliumcyanid, die Erdalkalimetallcyanide wie Magnesiumcyanid, oder auch Übergangsmetallcyanide wie Kupfercyanid.
   Üblicherweise arbeitet man in einem Ether wie Tetrahydrofuran, Dioxan und 1,2-Dimethoxyethan, oder in einem aprotischen, polaren Lösungsmittels, z.B. einem Alkylnitril wie Aceto-, Propio- und Butyronitril, einem Alkylharnstoff wie N,N,N',N'-Tetramethylharnstoff, einem offenkettigen oder cyclischen Dialkylamid wie Dimethylformamid, N-Methyl-2-pyrrolidon, 1,2-Dimethyl-imidazolidin-2-on und 1,2-Dimethyl-3,4,5,6-tetrahydro-2(1H)-pyrimidinon, einem Dialkylsulfoxid wie Dimethylsulfoxid, oder in Hexamethylphosphorsäuretriamid.
   Nach den bisherigen Erkenntnissen kann sich die Gegenwart eines Katalysators vorteilhaft auf den Reaktionsverlauf auswirken. Brauchbare Katalysatoren sind z.B. Übergangsmetalle und deren Komplexe oder Salze, z.B. Verbindungen des Kupfers wie Kupfer-(I)-chlorid, -iodid, -cyanid, oder des Nickels wie Nickel-bis-triphenylphospin-dibromid.
   Auf analoge Weise können auch die Ausgangsverbindungen V mit R³,R⁴ und/oder R⁵ = Halogen in die entsprechenden Verbindungen V mit R³/R⁴/R⁵ = CN übergeführt werden. Dabei ist es jedoch empfehlenswert, in Gegenwart einer Base zu arbeiten, wobei insbesondere schwach nucleophile Basen in Betracht kommen, und zwar sowohl anorganische Basen, z.B. Alkalimetallcarbonate wie Natrium- und Kaliumcarbonat, Alkalimetallhydrogencarbonate wie Natrium- und Kaliumhydrogencarbonat, oder Alkalimetallhydride wie Natriumhydrid und Kaliumhydrid, als auch organische Basen, z.B. Amine wie Triethylamin, Pyridin und N,N-Diethylanilin.
   Die Mengenverhältnisse sind normalerweise nicht kritisch. Im allgemeinen ist die etwa ein- bis 10-fache Menge an Cyanid und Base, bezogen auf die Menge an Ausgangsverbindung I oder V, ausreichend.
   Die Reaktionstemperatur liegt üblicherweise bei 50 bis 250°C; zur Erhöhung der Selektivität der Reaktion kann es aber auch empfehlenswert sein, bei tieferen Temperaturen, insbesondere bei etwa 20°C, zu arbeiten.
   Bezüglich verschiedener Ausführungsformen dieser Umsetzung sei auf Houben-Weyl, 'Methoden der Organischen Chemie', Georg Thieme Verlag, 4. Auflage, Stuttgart 1985, Bd. E5, S. 1444ff. sowie auf die dort angegebene Literatur verwiesen.
Verfahren K₎
   Halogenierung eines 1-Amino-3-benzyluracils der Formel I, bei dem R¹ Wasserstoff bedeutet: 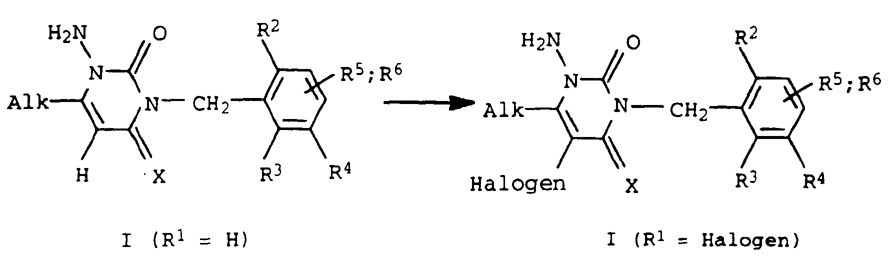
   Die Halogenierung erfolgt in der Regel in einem inerten organischen Lösungs- oder Verdünnungsmittel. Für die Chlorierung und Bromierung kommen beispielsweise aliphatische Carbonsäuren wie Essigsäure, oder chlorierte aliphatische Kohlenwasserstoffe wie Methylenchlorid, Chloroform und Tetrachlorkohlenstoff, in Betracht. Für die Iodierung sind niedrig siedende aliphatischen Carbonsäuren wie Essigsäure besonders bevorzugt.
   Für die Chlorierung und Bromierung eignen sich besonders elementares Chlor bzw. Brom, oder Sulfurylchlorid bzw. Sulfurylbromid, bei einer Reaktionstemperatur von vorzugsweise 0 bis 60°C, insbesondere 10 bis 30°C.
   Gewünschtenfalls kann die Chlorierung und Bromierung in Gegenwart eines säurebindenden Mittels erfolgen, wobei Natriumacetat und tertiäre Amine wie Triethylamin, Dimethylanilin und Pyridin besonders bevorzugt sind.
   Als Iodierungsmittel ist elementares Iod besonders bevorzugt, wobei in diesem Fall die Reaktionstemperatur bei ca. 0 bis 110°C, vorzugsweise bei 10 bis 30°C, liegt.
   Besonders vorteilhaft verläuft die Iodierung in Gegenwart einer Mineralsäure wie rauchende Salpetersäure.
   Die Menge an Halogenierungsmittel ist nicht kritisch; normalerweise verwendet man äquimolare Mengen an Halogenierungsmittel oder einen Überschuß bis etwa 200 mol-%, bezogen auf die Ausgangsverbindung (I mit R¹ = H).
   Überschüssiges Iod kann beispielsweise nach der Reaktion mittels gesättigter wäßriger Natriumhydrogensulfitlösung entfernt werden.
   Nach dieser Methode können auch die entsprechenden Vorprodukte V mit R¹ = H halogeniert werden.
Verfahren L₎
   Substitution der Nitrogruppe an R⁵ durch unsubstituiertes oder substituiertes Alkoxy, Cycloalkoxy, Alkenyloxy oder Alkinyloxy: 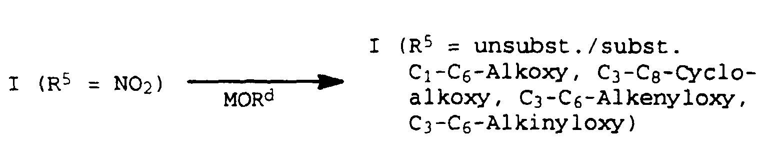 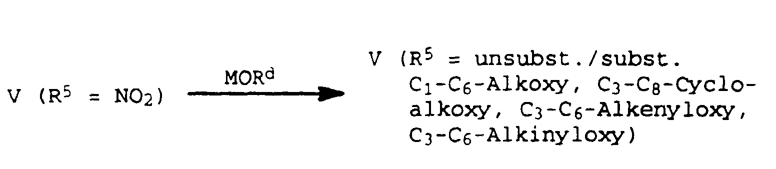
   Die Substitution der Nitrogruppe erfolgt üblicherweise durch Umsetzung von I oder V (R⁵ = NO₂) mit einem Alkoholat MOR^{d}, wobei M für ein Metallatom, vorzugsweise Lithium, Natrium oder Kalium steht und R^{d} unsubstituiertes oder substituiertes Alkoxy, Cycloalkoxy, Alkenyloxy oder Alkinyloxy bedeutet (vgl. z.B. Org. Synth. Coll. Vol. III, 293).
   Besonders gut geht diese Reaktion, wenn man von Verbindungen V mit R⁵ = NO₂ und R³, R⁴, R⁶ = elektronenziehende Substituenten wie Cyano ausgeht.
   In der Regel arbeitet man entweder in dem Alkohol HOR^{d}, dessen Alkoholat verwendet wird, oder in einem inerten organischen Lösungs- oder Verdünnungsmittel, z.B. in einem aromatischen Kohlenwasserstoff wie Toluol und die Xylole, in einem Ether wie Diethylether, Tetrahydrofuran und 1,2-Dimethoxyethan, oder in einem halogenierten Kohlenwasserstoff wie Dichlormethan und Chlorbenzol.
   Die Reaktionstemperatur liegt im allgemeinen bei 0 bis 150°C, vorzugsweise bei Raumtemperatur (etwa 20°C) bis zur Siedetemperatur des jeweiligen Reaktionsgemisches.
   Die Menge an Alkoholat ist normalerweise nicht kritisch; bevorzugt sind etwa 1 bis 3 Äquivalente Alkoholat pro Mol I (R⁵ = NO₂) oder V (R⁵ = NO₂).
Verfahren M₎
   Acetalisierung einer Verbindung I oder V, wobei R⁶ eine Gruppe -CO-R¹¹ bedeutet: 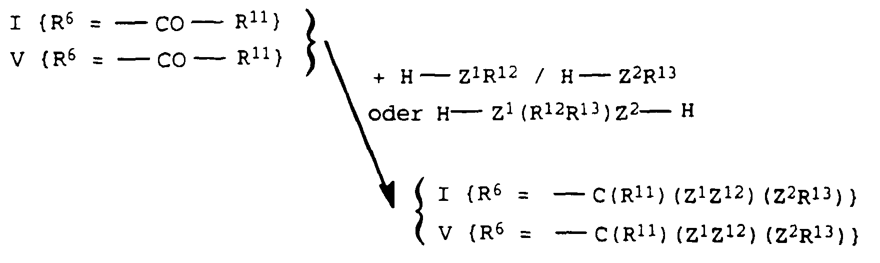
   Die Acetalisierung erfolgt allgemein in einem inerten aprotischen organischen Lösungsmittel, beispielsweise in einem aliphatischen oder cyclischen Ether wie Diethylether, 1,2-Dimethoxyethan, Tetrahydrofuran und Dioxan, in einem aromatischen Kohlenwasserstoff wie Benzol, Toluol, o-, m-, p-Xylol und Mesitylen, oder in einem chlorierten Kohlenwasserstoff wie Methylenchlorid, Chloroform und Chlorbenzol, sofern sie nicht ohne Lösungsmittel in einem Überschuß an H-Z¹R¹², H-Z²R¹³ oder H-Z¹(R¹²R¹³)Z²-H durchgeführt wird.
   Entstehendes Reaktionswasser kann wie üblich aus dem Reaktionsgemisch entfernt werden, z.B. mittels Wasserabscheidung.
   Vorzugsweise führt man die Acetalisierung in Gegenwart einer organischen Säure wie p-Toluolsulfonsäure und/oder einer Lewis-Säure wie Zinntetrachlorid, Zinn-II-chlorid, Eisen-III-chlorid, Tellurtetrachlorid und Bortrifluoretherat oder eines geeigneten Katalysators wie Montmorillonit-K 10 durch, wobei die Menge an Säure normalerweise zwischen 0,5 und 100 mol-%, bezogen auf die Menge an zu acetalisierendem Edukt, liegt.
   Die Mengenverhältnisse sind nicht kritisch. Für eine vollständige Umsetzung werden alle Reaktionspartner in etwa stöchiometrischen Mengen eingesetzt, bevorzugt verwendet man jedoch einen Überschuß an H-Z¹R¹² und H-Z²R¹³ bzw. H-Z¹(R¹²R¹³)Z²-H.
   Verwendet man die Edukte H-Z¹R¹² und H-Z²R¹³ bzw. H-Z¹(R¹²R¹³)Z²-H gleichzeitig als Verdünnungsmittel, so liegen sie in einem größeren Überschuß vor.
   Im allgemeinen arbeitet man bei Temperaturen von (-78) bis 180°C, vorzugsweise (-40) bis 150°C.
   Werden Produktgemische erhalten, z.B. wenn R¹² und R¹³ keinen gemeinsamen Rest bilden und Z¹R¹² und Z²R¹³ nicht gleich sind, so können diese gewünschtenfalls nach an sich bekannten Methoden wie Kristallisation und Chromatographie gereinigt und getrennt werden.
   Insbesondere Verbindungen der Formel I, wobei R⁶ für eine Gruppe -C(R¹¹) (Z¹R¹²) (Z²R¹³) steht, in der R¹² und R¹³ keinen gemeinsamen Rest bilden und Z¹R¹² und R²R¹³ nicht gleich sind, können auch nach anderen literaturbekannten Methoden dargestellt werden (vgl. z.B. Tetrahedron Lett. 32, 467-470 (1991) sowie die dort zitierte Literatur).
   In einigen Fällen kann es auch vorteilhaft sein, die Acetalisierung über den Umweg einer Acetalisierung zum Dialkylacetal, vorzugsweise Dimethylacetal, und anschließende Umacetalisierung in Gegenwart eines geeigneten Katalysators durchzuführen. Die für die Umacetalisierung verwendeten Lösungsmittel, Katalysatoren und sonstigen Reaktionsbedingungen entsprechen den bereits oben für die Acetalisierung aufgeführten.
Verfahren N₎
   Acetalspaltung einer Verbindung I oder V, wobei R⁶ eine Gruppe -C(R¹¹) (Z¹R¹²) (Z²R¹³) bedeutet: 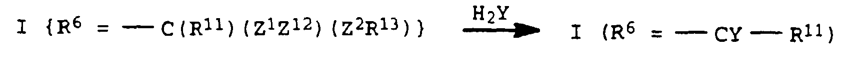 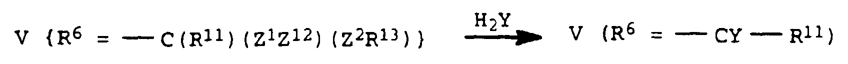
   Die Acetalspaltung kann ohne Säurezusatz, in Gegenwart einer Säure, z.B. einer Mineralsäure wie Salzsäure und Schwefelsäure, einer organischen Carbonsäure wie Ameisensäure, Essigsäure, Oxalsäure und Trifluoressigsäure, in Gegenwart eines sauren Ionenaustauschers wie Amberlite® (Warenzeichen der Fa. "Aldrich") IR120 oder IRC84, oder in Gegenwart eines Übergangsmetallsalzes wie Quecksilber-(II)-oxid, Kupfer-(I)-oxid und Eisen-(III)-chlorid, durchgeführt werden.
   Als Lösungs- oder Verdünnungsmittel eignen sich beispielsweise Aromaten wie Benzol, Toluol und o-, m-, p-Xylol, aliphatische oder cyclische Ether wie 1,2-Dimethoxyethan, Diethylether, Tetrahydrofuran und Dioxan, Alkohole wie Methanol, Ethanol und Isopropanol, polare organische Lösungsmittel wie Dimethylformamid, Dimethylsulfoxid und Acetonitril, Ketone wie Aceton und Butanon, oder auch Wasser.
   Vorzugsweise arbeitet man ohne Lösungsmittel in einem Überschuß der zur Acetalspaltung verwendeten Säure, wobei Ameisensäure besonders bevorzugt ist.
   Für eine vollständige Umsetzung werden die Edukte I bzw. V, wobei R⁶ eine Gruppe -C(R¹¹) (Z¹R¹²) (Z²R¹³) bedeutet, und H₂Y in mindestens stöchiometrischen Mengen eingesetzt, jedoch ist auch ein Überschuß an H₂Y, bis etwa 200 mol-%, möglich.
   Die Menge an Säure, Ionenaustauscher oder Übergangsmetallsalz ist nicht kritisch. Im allgemeinen ist eine Menge bis etwa 300 mol-%, bezogen auf die Menge an H₂Y, ausreichend.
   In der Regel liegt die Reaktionstemperatur bei (-78) bis 180°C, vorzugsweise 0°C bis Siedetemperatur des jeweiligen Verdünnungsmittels.
Verfahren O₎
   Olefinierung von Verbindungen I {R⁶ = -CO-R¹¹}: 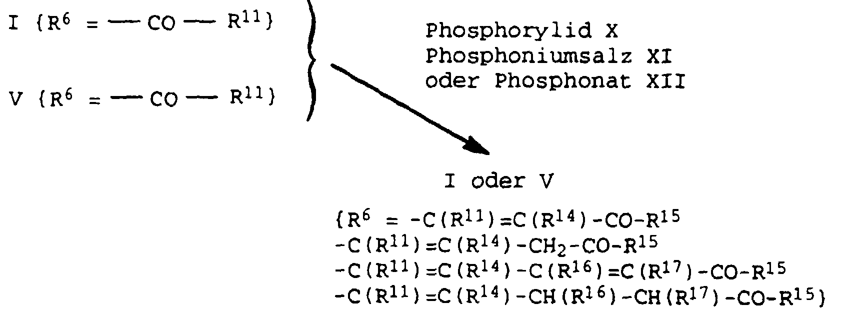
   Die Reaktion kann mit den folgenden Phosphoryliden Xa bis Xd, Phosphoniumsalzen XIa bis XId und Phosphonaten XIIa bis XIId durchgeführt werden:
   Phosphorylide X:

      R₃P=C(R¹⁴)-CO-R¹⁵ Xa,

      R₃P=C(R¹⁴)-CH₂-CO-R¹⁵ Xb,

      R₃P=C(R¹⁴)-C(R¹⁶)=C(R¹⁷)-CO-R¹⁵ Xc,

      R₃P=C(R¹⁴)-CH₂-CH(R¹⁸)-CO-R¹⁵ Xd;
   Phosphoniumsalze XI:

      R₃P^{⊕}-CH(R¹⁴)-CO-R¹⁵ Hal^{⊖} XIa,

      R₃P^{⊕}-CH(R¹⁴)-CH₂-CO-R¹⁵ Hal^{⊖} XIb,

      R₃P^{⊕}-CH(R¹⁴)-C(R¹⁶)=C(R¹⁷)-CO-R¹⁵ Hal^{⊖} XIc;

      R₃P^{⊕}-CH(R¹⁴)-CH₂-CH(R¹⁸)-CO-R¹⁵ Hal^{⊖} XId;
   Phosphonate XII:

      (RO)₂PO-CH(R¹⁴)-CO-R¹⁵ XIIa,

      (RO)₂PO-CH(R¹⁴)-CH₂-CO-R¹⁵ XIIb,

      (RO)₂PO-CH(R¹⁴)-C(R¹⁶)=C(R¹⁷)-CO-R¹⁵ XIIc,

      (RO)₂PO-CH(R¹⁴)-CH₂-CH(R¹)⁸-CO-R¹⁵ XIId.

   Wenig geeignet sind diejenigen Phosphorylide Xb und Xd, Phosphoniumsalze XIb und XId sowie Phosphonate XIIb und XIId, bei denen R¹⁵ Wasserstoff, Alkyl oder Cycloalkyl bedeutet.
   Die Reste R am Phosphor können gleich oder verschieden sein und stehen beispielsweise für verzweigte oder unverzweigte C₁-C₈-Alkylgruppen, C₅- oder C₆-Cycloalkylgruppen und insbesondere für Phenyl, das weitere (für die Umsetzung inerte Substituenten, beispielsweise C₁-C₄-Alkyl wie Methyl, Ethyl und tert.-Butyl, C₁-C₄-Alkoxy wie Methoxy oder Halogen wie Fluor, Chlor und Brom) tragen kann. Bevorzugt sind unsubstituierte Phenylreste, da der für die Herstellung der Phosphorylide X und Phosphoniumsalze XI verwendete Ausgangsstoff Triphenylphosphin besonders kostengünstig ist und bei den Umsetzungen zudem das sehr reaktionsträge und gut abtrennbare, feste Triphenylphosphinoxid entsteht.
   Zur Herstellung der Phosphonate XII eignen sich beispielsweise die in Houben-Weyl, Methoden der Organischen Chemie, Bd. E2, 1982, S. 345ff., beschriebenen Methoden.
   Als Lösungsmittel kommen inerte organische Lösungsmittel, z.B. Aromaten wie Toluol und o-, m-, p-Xylol, Ether wie 1,2-Dimethoxyethan, Diethylether, Tetrahydrofuran und Dioxan, polare organische Lösungsmittel wie Dimethylformamid und Dimethylsulfoxid, oder Alkohole wie Methanol, Ethanol und Isopropanol, in Betracht.
   Bei der Olefinierung von I bzw. V, wobei R⁶ -CO-R¹¹ bedeutet, mit einem Phosphoniumsalz XI oder einem Phosphonat XII arbeitet man in Gegenwart einer Base, wobei Alkalimetallalkyle wie n-Butyllithium, Alkalimetallhydride und -alkoholate wie Natriumhydrid, Natriumethanolat und Kalium-tert.-butanolat, sowie Alkalimetall- und Erdalkalimetallhydroxide wie Calciumhydroxid, besonders gut geeignet sind.
   Für eine vollständige Umsetzung werden alle Reaktionspartner in etwa stöchiometrischen Mengen eingesetzt; bevorzugt verwendet man jedoch einen Überschuß an Base, bis etwa 10 mol-%.
   Im allgemeinen liegt die Reaktionstemperatur bei (-40) bis 150°C.
   Die Verbindungen der Formel X, XI und XII sind bekannt oder lassen sich in auf bekannte Weise darstellen (vgl. z.B. Houben-Weyl, Methoden d. Org. Chemie, Bd. E1, S. 636 ff., Georg Thieme Verlag, Stuttgart 1982, Chem. Ber. 95, 3993 1962) oder Houben-Weyl, Methoden d. Org. Chemie, Bd. E2, S. 345ff., Georg Thieme Verlag, Stuttgart 1982).
   Eine weitere Möglichkeit zur Darstellung von 1-Amino-3-benzyluracilen I, wobei R⁶ für -C(R¹¹)=C(R¹⁴)-CO-R¹⁵ steht und R¹⁵ z.B. Wasserstoff, Alkyl, Alkenyl, Halogenalkyl, Cycloalkyl, Phenyl oder Alkoxyalkyl bedeutet, besteht in der an sich bekannten Aldolkondensation. Hierfür geeignete Bedingungen sind z.B. Nielsen, Org. React. 16, lff. (1968), zu entnehmen.
   Als weitere Methode zur Synthese von Verbindungen der Formel I, wobei R⁶ für -C(R¹¹)=C(R¹⁴)-CO-R¹⁵, -CH(R¹¹)-CH(R¹⁴)-CO-R¹⁵, -C(R¹¹)=C(R¹⁴)-CH₂-CO-R¹⁵, -C(R¹¹)=C(R¹⁴)-C(R¹⁶)=C(R¹⁷)-CO-R¹⁵ oder -C(R¹¹)=C(R¹⁴)-CH₂-CH(R¹⁸)-CO-R¹⁵ steht und R¹⁴ Wasserstoff, Cyano, Alkoxycarbonyl oder Alkylcarbonyl bedeutet, kommen sowohl die Knoevenagel-Kondensation als auch die Perkin-Kondensation in Betracht. Geeignete Bedingungen sind z.B. aus Org. React. 1967, 15, 204ff. (Knoevenagel) bzw. Johnson, Org. React. 1, 1942, 210ff. (Perkin), ersichtlich.
   Verbindungen, in denen R¹⁵ -SR²² oder -N(R²³)R²⁴ bedeutet, lassen sich z.B. in an sich bekannter Weise dadurch herstellen, daß man entsprechende Verbindungen, in denen R¹⁵ für Hydroxy steht, in ihre Säurehalogenide (Halogen anstelle von R¹⁵) überführt und die Verfahrensprodukte anschließend mit einem Amin H-N(R²³)R²⁴, Thiol H-SR²² oder mit einem reaktiven Derivat dieser Verbindungen umsetzt.
Verfahren P₎
   Umsetzung von Verbindungen I oder V {R⁶ = -CO-R¹¹} mit Aminen, Hydroxylaminen oder Hydrazinen: 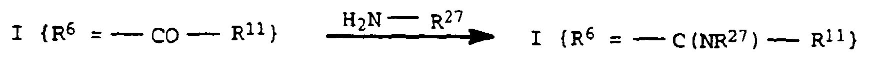 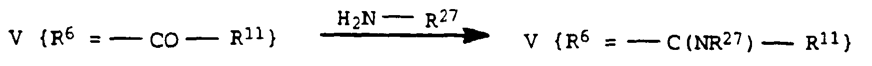
   Die Umsetzung erfolgt normalerweise in einem inerten organischen Lösungs- oder Verdünnungsmittel, z.B. in einem Aromaten wie Toluol und Xylol, in einem chlorierten Kohlenwasserstoff wie Dichlormethan, Chloroform und Chlorbenzol, in einem Ether wie Diethylether, 1,2-Dimethoxyethan und Tetrahydrofuran, in einem Alkohol wie Methanol und Ethanol, oder in einem Gemisch der genannten Lösungsmittel.
   Liegen die Amine H₂N-R²⁷ als Salze, z.B. als Hydrochloride oder Oxalate vor, so ist zu ihrer Freisetzung die Zugabe einer Base wie vorzugsweise Natriumcarbonat, Kaliumcarbonat, Natriumhydrogencarbonat, Triethylamin und Pyridin, empfehlenswert.
   Das entstehende Reaktionswasser kann gegebenenfalls destillativ oder mit Hilfe eines Wasserabscheiders aus dem Reaktionsgemisch entfernt werden.
   Üblicherweise liegt die Reaktionstemperatur bei (-30) bis 150°C, bevorzugt 0 bis 130°C.
Verfahren Q₎
   Spaltung von Verbindungen I oder V, wobei R⁶ -C(N^{R27})-R¹¹ bedeutet: 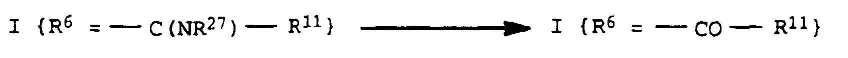 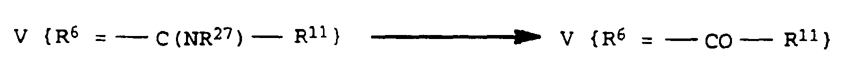
   Die Spaltung erfolgt lösungsmittelfrei oder in einem inerten Lösungs- oder Verdünnungsmittel mit Wasser oder einem reaktionsfähigen Derivat des Wassers.
   Die Umsetzung kann hydrolytisch oder unter oxidativen Bedingungen durchgeführt werden, wobei sich eine Reaktionstemperatur von (-78) bis 180°C, vorzugsweise 0°C bis Siedepunkt des Verdünnungsmittels, empfiehlt.
   Als Lösungs- oder Verdünnungsmittel kommen z.B. Aromaten wie Benzol, Toluol und o-, m-, p-Xylol, chlorierte Kohlenwasserstoffe wie Dichlormethan, Chloroform und Chlorbenzol, Ether wie Dialkylether, 1,2-Dimethoxyethan, Tetrahydrofuran und Dioxan, Alkohole wie Methanol und Ethanol, Ketone wie Aceton, Ester organischer Säuren wie Essigsäureethylester oder Wasser sowie Gemische der genannten Lösungsmittel in Betracht.
   Zweckmäßigerweise arbeitet man in Gegenwart einer Mineralsäure wie Salzsäure, Bromwasserstoffsäure und Schwefelsäure, einer Carbonsäure wie Essigsäure und Trifluoressigsäure oder einer Sulfonsäure wie p-Toluolsulfonsäure.
   Um das bei der Hydrolyse anfallende H₂N-R²⁷ abzufangen bzw. aus dem Gleichgewicht zu entfernen, kann es vorteilhaft sein, in Gegenwart einer anderen Carbonylverbindung, z.B. Aceton, Formaldehyd, Glyoxalsäure oder Phenylglyoxylsäure, bevorzugt Formaldehyd, zu arbeiten, die eine stabilere Verbindung mit H₂N-R²⁷ eingeht als I/V (R⁶ = CHO).
   Bei der Arbeitsweise unter oxidativen Bedingungen eignen sich insbesondere Oxidationsmittel wie Bleitetraacetat, Natriumhypochlorid und Wasserstoffperoxid.
   Gewünschtenfalls kann die Reaktionsführung zusätzlich in Gegenwart eines Katalysators wie Kupfer-(II)-sulfat, Titantetrachlorid und Bortrifluoretherat erfolgen.
   Die Mengen an Säure, Oxidationsmittel und Katalysator können in weiten Bereichen variiert werden. Normalerweise liegen sowohl Säure- als auch Katalysatormenge bei 5 bis 200 mol-%, und die Menge an Oxidationsmittel bei 25 bis 400 mol-%, bezogen auf die Menge der zu oxidierenden Verbindung; sie können aber auch in erheblich größerem Überschuß verwendet werden.
Verfahren R₎
   Reduktion eines 3-(Cyanobenzyl)uracils: 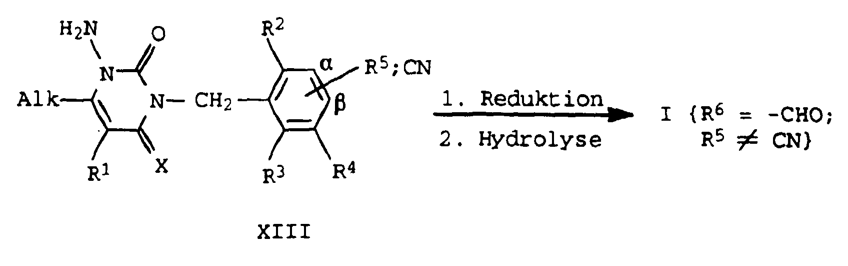
   Die Umsetzung erfolgt zweckmäßig in einem inerten organischen Lösungsmittel, z.B. einem Aromaten wie Toluol und o-, m-, p-Xylol, einem aliphatischen oder cyclischen Ether wie Diethylether, tert.-Butylmethylether, Tetrahydrofuran und Dioxan, einem chlorierten Kohlenwasserstoff wie Methylenchlorid, Chloroform und Chlorbenzol, oder in einer organischen Carbonsäure wie Ameisensäure.
   Als Reduktionsmittel eignen sich beispielsweise Wasserstoff oder Metallsalze wie Zinn-(II)-chlorid, Metallhydride wie Diisobutylaluminiumhydrid, Diisopropylaluminiumhydrid, Lithium-trisethoxyaluminiumhydrid und Lithium-bisethoxyaluminiumhydrid oder Triethylsilan. Bevorzugt ist die Verwendung von Diisobutylaluminiumhydrid, Ameisensäure oder Wasserstoff.
   Gewünschtenfalls kann die Reduktion in Gegenwart eines Katalysators, z.B. Triethyloxoniumtetrafluorborat oder Raney-Nickel, durchgeführt werden.
   Arbeitet man ohne Verdünnungsmittel in Ameisensäure als Reduktionsmittel, so kann diese auch in einem größeren Überschuß vorliegen.
   Die günstigste Reaktionstemperatur ist abhängig vom jeweiligen Reduktionsmittel, liegt aber im allgemeinen bei (-78) bis 150°C.
   Auf entsprechende Weise lassen sich auch Verbindungen I mit R⁵ = CN reduzieren.
Verfahren S₎
   Meerwein-Alkylierung eines Diaziniumsalzes XIVb: 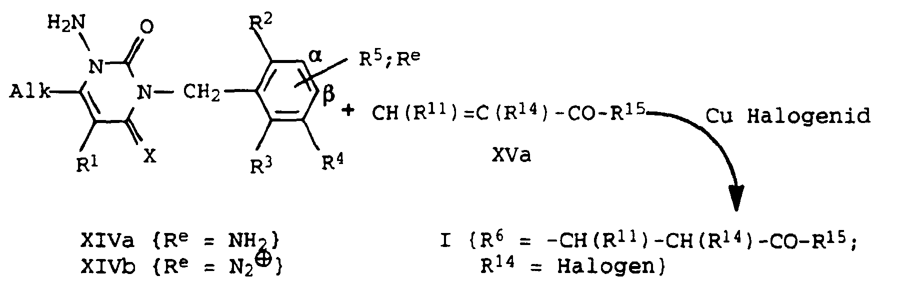
   Die Reaktionsbedingungen der Meerwein-Reaktion sind dem Fachmann an sich bekannt (vgl. z.B. M.P. Doyle et al., J. Org. Chem. 42, 1977, 2431; G. Theodoridis et al., J. Heterocyclic Chem. 28, 1991, 849; C.S. Rondestvedt Jr., Org. React. 24, 1976, 225 und dort zit. Literatur); die Umsetzung von XIVb mit XVa erfolgt zweckmäßig auf analoge Weise.
Verfahren T₎
   Metallkatalysierte Olefinkupplung mit einem Phenylhalogenid der Formel XV: 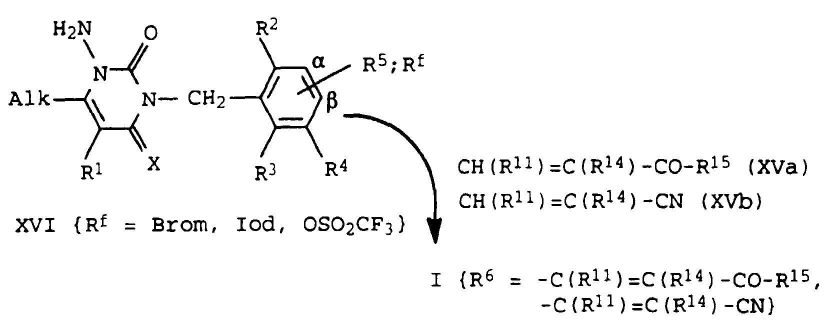
   Die Bedingungen dieser Heck- oder Heck-ähnlichen Reaktion sind dem Fachmann an sich bekannt (vgl. z.B. Comprehensive Organic Chemistry) und lassen sich analog auf obige Reaktion anwenden.
   Die Enaminester der Formel III sind neu. Ihre Herstellung und die der Enaminester VI kann nach an sich bekannten Methoden erfolgen, z.B. nach einem der folgenden Verfahren:
Verfahren U₎
   Umsetzung eines 3-Aminoalk-2-ensäureesters XVII mit einem Benzylisocyanat XVIII in Gegenwart einer Base: 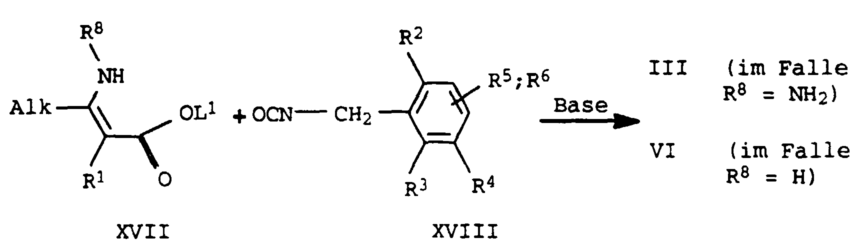 R⁸ steht für Wasserstoff oder Amino.
   Als 3-Aminoalk-2-ensäureester XVII hat sich bisher der Ethylester besonders bewährt, jedoch kann auch jeder andere Ester, vorzugsweise die Alkylester, verwendet werden.
   Die Umsetzung erfolgt zweckmäßig in Gegenwart eines im wesentlichen wasserfreien aprotischen organischen Lösungs- oder Verdünnungsmittels, beispielsweise eines aliphatischen oder cyclischen Ethers wie Diethylether, 1,2-Dimethoxyethan, Tetrahydrofuran und Dioxan, eines aliphatischen oder aromatischen Kohlenwasserstoffs wie n-Hexan, Benzol, Toluol und den Xylolen, eines halogenierten, aliphatischen Kohlenwasserstoffs wie Methylenchlorid, Chloroform, Tetrachlorkohlenstoff, 1,2-Dichlorethan und Chlorbenzol, eines aprotischen, polaren Lösungsmittel wie Dimethylformamid, Hexamethylphosphorsäuretriamid und Dimethylsulfoxid, oder eines Gemisches aus den genannten Solventien.
   Gewünschtenfalls kann auch in Gegenwart einer Metallhydridbase wie Natrium- und Kaliumhydrid, eines Alkalimetall- oder Erdalkalimetallalkoholates wie Natriummethanolat, -ethanolat und Kalium-tert.-butanolat, oder einer organischen tertiären Base wie Triethylamin und Pyridin gearbeitet werden, wobei die organische Base gleichzeitig als Lösungsmittel dienen kann.
   Zweckmäßig setzt man die Ausgangsverbindungen in stöchiometrischen Mengen ein oder man arbeitet mit einem geringen Überschuß der einen oder anderen Komponente bis etwa 10 mol-%. Beim Arbeiten ohne Lösungsmittel in Gegenwart einer organischen Base empfiehlt es sich, letztere in einem größeren Überschuß einzusetzen.
   Normalerweise ist eine Reaktionstemperatur von (-80) bis 50°C, insbesondere (-60) bis 30°C ausreichend.
   In einer besonders bevorzugten Ausführungsform wird der erhaltene Enaminester mit überschüssiger Base direkt (d.h. "in situ") in das entsprechende Wertprodukt I oder V übergeführt, dessen Reinigung dann mittels üblicher Trennverfahren wie Kristallisation und Chromatographie erfolgen kann.
Verfahren W₎
   Umsetzung eines β-Ketoesters XIX mit einem Benzylharnstoff XX: 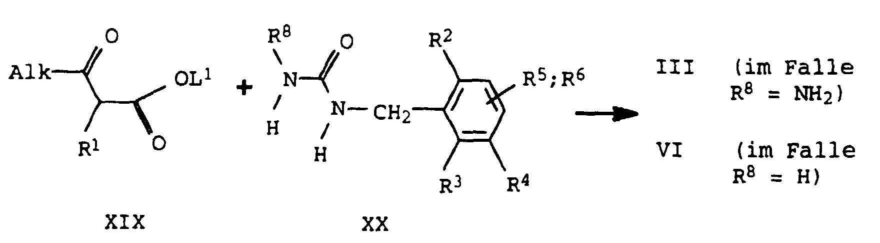
   Vorzugsweise arbeitet man im wesentlichen wasserfrei in einem inerten Lösungs- oder Verdünnungsmittel, besonders bevorzugt in Gegenwart eines sauren oder basischen Katalysators.
   Als Lösungs- oder Verdünnungsmittel kommen insbesondere mit Wasser azeotrop mischbare organische Lösungsmittel, beispielsweise Aromaten wie Benzol, Toluol und die Xylole, halogenierte Kohlenwasserstoffe wie Methylenchlorid, Chloroform, Tetrachlorkohlenstoff und Chlorbenzol, aliphatische und cyclische Ether wie 1,2-Dimethoxyethan, Tetrahydrofuran und Dioxan, oder Cyclohexan, aber auch Alkohole wie Methanol und Ethanol, in Betracht.
   Als saure Katalysatoren eignen sich bevorzugt starke Mineralsäuren wie Schwefelsäure und Salzsäure, Phosphor enthaltende Säuren wie Orthophosphorsäure und Polyphosphorsäure, organische Säuren wie p-Toluolsulfonsäure sowie saure Kationenaustauscher wie "Amberlyst 15" (Fa. Fluka).
   Als basische Katalysatoren eignen sich z.B. Metallhydride wie Natriumhydrid sowie besonders bevorzugt Metallalkoholate wie Natriummethanolat und -ethanolat.
   zweckmäßig setzt man β-Ketoester XIX und den Benzylharnstoff XX in etwa stöchiometrischen Mengen um oder man arbeitet mit einem geringen Überschuß der einen oder anderen Komponente, bis etwa 10 mol-%.
   Normalerweise ist es ausreichend, die halbe bis zweifache molare Menge an Katalysator, bezogen auf die Menge einer der Ausgangsverbindungen, einzusetzen.
   Im allgemeinen erfolgt die Reaktionsführung bei einer Temperatur von 60 bis 120°C, zur raschen Entfernung von entstehendem Wasser vorzugsweise bei der Siedetemperatur des Reaktionsgemisches.
Verfahren ψ₎: 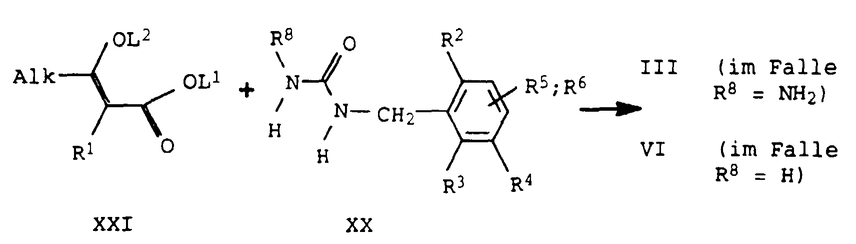
   L² steht für C₁-C₆-Alkyl oder Phenyl.
   Diese Umsetzung kann in einem inerten, mit Wasser mischbaren, organischen Lösungsmittel, beispielsweise einem aliphatischen oder cyclischen Ether wie 1,2-Dimethoxyethan, Tetrahydrofuran und Dioxan, oder einem niederen Alkohol, insbesondere Ethanol, durchgeführt werden, wobei die Reaktionstemperatur normalerweise bei 50 bis 100°C, vorzugsweise bei der Siedetemperatur des Reaktionsgemisches, liegt.
   Die Reaktion kann jedoch auch in einem aromatischen Verdünnungsmittel wie Benzol, Toluol und den Xylolen durchgeführt werden, wobei in diesem Fall der Zusatz entweder eines sauren Katalysators wie Salzsäure und p-Toluolsulfonsäure oder einer Base, z.B. eines Alkalimetallalkoholates wie Natriummethanolat und Natriumethanolat, empfehlenswert ist. Auch bei dieser Verfahrensvariante liegt die Reaktionstemperatur normalerweise bei 50 bis 100°C, bevorzugt jedoch bei 60 bis 80°C.
   Bezüglich der Mengenverhältnisse gelten die Angaben für Methode W).
   Die Enamincarboxylate der Formel IV sind ebenfalls neu; auch sie - und die Enamincarboxylate VII - können auf an sich bekannte Weise hergestellt werden, beispielsweise aus einem Benzylamin der Formel XXII nach folgendem allgemeinen Reaktionsschema φ): 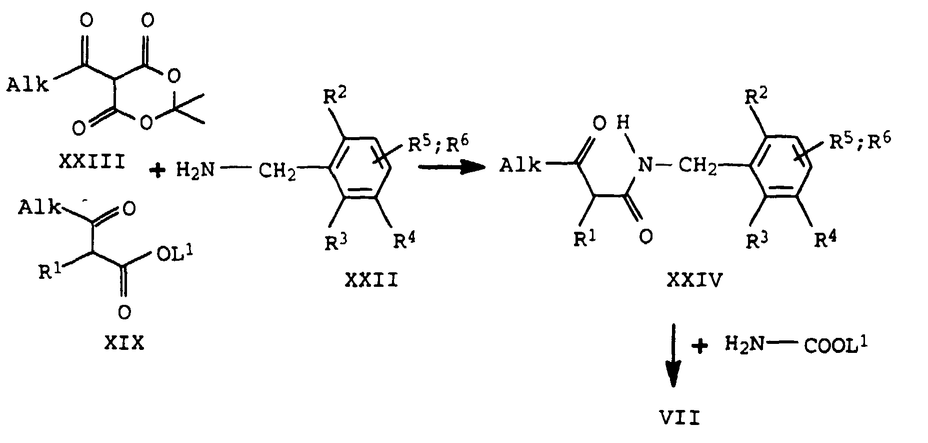
   Die Umsetzung von XXII mit XXIII erfolgt vorzugsweise in einem wasserfreien inerten aprotischen Lösungsmittel, beispielsweise in einem halogenierten Kohlenwasserstoff wie Methylenchlorid, Chloroform, Tetrachlorkohlenstoff und Chlorbenzol, einem aromatischen Kohlenwasserstoff wie Benzol, Toluol und den Xylolen, oder einem aliphatischen oder cyclischen Ether wie Diethylether, Dibutylether, 1,2-Dimethoxyethan, Tetrahydrofuran und Dioxan.
   Die Reaktionstemperatur liegt bei dieser Umsetzung (von XXII mit XXIII) im allgemeinen bei etwa 70 bis 140°C, insbesondere bei 100 bis 120°C.
   Bei der Umsetzung von XIX mit XXII handelt es sich um eine Aminolyse, die in der Regel entweder ohne Lösungsmittel [vgl. z.B. J. Soc. Dyes Col. 42, 81 (1926), Ber. 64, 970 (1931); Org. Synth., Coll. Vol. IV, 80 (1963) und J. Am. Chem. Soc. 70, 2402 (1948)] oder in einem inerten wasserfreien Lösungs-/Verdünnungsmittel, insbesondere in einem aprotischen Solvens, beispielsweise in einem Aromaten wie Toluol und den Xylolen, oder einem halogenierten Aromaten wie Chlorbenzol, durchgeführt wird.
   Hierbei empfiehlt sich das Arbeiten in Gegenwart eines basischen Katalysators, beispielsweise eines höher siedenden Amins [siehe z. B. Helv. Chim. Acta 11, 779 (1928) und U.S. 2,416,738] oder von Pyridin.
   Vorzugsweise liegt die Reaktionstemperatur bei ca. 20 bis 160°C, insbesondere bei 80°C bis zur Siedetemperatur des Reaktionsgemisches oder des basischen Katalysators.
   Zweckmäßigerweise setzt man die Ausgangsverbindungen jeweils in etwa stöchiometrischen Mengen um oder man arbeitet mit einem geringen Überschuß der einen oder anderen Komponente, bis etwa 10 mol-%. Arbeitet man in Gegenwart eines basischen Katalysators, so wird dieser normalerweise in der halben bis zur zweifachen molaren Menge, bezogen auf die Menge eines der Edukte, eingesetzt.
   Die anschließende Umsetzung der so hergestellten Verbindungen der Formel XXIV mit dem Amin H₂N-COOL¹ wird vorteilhaft in einem weitgehend wasserfreien Lösungs-/Verdünnungsmittel bei Normaldruck durchgeführt, besonders bevorzugt in Gegenwart eines sauren Katalysators.
   Als Lösungs-/Verdünnungsmittel kommen insbesondere mit Wasser azeotrop mischbare organische Flüssigkeiten, beispielsweise Aromaten wie Benzol, Toluol und die Xylole, oder halogenierte Kohlenwasserstoffe wie Tetrachlorkohlenstoff und Chlorbenzol, in Betracht.
   Geeignete Katalysatoren sind insbesondere starke Mineralsäuren wie Schwefelsäure, organische Säuren wie p-Toluolsulfonsäure, Phosphor enthaltende Säuren wie Orthophosphorsäure und Polyphosphorsäure oder saure Kationenaustauscher wie "Amberlyst 15" (Fa. Fluka).
   Im allgemeinen liegt die Reaktionstemperatur bei etwa 70 bis 150°C; zur raschen Entfernung des entstehenden Reaktionswassers arbeitet man jedoch zweckmäßigerweise bei der Siedetemperatur des jeweiligen Reaktionsgemisches.

Sofern nicht anders angegeben werden alle vorstehend beschriebenen Verfahren zweckmäßigerweise bei Atmosphärendruck oder unter dem Eigendruck des jeweiligen Reaktionsgemisches vorgenommen.

Die Aufarbeitung der Reaktionsgemische erfolgt in der Regel nach an sich bekannten Methoden, beispielsweise durch Verdünnen der Reaktionsmischung mit Wasser und anschließender Isolierung des Wertproduktes mittels Filtration, Kristallisation oder Lösungsmittelextraktion, oder durch Entfernen des Lösungsmittels, Verteilen des Rückstandes in einem Gemisch aus Wasser und einem geeigneten organischen Lösungsmittel und Aufarbeiten der organischen Phase auf das Wertprodukt hin.

Im allgemeinen sind die 1-Amino-3-benzyluracile I nach einem der vorstehend genannten Syntheseverfahren herstellbar. Aus wirtschaftlichen oder verfahrenstechnischen Gründen kann es jedoch zweckmäßiger sein, einige Verbindungen I aus ähnlichen 1-Amino-3-benzyluracilen I, die sich jedoch insbesondere in der Bedeutung der Reste R⁵ und/oder R⁶ unterscheiden, herzustellen, und zwar auf an sich bekannte Weise, z.B. durch Esterhydrolyse, Veresterung, Amidierung, Acetalisierung, Acetalhydrolyse, Kondensationsreaktion, Wittig-Reaktion, Peterson-Olefinierung, Veretherung, Alkylierung, Oxidation oder Reduktion.

Die 1-Amino-3-benzyluracile I können bei der Herstellung als Isomerengemische anfallen, die jedoch gewünschtenfalls nach den hierfür üblichen Methoden wie Kristallisation oder Chromatographie, auch an einem optisch aktiven Adsorbat, in die weitgehend reinen Isomeren getrennt werden können. Reine optisch aktive Isomere lassen sich vorteilhaft aus entsprechenden optisch aktiven Ausgangsprodukten herstellen.

Landwirtschftlich brauchbare Salze der Verbindungen I können durch Reaktion mit einer Base des entprechenden Kations, vorzugsweise einem Alkalimetallhydroxid oder -hydrid, oder durch Reaktion mit einer Säure des entprechenden Anions, vorzugsweise der Chlorwasserstoffsäure, Bromwasserstoffsäure, Schwefelsäure, Phosphorsäure oder Salpetersäure, gebildet werden.

Salze von I, deren Metallion kein Alkalimetallion ist, können auch durch Umsalzen des entsprechenden Alkalimetallsalzes in üblicher Weise hergestellt werden, ebenso Ammonium-, Phosphonium-, Sulfonium- und Sulfoxoniumsalze mittels Ammoniak, Phosphonium-, Sulfonium- oder Sulfoxoniumhydroxiden.

Die Verbindungen I und deren landwirtschaftlich brauchbaren Salze eignen sich - sowohl als Isomerengemische als auch in Form der reinen Isomeren - als Herbizide. Die I enthaltenden herbiziden Mittel bekämpfen Pflanzenwuchs auf Nichtkulturflächen sehr gut, besonders bei hohen Aufwandmengen. In Kulturen wie Weizen, Reis, Mais, Soja und Baumwolle wirken sie gegen Unkräuter und Schadgräser, ohne die Kulturpflanzen nennenswert zu schädigen. Dieser Effekt tritt vor allem bei niedrigen Aufwandmengen auf.

In Abhängigkeit von der jeweiligen Applikationsmethode können die Verbindungen I bzw. sie enthaltenden herbiziden Mittel noch in einer weiteren Zahl von Kulturpflanzen zur Beseitigung unerwünschter Pflanzen eingesetzt werden. In Betracht kommen beispielsweise folgende Kulturen:
Allium cepa, Ananas comosus, Arachis hypogaea, Asparagus officinalis, Beta vulgaris spec., altissima, Beta vulgaris spec., rapa, Brassica napus var. napus, Brassica napus var. napobrassica, Brassica rapa var. silvestris, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spec., Manihot esculenta, Medicago sativa, Musa spec., Nicotiana tabacum (N.rustica), Olea europaea, Oryza sativa, Phaseolus lunatus, Phaseolus vulgaris, Picea abies, Pinus spec., Pisum sativum, Prunus avium, Prunus persica, Pyrus communis, Ribes sylvestre, Ricinus communis, Saccharum officinarum, Secale cereale, Solanum tuberosum, Sorghum bicolor (s. vulgare), Theobroma cacao, Trifolium pratense, Triticum aestivum, Triticum durum, Vicia faba, Vitis vinifera und Zea mays.

Darüber hinaus können die Verbindungen I auch in Kulturen, die durch Züchtung einschließlich gentechnischer Methoden gegen die Wirkung von Herbiziden tolerant sind, verwandt werden.

Des weiteren eignen sich die 1-Amino-3-benzyluracile I auch zur Desikkation und/oder Defoliation von Pflanzen.

Als Desikkantien eignen sie sich insbesondere zur Austrocknung der oberirdischen Teile von Kulturpflanzen wie Kartoffel, Raps, Sonnenblume und Sojabohne. Damit wird ein vollständig mechanisches Beernten dieser wichtigen Kulturpflanzen ermöglicht.

Von wirtschaftlichem Interesse ist ferner die Ernteerleichterung, die durch das zeitlich konzentrierte Abfallen oder Vermindern der Haftfestigkeit am Baum bei Zitrusfrüchten, Oliven oder bei anderen Arten und Sorten von Kern-, Stein- und Schalenobst ermöglicht wird. Derselbe Mechanismus, das heißt die Förderung der Ausbildung von Trenngewebe zwischen Frucht- oder Blatt- und Sproßteil der Pflanzen ist auch für ein gut kontrollierbares Entblättern von Nutzpflanzen, insbesondere Baumwolle, wesentlich.

Außerdem führt die Verkürzung des Zeitintervalls, in dem die einzelnen Baumwollpflanzen reif werden, zu einer erhöhten Faserqualität nach der Ernte.

Die Verbindungen I bzw. die sie enthaltenden Mittel können beispielsweise in Form von direkt versprühbaren wäßrigen Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Als inerte Hilfsstoffe kommen im Wesentlichen in Betracht: Mineralölfraktionen von mittlerem bis hohem Siedepunkt wie Kerosin und Dieselöl, ferner Kohlenteeröle sowie Öle pflanzlichen oder tierischen Ursprungs, aliphatische, cyclische und aromatische Kohlenwasserstoffe, z.B. Paraffine, Tetrahydronaphthalin, alkylierte Naphthaline und deren Derivate, alkylierte Benzole und deren Derivate, Alkohole wie Methanol, Ethanol, Propanol, Butanol und Cyclohexanol, Ketone wie Cyclohexanon, stark polare Lösungsmittel, z.B. Amine wie N-Methylpyrrolidon und Wasser.

Wäßrige Anwendungsformen können aus Emulsionskonzentraten, Suspensionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe (Adjuvantien) kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Ricinusöl, Polyoxyethylen- oder Polyoxypropylenalkylether, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.

Die Konzentrationen der Wirkstoffe I in den anwendungsfertigen Zubereitungen können in weiten Bereichen variiert werden. Die Formulierungen enthalten im allgemeinen etwa von 0,001 bis 98 Gew.-%, vorzugsweise 0,01 bis 95 Gew.-%, mindestens eines Wirkstoffs. Die Wirkstoffe werden dabei in einer Reinheit von 90 % bis 100%, vorzugsweise 95 % bis 100 % (nach NMR-Spektrum) eingesetzt.

Die folgenden Formulierungsbeispiele verdeutlichen die Herstellung solcher Zubereitungen:
I. 20 Gewichtsteile der Verbindung Nr. Ia.436 werden in einer Mischung gelöst, die aus 80 Gewichtsteilen alkyliertem Benzol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100000 Gewichtsteilen Wasser erhält man eine wäßrige Dispersion, die 0,02 Gew.-% des Wirkstoffs enthält.
II. 20 Gewichtsteile der Verbindung Nr. Ia.436 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100000 Gewichtsteilen Wasser erhält man eine wäßrige Dispersion, die 0,02 Gew.-% des Wirkstoffs enthält.
III. 20 Gewichtsteile des Wirkstoffs Nr. Ia.436 werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100000 Gewichtsteilen Wasser erhält man eine wäßrige Dispersion, die 0,02 Gew.-% des Wirkstoffs enthält.
IV. 20 Gewichtsteile des Wirkstoffs Nr. Ia.436 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20000 Gewichtsteilen Wasser enthält man eine Spritzbrühe, die 0,1 Gew.-% des Wirkstoffs enthält.
V. 3 Gewichtsteile des Wirkstoffs Nr. Ia.436 werden mit 97 Gewichtsteilen feinteiligem Kaolin vermischt. Man erhält auf diese Weise ein Stäubemittel, das 3 Gew.-% des Wirkstoffs enthält.
VI. 20 Gewichtsteile des Wirkstoffs Nr. Ia.436 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.
VII. 1 Gewichtsteil des Wirkstoffs Nr. Ia.436 wird in einer Mischung gelöst, die aus 70 Gewichtsteilen Cyclohexanon, 20 Gewichtsteilen ethoxyliertem Isooctylphenol und 10 Gewichtsteilen ethoxyliertem Ricinusöl besteht. Man erhält ein stabiles Emulsionskonzentrat.
VIII. 1 Gewichtsteil des Wirkstoffs Nr. Ia.436 wird in einer Mischung gelöst, die aus 80 Gewichtsteilen Cyclohexanon und 20 Gewichtsteilen Wettol® EM 31 (= nichtionischer Emulgator auf der Basis von ethoxyliertem Ricinusöl) besteht. Man erhält ein stabiles Emulsionskonzentrat.

Die Applikation der Wirkstoffe I bzw. der herbiziden Mittel kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff I betragen je nach Bekämpfungsziel, Jahreszeit, Zielpflanzen und Wachstumsstadium 0,001 bis 3,0, vorzugsweise 0,01 bis 1,0 kg/ha aktive Substanz (a.S.).

Zur Verbreiterung des Wirkungsspektrums und zur Erzielung synergistischer Effekte können die 1-Amino-3-benzyluracile I mit zahlreichen Vertretern anderer herbizider oder wachstumsregulierender Wirkstoffgruppen gemischt und gemeinsam ausgebracht werden. Beispielsweise kommen als Mischungspartner 1,2,4-Thiadiazole, 1,3,4-Thiadiazole, Amide, Aminophosphorsäure und deren Derivate, Aminotriazole, Anilide, Aryloxy-/Heteroaryloxyalkansäuren und deren Derivate, Benzoesäure und deren Derivate, Benzothiadiazinone, 2-(Hetaroyl/Aroyl)-1,3-cyclohexandione, Heteroaryl-Aryl-Ketone, Benzylisoxazolidinone, meta-CF3-Phenylderivate, Carbamate, Chinolincarbonsäure und deren Derivate, Chloracetanilide, Cyclohexan-1,3-dionderivate, Diazine, Dichlorpropionsäure und deren Derivate, Dihydrobenzofurane, Dihydrofuran-3-one, Dinitroaniline, Dinitrophenole, Diphenylether, Dipyridyle, Halogencarbonsäuren und deren Derivate, Harnstoffe, 3-Phenyluracile, Imidazole, Imidazolinone, N-Phenyl-3,4,5,6-tetrahydrophthalimide, Oxadiazole, Oxirane, Phenole, Aryloxy- und Heteroaryloxyphenoxypropionsäureester, Phenylessigsäure und deren Derivate, 2-Phenylpropionsäure und deren Derivate, Pyrazole, Phenylpyrazole, Pyridazine, Pyridincarbonsäure und deren Derivate, Pyrimidylether, Sulfonamide, Sulfonylharnstoffe, Triazine, Triazinone, Triazolinone, Triazolcarboxamide und Uracile in Betracht.

Außerdem kann es von Nutzen sein, die Verbindungen I allein oder in Kombination mit anderen Herbiziden auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

### Herstellungsbeispiel

3-(2,3-Dichlorbenzyl)-2,4-dioxo-1-amino-6-trifluormethyl-1,2,3,4tetrahydropyrimidin (Nr. Ia.436)

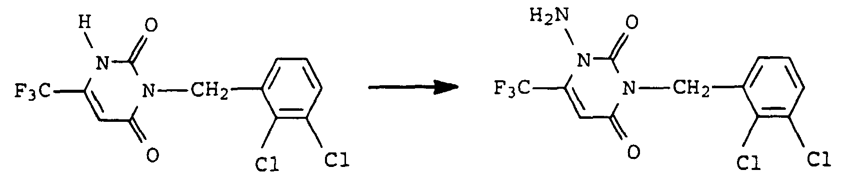

Zu einer Lösung von 0,0058 mol 3-(2,3-Dichlorbenzyl)-2,4-dioxo-1-H-6-trifluormethyl-1,2,3,4-tetrahydropyrimidin in 30 ml Essigester wurden 0,011 mol Kaliumcarbonat und dann 0,0062 mol 2,4-Dinitrophenoxyamin gegeben. Anschließend rührte man die Reaktionslösung 8 Std. bei 60°C, wonach der entstandene Feststoffanteil abgetrennt und mit Diisopropylether gewaschen wurde. Das Filtrat wurde zweimal mit Wasser gewaschen, dann über Natriumsulfat getrocknet und schließlich eingeengt. Der Rückstand wurde in 30 ml Diethylether aufgenommen. Durch Zugabe von Petrolether fällte man dann das Wertprodukt aus. Ausbeute: 0,6 g.

Herstellung des Vorprodukts 3-(2,3-Dichlorbenzyl)-2,4-dioxo-1-H-6-trifluormethyl-1,2,3,4-tetrahydropyrimidin

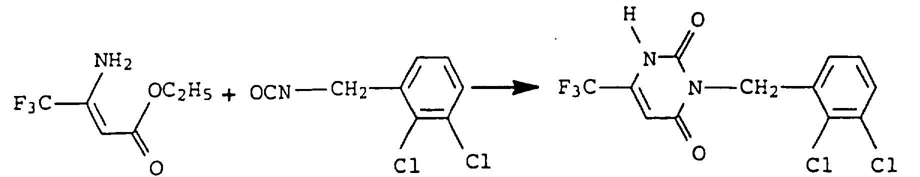

Zu 0,03 mol Natriumhydrid in 60 ml Dimethylformamid wurden bei 0°C 0,03 mol 3-Amino-4,4,4-trifluorbut-2-ensäureethylester getropft. Anschließend rührte man noch 30 Minuten bei dieser Temperatur. Dann wurde die Reaktionslösung auf (-10)°C abgekühlt und 0,03 mol 2,3-Dichlorbenzylisocyanat, gelöst in 5 ml Dimethylformamid, zugetropft. Anschließend erwärmte man die Reaktionslösung langsam auf Raumtemperatur und rührte noch 12 Std. Danach wurde noch 2 Std. bei 80°C gerührt, bevor man das Lösungsmittel weitgehend entfernte. Nach Zugabe von 100 ml Wasser wurde der entstandene Feststoffanteil abgetrennt. Das Filtrat wurde zweimal mit je 50 ml Toluol gewaschen. Nach ansäurern der wäßrigen Phase mit verdünnter Salzsäure extrahierte man das Produkt zweimal mit je 100 ml Methylenchlorid. Die organischen Phasen wurden mit Wasser gewaschen, dann über Natriumsulfat getrocknet und schließlich eingeengt. Aus dem Rückstand fällte man das Produkt durch Zugabe von Petrolether. Ausbeute: 6 g.

Herstellung des 2,3-Dichlorbenzylisocyanats:

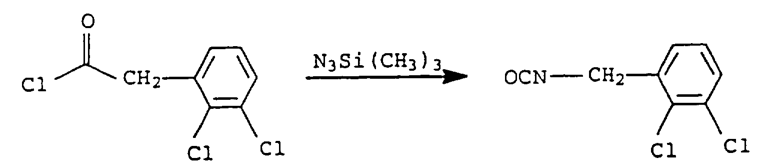

Zu einer auf Rückflußtemperatur erhitzten Mischung aus 0,065 mol 2,3-Dichlorbenzylcarbonsäurechlorid, 1,5 ml Triethylamin und 100 ml Toluol wurden langsam 0,071 mol Trimethylsilylazid getropft. Anschließend rührte man noch 30 min bei dieser Temperatur. Nach Entfernen des Lösungsmittels erhielt man ein Öl. Ausbeute: 13 g.

In den folgenden Tabellen 2 und 3 sind neben der vorstehend beschriebenen Verbindung noch weitere 1-Amino-3-benzyluracile I aufgeführt, die in analoger Weise hergestellt wurden oder herstellbar sind:

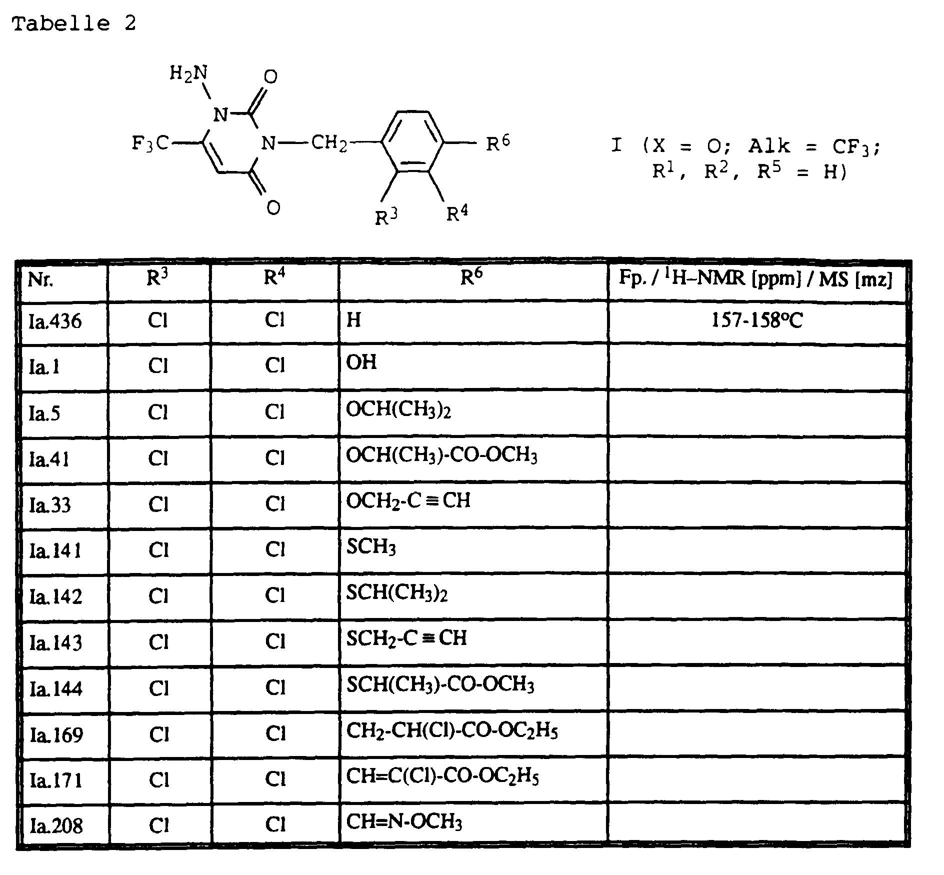

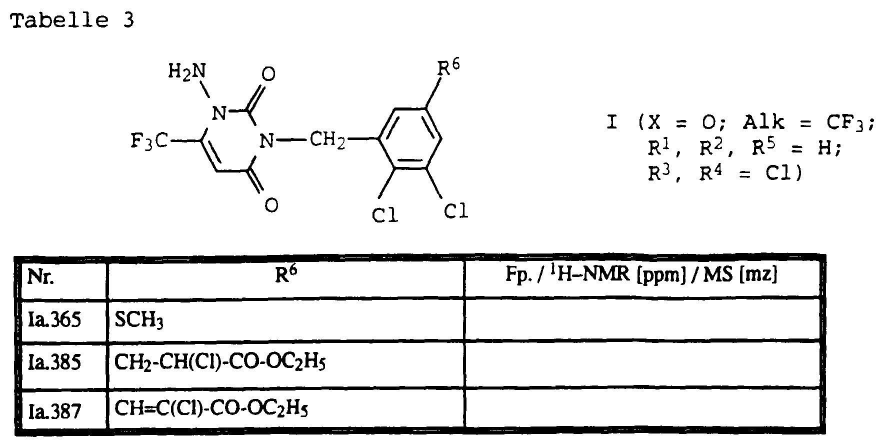

### Anwendungsbeispiele (herbizide Wirksamkeit)

Die herbizide Wirkung der 1-Amino-3-benzyluracile I ließ sich durch die folgenden Gewächshausversuche zeigen:

Als Kulturgefäße dienten Plastikblumentöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern, und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zweck der Nachauflaufbehandlung wurden die Testpflanzen je nach Wuchsform erst bis zu einer Wuchshöhe von 3 bis 15 cm angezogen und erst dann mit den in Wasser suspendierten oder emulgierten Wirkstoffen behandelt. Die Testpflanzen wurden dafür entweder direkt gesät und in den gleichen Gefäßen aufgezogen oder sie wurden erst als Keimpflanzen getrennt angezogen und einige Tage vor der Behandlung in die Versuchsgefäße verpflanzt. Die Aufwandmenge für die Nachauflaufbehandlung betrug 0,0312 oder 0,0156 kg/ha a.S. (aktive Substanz).

Die Pflanzen wurden artenspezifisch bei Temperaturen von 10 bis 25°C bzw. 20 bis 35°C gehalten. Die Versuchsperiode erstreckte sich über 2 bis 4 Wochen. Während dieser Zeit wurden die Pflanzen gepflegt, und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Lateinischer Name | Deutscher Name | Englischer Name |
|---|---|---|
| Abutilon theophrasti | Chinesischer Hanf | velvet leaf |
| Amaranthus retroflexus | Zurückgekrümmter Fuchsschwanz | redroot pigweed |
| Galium aparine | Klettenlabkraut | catchweed bedstraw |
| Ipomoea subspecies | Prunkwindearten | morningglory |

Bei einer Aufwandmenge von 0,0312 oder 0,0156 kg/ha a.S. zeigte die Verbindung Nr. Ia.436 im Nachauflaufverfahren eine sehr gute herbizide Wirkung gegen die o.g. Planzen.

### Anwendungsbeispiele (desikkative/defoliante Wirksamkeit)

Als Testpflanzen dienten junge, 4blättrige (ohne Keimblätter) Baumwollpflanzen, die unter Gewächshausbedingungen angezogen wurden (rel. Luftfeuchtigkeit 50 bis 70 %; Tag-/Nachttemperatur 27/20°C).

Die jungen Baumwollpflanzen wurden tropfnaß mit wässrigen Aufbereitungen der Wirkstoffe (unter Zusatz von 0,15 Gew.-% des Fettalkoholalkoxylats Plurafac® LF 700 ¹⁾, bezogen auf die Spritzbrühe) blattbehandelt. Die ausgebrachte Wassermenge betrug umgerechnet 1000 l/ha. Nach 13 Tagen wurde die Anzahl der abgeworfenen Blätter und der Grad der Entblätterung in % bestimmt.
¹⁾ ein schaumarmes, nichtionisches Tensid

Bei den unbehandelten Kontrollpflanzen trat kein Blattfall auf.

## Patentansprüche

1. 1-Amino-3-benzyluracile der allgemeinen Formel I 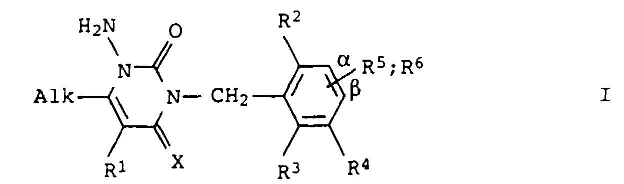 in der die Variablen folgende Bedeutung haben:
X Sauerstoff oder Schwefel;
Alk C₁-C₄-Halogenalkyl;
R¹ Wasserstoff oder Halogen;
R², R³ unabhängig voneinander Wasserstoff, Cyano, Thiocyanato, Halogen, C₁-C₄-Halogenalkyl, C₁-C₄-Halogenalkoxy oder C₁-C₄-Halogenalkylthio;
R⁴ Wasserstoff, Cyano, Thiocyanato, Halogen, C₁-C₄-Alkyl, C₁-C₄-Halogenalkyl, C₁-C₄-Alkoxy, C₁-C₄-Halogenalkoxy, C₁-C₄-Halogenalkylthio oder C₁-C₆-Alkylaminocarbonyl;
R⁵ an Position α oder β
Wasserstoff, Cyano, Nitro, Hydroxy, Amino, Halogen, C₁-C₄-Alkylamino, das durch C₁-C₄-Alkyl, (C₁-C₄-Alkyl)carboxyl oder (C₁-C₄-Alkoxy)carbonyl substituiert sein kann,
C₁-C₆-Halogenalkoxy, C₁-C₆-Halogenalkylthio, C₁-C₆-Alkoxy, C₁-C₆-Alkylthio, C₃-C₆-Cycloalkoxy, C₃-C₆-Cycloalkylthio, C₃-C₆-Alkenyloxy, C₃-C₆-Alkenylthio, C₃-C₆-Alkinyloxy, C₃-C₆-Alkinylthio, (C₁-C₆-Alkyl)carbonyloxy, (C₁-C₆-Alkyl)carbonylthio, (C₃-C₆-Alkenyl)carbonyloxy, (C₃-C₆-Alkenyl)carbonylthio, (C₃-C₆-Alkinyl)carbonyloxy, (C₃-C₆-Alkinyl)carbonylthio, C₁-C₆-Alkylsulfonyloxy oder C₁-C₆-Alkylsulfonyl, wobei jeder der letztgenannten 16 Reste gewünschtenfalls ein bis drei Substituenten tragen kann, jeweils ausgewählt aus der Gruppe bestehend aus
- Halogen, Nitro, Cyano, Hydroxy, C₃-C₆-Cycloalkyl, C₁-C₆-Alkoxy, C₃-C₆-Cycloalkoxy, C₃-C₆-Alkenyloxy, C₃-C₆-Alkinyloxy, C₁-C₆-Alkoxy-C₁-C₆-alkoxy, C₁-C₆-Alkylthio, C₁-C₆-Alkylsulfinyl, C₁-C₆-Alkylsulfonyl, C₁-C₆-Alkylidenaminoxy,
- der Phenyl-, Phenoxy- oder Phenylsulfonylgruppe, die unsubstituiert sein oder ein bis drei Substituenten tragen kann, jeweils ausgewählt aus der Gruppe bestehend aus Halogen, Nitro, Cyano, C₁-C₆-Alkyl, C₁-C₆-Alkoxy und C₁-C₆-Halogenalkyl,
- einer 3- bis 7gliedrigen Heterocyclyl- oder Heterocyclyloxygruppe mit ein bis drei Heteroatomen, ausgewählt aus der Gruppe bestehend aus zwei Sauerstoffatomen, zwei Schwefelatomen und 3 Stickstoffatomen, wobei der Heterocyclus gesättigt, partiell oder vollständig ungesättigt oder aromatisch sein und gewünschtenfalls ein bis drei Substituenten tragen kann, jeweils ausgewählt aus der Gruppe bestehend aus Halogen, Nitro, Cyano, C₁-C₆-Alkyl, C₁-C₆-Alkoxy, C₁-C₆-Halogenalkyl und (C₁-C₆-Alkyl)carbonyl,
- einer Gruppe -CO-R⁷, -COOR⁷, -COSR⁷, -CON(R⁷)R⁸, -OCO-R⁷, -OCOOR⁷, -OCOSR⁷, -OCON(R⁷)R⁸ oder -N(R⁷)R⁸, wobei
R⁷ für Wasserstoff, C₁-C₆-Alkyl, C₃-C₆-Cycloalkyl, C₃-C₆-Alkenyl, C₃-C₆-Alkinyl, C₁-C₆-Alkoxy-C₁-C₆-alkyl, (C₁-C₆-Alkoxy)carbonyl-C₁-C₆-alkyl, (C₃-C₆-Alkenyloxy)carbonyl-C₁-C₆-alkyl, Phenyl oder Phenyl-C₁-C₆-alkyl, wobei die Phenylgruppe und der Phenyl-Ring der Phenylalkylgruppe unsubstituiert sein oder einen bis drei Reste tragen können, jeweils ausgewählt aus der Gruppe bestehend aus Halogen, Nitro, Cyano, C₁-C₆-Alkyl, C₁-C₆-Halogenalkyl, C₁-C₆-Alkoxy und (C₁-C₆-Alkyl)carbonyl
und
R⁸ für Wasserstoff, Hydroxy, C₁-C₆-Alkyl, C₃-C₆-Cycloalkyl, C₁-C₆-Alkoxy, (C₁-C₆-Alkoxy) carbonyl-C₁-C₆-alkoxy, C₃-C₆-Alkenyl oder C₃-C₆-Alkenyloxy,
stehen, oder R⁷ und R⁸ bilden zusammen mit dem gemeinsamen Stickstoff einen 3- bis 7gliedrigen Heterocyclus mit ein bis drei Heteroatomen, ausgewählt aus einer Gruppe bestehend aus ein oder zwei Sauerstoffatomen, ein oder zwei Schwefelatomen und ein bis drei Stickstoffatomen, wobei der Heterocyclus gesättigt, partiell oder vollständig ungesättigt oder aromatisch sein und gewünschtenfalls ein bis drei Substituenten tragen kann, jeweils ausgewählt aus der Gruppe bestehend aus Halogen, Nitro, Cyano, C₁-C₆-Alkyl, C₁-C₆-Halogenalkyl C₁-C₆-Alkoxy und (C₁-C₆-Alkyl)carbonyl;
R⁶ an Position α, wobei R⁵ dann an Position β steht, oder an Position β, wobei R⁵ dann an Position α steht
1) Wasserstoff, Hydroxy, Mercapto, Halogen, C₁-C₆-Halogenalkoxy, C₁-C₆-Halogenalkylthio, C₁-C₆-Alkylthio-(C₁-C₆-alkyl)carbonyl, (C₁-C₆-Alkyl)iminooxycarbonyl,
2) C₁-C₆-Alkoxy, C₁-C₆-Alkylthio, C₃-C₆-Cycloalkoxy, C₃-C₆-Cycloalkylthio, C₂-C₆-Alkenyloxy, C₂-C₆-Alkenylthio, C₂-C₆-Alkinyloxy, C₂-C₆-Alkinylthio, (C₁-C₆-Alkyl)carbonyloxy, (C₁-C₆-Alkyl)carbonylthio, (C₁-C₆-Alkoxy)carboxyloxy, (C₂-C₆-Alkenyl)carbonyloxy, (C₂-C₆-Alkenyl)carbonylthio, (C₂-C₆-Alkinyl)carbonyloxy, (C₂-C₆-Alkinyl)carbonylthio, C₁-C₆-Alkylsulfonyloxy oder C₁-C₆-Alkylsulfonyl, wobei jeder der letztgenannten 17 Reste gewünschtenfalls ein bis drei Substituenten tragen kann, jeweils ausgewählt aus der Gruppe bestehend aus
- Halogen, Nitro, Cyano, Hydroxy, C₃-C₆-Cycloalkyl, C₁-C₆-Alkoxy, C₃-C₆-Cycloalkoxy, C₃-C₆-Alkenyloxy, C₃-C₆-Alkinyloxy, C₁-C₆-Alkoxy-C₁-C₆-alkoxy, C₁-C₆-Alkylthio, C₁-C₆-Alkylsulfinyl, C₁-C₆-Alkylsulfonyl, C₁-C₆-Alkylidenaminoxy,
- der Phenyl-, Phenoxy- oder Phenylsulfonylgruppe, die unsubstituiert sein oder ein bis drei Substituenten tragen kann, jeweils ausgewählt aus der Gruppe bestehend aus Halogen, Nitro, Cyano, C₁-C₆-Alkyl, C₁-C₆-Halogenalkyl, C₁-C₆-Alkoxy und (C₁-C₆-Alkoxy)carbonyl,
- einer 3- bis 7gliedrigen Heterocyclyl- oder Heterocyclyloxygruppe mit ein bis drei Heteroatomen, ausgewählt aus der Gruppe bestehend aus zwei Sauerstoffatomen, zwei Schwefelatomen und 3 Stickstoffatomen, wobei der Heterocyclus gesättigt, partiell oder vollständig ungesättigt oder aromatisch sein und gewünschtenfalls ein bis drei Substituenten tragen kann, jeweils ausgewählt aus der Gruppe bestehend aus Halogen, Nitro, Cyano, C₁-C₆-Alkyl, C₁-C₆-Alkoxy, C₁-C₆-Halogenalkyl und (C₁-C₆-Alkyl)carbonyl,
- einer Gruppe -CO-R⁹, -COOR⁹, -COSR⁹, -CON(R⁹)R¹⁰, -OCO-R⁹, -OCOOR⁹, -OCOSR⁹, -OCON(R⁹)R¹⁰ oder -N(R⁹)R¹⁰, wobei R⁹ für eine der Bedeutungen von R⁷ und R¹⁰ für eine der Bedeutungen von R⁸ steht,
- der Gruppe -C(R²¹)=N-OR²⁰;
3) -CY-R¹¹, -C(R¹¹) (Z¹R¹²) (Z²R¹³), wobei Z¹ und Z² jeweils für Sauerstoff oder Schwefel stehen, -C(R¹¹)=C(R¹⁴)-CN, -C(R¹¹)=C(R¹⁴)-CO-R¹⁵, -CH(R¹¹)-CH(R¹⁴)-CO-R¹⁵, -C(R¹¹)=C(R¹⁴)-CH₂-CO-R¹⁵, -C(R¹¹)=C(R¹⁴)-C(R¹⁶)=C(R¹⁷)-CO-R¹⁵, -C(R¹¹)=C(R¹⁴)-CH₂-CH(R¹⁸)-CO-R¹⁵, CO-OR¹⁹, -CO-SR¹⁹, -CO-N(R¹⁹)-OR²⁰, -C=C-CO-NH-OR²⁰, -C≡C-CO-N(R¹⁹)-OR²⁰, -C≡C-CS-NH-OR²⁰, -C≡C-CS-N(R¹⁹)-OR²⁰, -C(R¹¹)=C(R¹⁴)-CO-NH-OR²⁰, - C(R¹¹)=C(R¹⁴)-CO-N(R¹⁹)-OR²⁰, -C(R¹¹)=C(R¹⁴)-CS-NH-OR²⁰, -C(R¹¹)=C(R¹⁴)-CS-N(R¹⁹)-OR²⁰, -C(R¹¹)=C(R¹⁴)-C(R²¹)=N-OR²⁰, -C(R²¹)=N-OR²⁰, -C≡C-C(R²¹)=N-OR²⁰, -C(Z¹R¹²)(Z²R¹³)-OR¹⁹, -C(Z¹R¹²)(Z²R¹³)-SR¹⁹, -C(Z¹R¹²)(Z²R¹³)-N(R²³)R²⁴, -N(R²³)R^{24,} -CON(R²³)R²⁴, 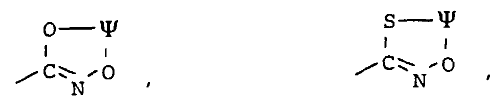 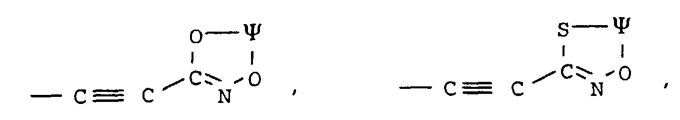 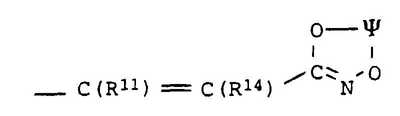 oder 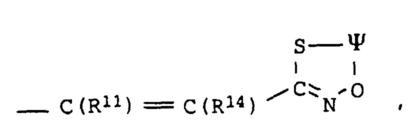 wobei Ψ für C₁-C₃-Alkylen steht, das einen C₁-C₆-Alkylsubstituenten tragen kann;
R¹¹ Wasserstoff, Cyano, C₁-C₆-Alkyl, C₁-C₆-Halogenalkyl, C₂-C₆-Alkenyl, C₂-C₆-Alkinyl, C₃-C₆-Cycloalkyl, C₁-C₆-Alkoxy-C₁-C₆-alkyl oder (C₁-C₆-Alkoxy)carbonyl;
R¹², R¹³ unabhängig voneinander C₁-C₆-Alkyl, C₁-C₆-Halogenalkyl, C₃-C₆-Alkenyl, C₃-C₆-Alkinyl, C₁-C₆-Alkoxy-C₁-C₆-alkyl oder
R¹² und R¹³ zusammen eine gesättigte oder ungesättigte, 2- bis 4gliedrige Kohlenstoffkette, die einen Oxosubstituenten tragen kann, wobei ein Glied dieser Kette durch ein den Variablen Z¹ und Z² nicht benachbartes Sauerstoff-, Schwefel- oder Stickstoffatom ersetzt sein kann, und wobei die Kohlenstoffkette noch ein bis drei Reste tragen kann, jeweils ausgewählt aus der Gruppe bestehend aus Cyano, Nitro, Amino, Halogen, C₁-C₆-Alkyl, C₂-C₆-Alkenyl, C₁-C₆-Alkoxy, C₂-C₆-Alkenyloxy, C₂-C₆-Alkinyloxy, C₁-C₆-Halogenalkyl, Cyano-C₁-C₆-alkyl, Hydroxy-C₁-C₆-alkyl, C₁-C₆-Alkoxy-C₁-C₆-alkyl, C₃-C₆-Alkenyloxy-C₁-C₆-alkyl, C₃-C₆-Alkinyloxy-C₁-C₆-alkyl, C₃-C₆-Cycloalkyl, C₃-C₆-Cycloalkoxy, Carboxy, (C₁-C₆-Alkoxy)carbonyl, (C₁-C₆-Alkyl)carbonyloxy-C₁-C₆-alkyl und Phenyl, das seinerseits unsubstituiert sein oder einen bis drei Substituenten tragen kann, jeweils ausgewählt aus der Gruppe bestehend aus Cyano, Nitro, Amino, Halogen, C₁-C₆-Alkyl, C₁-C₆-Halogenalkyl, C₁-C₆-Alkoxy und (C₁-C₆-Alkoxy)carbonyl, und wobei die Kohlenstoffkette auch durch einen ankondensierten oder spiroverknüpften 3- bis 7gliedrigen Ring substituiert sein kann, der ein oder zwei Heteroatome als Ringglieder enthalten kann, ausgewählt aus Sauerstoff, Schwefel, Stickstoff und durch C₁-C₆-Alkyl substituiertem Stickstoff, und der gewünschtenfalls seinerseits einen oder zwei der folgenden Substituenten tragen kann: Cyano, C₁-C₆-Alkyl, C₂-C₆-Alkenyl, C₁-C₆-Alkoxy, Cyano-C₁-C₆-alkyl, C₁-C₆-Halogenalkyl und (C₁-C₆-Alkoxy)carbonyl;
R¹⁴ Wasserstoff, Cyano, Halogen, C₁-C₆-Alkyl, C₁-C₆-Halogenalkyl, C₁-C₆-Alkoxy, (C₁-C₆-Alkyl)carbonyl oder (C₁-C₆-Alkoxy)carbonyl;
R¹⁵ Wasserstoff, O-R²², S-R²², C₁-C₆-Alkyl, das noch einen oder zwei C₁-C₆-Alkoxysubstituenten tragen kann, C₂-C₆-Alkenyl, C₂-C₆-Alkinyl, C₁-C₆-Halogenalkyl, C₃-C₆-Cycloalkyl, C₁-C₆-Alkylthio-C₁-C₆-alkyl, C₁-C₆-Alkyliminooxy, -N(R²³)R²⁴ oder Phenyl, das unsubstituiert sein oder ein bis drei Substituenten tragen kann, jeweils ausgewählt aus der Gruppe bestehend aus Cyano, Nitro, Halogen, C₁-C₆-Alkyl, C₂-C₆-Alkenyl, C₁-C₆-Halogenalkyl, C₁-C₆-Alkoxy und (C₁-C₆-Alkoxy)carbonyl,
wobei R²² für eine der Bedeutungen von R¹⁹ steht;
R¹⁶ Wasserstoff, Cyano, Halogen, C₁-C₆-Alkyl, C₃-C₆-Alkenyl, C₃-C₆-Alkinyl, C₁-C₆-Alkoxy-C₁-C₆-alkyl, (C₁-C₆-Alkyl)carbonyl, (C₁-C₆-Alkoxy)carbonyl, -N(R²⁵)R²⁶, wobei R²⁵ und R²⁶ für eine der Bedeutungen von R²³ und R²⁴ stehen, oder
Phenyl, das seinerseits noch einen bis drei Substituenten tragen kann, jeweils ausgewählt aus der Gruppe bestehend aus Cyano, Nitro, Halogen, C₁-C₆-Alkyl, C₁-C₆-Halogenalkyl, C₃-C₆-Alkenyl, C₁-C₆-Alkoxy und (C₁-C₆-Alkoxy)carbonyl;
R¹⁷ Wasserstoff, Cyano, Halogen, C₁-C₆-Alkyl, C₁-C₆-Alkoxy, C₁-C₆-Halogenalkyl, (C₁-C₆-Alkyl)carbonyl oder (C₁-C₆-Alkoxy)carbonyl;
R¹⁸ Wasserstoff, Cyano, C₁-C₆-Alkyl oder (C₁-C₆-Alkoxy)carbonyl;
R¹⁹ Wasserstoff, C₁-C₆-Alkyl, C₁-C₆-Halogenalkyl, C₂-C₆-Alkenyl oder C₂-C₆-Alkinyl, wobei die letztgenannten 4 Gruppen jeweils einen oder zwei der folgenden Reste tragen können: Cyano, Halogen, Hydroxy, Hydroxycarbonyl, C₁-C₆-Alkoxy, C₁-C₆-Alkylthio, (C₁-C₆-Alkyl)carbonyl, (C₁-C₆-Alkoxy)carbonyl, (C₁-C₆-Alkyl)carbonyloxy, (C₃-C₆-Alkenyloxy)carbonyl oder einen mit dem Stickstoffatom über eine Carbonylbrücke gebundenen 3- bis 7gliedrigen Azaheterocyclus, der neben Kohlenstoffringgliedern noch ein Sauerstoff- oder Schwefelatom als Ringglied enthalten kann;
oder (C₁-C₆-Alkyl) carbonyl, (C₁-C₆-Halogenalkyl)carbonyl, (C₁-C₆-Alkoxy) carbonyl, C₁-C₆-Alkylaminocarbonyl, Di-(C₁-C₆-alkyl)aminocarbonyl, C₁-C₆-Alkyloximino-C₁-C₆-alkyl, C₃-C₆-Cycloalkyl,
Phenyl oder Phenyl-C₁-C₆-alkyl, wobei die Phenylringe unsubstituiert sein oder ihrerseits ein bis drei Substituenten tragen können, jeweils ausgewählt aus der Gruppe bestehend aus Cyano, Nitro, Halogen, C₁-C₆-Alkyl, C₁-C₆-Halogenalkyl, C₁-C₆-Alkoxy und (C₁-C₆-Alkoxy)carbonyl;
R²⁰ Wasserstoff, C₁-C₆-Alkyl, C₁-C₆-Halogenalkyl, C₃-C₆-Cycloalkyl, C₃-C₆-Alkenyl, C₃-C₆-Alkinyl, Hydroxy-C₁-C₆-alkyl, C₁-C₆-Alkoxy-C₁-C₆-alkyl, C₁-C₆-Alkylthio-C₁-C₆-alkyl, Cyano-C₁-C₆-alkyl, (C₁-C₆-Alkyl)carbonyl-C₁-C₆-alkyl, (C₁-C₆-Alkoxy)carbonyl-C₁-C₆-alkyl, (C₁-C₆-Alkoxy)carbonyl-C₂-C₆-alkenyl, (C₁-C₆-Alkyl)carbonyloxy-C₁-C₆-alkyl oder Phenyl-C₁-C₆-alkyl, wobei der Phenylring gewünschtenfalls ein bis drei Substituenten tragen kann, jeweils ausgewählt aus der Gruppe bestehend aus Cyano, Nitro, Halogen, C₁-C₆-Alkyl, C₁-C₆-Halogenalkyl, C₁-C₆-Alkoxy und (C₁-C₆-Alkoxy)carbonyl;
R²¹
- Wasserstoff, Halogen,
- C₁-C₆-Alkyl, C₁-C₆-Halogenalkyl, C₁-C₆-Alkoxy, C₁-C₆-Halogenalkoxy, C₃-C₆-Alkenyloxy, C₁-C₆-Alkylthio, C₁-C₆-Halogenalkylthio, (C₁-C₆-Alkyl)carbonyloxy, (C₁-C₆-Halogenalkyl)carbonyloxy, C₁-C₆-Alkylsulfonyloxy oder C₁-C₆-Halogenalkylsulfonyloxy, wobei die letztgenannten 11 Reste einen der folgenden Substituenten tragen können: Hydroxy, Cyano, Hydroxycarbonyl, C₁-C₆-Alkoxy, C₁-C₆-Alkylthio, (C₁-C₆-Alkyl)carbonyl, (C₁-C₆-Alkoxy)carbonyl, (C₁-C₆-Alkyl)aminocarbonyl, Di-(C₁-C₆-alkyl)aminocarbonyl, (C₁-C₆-Alkyl) carbonyloxy,
- einen mit dem Stickstoffatom über eine Carbonylbrücke gebundenen 3- bis 7gliedrigen Azaheterocyclus, der neben Kohlenstoffringgliedern noch ein Sauerstoff- oder Schwefelatom als Ringglied enthalten kann,
- (C₁-C₆-Alkyl)carbonyl, (C₁-C₆-Halogenalkyl)carbonyl, (C₁-C₆-Alkoxy)carbonyl, (C₁-C₆-Alkoxy)carbonyloxy, (C₁-C₆-Alkyl)carbonylthio, (C₁-C₆-Halogenalkyl)carbonylthio, (C₁-C₆-Alkoxy)carbonylthio, C₂-C₆-Alkenyl, C₂-C₆-Alkenylthio, C₃-C₆-Alkinyl, C₃-C₆-Alkinyloxy, C₃-C₆-Alkinylthio, (C₂-C₆-Alkinyl)carbonyloxy, C₃-C₆-Alkinylsulfonyloxy, C₃-C₆-Cycloalkyl, C₃-C₆-Cycloalkyloxy, C₃-C₆-Cycloalkylthio, (C₃-C₆-Cycloalkyl)carbonyloxy, C₃-C₆-Cycloalkylsulfonyloxy,
- Phenyl, Phenoxy, Phenylthio, Benzoyloxy, Phenylsulfonyloxy, Phenyl-C₁-C₆-alkyl, Phenyl-C₁-C₆-alkoxy, Phenyl-C₁-C₆-alkylthio, Phenyl-(C₁-C₆-alkyl)carbonyloxy oder Phenyl-(C₁-C₆-alkyl) sulfonyloxy, wobei die Phenylringe der letztgenannten 10 Reste unsubstituiert sein oder ihrerseits ein bis drei Substituenten tragen können, jeweils ausgewählt aus der Gruppe bestehend aus Cyano, Nitro, Halogen, C₁-C₆-Alkyl, C₁-C₆-Halogenalkyl, C₁-C₆-Alkoxy und (C₁-C₆-Alkoxy)carbonyl;
R²³, R²⁴ unabhängig voneinander
Wasserstoff, C₁-C₆-Alkyl, C₂-C₆-Alkenyl, C₂-C₆-Alkinyl, C₃-C₆-Cycloalkyl, C₁-C₆-Halogenalkyl, C₁-C₆-Alkoxy-C₁-C₆-alkyl, (C₁-C₆-Alkyl)carbonyl, (C₁-C₆-Alkoxy)carbonyl, (C₁-C₆-Alkoxy) carbonyl-C₁-C₆-alkyl, (C₁-C₆-Alkoxy)carbonyl-C₂-C₆-alkenyl, wobei die Alkenylkette zusätzlich ein bis drei Halogen- und/oder Cyano-Reste tragen kann,
oder für C₁-C₆-Alkylsulfonyl, (C₁-C₆-Alkoxy)carbonyl-C₁-C₆-alkylsulfonyl, Phenyl oder Phenylsulfonyl, wobei die beiden Phenylringe unsubstituiert sein oder ihrerseits einen bis drei Substituenten tragen können, jeweils ausgewählt aus der Gruppe bestehend aus Cyano, Nitro, Halogen, C₁-C₆-Alkyl, C₁-C₆-Halogenalkyl, C₃-C₆-Alkenyl, C₁-C₆-Alkoxy und (C₁-C₆-Alkoxy)carbonyl,
oder R²³ und R²⁴ zusammen mit dem gemeinsamen Stickstoffatom einen gesättigten oder ungesättigten 4- bis 7gliedrigen Azaheterocyclus, der neben Kohlenstoffringgliedern gewünschtenfalls eines der folgenden Glieder enthalten kann: -O-, -S-, -N=, -NH- oder -N(C₁-C₆-Alkyl)-;
Y Sauerstoff, Schwefel oder -N(R²⁷)-;
R²⁷ Wasserstoff, Hydroxy, C₁-C₆-Alkyl, C₃-C₆-Alkenyl, C₃-C₆-Alkinyl, C₃-C₇-Cycloalkyl, C₁-C₆-Halogenalkyl, C₁-C₆-Alkoxy-C₁-C₆-alkyl, C₁-C₆-Alkoxy, C₃-C₆-Alkenyloxy, C₃-C₆-Alkinyloxy, C₃-C₆-Cycloalkoxy, C₅-C₇-Cycloalkenyloxy, C₁-C₆-Halogenalkoxy, C₃-C₆-Halogenalkenyloxy, Hydroxy-C₁-C₆-alkoxy, Cyano-C₁-C₆-alkoxy, C₃-C₆-Cycloalkyl-C₁-C₆-alkoxy, C₁-C₆-Alkoxy-C₁-C₆-alkoxy,
C₁-C₆-Alkoxy-C₃-C₆-alkenyloxy, (C₁-C₆-Alkyl)carbonyloxy, (C₁-C₆-Halogenalkyl)carbonyloxy, (C₁-C₆-Alkyl)carbamoyloxy, (C₁-C₆-Halogenalkyl)carbamoyloxy, (C₁-C₆-Alkyl)carbonyl-C₁-C₆-alkyl, (C₁-C₆-Alkyl)carbonyl-C₁-C₆-alkoxy, (C₁-C₆-Alkoxy)carbonyl-C₁-C₆-alkyl, (C₁-C₆-Alkoxy)carbonyl-C₁-C₆-alkoxy, C₁-C₆-Alkylthio-C₁-C₆-alkoxy, Di-(C₁-C₆-alkyl)amino-C₁-C₆-alkoxy,
Phenyl, das seinerseits noch einen bis drei Substituenten tragen kann, jeweils ausgewählt aus der Gruppe bestehend aus Cyano, Nitro, Halogen, C₁-C₆-Alkyl, C₁-C₆-Halogenalkyl, C₂-C₆-Alkenyl, C₁-C₆-Alkoxy und (C₁-C₆-Alkoxy)carbonyl, Phenyl-C₁-C₆-alkoxy, Phenyl-C₃-C₆-alkenyloxy oder Phenyl-C₃-C₆-alkinyloxy, wobei jeweils eine oder zwei Methylengruppen der Kohlenstoffketten durch -O- , -S- oder -N(C₁-C₆-Alkyl)- ersetzt sein können und wobei jeder Phenylring unsubstituiert oder seinerseits einen bis drei Substituenten tragen kann, jeweils ausgewählt aus der Gruppe bestehend aus Cyano, Nitro, Halogen, C₁-C₆-Alkyl, C₁-C₆-Halogenalkyl, C₂-C₆-Alkenyl, C₁-C₆-Alkoxy und (C₁-C₆-Alkoxy)carbonyl,
Heterocyclyl, Heterocyclyl-C₁-C₆-alkoxy, Heterocyclyl-C₃-C₆-alkenyloxy oder Heterocyclyl-C₃-C₆-alkinyloxy, wobei jeweils eine oder zwei Methylengruppen der Kohlenstoffketten durch -O- , -S- oder -N(C₁-C₆-Alkyl)- ersetzt sein können und wobei jeder Heterocyclus 3- bis 7gliedrig,
gesättigt, ungesättigt oder aromatisch sein kann und ein bis vier Heteroatome enthält, ausgewählt aus der Gruppe bestehend aus zwei Sauerstoffatomen, zwei Schwefelatomen und 4 Stickstoffatomen,
und entweder unsubstituiert ist oder seinerseits einen bis drei Substituenten trägt, jeweils ausgewählt aus der Gruppe bestehend aus Cyano, Nitro, Halogen, C₁-C₆-Alkyl, C₁-C₆-Halogenalkyl, C₂-C₆-Alkenyl, C₁-C₆-Alkoxy und (C₁-C₆-Alkoxy)carbonyl,
oder -N(R²⁸)R²⁹, wobei R²⁸ und R²⁹ jeweils für Wasserstoff, C₁-C₆-Alkyl, C₃-C₆-Alkenyl, C₃-C₆-Alkinyl, C₃-C₆-Cycloalkyl, C₁-C₆-Halogenalkyl, C₁-C₆-Alkoxy-C₁-C₆-alkyl, (C₁-C₆-Alkyl)carbonyl, (C₁-C₆-Alkoxy)carbonyl, (C₁-C₆-Alkoxy)carbonyl-C₁-C₆-alkyl,
(C₁-C₆-Alkoxy)carbonyl-C₂-C₆-alkenyl, wobei die Alkenylkette zusätzlich ein bis drei Halogen- und/oder Cyano-Reste tragen kann,
oder für Phenyl, das unsubstituiert sein oder seinerseits noch einen bis drei Substituenten tragen kann, jeweils ausgewählt aus der Gruppe bestehend aus Cyano, Nitro,
Halogen, C₁-C₆-Alkyl, C₁-C₆-Halogenalkyl, C₃-C₆-Alkenyl, C₁-C₆-Alkoxy und (C₁-C₆-Alkoxy)carbonyl,
stehen,
oder wobei R²⁸ und R²⁹ zusammen mit dem gemeinsamen Stickstoffatom einen gesättigten oder ungesättigten 4- bis 7gliedrigen Heterocyclus bilden, der neben Kohlenstoffringgliedern gewünschtenfalls noch eines der folgenden Glieder enthalten kann: -O-, -S-, -N=, -NH- oder -N(C₁-C₆-Alkyl)-;
sowie die landwirtschaftlich brauchbaren Salze der Verbindungen I.

2. Verwendung der 1-Amino-3-benzyluracile der Formel I und ihrer landwirtschaftlich brauchbaren Salze, gemäß Anspruch 1, als Herbizide oder zur Desikkation/Defoliation von Pflanzen.

3. Herbizides Mittel, enthaltend eine herbizid wirksame Menge mindestens eines 1-Amino-3-benzyluracils der Formel I oder eines landwirtschaftlich brauchbaren Salzes von I, gemäß Anspruch 1, und mindestens einen inerten flüssigen und/oder festen Trägerstoff sowie gewünschtenfalls mindestens einen oberflächenaktiven Stoff.

4. Mittel zur Desikkation und/oder Defoliation von Pflanzen, enthaltend eine desikkant und/oder defoliant wirksame Menge mindestens eines 1-Amino-3-benzyluracils der Formel I oder eines landwirtschaftlich brauchbaren Salzes von I, gemäß Anspruch 1, und mindestens einen inerten flüssigen und/oder festen Trägerstoff sowie gewünschtenfalls mindestens einen oberflächenaktiven Stoff.

5. Verfahren zur Herstellung von herbizid wirksamen Mitteln, dadurch gekennzeichnet, daß man eine herbizid wirksame Menge mindestens eines 1-Amino-3-benzyluracils der Formel I oder eines landwirtschaftlich brauchbaren Salzes von I, gemäß Anspruch 1, und mindestens einen inerten flüssigen und/oder festen Trägerstoff sowie gewünschtenfalls mindestens einen oberflächenaktiven Stoff mischt.

6. Verfahren zur Herstellung von desikkant und/oder defoliant wirksamen Mitteln, dadurch gekennzeichnet, daß man eine desikkant und/oder defoliant wirksame Menge mindestens eines 1-Amino-3-benzyluracils der Formel I oder eines landwirtschaftlich brauchbaren Salzes von I, gemäß Anspruch 1, und mindestens einen inerten flüssigen und/oder festen Trägerstoff sowie gewünschtenfalls mindestens einen oberflächenaktiven Stoff mischt.

7. Verfahren zur Bekämpfung von unerwünschtem Pflanzenwuchs, dadurch gekennzeichnet, daß man eine herbizid wirksame Menge mindestens eines 1-Amino-3-benzyluracils der Formel I oder eines landwirtschaftlich brauchbaren Salzes von I, gemäß Anspruch 1, auf Pflanzen, deren Lebensraum oder auf Saatgut einwirken läßt.

8. Verfahren zur Desikkation und/oder Defoliation von Pflanzen, dadurch gekennzeichnet, daß man eine desikkant und/oder defoliant wirksame Menge mindestens eines 1-Amino-3-benzyluracils der Formel I oder eines landwirtschaftlich brauchbaren Salzes von I, gemäß Anspruch 1, auf Pflanzen einwirken läßt.

9. Verfahren nach Anspruch 8, dadurch gekennzeichnet, daß man Baumwolle behandelt.

10. Verfahren zur Herstellung von 1-Amino-3-benzyluracilen der Formel I gemäß Anspruch 1, wobei X für Sauerstoff steht, dadurch gekennzeichnet, daß man einen Enaminester der Formel III 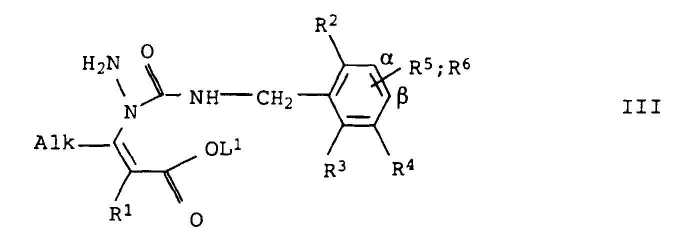 oder ein Enamincarboxylat der Formel IV 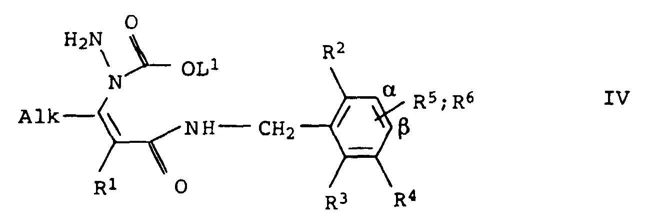 wobei L¹ jeweils für niedermolekulares Alkyl oder Phenyl steht und die Substituenten Alk und R¹ bis R⁶ die in Anspruch 1 angegebenen Bedeutungen haben,
in Gegenwart einer Base cyclisiert.

11. Verfahren zur Herstellung von 1-Amino-3-benzyluracilen der Formel I gemäß Anspruch 1, dadurch gekennzeichnet, daß man ein 1-H-3-Phenyluracil der Formel V 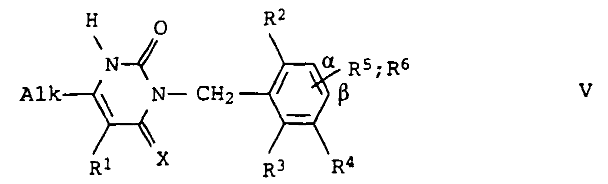 in der die Substituenten Alk und R¹ bis R⁶ die in Anspruch 1 angegebenen Bedeutungen haben,
in Gegenwart einer Base mit einem elektrophilen Aminierungsreagenz umsetzt.

12. Verfahren zur Herstellung von 1-Amino-3-benzyluracilen der Formel I gemäß Anspruch 1, dadurch gekennzeichnet, daß man ein 1-Amino-6-halogenalkyluracil der Formel VIII 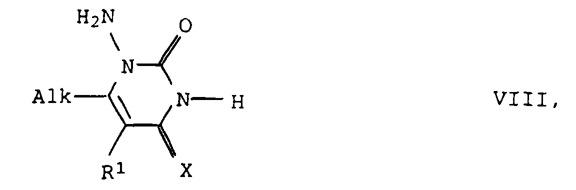 wobei Alk und R¹ die in Anspruch 1 angegebenen Bedeutungen haben, in Gegenwart einer Base, oder ein Alkalimetallsalz von VIII, mit einem Benzylhalogenid der Formel IX 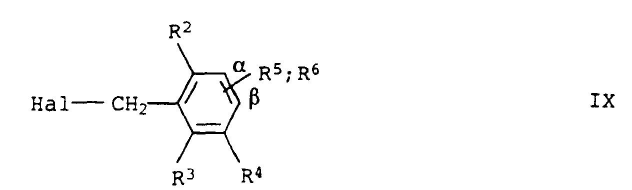 wobei Hal für Halogen steht und die Substituenten R² bis R⁶ die in Anspruch 1 angegebenen Bedeutungen haben, umsetzt.

13. Enaminester der Formel III 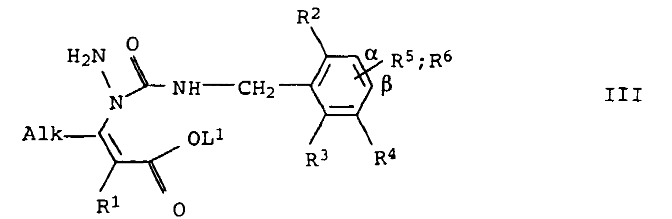 in der L¹ für C₁-C₆-Alkyl oder Phenyl steht und die Substituenten Alk und R¹ bis R⁶ die in Anspruch 1 angegebenen Bedeutungen haben.

14. Enamincarboxylate der Formel IV 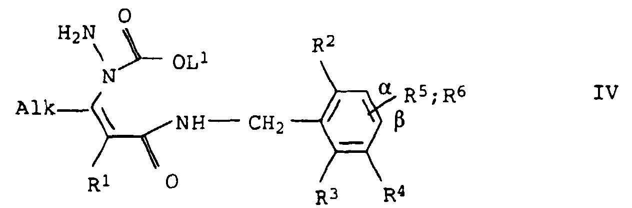 in der L¹ für C₁-C₆-Alkyl oder Phenyl steht und die Substituenten Alk und R¹ bis R⁶ die in Anspruch 1 angegebenen Bedeutungen haben.

## Claims

1. A 1-amino-3-benzyluracil of the formula I 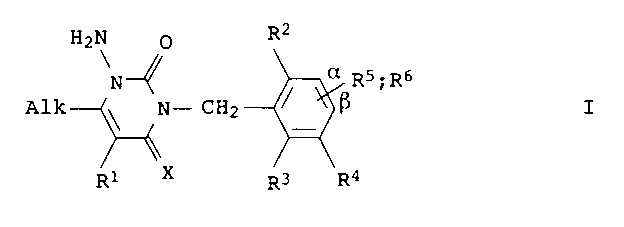 where the variables have the following meanings:
X is oxygen or sulfur;
Alk is C₁-C₄-haloalkyl;
R¹ is hydrogen or halogen;
R² and R³ independently of one another are hydrogen, cyano, thiocyanato, halogen, C₁-C₄-haloalkyl, C₁-C₄-haloalkoxy or C₁-C₄-haloalkylthio;
R⁴ is hydrogen, cyano, thiocyanato, halogen, C₁-C₄-alkyl, C₁-C₄-haloalkyl, C₁-C₄-alkoxy, C₁-C₄-haloalkoxy, C₁-C₄-haloalkylthio or C₁-C₆-alkylaminocarbonyl;
R⁵ in the a or β position is hydrogen, cyano, nitro, hydroxyl, amino, halogen, C₁-C₄-alkylamino which can be substituted by C₁-C₄-alkyl, (C₁-C₄-alkyl)carboxyl or (C₁-C₄-alkoxy)carbonyl, C₁-C₆-haloalkoxy, C₁-C₆-haloalkylthio, C₁-C₆-alkoxy, C₁-C₆-alkylthio, C₃-C₆-cycloalkoxy, C₃-C₆-cycloalkylthio, C₃-C₆-alkenyloxy, C₃-C₆-alkenylthio, C₃-C₆-alkynyloxy, C₃-C₆-alkynylthio, (C₁-C₆-alkyl)carbonyloxy, (C₁-C₆-alkyl)carbonylthio, (C₃-C₆-alkenyl)carbonyloxy, (C₃-C₆-alkenyl)carbonylthio, (C₃-C₆-alkynyl)carbonyloxy, (C₃-C₆-alkynyl)carbonylthio, C₁-C₆-alkylsulfonyloxy or C₁-C₆-alkylsulfonyl, it being possible, if desired, for each of the last-mentioned 16 radicals to have attached to them one to three substituents, in each case selected from the group consisting of
- halogen, nitro, cyano, hydroxyl, C₃-C₆-cycloalkyl, C₁-C₆-alkoxy, C₃-C₆-cycloalkoxy, C₃-C₆-alkenyloxy, C₃-C₆-alkynyloxy, C₁-C₆-alkoxy-C₁-C₆-alkoxy, C₁-C₆-alkylthio, C₁-C₆-alkylsulfinyl, C₁-C₆-alkylsulfonyl, C₁-C₆-alkylideneaminoxy,
- the phenyl, phenoxy or phenylsulfonyl group which can be unsubstituted or have attached to it one to three substituents, in each case selected from the group consisting of halogen, nitro, cyano, C₁-C₆-alkyl, C₁-C₆-alkoxy and C₁-C₆-haloalkyl,
- a 3- to 7-membered heterocyclyl or heterocyclyloxy group having one to three hetero atoms selected from the group consisting of two oxygen atoms, two sulfur atoms and three nitrogen atoms, it being possible for the heterocycle to be saturated, partially or fully unsaturated or aromatic and, if desired, to have attached to it one to three substituents in each case selected from the group consisting of halogen, nitro, cyano, C₁-C₆-alkyl, C₁-C₆-alkoxy, C₁-C₆-haloalkyl and (C₁-C₆-alkyl)carbonyl,
- a group -CO-R⁷, -COOR⁷, -COSR⁷, -CON(R⁷)R⁸, -OCO-R⁷, -OCOOR⁷, -OCOSR⁷, -OCON(R⁷)R⁸ or -N(R⁷)R⁸ where
R⁷ is hydrogen, C₁-C₆-alkyl, C₃-C₆-cycloalkyl, C₃-C₆-alkenyl, C₃-C₆-alkynyl, C₁-C₆-alkoxy-C₁-C₆-alkyl,
(C₁-C₆-alkoxy)carbonyl-C₁-C₆-alkyl,
(C₃-C₆-alkenyloxy)carbonyl-C₁-C₆-alkyl, phenyl or phenyl-C₁-C₆-alkyl, it being possible for the phenyl group and the phenyl ring of the phenyl alkyl group to be unsubstituted or to have attached to them one to three radicals, in each case selected from the group consisting of halogen, nitro, cyano, C₁-C₆-alkyl, C₁-C₆-haloalkyl, C₁-C₆-alkoxy and (C₁-C₆-alkyl)carbonyl
and
R⁸ is hydrogen, hydroxyl, C₁-C₆-alkyl, C₃-C₆-cycloalkyl, C₁-C₆-alkoxy, (C₁-C₆-alkoxy)carbonyl-C₁-C₆-alkoxy, C₃-C₆-alkenyl or C₃-C₆-alkenyloxy,
or R⁷ and R⁸ together with the joint nitrogen form a 3- to 7-membered heterocycle having one to three hetero atoms selected from a group consisting of one or two oxygen atoms, one or two sulfur atoms and one to three nitrogen atoms, it being possible for the heterocycle to be saturated, partially or fully unsaturated or aromatic and, if desired, to have attached to it one to three substituents, in each case selected from the group consisting of halogen, nitro, cyano, C₁-C₆-alkyl, C₁-C₆-haloalkyl C₁-C₆-alkoxy and (C₁-C₆-alkyl)carbonyl;
R⁶ in the a position, in which case R⁵ is in the β position, or in the β position, in which case R⁵ is in the a position, is
1) hydrogen, hydroxyl, mercapto, halogen, C₁-C₆-haloalkoxy, C₁-C₆-haloalkylthio, C₁-C₆-alkylthio-(C₁-C₆-alkyl)carbonyl, (C₁-C₆-alkyl)iminooxycarbonyl,
2) C₁-C₆-alkoxy, C₁-C₆-alkylthio, C₃-C₆-cycloalkoxy, C₃-C₆-cycloalkylthio, C₂-C₆-alkenyloxy, C₂-C₆-alkenylthio, C₂-C₆-alkynyloxy, C₂-C₆-alkynylthio, (C₁-C₆-alkyl)carbonyloxy, (C₁-C₆-alkyl)carbonylthio, (C₁-C₆-alkoxy)carboxyloxy, (C₂-C₆-alkenyl)carbonyloxy, (C₂-C₆-alkenyl)carbonylthio, (C₂-C₆-alkynyl)carbonyloxy, (C₂-C₆-alkynyl)carbonylthio, C₁-C₆-alkylsulfonyloxy or C₁-C₆-alkylsulfonyl, it being possible for each of the last-mentioned 17 radicals, if desired, to have attached to it one to three substituents, in each case selected from the group consisting of
- halogen, nitro, cyano, hydroxyl, C₃-C₆-cycloalkyl, C₁-C₆-alkoxy, C₃-C₆-cycloalkoxy, C₃-C₆-alkenyloxy, C₃-C₆-alkynyloxy, C₁-C₆-alkoxy-C₁-C₆-alkoxy, C₁-C₆-alkylthio, C₁-C₆-alkylsulfinyl, C₁-C₆-alkylsulfonyl, C₁-C₆-alkylideneaminoxy,
- the phenyl, phenoxy or phenylsulfonyl group which can be unsubstituted or have attached to it one to three substituents, in each case selected from the group consisting of halogen, nitro, cyano, C₁-C₆-alkyl, C₁-C₆-haloalkyl, C₁-C₆-alkoxy and (C₁-C₆-alkoxy)carbonyl,
- a 3- to 7-membered heterocyclyl or heterocyclyloxy group having one to three hetero atoms selected from the group consisting of two oxygen atoms, two sulfur atoms and three nitrogen atoms, it being possible for the heterocycle to be saturated, partially or fully unsaturated or aromatic and, if desired, to have attached to it one to three substituents in each case selected from the group consisting of halogen, nitro, cyano, C₁-C₆-alkyl, C₁-C₆-alkoxy, C₁-C₆-haloalkyl and (C₁-C₆-alkyl)carbonyl,
- a group -CO-R⁹, -COOR⁹, -COSR⁹, -CON(R⁹)R¹⁰, -OCO-R⁹, -OCOOR⁹, -OCOSR⁹, -OCON(R⁹)R¹⁰ or -N(R⁹)R¹⁰, R⁹ having one of the meanings of R⁷ and R¹⁰ having one of the meanings of R⁸,
- the group -C(R²¹)=N-OR²⁰ ;
3) -CY-R¹¹, -C(R¹¹)(Z¹R¹²)(Z²R¹³) where Z¹ and Z² in each case are oxygen or sulfur,
-C(R¹¹)=C(R¹⁴)-CN, -C(R¹¹)=C(R¹⁴)-CO-R¹⁵,
-CH(R¹¹)-CH(R¹⁴)-CO-R¹⁵, -C(R¹¹)=C(R¹⁴)-CH₂-CO-R¹⁵,
-C(R¹¹)=C(R¹⁴)-C(R¹⁶)=C(R¹⁷)-CO-R¹⁵,
-C(R¹¹)=C(R¹⁴)-CH₂-CH(R¹⁸)-CO-R¹⁵, -CO-OR¹⁹, -CO-SR¹⁹,
-CO-N(R¹⁹)-OR²⁰, -C≡C-CO-NH-OR²⁰,
-C≡C-CO-N(R19)-OR²⁰, -C≡C-CS-NH-OR²⁰,
-C≡C-CS-N(R¹⁹)-OR²⁰, -C(R¹¹)=C(R¹⁴)-CO-NH-OR²⁰,
-C(R¹¹)=C(R¹⁴)-CO-N(R¹⁹)-OR²⁰,
-C(R¹¹)=C(R¹⁴)-CS-NH-OR²⁰,
-C(R¹¹)=C(R¹⁴)-CS-N(R¹⁹)-OR²⁰,
-C(R¹¹)=C(R¹⁴)-C(R²¹)=N-OR²⁰, -C(R²¹)=N-OR²⁰,
-C≡C-C(R²¹)=N-OR²⁰, -C(Z¹R¹²)(Z²R¹³)-OR¹⁹,
-C(Z¹R¹²)(Z²R¹³)-SR¹⁹, -C(Z¹R¹²)(Z²R¹³)-N(R²³)R²⁴,
-N(R²³)R²⁴, -CON(R²³)R²⁴, 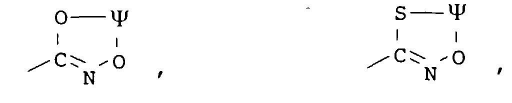 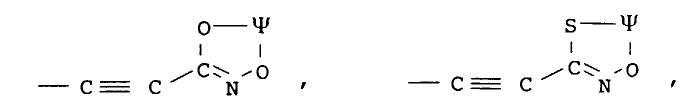 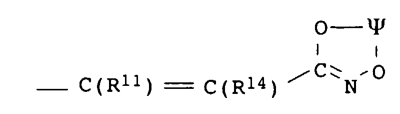 or 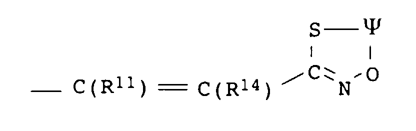 where Ψ is C₁-C₃-alkylene which can have attached to it a C₁-C₆-alkyl substituent;
R¹¹ is hydrogen, cyano, C₁-C₆-alkyl, C₁-C₆-haloalkyl, C₂-C₆-alkenyl, C₂-C₆-alkynyl, C₃-C₆-cycloalkyl, C₁-C₆-alkoxy-C₁-C₆-alkyl or (C₁-C₆-alkoxy)carbonyl;
R¹² and R¹³ independently of one another are C₁-C₆-alkyl, C₁-C₆-haloalkyl, C₃-C₆-alkenyl, C₃-C₆-alkynyl, C₁-C₆-alkoxy-C₁-C₆-alkyl or
R¹² and R¹³ together are a saturated or unsaturated, 2- to 4-membered carbon chain which can have attached to it an oxo substituent, it being possible for one member of this chain to be replaced by an oxygen, sulfur or nitrogen atom which is not neighboring to the variables Z¹ and Z² and it being possible for the carbon chain additionally to have attached to it one to three radicals, in each case selected from the group consisting of cyano, nitro, amino, halogen, C₁-C₆-alkyl, C₂-C₆-alkenyl, C₁-C₆-alkoxy, C₂-C₆-alkenyloxy, C₂-C₆-alkynyloxy, C₁-C₆-haloalkyl, cyano-C₁-C₆-alkyl, hydroxy-C₁-C₆-alkyl, C₁-C₆-alkoxy-C₁-C₆-alkyl, C₃-C₆-alkenyloxy-C₁-C₆-alkyl, C₃-C₆-alkynyloxy-C₁-C₆-alkyl, C₃-C₆-cycloalkyl, C₃-C₆-cycloalkoxy, carboxy, (C₁-C₆-alkoxy)carbonyl, (C₁-C₆-alkyl)carbonyloxy-C₁-C₆-alkyl and phenyl which, in turn, can be unsubstituted or have attached to it one to three substituents, in each case selected from the group consisting of cyano, nitro, amino, halogen, C₁-C₆-alkyl, C₁-C₆-haloalkyl, C₁-C₆-alkoxy and (C₁-C₆-alkoxy)carbonyl, and it also being possible for the carbon chain to be substituted by a fused or spiro-linked 3- to 7-membered ring which can contain, as ring members, one or two hetero atoms selected from amongst oxygen, sulfur, nitrogen and C₁-C₆-alkyl-substituted nitrogen and which, in turn, can have attached to it, if desired, one or two of the following substituents: cyano, C₁-C₆-alkyl, C₂-C₆-alkenyl, C₁-C₆-alkoxy, cyano-C₁-C₆-alkyl, C₁-C₆-haloalkyl and (C₁-C₆-alkoxy)carbonyl;
R¹⁴ is hydrogen, cyano, halogen, C₁-C₆-alkyl, C₁-C₆-haloalkyl, C₁-C₆-alkoxy, (C₁-C₆-alkyl)carbonyl or (C₁-C₆-alkoxy)carbonyl;
R¹⁵ is hydrogen, O-R²², S-R²², C₁-C₆-alkyl which can additionally have attached to it one or two C₁-C₆-alkoxy substituents, or is C₂-C₆-alkenyl, C₂-C₆-alkynyl, C₁-C₆-haloalkyl, C₃-C₆-cycloalkyl, C₁-C₆-alkylthio-C₁-C₆-alkyl, C₁-C₆-alkyliminooxy, -N(R²³)R²⁴ or phenyl which can be unsubstituted or have attached to it one to three substituents, in each case selected from the group consisting of cyano, nitro, halogen, C₁-C₆-alkyl, C₂-C₆-alkenyl, C₁-C₆-haloalkyl, C₁-C₆-alkoxy and (C₁-C₆-alkoxy)carbonyl,
R²² having one of the meanings of R¹⁹;
R¹⁶ is hydrogen, cyano, halogen, C₁-C₆-alkyl, C₃-C₆-alkenyl, C₃-C₆-alkynyl, C₁-C₆-alkoxy-C₁-C₆-alkyl, (C₁-C₆-alkyl)carbonyl, (C₁-C₆-alkoxy)carbonyl, -N(R²⁵)R²⁶, R²⁵ and R²⁶ having one of the meanings of R²³ and R²⁴, or is phenyl which, in turn, can additionally have attached to it one to three substituents, in each case selected from the group consisting of cyano, nitro, halogen, C₁-C₆-alkyl, C₁-C₆-haloalkyl, C₃-C₆-alkenyl, C₁-C₆-alkoxy and (C₁-C₆-alkoxy)carbonyl;
R¹⁷ is hydrogen, cyano, halogen, C₁-C₆-alkyl, C₁-C₆-alkoxy, C₁-C₆-haloalkyl, (C₁-C₆-alkyl)carbonyl or (C₁-C₆-alkoxy)carbonyl;
R¹⁸ is hydrogen, cyano, C₁-C₆-alkyl or (C₁-C₆-alkoxy)carbonyl;
R¹⁹ is hydrogen, C₁-C₆-alkyl, C₁-C₆-haloalkyl, C₂-C₆-alkenyl or C₂-C₆-alkynyl, it being possible for each of the last-mentioned 4 groups to have attached to it one or two of the following radicals: cyano, halogen, hydroxyl, hydroxycarbonyl, C₁-C₆-alkoxy, C₁-C₆-alkylthio, (C₁-C₆-alkyl)-carbonyl, (C₁-C₆-alkoxy)carbonyl, (C₁-C₆-alkyl)carbonyloxy, (C₃-C₆-alkenyloxy)carbonyl or a 3- to 7-membered aza heterocycle which is bonded to the nitrogen atom via a carbonyl bridge and which, in addition to carbon ring members, can additionally contain an oxygen or sulfur atom as ring member; or is (C₁-C₆-alkyl)carbonyl, (C₁-C₆-haloalkyl)carbonyl, (C₁-C₆-alkoxy)carbonyl, C₁-C₆-alkylaminocarbonyl,
di(C₁-C₆-alkyl)aminocarbonyl,
C₁-C₆-alkyloximino-C₁-C₆-alkyl, C₃-C₆-cycloalkyl,
phenyl or phenyl-C₁-C₆-alkyl, it being possible for the phenyl rings to be unsubstituted or to have attached to them, in turn, one to three substituents, in each case selected from the group consisting of cyano, nitro, . halogen, C₁-C₆-alkyl, C₁-C₆-haloalkyl, C₁-C₆-alkoxy and (C₁-C₆-alkoxy)carbonyl;
R²⁰ is hydrogen, C₁-C₆-alkyl, C₁-C₆-haloalkyl, C₃-C₆-cycloalkyl, C₃-C₆-alkenyl, C₃-C₆-alkynyl, hydroxy-C₁-C₆-alkyl, C₁-C₆-alkoxy-C₁-C₆-alkyl, C₁-C₆-alkylthio-C₁-C₆-alkyl, cyano-C₁-C₆-alkyl, (C₁-C₆-alkyl)carbonyl-C₁-C₆-alkyl, (C₁-C₆-alkoxy)carbonyl-C₁-C₆-alkyl, (C₁-C₆-alkoxy)carbonyl-C₂-C₆-alkenyl, (C₁-C₆-alkyl)carbonyloxy-C₁-C₆-alkyl or phenyl-C₁-C₆-alkyl, it being possible, if desired, for the phenyl ring to have attached to it one to three substituents, in each case selected from the group consisting of cyano, nitro, halogen, C₁-C₆-alkyl, C₁-C₆-haloalkyl, C₁-C₆-alkoxy and (C₁-C₆-alkoxy)carbonyl;
R²¹
- is hydrogen, halogen,
- C₁-C₆-alkyl, C₁-C₆-haloalkyl, C₁-C₆-alkoxy, C₁-C₆-haloalkoxy, C₃-C₆-alkenyloxy, C₁-C₆-alkylthio, C₁-C₆-haloalkylthio, (C₁-C₆-alkyl)carbonyloxy, (C₁-C₆-haloalkyl)carbonyloxy, C₁-C₆-alkylsulfonyloxy or C₁-C₆-haloalkylsulfonyloxy,
it being possible for the last-mentioned 11 radicals to have attached to them one of the following substituents: hydroxyl, cyano, hydroxycarbonyl, C₁-C₆-alkoxy, C₁-C₆-alkylthio, (C₁-C₆-alkyl)carbonyl, (C₁-C₆-alkoxy)carbonyl, (C₁-C₆-alkyl)aminocarbonyl, di(C₁-C₆-alkyl)aminocarbonyl, (C₁-C₆-alkyl)-carbonyloxy,
- a 3- to 7-membered aza heterocycle which is bonded to the nitrogen atom via a carbonyl bridge and which, in addition to carbon ring members, can also contain an oxygen or sulfur atom as ring member,
- (C₁-C₆-alkyl)carbonyl, (C₁-C₆-haloalkyl)carbonyl, (C₁-C₆-alkoxy)carbonyl, (C₁-C₆-alkoxy)carbonyloxy, (C₁-C₆-alkyl)carbonylthio, (C₁-C₆-haloalkyl)carbonylthio, (C₁-C₆-alkoxy)carbonylthio, C₂-C₆-alkenyl, C₂-C₆-alkenylthio, C₃-C₆-alkynyl, C₃-C₆-alkynyloxy, C₃-C₆-alkynylthio, (C₂-C₆-alkynyl)carbonyloxy, C₃-C₆-alkynylsulfonyloxy, C₃-C₆-cycloalkyl, C₃-C₆-cycloalkyloxy, C₃-C₆-cycloalkylthio, (C₃-C₆-cycloalkyl)carbonyloxy, C₃-C₆-cycloalkylsulfonyloxy,
- phenyl, phenoxy, phenylthio, benzoyloxy, phenylsulfonyloxy, phenyl-C₁-C₆-alkyl, phenyl-C₁-C₆-alkoxy, phenyl-C₁-C₆-alkylthio, phenyl-(C₁-C₆-alkyl)carbonyloxy or phenyl-(C₁-C₆-alkyl)sulfonyloxy, it being possible for the phenyl rings of the last-mentioned 10 radicals to be unsubstituted or to have attached to them, in turn, one to three substituents, in each case selected from the group consisting of cyano, nitro, halogen, C₁-C₆-alkyl, C₁-C₆-haloalkyl, C₁-C₆-alkoxy and (C₁-C₆-alkoxy)carbonyl;
R²³ and R²⁴ independently of one another are hydrogen, C₁-C₆-alkyl, C₂-C₆-alkenyl, C₂-C₆-alkynyl, C₃-C₆-cycloalkyl, C₁-C₆-haloalkyl, C₁-C₆-alkoxy-C₁-C₆-alkyl, (C₁-C₆-alkyl)carbonyl, (C₁-C₆-alkoxy)carbonyl, (C₁-C₆-alkoxy)carbonyl-C₁-C₆-alkyl, (C₁-C₆-alkoxy)carbonyl-C₂-C₆-alkenyl, it being possible for the alkenyl chain additionally to have attached to it one to three halogen and/or cyano radicals, or are C₁-C₆-alkylsulfonyl, (C₁-C₆-alkoxy)carbonyl-C₁-C₆-alkylsulfonyl, phenyl or phenylsulfonyl, it being possible for the two phenyl rings to be unsubstituted or, in turn, to have attached to them one to three substituents, in each case selected from the group consisting of cyano, nitro, halogen, C₁-C₆-alkyl, C₁-C₆-haloalkyl, C₃-C₆-alkenyl, C₁-C₆-alkoxy and (C₁-C₆-alkoxy)carbonyl,
or R²³ and R²⁴ together with the joint nitrogen atom are a saturated or unsaturated 4- to 7-membered aza heterocycle which, in addition to carbon ring members, can contain, if desired, one of the following members: -O-, -S-, -N=, -NH- or -N(C₁-C₆-alkyl)-;
Y is oxygen, sulfur or -N(R²⁷)-;
R²⁷ is hydrogen, hydroxyl, C₁-C₆-alkyl, C₃-C₆-alkenyl, C₃-C₆-alkynyl, C₃-C₇-cycloalkyl, C₁-C₆-haloalkyl, C₁-C₆-alkoxy-C₁-C₆-alkyl, C₁-C₆-alkoxy, C₃-C₆-alkenyloxy, C₃-C₆-alkynyloxy, C₃-C₆-cycloalkoxy, C₅-C₇-cycloalkenyloxy, C₁-C₆-haloalkoxy, C₃-C₆-haloalkenyloxy, hydroxy-C₁-C₆-alkoxy, cyano-C₁-C₆-alkoxy, C₃-C₆-cycloalkyl-C₁-C₆-alkoxy, C₁-C₆-alkoxy-C₁-C₆-alkoxy, C₁-C₆-alkoxy-C₃-C₆-alkenyloxy, (C₁-C₆-alkyl)carbonyloxy, (C₁-C₆-haloalkyl)carbonyloxy, (C₁-C₆-alkyl)carbamoyloxy, (C₁-C₆-haloalkyl)carbamoyloxy, (C₁-C₆-alkyl)carbonyl-C₁-C₆-alkyl, (C₁-C₆-alkyl)carbonyl-C₁-C₆-alkoxy, (C₁-C₆-alkoxy)carbonyl-C₁-C₆-alkyl, (C₁-C₆-alkoxy)carbonyl-C₁-C₆-alkoxy, C₁-C₆-alkylthio-C₁-C₆-alkoxy, di(C₁-C₆-alkyl)amino-C₁-C₆-alkoxy,
phenyl, which, in turn, can additionally have attached to it one to three substituents, in each case selected from the group consisting of cyano, nitro, halogen,
C₁-C₆-alkyl, C₁-C₆-haloalkyl, C₂-C₆-alkenyl, C₁-C₆-alkoxy and (C₁-C₆-alkoxy)carbonyl,
phenyl-C₁-C₆-alkoxy, phenyl-C₃-C₆-alkenyloxy or phenyl-C₃-C₆-alkynyloxy, it being possible for in each case one or two methylene groups of the carbon chains to be replaced by -O- , -S- or -N(C₁-C₆-alkyl)- and it being possible for each phenyl ring to be unsubstituted or, in turn, to have attached to it one to three substituents, in each case selected from the group consisting of cyano, nitro, halogen, C₁-C₆-alkyl, C₁-C₆-haloalkyl,
C₂-C₆-alkenyl, C₁-C₆-alkoxy and (C₁-C₆-alkoxy)carbonyl, heterocyclyl, heterocyclyl-C₁-C₆-alkoxy, heterocyclyl-C₃-C₆-alkenyloxy or heterocyclyl-C₃-C₆-alkynyloxy, it being possible for in each case one or two methylene groups of the carbon chains to be replaced by -O- , -S- or -N(C₁-C₆-alkyl)- and it being possible for each heterocycle to be 3- to 7-membered, saturated,
unsaturated or aromatic and to contain one to four hetero atoms selected from the group consisting of two oxygen atoms, two sulfur atoms and 4 nitrogen atoms and to be either unsubstituted or, in turn, to have attached to it one to three substituents, in each case selected from the group consisting of cyano, nitro, halogen, C₁-C₆-alkyl, C₁-C₆-haloalkyl, C₂-C₆-alkenyl, C₁-C₆-alkoxy and (C₁-C₆-alkoxy)carbonyl,
or -N(R²⁸)R²⁹, where R²⁸ and R²⁹ are in each case hydrogen, C₁-C₆-alkyl, C₃-C₆-alkenyl, C₃-C₆-alkynyl, C₃-C₆-cycloalkyl, C₁-C₆-haloalkyl, C₁-C₆-alkoxy-C₁-C₆-alkyl, (C₁-C₆-alkyl)carbonyl, (C₁-C₆-alkoxy)carbonyl, (C₁-C₆-alkoxy)carbonyl-C₁-C₆-alkyl, (C₁-C₆-alkoxy)carbonyl-C₂-C₆-alkenyl, it being possible for the alkenyl chain additionally to have attached to it one to three halogen and/or cyano radicals,
or is phenyl which can be unsubstituted or, in turn, additionally have attached to it one to three substituents, in each case selected from the group consisting of cyano, nitro, halogen, C₁-C₆-alkyl, C₁-C₆-haloalkyl, C₃-C₆-alkenyl, C₁-C₆-alkoxy and (C₁-C₆-alkoxy)carbonyl,
or where R²⁸ and R²⁹ together with the joint nitrogen atom form a saturated or unsaturated 4- to 7-membered heterocycle which, in addition to carbon ring members, can additionally have, if desired, one of the following members: -O-, -S-, -N=, -NH- or -N(C₁-C₆-alkyl)-;
or an agriculturally useful salt of a compound I.

2. The use of a 1-amino-3-benzyluracil of the formula I and an agriculturally useful salt thereof as claimed in claim 1 as a herbicide or for the desiccation/defoliation of plants.

3. A herbicidal composition comprising a herbicidally active amount of at least one 1-amino-3-benzyluracil of the formula I or of an agriculturally useful salt of I as claimed in claim 1 and at least one inert liquid and/or solid carrier and, if desired, at least one surfactant.

4. A composition for the desiccation and/or defoliation of plants, comprising an amount of at least one 1-amino-3-benzyluracil of the formula I or of an agriculturally useful salt of I as claimed in claim 1 such that it acts as a desiccant and/or defoliant and at least one inert liquid and/or solid carrier and, if desired, at least one surfactant.

5. A process for the preparation of herbicidally active compositions, which comprises mixing a herbicidally active amount of at least one 1-amino-3-benzyluracil of the formula I or of an agriculturally useful salt of I as claimed in claim 1 and at least one inert liquid and/or solid carrier and, if desired, at least one surfactant.

6. A process for the preparation of compositions which act as desiccants and/or defoliants, which comprises mixing an amount of at least one 1-amino-3-benzyluracil of the formula I or of an agriculturally useful salt of I as claimed in claim 1 such that it acts as a desiccant and/or defoliant and at least one inert liquid and/or solid carrier and, if desired, at least one surfactant.

7. A method of controlling undesirable vegetation, which comprises allowing a herbicidally active amount of at least one 1-amino-3-benzyluracil of the formula I or of an agriculturally useful salt of I as claimed in claim 1 to act on plants, their environment or on seed.

8. A method for the desiccation and/or defoliation of plants, which comprises allowing an amount of at least one 1-amino-3-benzyluracil of the formula I or of an agriculturally useful salt of I as claimed in claim 1 to act on plants such that it acts as a desiccant and/or defoliant.

9. A method as claimed in claim 8, wherein cotton is treated.

10. A process for the preparation of a 1-amino-3-benzyluracil of the formula I as claimed in claim 1, where X is oxygen, which comprises cyclizing, in the presence of a base, an enamine ester of the formula III 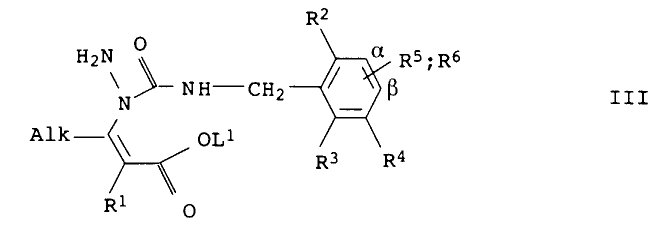 or an enamine carboxylate of the formula IV 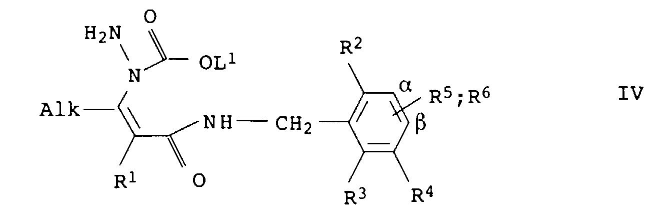 where L¹ in each case is low-molecular-weight alkyl or phenyl and the substituents Alk and R¹ to R⁶ have the meanings given in claim 1.

11. A process for the preparation of a 1-amino-3-benzyluracil of the formula I as claimed in claim 1, wherein a 1-H-3-phenyluracil [sic] of the formula V 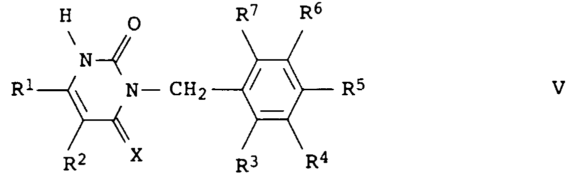 where the substituents Alk and R¹ to R⁶ have the meanings given in claim 1 is reacted with an electrophilic aminating reagent in the presence of a base.

12. A process for the preparation of a 1-amino-3-benzyluracil of the formula I as claimed in claim 1, wherein a 1-amino-6-haloalkyluracil of the formula VIII 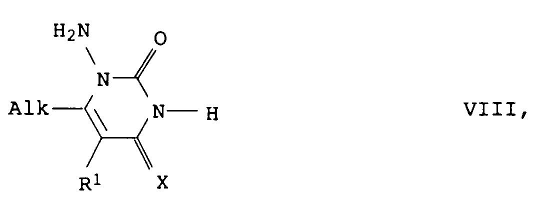 where Alk and R¹ have the meanings given in claim 1 is reacted in the presence of a base, or an alkali metal salt of VIII is reacted with a benzyl halide of the formula IX 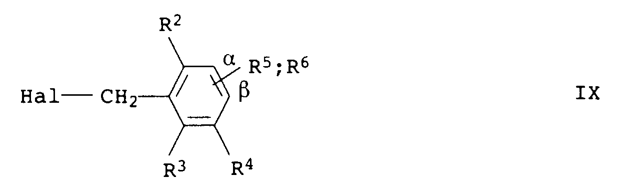 where Hal is halogen and the substituents R² to R⁶ have the meanings given in claim 1.

13. An enamine ester of the formula III 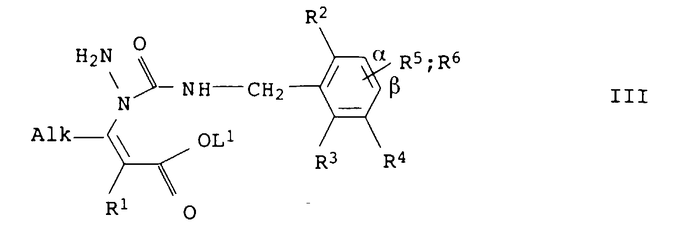 where L¹ is C₁-C₆-alkyl or phenyl and the substsituents Alk and R¹ to R⁶ have the meanings given in claim 1.

14. An enamine carboxylate of the formula IV 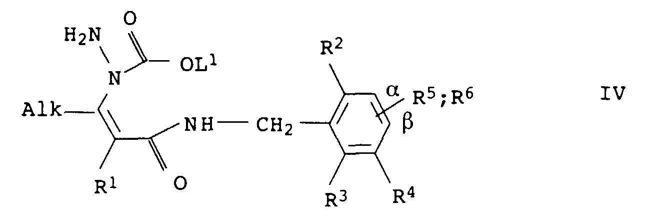 where L¹ is C₁-C₆-alkyl or phenyl and the substituents Alk and R¹ to R⁶ have the meanings given in claim 1.

## Revendications

1. 1-amino-3-benzyluraciles de formule générale I 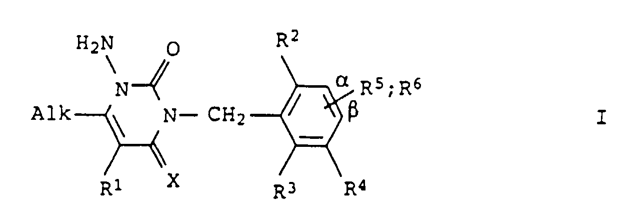 dans laquelle les variables ont la signification suivante:
X oxygène ou soufre;
Alk halogénoalkyle en C₁-C₄;
R¹ hydrogène ou halogéno;
R², R³, indépendamment l'un de l'autre, hydrogène, cyano, thiocyanato, halogéno, halogénoalkyle en C₁-C₄ ou halogénoalkylthio en C₁-C₄;
R⁴ hydrogène, cyano, thiocyanato, halogéno, alkyle en C₁-C₄, halogénoalkyle en C₁-C₄, alcoxy en C₁-C₄, halogénoalcoxy en C₁-C₄, halogénoalkylthio en C₁-C₄ ou alkylaminocarbonyle en C₁-C₆;
R⁵ sur la position α ou β
hydrogène, cyano, nitro, hydroxy, amino, halogéno, alkylamino en C₁-C₄, qui peut être substitué par des groupes alkyle en C₁-C₄, (alkyl en C₁-C₄)carboxyle ou (alcoxy en C₁-C₄)carbonyle,
halogénoalcoxy en C₁-C₆, halogénoalkylthio en C₁-C₆, alcoxy en C₁-C₆, alkylthio en C₁-C₆, cycloalcoxy en C₃-C₆, cycloalkylthio en C₃-C₆, alcényloxy en C₃-C₆, alcénylthio en C₃-C₆, alcynyloxy en C₃-C₆, alcynylthio en C₃-C₆, (alkyl en C₁-C₆)carbonyloxy, (alkyl en C₁-C₆)carbonylthio, (alcényl en C₃-C₆)carbonyloxy, (alcényl en C₃-C₆)carbonylthio, (alcynyl en C₃-C₆)carbonyloxy, (alcynyl en C₃-C₆)carbonylthio, alkylsulfonyloxy en C₁-C₆ ou alkylsulfonyle en C₁-C₆, tandis que chacun des 16 restes mentionnés en dernier peut éventuellement porter un à trois substituants, chacun choisi dans le groupe consistant en
- halogéno, nitro, cyano, hydroxy, cycloalkyl en C₃-C₆, alcoxy en C₁-C₆, cycloalcoxy en C₃-C₆, alcényloxy en C₃-C₆, alcynyloxy en C₃-C₆, alcoxy(C₁-C₆)-alcoxy(C₁-C₆), alkylthio en C₁-C₆, alkylsulfinyle en C₁-C₆, alkylsulfonyle en C₁-C₆, alkylidèneaminoxy en C₁-C₆,
- le groupe phényle, phénoxy ou phénylsulfonyle, qui peut être non-substitué ou porter un à trois substituants, chacun choisi dans le groupe consistant en halogéno, nitro, cyano, alkyle en C₁-C₆, alcoxy en C₁-C₆ et halogénoalkyle en C₁-C₆,
- un groupe hétérocyclyle ou hétérocyclyloxy à 3 à 7 chaînons ayant un à trois hétéroatomes, choisi dans le groupe consistant en deux atomes d'oxygène, deux atomes de soufre et 3 atomes d'azote, tandis que l'hétérocycle peut être saturé, partiellement ou totalement insaturé ou aromatique et peut éventuellement porter un à trois substituants, chacun choisi dans le groupe consistant en halogéno, nitro, cyano, alkyle en C₁-C₆, alcoxy en C₁-C₆, halogénoalkyle en C₁-C₆ et (alkyl en C₁-C₆)carbonyle,
- un groupe -CO-R⁷, -COOR⁷, -COSR⁷, - CON(R⁷)R⁸, -OCO-R⁷, -OCOOR⁷, -OCOSR⁷, -OCON(R⁷)R⁸ ou -N(R⁷)R⁸, tandis que
R⁷ désigne l'hydrogène ou un groupe alkyle en C₁-C₆, cycloalkyle en C₃-C₆, alcényle en C₃-C₆, alcynyle en C₃-C₆, alcoxy(C₁-C₆)-alkyle(C₁-C₆), (alcoxy en C₁-C₆)carbonyle-alkyle(C₁-C₆), (alcényloxy en C₁-C₆)carbonyl-alkyle(C₁-C₆), phényle ou phényl-alkyle(C₁-C₆), tandis que le groupe phényle et le cycle phényle du groupe phénylalkyle peuvent être non-substitués ou peuvent porter un à trois restes, à chaque fois choisis dans le groupe consistant en halogéno, nitro, cyano, alkyle en C₁-C₆, halogénoalkyle en C₁-C₆, alcoxy en C₁-C₆ et (alkyle en C₁-C₆)-carbonyle
et
R⁸ désigne l'hydrogène ou un groupe hydroxy, alkyle en C₁-C₆, cycloalkyle en C₃-C₆, alcoxy en C₁-C₆, (alcoxy en C₁-C₆)carbonylalcoxy(C₁-C₆), alcényle en C₃-C₆ ou alcényloxy en C₃-C₆,
ou R⁷ et R⁸ forment conjointement avec l'azote commun un hétérocycle à 3 à 7 chaînons ayant un à trois hétéroatomes, choisis dans un groupe consistant en un ou deux atomes d'oxygène, un ou deux atomes de soufre et un à trois atomes d'azote, tandis que l'hétérocycle peut être saturé, partiellement ou totalement insaturé ou aromatique et peut éventuellement porter un à trois substituants, chacun choisi dans le groupe consistant en un groupe halogéno; nitro, cyano, alkyle en C₁-C₆, halogénoalkyle en C₁-C₆, alcoxy en C₁-C₆ ou (alkyl en C₁-C₆)carbonyle;
R⁶ fixé sur la position a tandis que R⁵ est alors sur la position β, ou sur la position β tandis que R⁵ est alors sur la position α,désigne
1) hydrogène, hydroxy, mercapto, halogéno, halogénoalcoxy en C₁-C₆, halogénoalkylthio en C₁-C₆, alkylthio en C₁-C₆-(alkyl en C₁-C₆)-carbonyle, (alkyl en C₁-C₆)iminooxycarbonyle,
2) alcoxy en C₁-C₆, alkylthio en C₁-C₆, cycloalcoxy en C₃-C₆, cycloalkylthio en C₃-C₆, alcényloxy en C₂-C₆, alcénylthio en C₂-C₆, alcynyloxy en C₂-C₆, alcynylthio en C₂-C₆, (alkyl en C₁-C₆)carbonyloxy, (alkyl en C₁-C₆)carbonylthio, (alcoxy en C₁-C₆)carbonyloxy, (alcényl en C₂-C₆)carbonyloxy, (alcényl en C₂-C₆)carbonylthio, (alcynyl en C₂-C₆)carbonyloxy, (alcynyl en C₂-C₆)carbonylthio, alkylsulfonyloxy en C₁-C₆ ou alkylsulfonyle en C₁-C₆, tandis que chacun des 17 restes mentionnés en dernier peut éventuellement porter un à trois substituants, chacun choisi dans le groupe consistant en
- halogéno, nitro, cyano, hydroxy, cycloalkyle en C₃-C₆, alcoxy en C₁-C₆, cycloalcoxy en C₃-C₆, alcényloxy en C₃-C₆, alcynyloxy en C₃-C₆, alcoxy-(C₁-C₆)-alcoxy en C₁-C₆, alkylthio en C₁-C₆, alkylsulfinyle en C₁-C₆, alkylsulfonyle en C₁-C₆, alkylidèneaminoxy en C₁-C₆,
- le groupe phényle, phénoxy ou phénylsulfonyle, qui peut être non-substitué ou porter un à trois substituants, chacun choisi dans le groupe consistant en halogéno, nitro, cyano, alkyle en C₁-C₆, halogénoalkyle en C₁-C₆, alcoxy en C₁-C₆ et (alcoxy en C₁-C₆)-carbonyle,
- un groupe hétérocyclyle ou hétérocyclyloxy à 3 à 7 chaînons ayant un à trois hétéroatomes, choisi dans le groupe consistant en deux atomes d'oxygène, deux atomes de soufre et 3 atomes d'azote, tandis que l'hétérocycle peut être saturé, partiellement ou totalement insaturé ou aromatique et peut éventuellement porter un à trois substituants, chacun choisi dans le groupe consistant en halogéno, nitro, cyano, alkyle en C₁-C₆, alcoxy en C₁-C₆, halogénoalkyle en C₁-C₆ et (alkyl en C₁-C₆)carbonyle,
- un groupe -CO-R⁹, -COOR⁹, -COSR⁹, -CON(R⁹)R¹⁰, -OCO-R⁹, -OCOOR⁹, -OCOSR⁹, -OCON(R⁹)R¹⁰ ou -N(R⁹)R¹⁰, tandis que R⁹ a l'une des significations de R⁷ et R¹⁰ a l'une des significations de R⁸,
- le groupe -C(R²¹)=N-OR²⁰;
3) -CY-R¹¹, -C(R¹¹)(Z¹R¹²)(Z²R¹³), tandis que Z¹ et Z² représentent chacun l'oxygène ou le soufre, 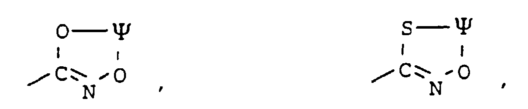 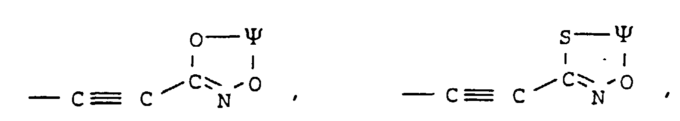 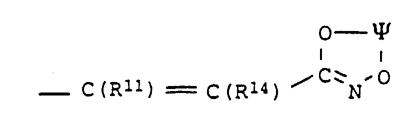 ou 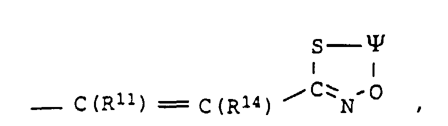 tandis que ψ désigne un alkylène en C₁-C₃, qui peut porter un substituant alkyle en C₁-C₆;
R¹¹ hydrogène, cyano, alkyle en C₁-C₆, halogéno-alkyle en C₁-C₆, alcényle en C₂-C₆, alcynyle en C₂-C₆, cycloalkyle en C₃-C₆, alcoxy(C₁-C₆)alkyle en C₁-C₆ ou (alcoxy en C₁-C₆)carbonyle;
R¹², R¹³, indépendamment l'un de l'autre, alkyle en C₁-C₆, halogénoalkyle en C₁-C₆, alcényle en C₃-C₆, alcynyle en C₃-C₆, alcoxy(C₁-C₆)-alkyle en C₁-C₆ ou
R¹² et R¹³ représentent ensemble une chaîne hydrocarbonée à 2 à 4 chaînons, saturée ou insaturée, qui peut porter un substituant oxo, tandis qu'un membre de cette chaîne peut être remplacé par un atome d'oxygène, de soufre ou d'azote qui ne soit pas voisin des variables Z¹ et Z², et tandis que la chaîne hydrocarbonée peut encore porter un à trois restes choisis chacun dans le groupe consistant en un reste cyano, nitro, amino, halogéno, alkyle en C₁-C₆, alcényle en C₂-C₆, alcoxy en C₁-C₆, alcényloxy en C₂-C₆, alcynyloxy en C₂-C₆, halogénoalkyle en C₁-C₆, cyano-alkyle(C₁-C₆), hydroxyalkyle en C₁-C₆, alcoxy(C₁-C₆)-alkyle en C₁-C₆, alcényloxy(C₃-C₆)-alkyle en C₁-C₆, alcynyloxy(C₃-C₆)alkyle en C₁-C₆, cycloalkyle en C₃-C₆, cycloalcoxy en C₃-C₆, carboxy, (alcoxy en C₁-C₆)carbonyle, (alkyl en C₁-C₆)carbonyloxyalkyle en C₁-C₆ et phényle, qui peut à son tour être non-substitué ou porter un à trois substituants, dont chacun est choisi dans le groupe consistant en un reste cyano, nitro, amino, halogéno, alkyle en C₁-C₆, halogénoalkyle en C₁-C₆, alcoxy en C₁-C₆ et (alcoxy en C₁-C₆)carbonyle, et tandis que la chaîne hydrocarbonée peut également être substituée par un cycle à 3 à 7 chaînons, condensé ou spiro, qui peut contenir en tant que membres du cycle un ou deux hétéroatomes choisis parmi l'oxygène, le soufre, l'azote et l'azote substitué par un groupe alkyle en C₁-C₆, et qui peut si on le souhaite porter à son tour un ou deux des substituants suivants: cyano, alkyle en C₁-C₆, alcényle en C₂-C₆, alcoxy en C₁-C₆, cyano-alkyle(C₁-C₆), halogénoalkyle en C₁-C₆ et (alcoxy en C₁-C₆)carbonyle;
R¹⁴ hydrogène, cyano, halogéno, alkyle en C₁-C₆, halogénoalkyle en C₁-C₆, alcoxy en C₁-C₆, (alkyl en C₁-C₆)carbonyle ou (alcoxy en C₁-C₆)carbonyle;
R¹⁵ hydrogène, O-R²², S-R²², alkyle en C₁-C₆ - qui peut encore porter un ou deux substituants alcoxy en C₁-C₆ - alcényle en C₂-C₆, alcynyle en C₂-C₆, halogénoalkyle en C₁-C₆, cycloalkyle en C₃-C₆, alkylthio(C₁-C₆)-alkyle en C₁-C₆, alkyliminooxy en C₁-C₆, -N(R²³)R²⁴ ou phényle, qui peut être non-substitué ou porter un à trois substituants, chacun choisi dans le groupe consistant en cyano, nitro, halogéno, alkyle en C₁-C₆, alcényle en C₂-C₆, halogénoalkyle en C₁-C₆, alcoxy en C₁-C₆ et (alcoxy en C₁-C₆)carbonyle,
tandis que R²² a l'une des significations de R¹⁹;
R¹⁶ hydrogène, cyano, halogéno, alkyle en C₁-C₆, alcényle en C₃-C₆, alcynyle en C₃-C₆, alcoxy(C₁-C₆)alkyle en C₁-C₆, (alkyl en C₁-C₆)carbonyle, (alcoxy en C₁-C₆)carbonyle,-N(R²⁵)R²⁶, tandis que R²⁵ et R²⁶ ont l'une des significations de R²³ et R²⁴,
ou
phényle, qui à son tour peut encore porter un à trois substituants, à chaque fois choisi dans le groupe consistant en cyano, nitro, halogéno, alkyle en C₁-C₆, halogénoalkyle en C₁-C₆, alcényle en C₃-C₆, alcoxy en C₁-C₆ et (alcoxy en C₁-C₆)carbonyle;
R¹⁷ hydrogène, cyano, halogéno, alkyle en C₁-C₆, alcoxy en C₁-C₆, halogénoalkyle en C₁-C₆, (alkyl en C₁-C₆)carbonyle ou (alcoxy en C₁-C₆)carbonyle;
R¹⁸ hydrogène, cyano, alkyle en C₁-C₆ ou (alcoxy en C₁-C₆)carbonyle;
R¹⁹ hydrogène, alkyle en C₁-C₆, halogénoalkyle en C₁-C₆, alcényle en C₂-C₆ ou alcynyle en C₂-C₆, tandis que les quatre groupes mentionnés en dernier peuvent chacun porter un ou deux des restes suivants: cyano, halogéno, hydroxy, hydroxycarbonyle, alcoxy en C₁-C₆, alkylthio en C₁-C₆, (alkyl en C₁-C₆)carbonyle, (alcoxy en C₁-C₆)carbonyle, (alkyl en C₁-C₆)carbonyloxy, (alcényloxy en C₃-C₆)carbonyle ou un azahétérocycle à 3 à 7 chaînons lié à l'atome d'azote par un pont carbonyle, qui peut contenir, outre les membres cycliques carbonés, encore un atome d'oxygène ou de soufre en tant que membre du cycle;
ou (alkyl en C₁-C₆)carbonyle, (halogénoalkyl en C₁-C₆)carbonyle, (alcoxy en C₁-C₆)carbonyle, alkylaminocarbonyle en C₁-C₆, di-(alkyl en C₁-C₆)aminocarbonyle, alkyloximino(C₁-C₆)alkyle en C₁-C₆, cycloalkyle en C₃-C₆,
phényle ou phényl-alkyle(C₁-C₆), tandis que les cycles phényle peuvent être non-substitués ou porter à leur tour un à trois substituants, chacun choisi dans le groupe consistant en cyano, nitro, halogéno, alkyle en C₁-C₆, halogénoalkyle en C₁-C₆, alcoxy en C₁-C₆ et (alcoxy en C₁-C₆)carbonyle;
R²⁰ hydrogène, alkyle en C₁-C₆, halogénoalkyle en C₁-C₆, cycloalkyle en C₃-C₆, alcényle en C₃-C₆, alcynyle en C₃-C₆, hydroxyalkyle en C₁-C₆, alcoxy(C₁-C₆)-alkyle en C₁-C₆, alkylthio(C₁-C₆)-alkyle en C₁-C₆, cyano-alkyle(C₁-C₆), (alkyl en C₁-C₆)carbonyl-alkyle(C₁-C₆), (alcoxy en C₁-C₆)carbonyl-alkyle(C₁-C₆), (alcoxy en C₁-C₆)carbonyl-alcényle(C₁-C₆), (alkyl en C₁-C₆)carbonyloxy-alkyle(C₁-C₆) ou phénylalkyle(C₁-C₆), tandis que le cycle phényle peut en option porter un à trois substituants, chacun choisi dans le groupe consistant en cyano, nitro, halogéno, alkyle en C₁-C₆, halogénoalkyle en C₁-C₆, alcoxy en C₁-C₆ et (alcoxy en C₁-C₆)carbonyle;
R²¹
- hydrogène, halogéno,
- alkyle en C₁-C₆, halogénoalkyle en C₁-C₆, alcoxy en C₁-C₆, halogénoalcoxy en C₁-C₆, alcényloxy en C₃-C₆, alkylthio en C₁-C₆, halogénoalkylthio en C₁-C₆, (alkyl en C₁-C₆)carbonyloxy, (halogénoalkyl en C₁-C₆)carbonyloxy, alkylsulfonyloxy en C₁-C₆ ou halogénoalkylsulfonyloxy en C₁-C₆, tandis que les 11 restes mentionnés en dernier peuvent porter l'un des substituants suivants: hydroxy, cyano, hydroxycarbonyle, alcoxy en C₁-C₆, alkylthio en C₁-C₆, (alkyl en C₁-C₆)carbonyle, (alcoxy en C₁-C₆)carbonyle, (alkyl en C₁-C₆)aminocarbonyle, di-(alkyl en C₁-C₆)aminocarbonyle, (alkyl en C₁-C₆)carbonyloxy, un azahétérocycle à 3 à 7 chaînons lié à l'atome d'azote par un pont carbonyle, qui peut encore contenir, outre les membres du cycle hydrocarboné, un atome d'oxygène ou un atome de soufre en tant que membre du cycle, (alkyl en C₁-C₆)carbonyle, (halogénoalkyl en C₁-C₆)carbonyle, (alcoxy en C₁-C₆)carbonyle, (alcoxy en C₁-C₆)carbonyloxy, (alkyl en C₁-C₆)carbonylthio, (halogénoalkyl en C₁-C₆)carbonylthio, (alcoxy en C₁-C₆)carbonylthio, alcényl en C₂-C₆, alcénylthio en C₂-C₆, alcynyle en C₃-C₆, alcynyloxy en C₃-C₆, alcynylthio en C₃-C₆, (alcynyl en C₂-C₆)carbonyloxy, alcynylsulfonyloxy en C₃-C₆, cycloalkyle en C₃-C₆, cycloalkyloxy en C₃-C₆, cycloalkylthio en C₃-C₆, (cycloalkyl en C₃-C₆)carbonyloxy, cycloalkylsulfonyloxy en C₃-C₆,
- phényle, phénoxy, phénylthio, benzoyloxy, phénylsulfonyloxy, phényl-alkyle(C₁-C₆), phényl-alcoxy(C₁-C₆), phénylalkylthio(C₁-C₆), phényl-(alkyl en C₁-C₆)carbonyloxy ou phényl-(alkyl en C₁-C₆)sulfonyloxy, tandis que les cycles phényle des 10 restes mentionnés en dernier peuvent être non-substitués ou peuvent à leur tour porter un à trois substituants, chacun choisi dans le groupe consistant en cyano, nitro, halogéno, alkyle en C₁-C₆, halogénoalkyle en C₁-C₆, alcoxy en C₁-C₆ et (alcoxy en C₁-C₆)carbonyle;
R²³, R²⁴, indépendamment l'un de l'autre,
hydrogène, alkyle en C₁-C₆, alcényle en C₂-C₆, alcynyle en C₂-C₆, cycloalkyle en C₃-C₆, halogénoalkyle en C₁-C₆, alcoxy(C₁-C₆)-alkyle en C₁-C₆, (alkyl en C₁-C₆)carbonyle, (alcoxy en C₁-C₆)carbonyle, (alcoxy en C₁-C₆)carbonylalkyle(C₁-C₆), (alcoxy en C₁-C₆)carbonylalcényle(C₂-C₆), tandis que la chaîne alcényle peut porter en outre un à trois restes halogéno et/ou cyano,
ou alkylsulfonyle en C₁-C₆, (alcoxy en C₁-C₆)carbonyl-alkylsulfonyle en C₁-C₆, phényle ou phénylsulfonyle, tandis que les deux noyaux phényle peuvent être non-substitués ou porter à leur tour un à trois substituants, chacun choisi dans le groupe consistant en cyano, nitro, halogéno, alkyle en C₁-C₆, halogénoalkyle en C₁-C₆, alcényle en C₃-C₆, alcoxy en C₁-C₆ et (alcoxy en C₁-C₆)carbonyle,
ou R²³ et R²⁴, conjointement avec l'atome d'azote commun, un azahétérocycle à 4 à 7 chaînons, saturé ou insaturé, qui, outre les membres hydrocarbonés du cycle, peut en option contenir un des membres suivants: -O-, -S-, -N=, -NH- ou -N(alkyle en C₁-C₆)-;
Y oxygène, soufre ou -N(R²⁷)-;
R²⁷ hydrogène, hydroxy, alkyle en C₁-C₆, alcényle en C₃-C₆, alcynyle en C₃-C₆, cycloalkyle en C₃-C₇, halogénoalkyle en C₁-C₆, alcoxy(C₁-C₆)alkyle en C₁-C₆, alcoxy en C₁-C₆, alcényloxy en C₃-C₆, alcynyloxy en C₃-C₆, cycloalcoxy en C₃-C₆, cycloalcényloxy en C₅-C₇, halogénoalcoxy en C₁-C₆, halogénoalcényloxy en C₃-C₆, hydroxyalcoxy(C₁-C₆), cyano-alcoxy(C₁-C₆), cycloalkyl(C₃-C₆)-alcoxy en C₁-C₆, alcoxy(C₁-C₆)-alcoxy en C₁-C₆, alcoxy(C₁-C₆)-alcényloxy en C₃-C₆, (alkyl en C₁-C₆)carbonyloxy, (halogénoalkyl en C₁-C₆)carbonyloxy, (alkyl en C₁-C₆)carbamoyloxy, (halogénoalkyl en C₁-C₆)carbamoyloxy, (alkyl en C₁-C₆)carbonyl-alkyle(C₁-C₆), (alkyl en C₁-C₆,)carbonyl-alcoxy(C₁-C₆), (alcoxy en C₁-C₆)carbonyl-alkyle(C₁-C₆), (alcoxy en C₁-C₆)carbonyl-alcoxy(C₁-C₆), alkylthio(C₁-C₆)alcoxy en C₁-C₆, di-(alkyl en C₁-C₆)aminoalcoxy en C₁-C₆,
phényle, qui à son tour peut encore porter un à trois substituants, chacun choisi dans le groupe consistant en cyano, nitro, halogéno, alkyle en C₁-C₆, halogénoalkyle en C₁-C₆, alcényle en C₂-C₆, alcoxy en C₁-C₆ et (alcoxy en C₁-C₆)carbonyle,
phényl-alcoxy(C₁-C₆), phényl-alcényloxy(C₃-C₆) ou phényl-alcynyloxy(C₃-C₆), tandis qu'à chaque fois un ou deux groupes méthylène des chaînes hydrocarbonées peuvent être remplacés par -O-, -S- ou -N(alkyle en C₁-C₆)- et tandis que chaque noyau phényle peut être non-substitué ou porter à son tour un à trois substituants, chacun choisi dans le groupe consistant en cyano, nitro, halogéno, alkyle en C₁-C₆, halogénoalkyle en C₁-C₆, alcényle en C₂-C₆, alcoxy en C₁-C₆ et (alcoxy en C₁-C₆)carbonyle, hétérocyclyle, hétérocyclyl-alcoxy(C₁-C₆), hétérocyclyl-alcényloxy(C₃-C₆) ou hétérocyclylalcynyloxy(C₃-C₆), tandis qu'à chaque fois un ou deux groupes méthylène des chaînes hydrocarbonées peuvent être remplacés par -O-, -S- ou -N(alkyle en C₁-C₆)- et tandis que chaque hétérocycle peut être saturé à 3 à 7 chaînons, insaturé ou aromatique et contient un à quatre hétéroatomes, choisis dans le groupe consistant en deux atomes d'oxygène, deux atomes de soufre et 4 atomes d'azote,
et soit est non-substitué, soit porté à son tour un à trois substituants, chacun choisi dans le groupe consistant en cyano, nitro, halogéno, alkyle en C₁-C₆, halogéno-alkyle en C₁-C₆, alcényle en C₂-C₆, alcoxy en C₁-C₆ et (alcoxy en C₁-C₆)carbonyle,
ou -N(R²⁸)R²⁹, tandis que R²⁸ et R²⁹ désignent chacun l'hydrogène ou un groupe alkyle en C₁-C₆, alcényle en C₃-C₆, alcynyle en C₃-C₆, cycloalkyle en C₃-C₆, halogénoalkyle en C₁-C₆, alcoxy(C₁-C₆)-alkyle en C₁-C₆, (alkyl en C₁-C₆)carbonyle, (alcoxy en C₁-C₆)carbonyle, (alcoxy en C₁-C₆)carbonyl-alkyle en C₁-C₆, (alcoxy en C₁-C₆)carbonyl-alcényle en C₂-C₆, tandis que la chaîne alcényle peut en outre porter un à trois restes halogéno et/ou cyano,
ou phényle, qui peut être non-substitué ou porter encore à son tour un à trois substituants, chacun choisi dans le groupe consistant en cyano, nitro, halogéno, alkyle en C₁-C₆, halogénoalkyle en C₁-C₆, alcényle en C₃-C₆, alcoxy en C₁-C₆ et (alcoxy en C₁-C₆)carbonyle,
ou tandis que R²⁸ et R²⁹ forment, conjointement avec l'atome d'azote commun, un hétérocycle à 4 à 7 chaînons, saturé ou insaturé, qui, outre les membres hydrocarbonés du cycle, peut en option contenir encore un des membres suivants: -O-, -S-, -N=, -NH- ou -N(alkyle en C₁-C₆)-;
ainsi que les sels utilisables en agriculture des composés I.

2. Utilisation des 1-amino-3-benzyluraciles de formule I et de leurs sels pouvant servir en agriculture, selon la revendication 1, comme herbicides'ou pour la dessiccation/défoliation de plantes.

3. Agent herbicide, contenant une quantité efficace du point de vue herbicide d'au moins un 1-amino-3-benzyluracile de formule I ou d'un sel de I utilisable en agriculture, selon la revendication 1, et au moins un support inerte, liquide et/ou solide, ainsi que, en option, au moins une substance tensio-active.

4. Agent pour la dessiccation et/ou la défoliation de plantes, contenant une quantité efficace du point de vue desséchant et/ou défoliant d'au moins un 1-amino-3-benzylurcile de formule I ou d'un sel de I utilisable en agriculture, selon la revendication 1, et au moins un support inerte, liquide et/ou solide, ainsi que, en option, au moins une substance tensio-active.

5. Procédé pour la préparation d'agents à activité herbicide, caractérisé par le fait qu'on mélange une quantité efficace du point de vue herbicide d'au moins un 1-amino-3-benzyluracile de formule I ou d'un sel de I utilisable en agriculture, selon la revendication 1, et au moins un support inerte, liquide et/ou solide, ainsi que, en option, au moins une substance tensio-active.

6. Procédé pour la préparation d'agents à activité desséchante et/ou défoliante, caractérisé par le fait qu'on mélange une quantité efficace du point de vue desséchant et/ou défoliant d'au moins un 1-amino-3-benzyluracile de formule I ou d'un sel de I utilisable en agriculture, selon la revendication 1, et au moins un support inerte, liquide et/ou solide, ainsi que, en option, au moins une substance tensio-active.

7. Procédé pour lutter contre la croissance parasite de plantes, caractérisé par le fait qu'on fait agir sur des plantes, leur biotope ou sur des semences une quantité efficace du point de vue herbicide d'au moins un 1-amino-3-benzyluracile de formule I ou d'un sel de I utilisable en agriculture, selon la revendication 1.

8. Procédé pour la dessiccation et/ou la défoliation de plantes, caractérisé par le fait qu'on fait agir sur des plantes une quantité efficace du point de vue desséchant et/ou défoliant d'au moins un 1-amino-3-benzyluracile de formule I ou d'un sel de I utilisable en agriculture, selon la revendication 1.

9. Procédé selon la revendication 8, caractérisé par le fait qu'on traite du coton.

10. Procédé pour la préparation de 1-amino-3-benzyluraciles de formule I selon la revendication 1, où X désigne l'oxygène, caractérisé par le fait qu'on cyclise en présence d'une base un ester d'énamine de formule III 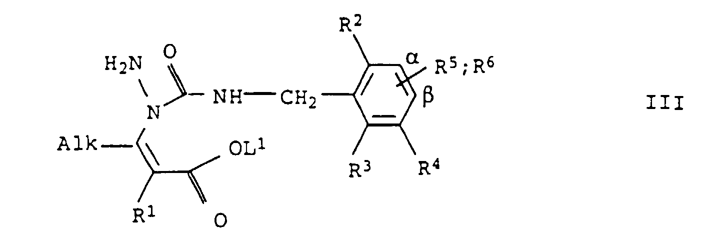 ou un carboxylate d'énamine de formule IV 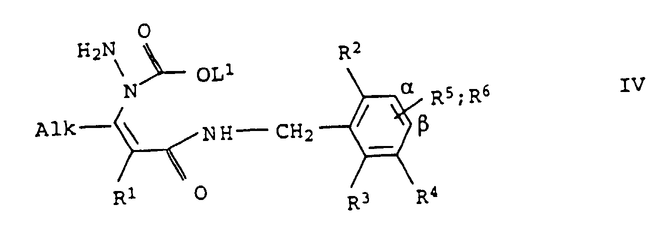 où L¹ représente à chaque fois un alkyle de faible masse moléculaire ou un phényle et les substituants Alk et R¹ à R⁶ ont les significations indiquées dans la revendication 1.

11. Procédé pour la préparation de 1-amino-3-benzyluraciles de formule I selon la revendication 1, caractérisé par le fait qu'on fait réagir un 1-H-3-phényluracile de formule V 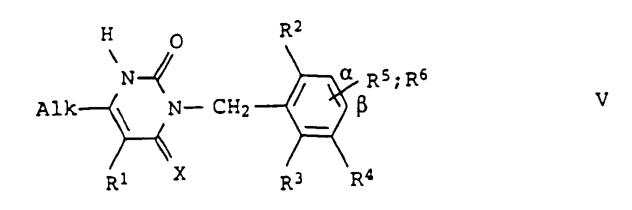 où les substituants Alk et R¹ à R⁶ ont les significations indiquées dans la revendication 1, en présence d'une base avec un réactif d'amination électrophile.

12. Procédé pour la préparation de 1-amino-3-benzyluraciles de formule I selon la revendication 1, caractérisé par le fait qu'on fait réagir un 1-amino-6-halogénoalkyluracile de formule VIII 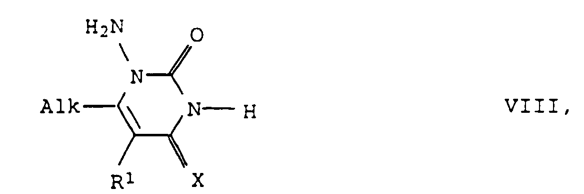 où Alk et R¹ ont les significations indiquées dans la revendication 1, en présence d'une base, ou un sel de métal alcalin de VIII, avec un halogénure de benzyle de formule IX 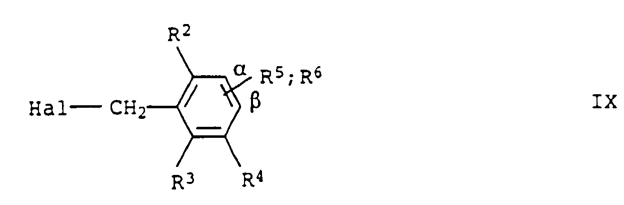 où Hal désigne un halogène et les substituants R² à R⁶ ont les significations indiquées dans la revendication 1.

13. Ester d'énamine de formule III 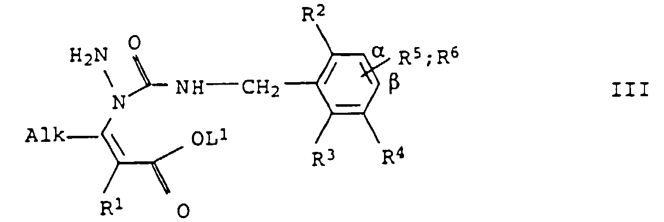 dans laquelle L¹ désigne un alkyle en C₁-C₆ ou un phényle et les substituants Alk et R¹ à R⁶ ont les significations indiquées dans la revendication 1.

14. Carboxylates d'énamine de formule IV 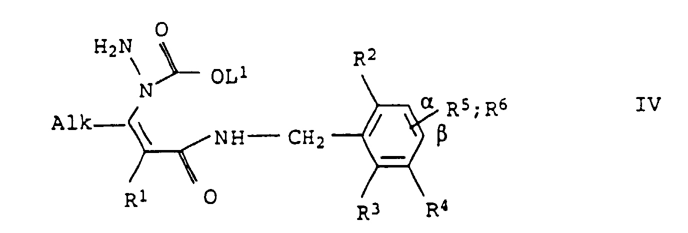 dans laquelle L¹ désigne un alkyle en C₁-C₆ ou un phényle et les substituants Alk et R¹ à R⁶ ont les significations indiquées dans la revendication 1.
